# EUROPEAN PATENT APPLICATION

(11) **EP 4 067 381 A1**
(43) Date of publication of application: **05.10.2022**
(21) Application number: 21166756.3
(22) Date of filing: 01.04.2021
(51) Int. Cl.: C07K 16/28, A61K 39/00

(54) **NOVEL TNFR2 BINDING MOLECULES**

(71) Applicant: Julius-Maximilians-Universität Würzburg, 97070 Würzburg (DE)
(72) Inventor: The designation of the inventor has not yet been filed
(74) Representative: J A Kemp LLP

(57) **Abstract**

The present disclosure provides a binding molecule that specifically binds TNFR2, but not TNFR1. Preferably the binding molecule is an FcyR-independent agonist of TNFR2 and more preferably has a valency of at least two for binding TNFR2. The binding molecules have a variety of uses including diagnostic and therapeutic. The ability of the binding molecules to be able to modulate immune responses means that they are particularly suited for treating autoimmune diseases, or inflammatory conditions.

## Description

### Field of Invention

The present invention relates to binding molecules specific for TNFR2 (Tumor Necrosis Factor Receptor 2, also known as TNFRSF1B, and CD120B). The binding molecules are typically able to bind to TNFR2 and trigger TNFR2 signalling independent from FcyR (Fc gamma receptor) binding. The binding molecules may be used to target cells expressing TNFR2. The present invention also relates to the use of the binding molecules in treating conditions such as inflammatory disorders, and autoimmune disease.

### Background of Invention

TNFα is a cytokine which plays a wide range of roles in inflammation, differentiation and tissue homeostasis and occurs in soluble and transmembrane forms. It is a key inflammatory mediator which is targeted in the clinic with TNF blocking reagents to suppress unwanted immune responses in inflammatory and autoimmune conditions. TNFR1 and TNFR2 are distinct receptors for Tumor Necrosis Factor-alpha (TNFα) with signalling through each receptor eliciting different effects. Signalling via TNFR1 triggers strong and manifold pro-inflammatory effects, whilst signalling via TNFR2 has a wider range of effects ranging from immune stimulation, to suppression of immune responses, to tissue regeneration. The two receptors also differ from each other in terms of which cells they are expressed on. TNFR1 is ubiquitously expressed, whilst TNFR2 is found on a more restricted group of cells, in particular subsets of immune cells, endothelial cells, and neurons. Immune cells expressing TNFR2 include immune suppressive cell types such as T regulatory cells (Tregs), B regulatory cells (B regs), and MDSC (myeloid derived suppressor cells), as well as other cell types, such as CD8+ T cells.

TNFR1 and TNFR2 have a extracellular domain containing four characteristic cystein rich domains (CRDs), a transmembrane segment, and an intracellular domain. Unlike TNFR1, TNFR2 does not include a cytoplasmic death domain, but can still play a role in apoptosis by controlling cell death regulatory factors such as TRAF2 and cIAPs. TNFR2 molecules are able to associate with each other, forming dimeric and trimeric complexes of TNFR2 which can bind TNFα with high affinity, resulting in the formation of oligomers of trimeric TNF-TNFR2 complexes capable of intracellular signalling. Though both TNFR1 and TNFR2 may be expressed on the surface of the same cell type, the two do not form complexes with each other. TNFR2 is able to bind both soluble and membrane bound TNFα. Binding of membrane TNFα is able to activate TNFR2 effectively, but with soluble TNFα the oligomerisation of liganded TNFR2 trimers to hexamers or higher complexes is inadequate and thus fails to trigger effective TNFR2 activation. As well as TNFα, lymphotoxin-alpha (LTα) is also thought to act as a ligand for TNFR2.

When TNFR2 is activated by membrane TNFα, the intracellular domains of the receptor are thought to recruit existing cytoplasmic TRAF2 trimers, resulting in recruitment of the E3 ligases cIAP1 and cIAP2 to TNFR2 complexes, which leads to classical NEκB signalling. TRAF2 recruitment is also associated with activating the alternative NEκB signalling pathway. Binding of membrane TNFα to TNFR2 is also thought to trigger signalling through the PI3K/Akt pathway, maintaining survival and enhancing proliferation, as well as promoting other functions such as cellular adhesion and migration. TNFR2 knockout mice are more susceptible to inflammatory disease and, whilst they have Treg cells, they are less responsive. Naturally occurring polymorphisms of TNFR2 also exist and are associated with a wide variety of autoimmune diseases

The expression and function of TNFR2 on Treg cells make it a desirable target for modulating the immune system, especially as TNFR2 is found on a more limited subset of cells than TNFR1. The ability to stimulate Treg cells offers a way to control unwanted immune responses in a wide range of conditions where their ability to suppress immune responses may be useful including allergy, autoimmunity, transplantation, graft versus host disease (GVHD) and infectious disease. As well as Treg cells, other cell types expressing TNFR2 may be targeted to bring about desired functions. There is therefore an ongoing need for agents that target and activate TNFR2.

### Summary of the Invention

The present invention provides binding molecules which are able to bind the extracellular domain of TNFR2. In a preferred embodiment, a binding molecule of the present invention is able to bind TNFR2 and trigger signalling through the receptor thus acting as an agonist. In a further particularly preferred embodiment the binding molecules may act as agonists of TNFR2 independent of FcyR binding. In a particularly preferred embodiment, the binding molecules of the present invention are antibodies or antibody fragments. In a further particularly preferred embodiment of the present invention the binding molecules are selective in that they specifically bind TNFR2, but not TNFR1.

The binding molecules can have a variety of uses from diagnostic applications to therapeutic uses. As the binding molecules have the ability to modulate the immune system they are particularly useful in treating autoimmune conditions, and inflammatory conditions. The binding molecules may be used to downregulate the immune system due to their action on Treg cells and so are especially useful in treating such conditions, including in particular Graft versus Host Disease (GvHD).

Accordingly, in one embodiment the present invention provides a binding molecule that specifically binds TNFR2, but not TNFR1, where the binding molecule is an FcyR independent agonist of TNFR2 and has a valency of at least two for binding TNFR2.

The present invention also provides:
- The binding molecule of the invention for use as a medicament.
- The binding molecule of the invention for use in a method of treating or preventing an autoimmune disorder, or an inflammatory disorder.
- A method of treating or preventing an autoimmune disorder, or an inflammatory disorder comprising administering a binding molecule of the invention to a subject suffering from, or at risk of, the disorder.
- Use of a binding molecule of the invention for manufacturing a medicament for use in a method of treating or preventing an autoimmune disorder, or an inflammatory disorder.
- A method of stimulating cell proliferation comprising contacting a target cell expressing TNFR2 with a binding molecule of the invention.
- A pharmaceutical composition comprising a binding molecule of the invention and a pharmaceutically acceptable carrier.
- A method of detecting TNFR2 comprising contacting a test sample with a binding molecule of the invention and detecting binding of the binding molecule to TNFR2.
- A diagnostic method comprising administering a binding molecule of the invention to a subject and detecting binding of the binding molecule to TNFR2.
- A binding molecule of the invention for use in a method of diagnosis, the method comprising administering a binding molecule of the invention to a subject and detecting binding of the binding molecule to TNFR2.

### Sequence Listing

The present application includes a sequence listing in electronic form which forms part of the application as filed.

### Brief Description of the Figures

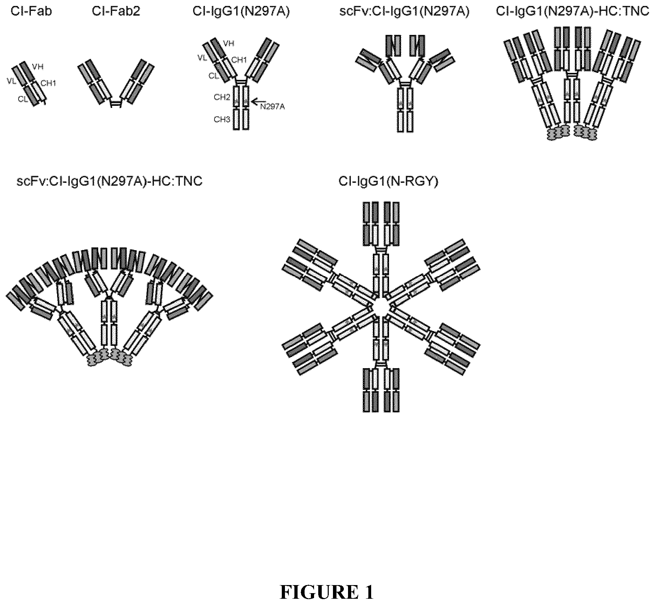

**Figure 1** **- Nomenclature and schemes of the domain architecture of exemplary anti-TNFR2 antibody variants with N-terminal organized TNFR2 binding sites (Fab or scFv domains).** CI = clone identifier of parental anti-TNFR2 antibody, e.g. the mouse anti-human TNFR2 antibodies C4, C19, C9, C40 *etc.;* N297A refers to the corresponding mutation in the heavy chain of human IgGI which reduces FcyR interaction; N-RGY refers to the N297A mutation plus mutations promoting IgG1 hexamerization.

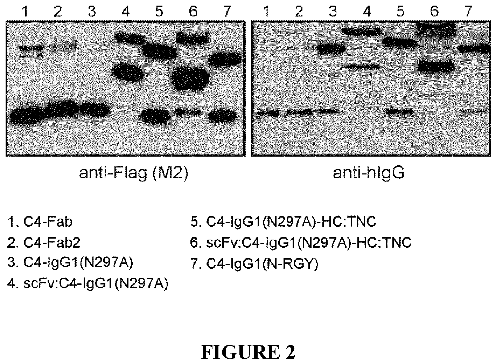

**Figure 2** **- Western blot analysis of variants of the anti-TNFR2 antibody C4 with N-terminal unidirectionally orientated TNFR2 binding sites.** Flag-tagged variants of the indicated C4 constructs (200 ng) were analyzed by Western blotting with anti-human IgG and anti-Flag antibodies.

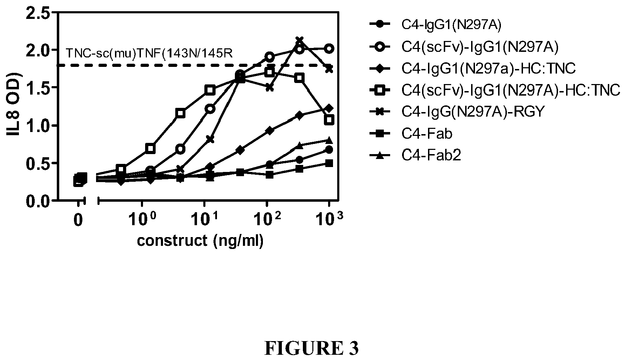

**Figure 3** **- TNFR2-mediated IL8 production in HT1080-Bcl2-TNFR2 cells after treatment with C4 variants with unidirectionally organized N-terminal TNFR2 binding sites.** HT1080-Bcl2-TNFR2 cells, stably expressing human TNFR2, were stimulated with supernatants containing the indicated concentrations of the various C4 constructs. Next day, supernatants were harvested and analyzed by ELISA for their IL8 content. The data shown are derived from parallel processed experiments which for better resolution were shown in three diagrams. The maximum response induced with a highly active oligomeric form of the TNFR2-specific TNF mutant TNF(143N/145R) positive control was indicated by a dotted line.

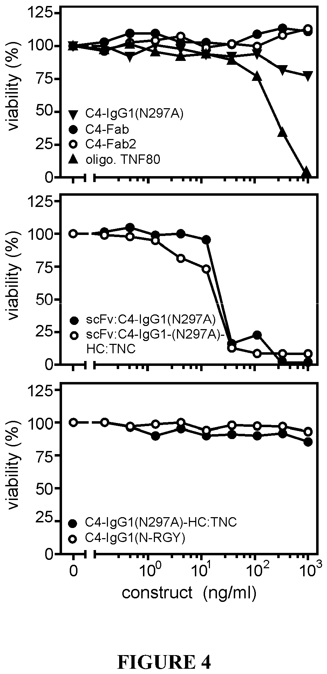

**Figure 4****. TNFR2-mediated cell death in Kym-1 cells after treatment with C4 variants with unidirectionally orientated N-terminal TNFR2 binding sites.** Kym-1 cells were stimulated with supernatants containing the indicated concentrations of the various C4 constructs. Next day, cell viability was determined using crystal violet staining. A highly active oligomeric form of the TNFR2-specific TNF mutant TNF(143N/145R) (oligo. TNF80) served as a positive control for TNFR2 engagement.

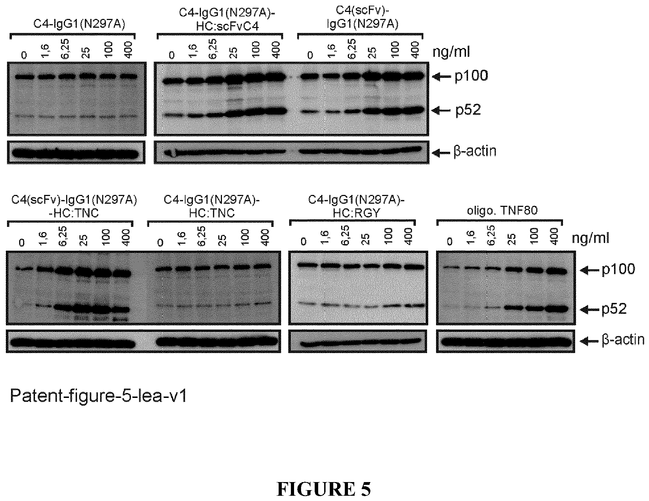

**Figure 5** **- TNFR2-mediated p100 processing in Kym-1 cells after treatment with C4 variants with N-terminal unidirectionally orientated TNFR2 binding sites.** Kym-1 cells were stimulated overnight with supernatants containing the indicated concentrations of the various C4 variants. Treatment was performed in the presence of 20 µM ZVAD to prevent TNFR2-induced cell death. A nonameric TNFR2-specific mutant of mouse TNF (oligo. TNF80), which potently activate human and murine TNFR2, was used as a positive control.

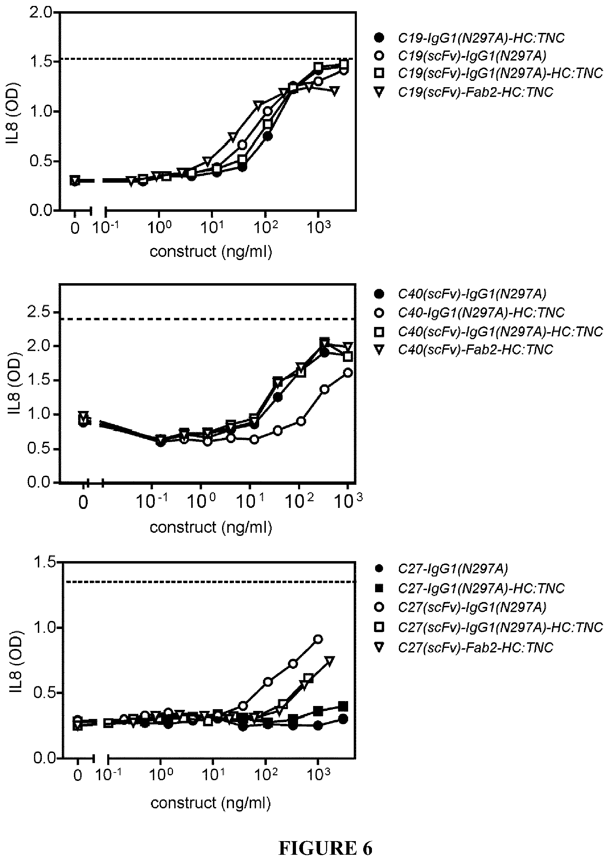

**Figure 6****. TNFR2-mediated IL8 production in HT1080-Bcl2-TNFR2 cells after treatment with variants of C19, C27 and C40 with N-terminal unidirectionally orientated TNFR2 binding sites.** HT1080-Bcl2-TNFR2 cells were stimulated with supernatants containing the indicated concentrations of the various constructs. Next day, supernatants were harvested and analyzed by ELISA for their IL8 content. The maximum response induced with a highly active oligomeric form of the TNFR2-specific TNF mutant TNF(143N/145R) positive control was indicated by a dotted line. Note: even constructs derived of the lower affinity antibody C27 showed significant agonistic activity (lower panel).

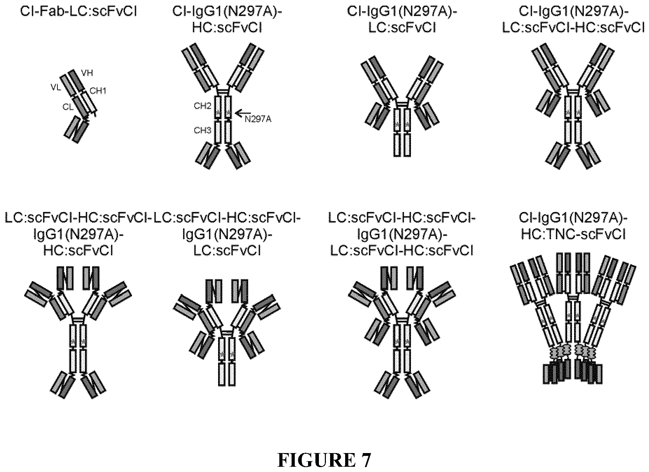

**Figure** 7 - **Nomenclature and schemes of the domain architecture of anti-TNFR2 antibody variants with N-terminal and C-terminal organized TNFR2 binding sites (Fab or scFv domains).** CI = clone identifier, e.g. C4, C19 etc.; N297A refers to the corresponding mutation in the heavy chain of human IgGI reducing FcyR binding.

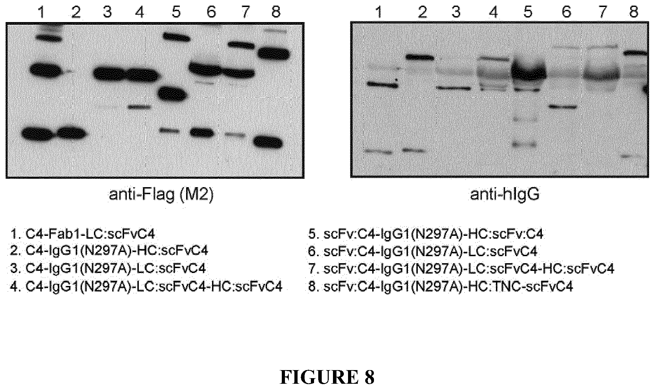

**Figure 8** - **Western blot analysis of C4 variants with N-terminal and C-terminally fused TNFR2 binding sites.** Flag-tagged variants of the indicated C4 constructs (200 ng) were analyzed by Western blotting with anti-human IgG and anti-Flag antibodies. Note that the heavy chain of C4-IgG1(N297A)-HC:scFvC4 does not contain a Flag tag.

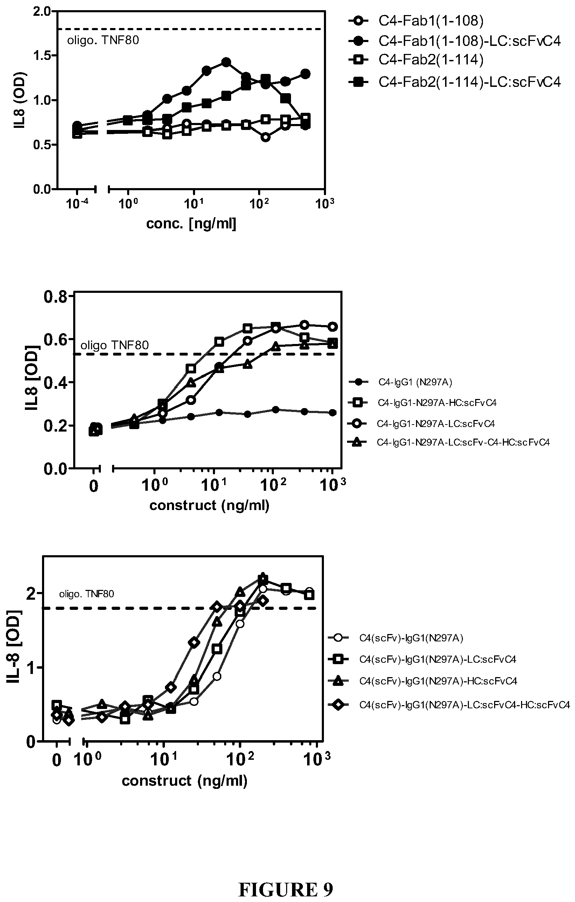

**Figure 9** **- TNFR2-mediated IL8 production in HT1080-Bcl2-TNFR2 cells after** treatment **with C4 constructs with N-terminal and C-terminal TNFR2 binding sites.** HT1080-Bcl2-TNFR2 cells were stimulated with supernatants containing the indicated concentrations of the various C4 constructs. Next day, supernatants were harvested and analyzed by ELISA for their IL8 content. The data shown are derived from parallel processed experiments which for better resolution were shown in three diagrams. The maximum response induced with a positive control (oligo. TNF80; highly active oligomeric form of the TNFR2-specific TNF mutant TNF(143N/145R)) was indicated by a dotted line. Corresponding constructs with only N-terminal binding sites were analyzed in parallel for comparison.

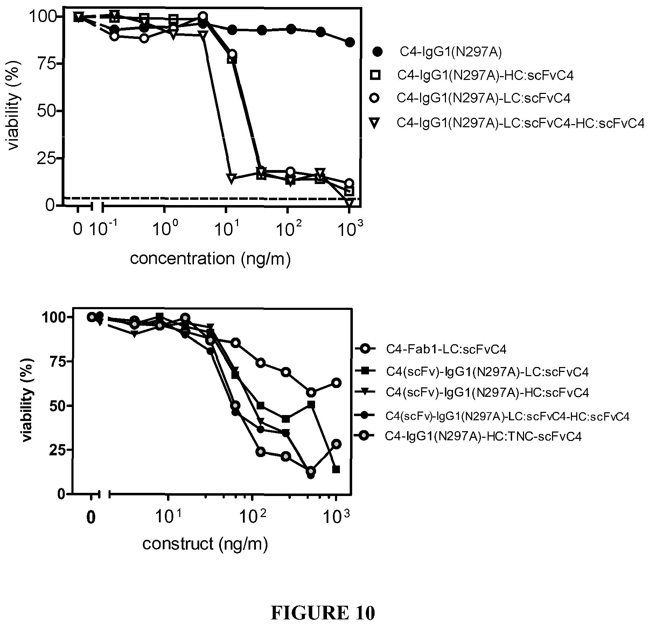

**Figure 10** - **TNFR2-mediated cell death in Kym-1 cells after treatment with C4 constructs with N-terminal and C-terminal organized TNFR2 binding sites.** Kym-1 cells were stimulated with supernatants from HEK293 transfected cells containing the indicated approximate concentrations of the various C4 constructs. Next day, cell viability was determined using the MTT assay. A highly active oligomeric form of the TNFR2-specific TNF mutant TNF(143N/145R) was used as a positive control.

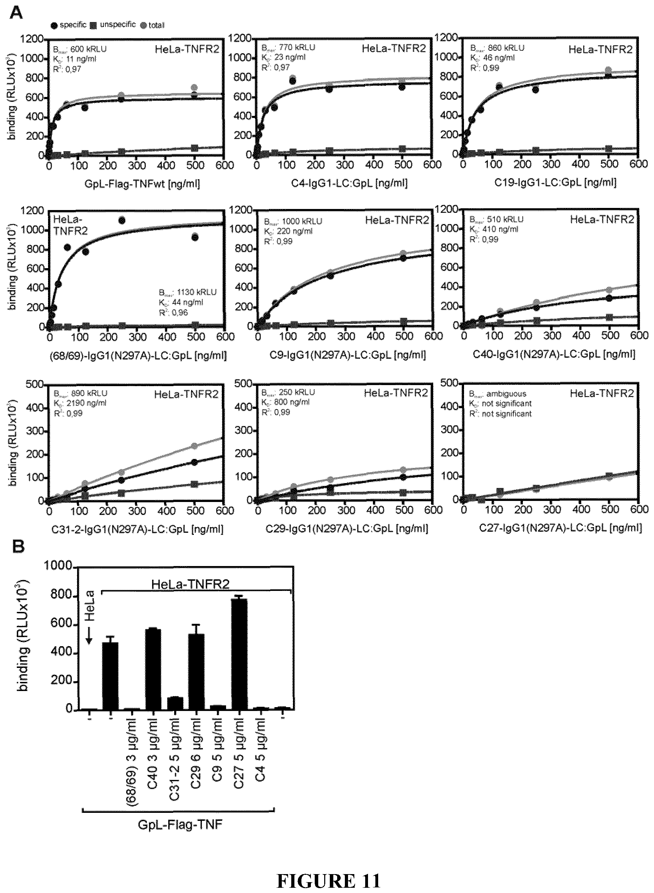

**Figure 11** **- Binding properties of TNFR2-specific antibodies. (A)** Equilibrium binding of the indicated anti-TNFR2 antibody GpL fusion proteins to cell expressed human TNFR2 at 37°C. **(B)** HeLa-TNFR2 cells were pretreated with the indicated amounts of anti-TNFR2 antibodies or remained untreated. 5 ng/ml GpL-TNF were added and binding was measured after 1 hour incubation at 37°C. Binding of the various constructs to untransfected HeLa cells was analyzed to obtain unspecific binding values.

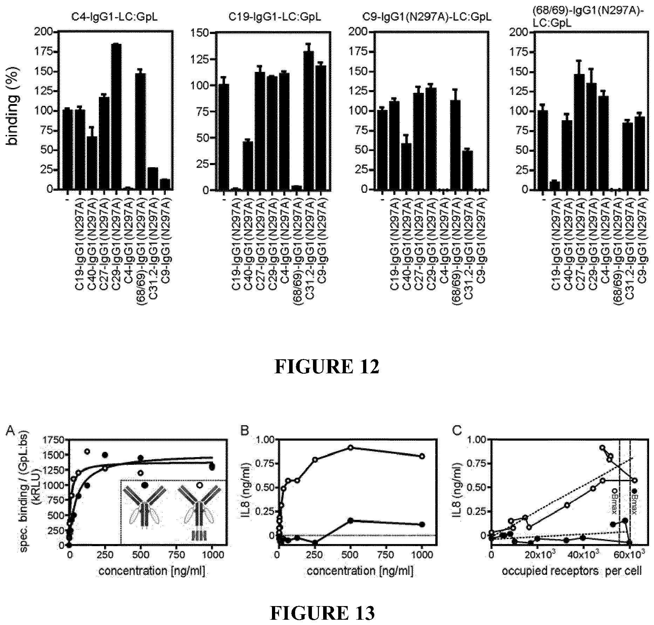

**Figure 12** - **Competition of αTNFR2 antibodies.** HeLa-TNFR2 cells were pretreated with 3 - 10 µg/ml of the indicated anti-TNFR2 antibodies or remained untreated. 50 ng/ml of the various αTNFR2-IgG1(N297A)-LC:GpL variants were added and binding was measured after 1 hour
**Figure 13** **- Tetravalent mAb binding TNFR2 molecules trigger much stronger IL8 induction than TNFR2 molecules bound by the conventional bivalent mAb variant. (A,B)** TNFR2-negative Hela cells and HeLa-TNFR2 cells were pairwise stimulated for 8 hours with the indicated concentrations of the GpL fusion proteins of the bi- and tetravalent version of the TNFR2-specific mAb C4 (see inset for scheme). Supernatants of HeLa-TNFR2 cells were then analyzed for TNFR2-induced IL8 production (B) and repeatedly washed cells (HeLa, and HeLa-TNFR2) were analyzed for cell-associated GpL activity. Specific binding to TNFR2, shown in (A) were calculated by subtraction of the unspecific binding values obtained from the HeLa cells from the total binding values derived of the HeLa-TNFR2 cells. Please note luciferase activity was normalized according to the ratio of the number of GpL reporter domains to the number of TNFR2-binding domains within the two mAb variants (1 versus 0.5). (C) IL8 production by the mAb variants were directly plotted as a function of their specific binding. The latter was transformed into "occupied receptors per cells" by help of the number of cells in the assay and the measured specific activity (RLU/molecule) of the GpL constructs. Maximum binding of the two constructs obtained from A is indicated by dashed vertical lines. The dotted lines indicate linear regression of the IL8 production as function of receptor occupancy.

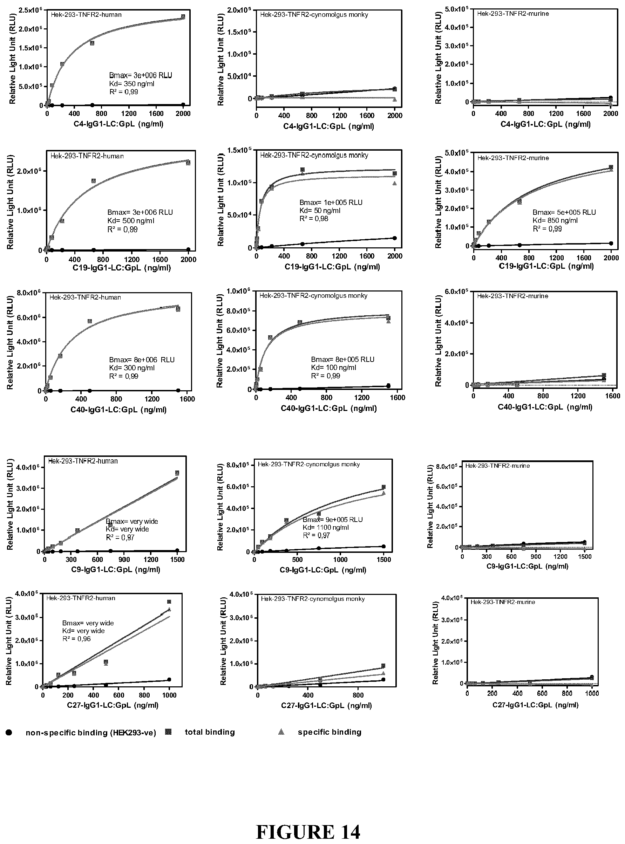

**Figure 14** **- Binding of TNFR2-specific antibodies to human, murine and cynomolgus TNFR2.** HEK293 cells were transiently transfected with human, murine and cynomologus TNFR2 or empty vector. Equilibrium binding of the indicated anti-TNFR2 antibody GpL fusion proteins were then determined at 37°C. Binding to empty vector transfected cells was determined to obtain unspecific binding values.

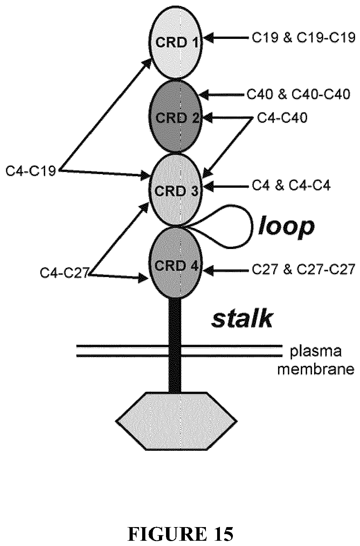

**Figure 15** **- Scheme of the domain architecture of TNFR2 and indication of the extracellular TNFR2 domains recognized by the anti-TNFR2 antibodies C4, C19, C27 and C40 and tetravalent CI-IgG1(N297A)-HC:scFvCI variants derived thereof.** CI, anti-TNFR2 clone identifier; CRD, cysteine-rich domain.

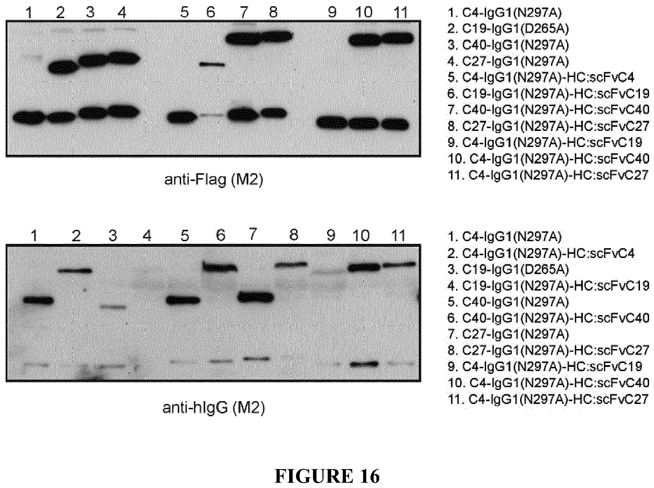

**Figure 16** - **Western blot analysis of mono- and bispecific TNFR2-specific CI-IgG-HC:scFvCI variants.** Flag-tagged variants of the indicated antibody constructs (200 ng) were analyzed by Western blotting with anti-human IgG (lower panel) and anti-Flag antibodies (upper panel). Conventional IgG variants were included for comparison. Please note: i) The heavy chains of C4-IgG1(N297A), C4-IgG1(N297A)-HC:scFvC4 and C4-IgG1(N297A)-HC:scFvC19 do not contain a Flag tag, ii) the conventional C19 variant used was in the murine IgG1(D265A) isoform.

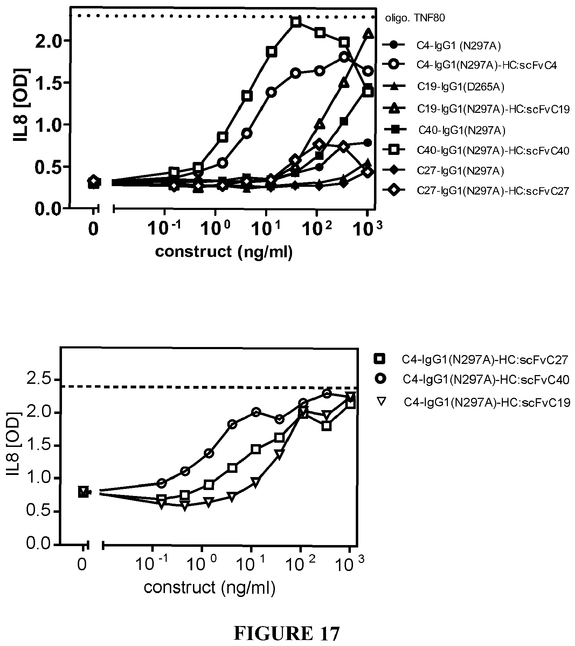

**Figure 17** - **TNFR2-mediated IL8 production in HT1080-Bcl2-TNFR2 cells after treatment with mono- (upper level) and biparatopic (lower panel) TNFR2-specific CI-IgG-HC:scFvCI variants.** The response induced by 200 ng/ml of a highly agonistic oligomerized TNFR2-specific TNF variant is indicated (dotted line). Please note, in the upper panel, the C27 antibody which has lower affinity still gave some agonistic activity in the tetravalent form.

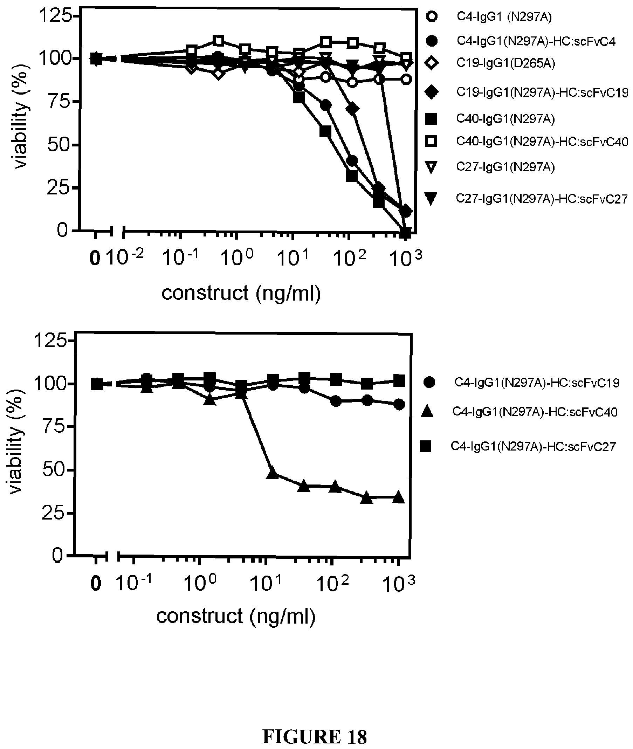

**Figure 18** - **TNFR2-mediated cell death in Kym-1 cells after treatment with mono-(upper level) and bispecific (lower panel) TNFR2-specific CI-IgG-HC:scFvCI variants.** Kym-1 Cells were stimulated with supernatants containing the indicated approximate concentrations of the various constructs. Next day, cell viability was determined using crystal violet staining. Conventional antibodies (upper panel) were analyzed in parallel for comparison.

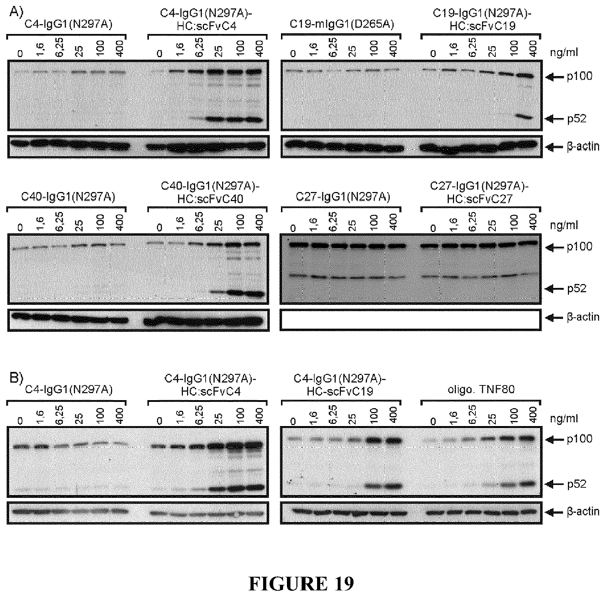

**Figure 19** **- TNFR2-mediated p100 processing in Kym-1 cells after treatment with mono-** (A) **and bispecific (B) TNFR2-specific CI-IgG-HC:scFvCI variants.** Cells were stimulated overnight with supernatants containing the indicated approximate concentrations of the various constructs. Treatment was performed in the presence of 20 µM ZVAD to prevent TNFR2-induced cell death.

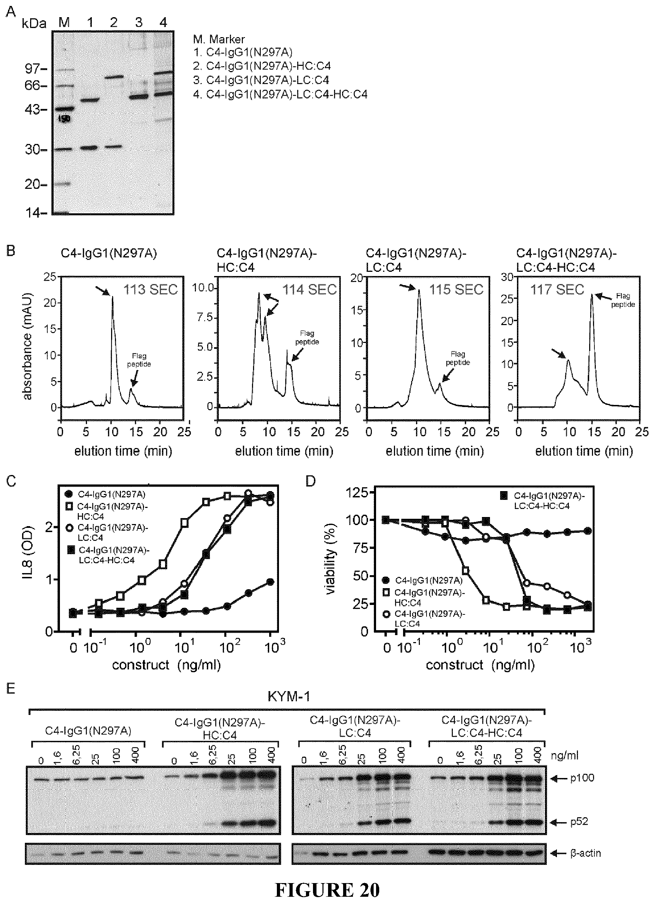

**Figure 20** **- Purification and functional analysis of C4-IgG1(N297A)-HC:scFvC4, C4-IgG1(N297A)-LC:scFvC4 and C4-IgG1(N297A)-LC:scFvC4-HC:scFvC4.** (A) Silver stained SDS-PAGE gel. (B) Gelfiltration analysis. (C) IL8 induction in HT1080-Bcl2-TNFR2 cells. (D) Cell death induction in Kym-1 cells. (E) Induction of p100 processing in Kym-1 cells. ZVAD was added to prevent cell death.

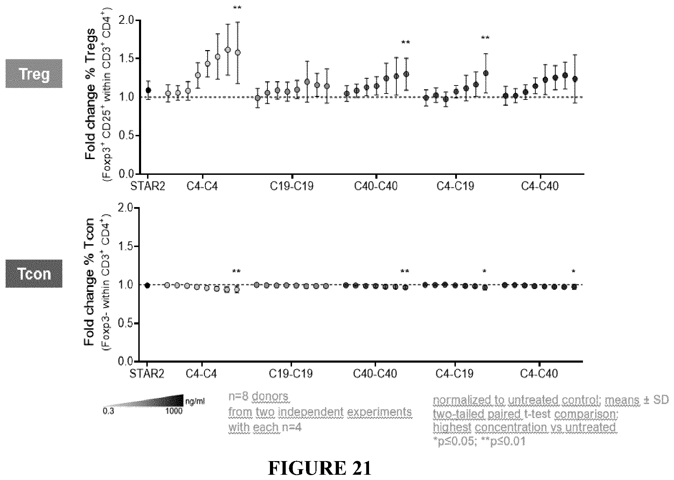

**Figure 21** - **Ex vivo assessment of Treg and Tcon cells after treatment with tetravalent TNFR2 agonists.** PBMCs were collected from donors, cultured, harvested, then treated with tetravalent antibodies (mono- or bi-specific; Fab-HC-scFv format). Untreated negative control cells were also assessed as was a positive control with the Star2 (also named TNC-sc(mu)TNF(143N/145R) TNFR2 agonist (oligo TNF80), an oligomerized highly potent TNFR2-selective TNF variant (Chopra *et al.,* 2016). The top panel shows percentage fold change in Tregs, the bottom in Tcon cells.

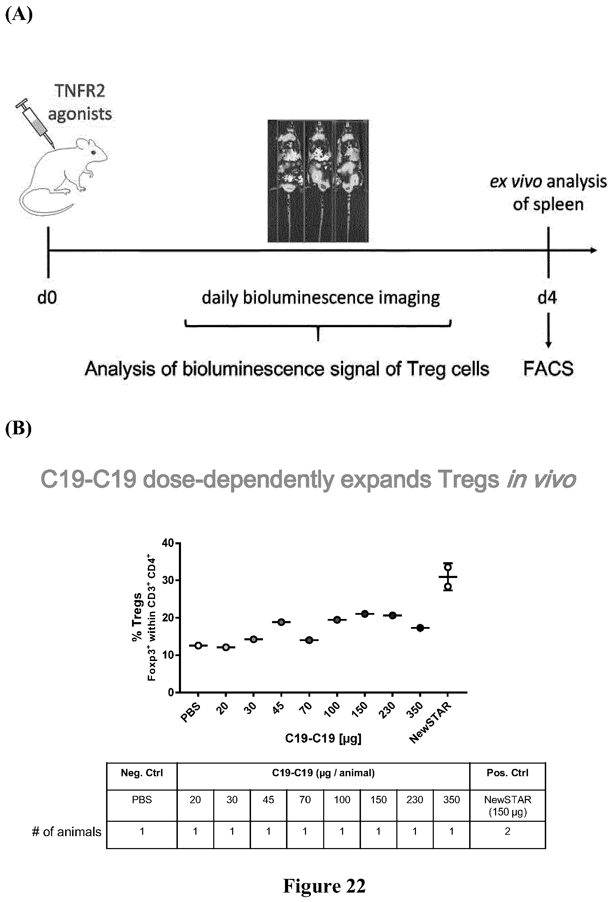

**Figure 22** **- Dose-dependent increase of Tregs in syngeneic FoxP3-Luci mice by a human-mouse crossreactive tetravalent TNFR2 agonist.** Transgenic FoxP3-Luci mice were administered 8 doses of a TNFR2 agonist (N=1) and then assessed daily by bioluminescence imaging. After four days the mice were sacrified and cells from the spleen analysed. Panel (A) shows the time line for the experiment. Panel (B) shows the results of the FACS analysis of splenocytes.

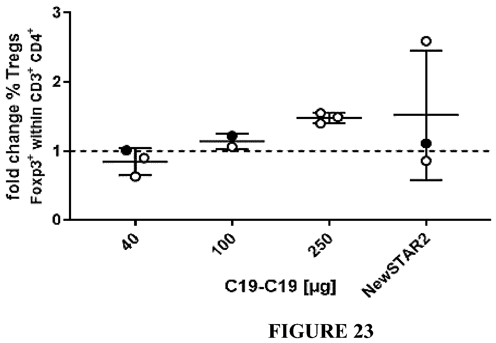

Figure 23 - Dose-dependent increase of Tregs in syngeneic FoxP3-Luci mice by a **human-mouse crossreactive tetravalent TNFR2 agonist.** Transgenic FoxP3-Luci mice were administered three doses of a TNFR2 agonist and then assessed daily by bioluminescence imaging. After four days the mice were sacrified and cells from the spleen analysed. Panel (A) shows the time line for the experiment. Panel (B) shows the results of the FACS analysis of splenocytes.

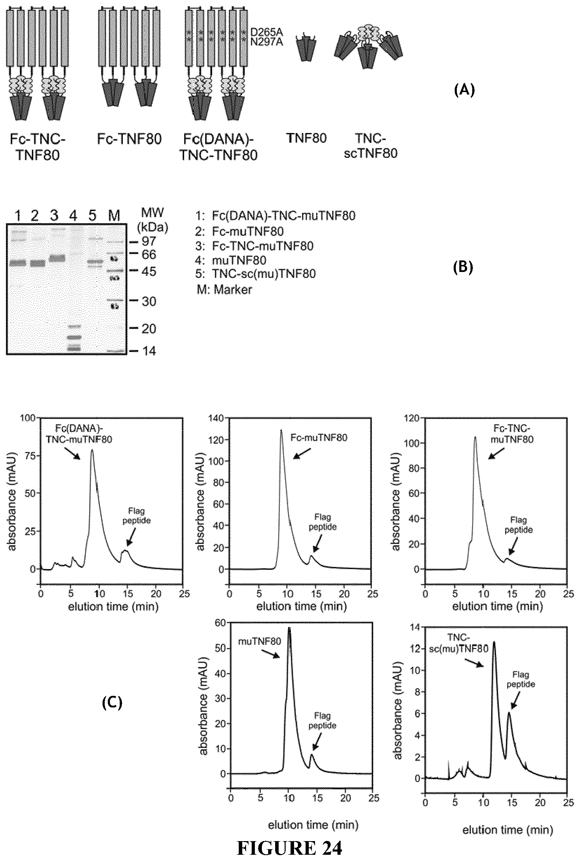

**Figure 24** - **Production and analysis of TNFR2-specific TNF constructs. Panel (A):** Domain architecture of TNF-based molecules. TNF80 refers to a TNFR2-specific mutant of soluble TNF. **Panel (B):** murine soluble TNF80 based constructs were purified by anti-Flag affinity purification by help of a Flag tag contained in all constructs and analyzed by SDS-PAGE and silver staining. Panel (C): Gel filtration analysis of the purified protein shown in "B".

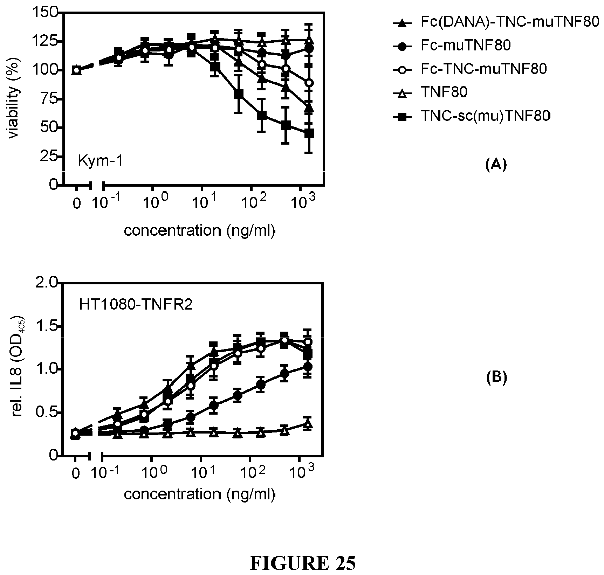

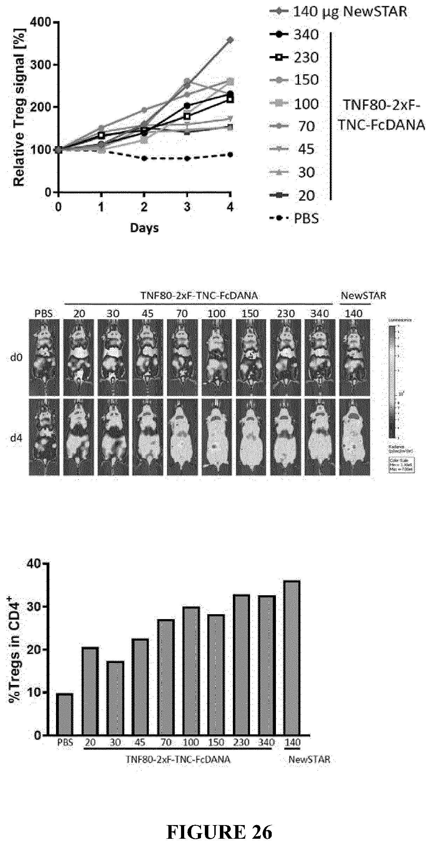

**Figure 25** **- Agonistic activity of different formats of soluble murine TNF80. Panel (A):** Kym-1 cells were stimulated with the indicated concentrations of the various muTNF80 variants. Next day, cell viability was determined using crystal violet staining. **Panel (B):** HT1080-Bcl2-TNFR2 cells were stimulated with the indicated concentrations of the various TNF80 variants shown in Figure 26. Next day, supernatants were harvested and analyzed by ELISA for their IL8 content. The data shown are derived from four independent experiments.
**Figure 26** **- In vivo Treg expansion by Fc(DANA)-TNC-muTNF80.** Mice with luciferase under the control of the Foxp3 promoter were injected i.v with a single injection of the indicated amount of Fc(DANA)-TNC-muTNF80 and were analyzed daily by BLI **(upper panel).** Day 0 and day 4 BLI pictures are shown in the **middle panel.** The lower panel shows FACS analysis based quantification of Treg frequency on day 4.

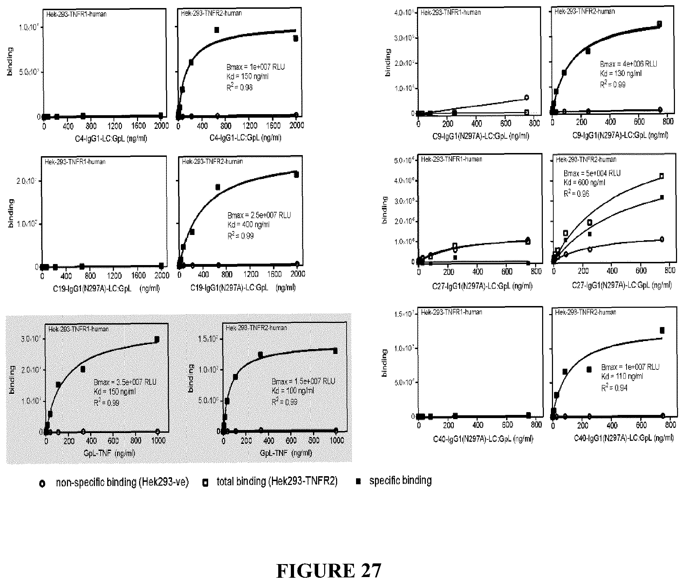

**Figure 27** **- Demonstration of selective binding of TNFR2, but not TNFR1, by binding molecules of the invention.** HEK293 cells were transiently transfected with: (i) a TNFR2 expression plasmid; (ii) an expression plasmid encoding the extracellular domain of TNFR1 with a GPI anchor domain; or (iii) empty vector (ev). Next day, the indicated GpL tagged binding molecules were added at the indicated concentration to the cells. After incubation for 1 hour at 37°C, followed by two washes with PBS, the remaining cell bound GpL activity was determined. GpL-TNF served as positive control binding to both TN receptor types.

### Detailed Description of the Invention

### TNFR2 and TNFR2 Target cells

The present invention provides binding molecules that bind TNFR2 and in particular which bind the extracellular domain of TNFR2. TNFR2 comprises a characteristic extracellular domain, a transmembrane segment, and an intracellular domain. The extracellular region contains four cysteine rich domains (CRD) that are repeated. The TNFR2 bound by a binding molecule of the present invention is typically a mammalian TNFR2. In a particularly preferred embodiment, the TNFR2 bound by a binding molecule of the present invention is human TNFR2. The amino acid sequence of human TNFR2 is provided as SEQ ID NO: 1. The human TNFR2 amino acid sequence provided in SEQ ID NO: 1 is subdivided into the signal peptide comprising amino acids 1 to 22; the extracellular domain of amino acids 23 to 257 and the transmembrane region of amino acids 258 to 287. A binding molecule of the present invention typically binds the extracellular domain of TNFR2. In one embodiment, a binding molecule of the present invention may bind human TNFR2, but also TNFR2 of a different species such as a mouse TNFR2 and/or a monkey TNFR2. In one embodiment, it binds human, mouse, and a monkey TNFR2.

A binding molecule of the present invention will typically be used to bind a target cell, in particular a target cell expressing TNFR2. The target cell may be any cell type expressing TNFR2. In one embodiment the target cell is an immune cell, endothelial cell, mesenchymal stem cell, or neuron. In a particularly preferred embodiment, the target cell is an immune cell. In an especially preferred embodiment, the target cell is a Treg cell. In one embodiment the immune cells expressing TNFR2 are selected from a T regulatory cells (Tregs), B regulatory cells (B regs), and MDSC (myeloid derived suppressor cells). In one embodiment, the target cell may be a Treg cell which is a Foxp3+ CD25+ regulatory T cell.

### Binding Molecules

The present invention provides binding molecules that bind TNFR2. In one preferred embodiment, a binding molecule of the present invention will bind to TNFR2 and stimulate signalling through TNFR2. In one preferred embodiment, the binding molecules are agonistic antibodies or antibody fusion proteins or antibody fragments or antibody fragment fusion proteins against TNFR2 and so activate the receptor. In a preferred embodiment, the binding molecules are antibodies that specifically bind TNFR2, preferably they are antibodies that specifically bind TNFR2 and act as an agonist of it without FcgR binding. In one preferred embodiment, a binding molecule of the present invention will bind TNFR2 to a greater extent than to TNFR1. For instance, the strength of binding for TNFR2 compared to TNFR1 may be at least 10, 50, 100, 500, 1000 or more times higher. In one embodiment, the strength of binding for TNFR2 compared to TNFR1 may be at least 10,000, or at least100,000 times greater. In one embodiment, the binding molecule does not bind TNFR1 at all. In one preferred embodiment, a binding molecule, and in particular of an antibody or antibody fragment or fusion proteins thereof of the present invention, is said to specifically bind TNFR2 when the KD for the binding has a value below 10⁻⁶M, 10⁻⁷M, 10⁻⁸M, 10⁻⁹M, or 10⁻¹⁰M. In one embodiment the KD value is meaured using cells expressing TNFR2, for example HeLa transfectants expressing TNFR2. In one preferred embodiment, KD values are determined using the approach set out in Kums J, Nelke J, Rüth B, Schäfer V, Siegmund D, Wajant H.MAbs. 2017 Apr;9(3):506-520. Hence, in one embodiment, a fusion of the binding molecule with *Gaussia princeps* luciferase as a reporter domain is employed to measure the KD value of the binding molecule as described in Kums *et al., supra.* In one embodiment, cell lines expressing TNFR2 may be used in such measurement, for example HeLa or HEK293 cells that stably express TNFR2 on their surface. Typically, the binding molecules of the invention bind to TNFR2 and trigger TNFR2 signalling. In one embodiment, the binding molecules, and in particular antibodies, provided are agonistic for TNFR2, but are not agonistic for TNFR1. In one embodiment, a binding molecule of the present invention binds TNFR2, but does not bind any other TNFR super family members.

In a particularly preferred embodiment, the binding molecule of the present invention is an antibody. The term "antibody" as used herein typically refers to any functional antibody or antibody fusion protein or antibody fragment that is capable of specific binding to the antigen of interest, as generally outlined in chapter 7 of Paul, W.E. (Ed.).: Fundamental Immunology 2nd Ed. Raven Press, Ltd., New York 1989, which is incorporated herein by reference or shown in the "Periodic Table of Antibodies" (https://absoluteantibody.com/periodic-table-of-antibodies/). Reference to an antibody herein includes antibody fragments unless specifically stated. An antibody of the present invention typically specifically binds TNFR2. Reference to an antibody includes all of the various antibody formats discussed herein including antibodies that do not have the naturally occurring four chain structure of an antibody seen in humans, for example in one preferred embodiment an antibody of the present invention may be, or may comprise, a single chain antibody. An antibody of the present invention may also comprise non-antibody sequences such as a ligand based molecule that binds TNFR2. In one embodiment an antibody of the present invention may comprise polypeptide(s) which are a fusion of antibody sequences and non-antibody sequences, for example TNC peptide and TNF-alpha polypeptides (particularly TNFR2-specific mutants such as TNF80 (or TNF(143N/145R) as discussed herein. An antibody of the invention also includes a portion or fragment of an antibody. For example, an antibody of the present invention may comprise just an antibody Fc region modified so that it does not bind Fc receptors and ligand based binding molecules joined to the Fc region. In one embodiment, the ligand based molecules present in an antibody of the present invention is a TNF-based ligand for TNFR2, e.g. a TNF protomer or a mutant thereof. In one embodiment it is a single-chain version of a TNF trimer. An antibody of the present invention also includes antibody formats such as single domain antibodies, for example VHH domain antibodies. In a preferred embodiment, an antibody of the present invention may be a molecule that comprises such a single domain antibody or single domain antibodies where the overall valency of the binding molecule is at least two. For example, an antibody of the present invention may comprise two such single domain antibodies joined together as part of the overall antibody. In one embodiment, an antibody of the present invention may comprise an Fc region, but not the variable regions that would normally be found in a naturally occurring four chain antibody. In one preferred embodiment, such Fc regions may though comprise other sequences as part of the polypeptides comprising the heavy chains of the Fc region, in particular TNC peptide and/or TNF-alpha (particularly TNF80) and preferably both, in particular in the C-terminal portion of the polypeptides.

The "valency" of a binding molecule as used herein denotes the number of binding sites that a binding molecule has, with the valency for TNFR2 denoting the number of binding sites for TNFR2 that a binding molecule has. The "specificity" of a binding site denotes what it binds to, for instance it may denote what target molecule the binding molecule binds, but it may also denote where on the target it binds. In respect of an antibody, the specificity of an antibody denotes what target the antibody binds, it may also be used to denote where on the antigen it binds in terms of the epitope bound on that target molecule. Binding molecules may have different specificities in the sense that they bind epitopes on different target molecules (antigens). However, binding molecules may have different specificities in the sense that they bind different epitopes on the same target molecule (antigen). For example, a bispecific binding molecule may be bispecific in the sense it can bind two different target molecules, but alternatively it may be bispecific in the sense it can bind two different epitopes of the same target molecule and in particular of TNFR2.

In one embodiment, all of the binding sites of a binding molecule of the present invention will bind the same target. In one embodiment, where the binding molecule of the present invention is an antibody, it may be that all of the antigen-binding sites of the antibody bind the same epitope of TNFR2. In another embodiment, it may be that a binding molecule of the present invention will comprise at least two different target binding sites that each bind a different target site on TNFR2, for example which each recognise a different epitope of TNFR2. In one embodiment, a binding molecule of the present invention is an antibody which comprises at least two antigen-binding sites that bind a different epitope of TNFR2. In one embodiment, at least one of the binding sites of the molecule will bind a target other than TNFR2. For example, in one embodiment a binding molecule of the present invention may include a binding site with a different specificity that recognises a target of a different cell type. For example, the different specificity can be used as a homing entity to bring the target the cell expressing TNFR2, together with a different cell type. In one embodiment, a binding molecule of the present invention may include at least one binding site that binds a target that alters the serum half-life of the binding molecule, for example in one embodiment it may bind serum albumin. In one embodiment, only one of the binding sites of the molecule will target something other than TNFR2.

A binding molecule of the present invention will have a valency of at least two for TNFR2. In one embodiment, a binding molecule of the present invention will have a valency of two, three, four, five, six, seven, eight, nine, ten, eleven or twelve, or at least those valencies for TNFR2. In one preferred embodiment, a binding molecule of the present invention will have a valency of two. In another preferred embodiment, it will have a valency of three. In one embodiment, a binding molecule of the present invention is trispecific. In another preferred embodiment, a binding molecule of the present invention will have a valency of at least four. Hence, in a preferred embodiment, a binding molecule of the present invention is tetravalent. In one preferred embodiment, a binding molecule of the present invention will have a valency of four. In one embodiment, a binding molecule of the present invention will have a valency of at least six. In a preferred embodiment, it will have a valency of six. In one preferred embodiment, a binding molecule of the present invention will have a valency of twelve. In one embodiment, a binding molecule of the present invention is multi-specific, for instance as it has a valency of at least two, and preferably at least four.

As well as the "valency" of a binding molecule of the present invention, a further point is the orientation/localization of the binding sites of the binding molecules. That may be best considered using the examples of the antibodies shown in Figures 1 and 7. Figure 1 depicts antibodies with (N-terminal) unidirectional oriented antigen-binding sites, solely in what can be thought of as "North" orientation, for example in one embodiment such binding molecules may be thought of having the antigen-binding sites that bind TNFR2 at what would be considered the N-terminal portion of the heavy and light chains of a conventional four chain antibody and they may be located at the N-terminal portions of the polypeptides of the binding molecules. The orientation of the binding sites may also be defined by referring to binding sites in the same orientation as "parallel" binding sites, for example where all of the binding sites are at the N-terminal portions of the antibody chains or all at the C-terminal portion of the antibody chains, the binding sites may be considered as parallel. Such binding sites may also be considered as unidirectional orientated. Figure 7 of the present application depicts antibodies with dual N- and C-terminal antigen binding sites in what can be thought of as "North"+"South" orientations or "opposite" orientations. In one embodiment, such binding sites may be thought of as having antigen-binding sites in more than one plane or more than one direction. In one embodiment, a construct of the invention is one where the antigen binding sites are solely in the "North" orientation, so the antigen-binding sites are N-terminal. In one embodiment, a binding molecule of the present invention is a North construct with scFvs replacing the variable domains of the HC and LC, for example (scFv-IgG(N297A) and scFv-IgG(N297A)-HC:TNC). In another embodiment, a binding molecule of the present invention may have binding sites in both the "North" and "South" orientation, so effectively have antigen-binding sites at both the N-terminal and C-terminal locations..

In one embodiment, "North" facing antigen-binding sites may be thought of as in the same orientation or plane as the binding site of a conventional monomeric IgG molecule, in particular what would be normally considered the N-terminal portion of the light and heavy chain variable regions of such a conventional IgG molecule. In one embodiment, "South" facing antigen binding sites may be thought of those in a direction or a plane that a conventional monomeric IgG molecule does not have antigen binding sites. In one embodiment the orientation is the opposite from that of the Fab subdomains (VH+VL) domain in a conventional monomeric IgG molecule thus having the TNFR2 binding domain at the Fc domain C-terminus directing to neighboring cells of the antigen expressing cell. Such binding sites can be thought of as in the opposite to the "normal" orientation of a Fab domain in an antibody. Whilst the hexameric IgG molecule depicted in Figure 1 has antigen-binding sites in the top and bottom of the hexamer it is not considered to have "North" and "South" or "opposite" orientated antigen-binding sites, because all of the antigen-binding sites are in the same format as a conventional monomeric IgG molecules, for example in the sense that it does not have antigen-binding sites at what would be considered the C-terminal portion of a conventional monomeric IgG molecule. In one preferred embodiment, an antibody of the present invention comprises antigen binding sites at the N-terminal ends of the polypeptides making up the antibody. In one embodiment, it may be that a binding molecule of the present invention is an antibody in the sense that it has antigen binding sites at both the N-terminus and C-terminus of polypeptide chains of the antibody.

In one preferred embodiment, the binding molecule employed is a multimer of individual subunits. For example, in one preferred embodiment the binding molecule of the present invention is a dimer. In one embodiment of the present invention the dimer is form of two scFv-Fab molecules. In one preferred embodiment the binding molecule of the present invention is a trimer. In another preferred embodiment, a binding molecule of the present invention is a hexamer. In one embodiment, the binding molecule employed may be an antibody. In one embodiment the binding molecule employed may be an antibody which is an antibody fusion protein hexamer. In one preferred embodiment, the heavy chains of the individual IgG molecules making up the hexamers comprise an RGY peptide motif and in particular may do so at their C-terminus. In one particularly preferred embodiment an antibody of the present invention comprises the mutations of the RGY motif which are E345R/E430G/S440Y in the heavy chain and in particular in the Fc tail. The RGY motif is described, for instance, by de Jong et al (2016) PLOSBiology IDOI:10. 1371/journal.pbio.1002344 which is incorporated by reference in its entirety, as well as specifically in relation to the RGF motif and its use in the formation of hexamers.

Typically, where a binding molecule of the invention comprises a multimer or assembly of smaller units it will still be considered a "binding molecule" or "antibody" of the present invention, for instance even though it may comprise several subunits that might individually be considered an antibody, such as an IgG and scFv. So, for instance, an "antibody" of the present invention may comprise an antibody fragment or fragments as part of the overall "antibody" of the present invention. As discussed below, it may comprise other entities such as ligands for TNFR2, such as TNF-alpha and in particular TNF80 and other TNFR2-specific TNF mutants.

In one embodiment, an antibody of the present invention will comprise at least a heavy chain modification at E345 and in particular E345R. In another embodiment, an antibody of the present invention may comprise at least a heavy chain modification at E430 and in particular E430G. In another embodiment, an antibody of the present invention may comprise at least a heavy chain modification at S440 and in particular S440Y. In another embodiment, an antibody of the present invention may comprise at least heavy chain modifications at E345 and E430 and in particular E345R and E430G. In a preferred embodiment, an antibody of the present invention may comprise at least heavy chain modifications at E345, E439, and S440, in particular E345R, E439G and S440Y. In a preferred embodiment, an antibody of the invention will comprise such modifications and comprise a hexamer of IgG units. In a further preferred embodiment, an antibody of the present invention will undergo on-target hexamerisation once it has bound to TNFR2.

In one preferred embodiment, a binding molecule of the present invention may comprise sequences that bring about trimerization. Examples of such sequences include trimerisation domains from collagen, leucine zipper, T4 fibritin, and trimerization domains of TRAF family members. A binding molecule of the present invention in some embodiments may comprise such trimerization sequences. In one embodiment, a binding molecule of the resent invention may comprise, or may be, a trimer due to the presence of such domains. In one preferred embodiment, the trimerization sequence may be that from tenascin-C (TNC). However, in any of the embodiments discussed herein where TNC is referred to a trimerization sequence in general may be employed, for example any of the specific ones referred to herein and not just TNC, though in a preferred embodiment TNC is used. In one preferred embodiment the TNC sequence used in from chicken TNC. In another preferred embodiment, it is from a mammalian TNC. In a further preferred embodiment, the TNC is human TNC.

In another preferred embodiment, the tenascin-C (TNC) trimerization domain may be used to assemble individual antibody or other subunits into a multimer. Hence, a binding molecule, and in particular an antibody of the present invention, may comprise a TNC trimerization domain peptide such as that in SEQ ID NO: 2. It may comprise a variant TNC peptide, for example one with five, four, three, two, or one amino acid sequence change relative to SEQ ID NO:2, but which is still able to bring about the formation of multimers. In one preferred embodiment, the C-terminus of the heavy chains of the antibody comprise, or are linked to, the TNC peptide sequence. In one embodiment, such an approach is used to bring together a trimer of individual TNFR2 binding moieties such as antibody subunits antibodies, and/or ligands. Any of the binding molecules set out herein, unless otherwise stated, may comprise a TNC peptide as a way to allow association of individual units and in particular as a way to form multimers.

In one embodiment, a binding molecule of the present invention is an FcyR independent agonist of TNFR2. For example, in the case where the binding molecule is an antibody, it may be that the antibody lacks an Fc region and so does not bind FcyR for that region. Alternatively, in another embodiment, it may be that the antibody does have an Fc region, but that it has been modified so that it is unable to bind FcyR. Various Fc modifications are discussed herein that may be used to accomplish that.

In one embodiment, a binding molecule of the present invention will be an antibody selected from an IgG, IgM, IgA, IgE and IgD antibody or comprise such an antibody. It may comprise the Fc modifications discussed herein that render it Fc gamma Receptor independent. In one particularly preferred embodiment, it will be, or comprise, an IgG antibody or comprise such an antibody. In one particularly preferred embodiment it will be an IgG1 antibody or comprise such an antibody. The antibody may be an IgG2, IgG3, or IgG4 antibody or comprise such an antibody. In a particularly preferred embodiment, an antibody of the present invention may comprise single domain antibodies, for example VHH single domain antibodies. In one embodiment, an antibody of the present invention will comprise at least two such VHH single domain antibodies.

In one particularly preferred embodiment of the present invention, the binding molecule is, or comprises, an antibody. As discussed further below, reference to an antibody encompasses antibodies in various formats, including antibodies that comprise, or have linked to them, ligand based molecules for binding TNFR2.

### Binding molecule formats

In a preferred embodiment, the present invention provides binding molecules that specifically bind TNFR2, but not TNFR1. In a further preferred embodiment, the binding molecule is an FcyR independent agonist of TNFR2. The binding molecule has a valency of at least two for binding TNFR2. Such a binding molecule of the present invention may be provided in a variety of different formats.

In a preferred embodiment, the binding sites of a binding molecule of the present invention may be selected from antibody antigen-binding sites and ligands that bind to TNFR2. In an especially preferred embodiment, a binding molecule of the present invention is an antibody or comprises at least part of an antibody. As defined herein an antibody includes various formats that include antigen-binding sites, but are not in a naturally occurring antibody format. An antibody does not necessarily have to be a whole antibody, for example an antibody of the present invention may be, or comprise, an antibody fragment, such as Fab2 antibody fragment. Antibodies of the present invention may comprise antibody fragments, for example in one preferred embodiment an antibody of the present invention may comprise an antigen-binding site provided by a scFv fragment or Fab as part of the antibody. An antibody of the present invention may be, or comprise, a single chain antibody.

Reference to an antibody includes antibody molecules that comprise, are linked to, or conjugated to moieties that do not themselves originate from antibody based sequences. So, for instance, reference to an antibody herein includes an antibody which is linked or conjugated to a TNFR2 ligand or a modified version of a TNFR2 ligand. Examples of TNFR2 ligands that may be employed, or be modified to be employed, include TNFα and LTα, as well as variants thereof. In one embodiment, a binding molecule, and in particular an antibody, of the present invention may comprise sequences that bring about multimerisation. An example of one such sequence is that an antibody of the present invention may comprise a heavy chain with a sequence that brings about multimerisation, for example by including the RGY mutations that bring about multimerisation. In one preferred embodiment, it is the Fc tailpiece which comprises the RGY modifications to bring about hexamerisation. In another particularly preferred embodiment, an antibody of the present invention may comprise a tenascin-C trimerization domain peptide sequence (TNC) at the C-terminus of heavy chains. Hence, in one embodiment, a binding molecule of the present invention, particularly an antibody, may comprise a peptide sequence from Tenascin-C which is provided as SEQ ID NO: 2 or a variant TNC peptide which is still able to bring about multimerisation. In another embodiment, it may comprise the human TNC sequence which brings about trimerization. In another embodiment, it may comprise a trimerization domain, such as any of the specific ones mentioned herein.

In one embodiment all of the binding sites of a TNFR2 binding molecule of the present invention may be antibody antigen-binding sites. In another embodiment, a binding molecule of the present invention comprises at least one antibody antigen-binding site for TNFR2 and at least one ligand, or ligand derivative, that binds TNFR2, in other words it may comprise a mixture of such binding sites. In one embodiment, a binding molecule of the present invention comprises an antibody which further comprises ligand based binding sites for TNFR2. In one preferred embodiment, a binding molecule, and in particular an antibody, of the present invention does not comprise ligand based TNFR2 binding sites. In embodiments where the binding molecule is an antibody, an antigen binding site will generally comprise six CDRs, three light chain variable region CDRs (LCDR1, LCDR2, and LCDR2) and three heavy chain CDRs (HCDR1, HCDR2, and HCDR3).

In one preferred embodiment, the antigen binding sites of the antibody and the ligand based binding sites are in different orientations, so that the two may be in opposite orientations, for example the antigen-binding sites may be in "North" orientation and the ligand based binding sites in "South" or "opposite" orientation. In another embodiment, the binding molecule may have antibody antigen-binding sites in both "North" and "South" orientations, but ligand based binding sites in only one orientation, for example solely in "South" orientation.

In one embodiment of the invention an antibody with only "North" orientated binding sites is employed. Figure 1 of the present application shows illustrative examples of preferred TNFR2 binding molecules, as well as Fab which has a valency of one and so on its own is not a binding molecule of the present invention.

In the formats shown by the Figures, in particular Figures 1 and 7, the structures are indicated with a "CI" or "Clone Identifier" where an parental TNFR2-specific IgG molecule is referred to, with the Clone Identifier indicating from which clone discussed in the Examples the CDRs of the IgG antigen-binding sites originate. Where reference is made herein to such structures, but without the Clone Identifier being used, the structure is then not limited to reference to a specific clone, so is not limited to the CDRs being from a particular clone from the Examples, and the antigen-binding sites may be from any suitable TNFR2 antibody. So, for instance, reference to scFv-C4-IgG1(N297A) includes the clone identifier (CI) for Clone 4, but where scFv-IgG1(N297A) is referred to it indicates such a format binding molecule but without the antigen-binding site being limited to sequences from a particular clone.

In preferred embodiments, a binding molecule of the present invention is selected from the following:
i. A Fab2 antibody where both specificities are specific for TNFR2.
ii. An IgG antibody which comprises an Fc region that is not able to activate Fc receptors and in particular does not bind FcyR. Both valencies of the molecule are specific for TNFR2 and may be thought of as "North" orientated. In one embodiment, the Fc region of the antibody comprises a modification that means it does not bind to Fc receptors, such as any of the specific modifications set out herein. In one embodiment, the antibody is an IgG1 antibody. In a preferred embodiment it has the format IgG1(N297A) shown in Figure 1, but not limited to a specific clone.
iii. A tetravalent antibody molecule, comprising four antigen-binding sites specific for TNFR2 which are all "North" orientated, where the antibody has the basic structure of an IgG molecule modified so that each light and heavy chain has at its N terminus an antigen binding site linked to it. In one embodiment, each antigen-binding site comprises a pair of heavy and light chain variable regions (scFv domain) which are joined to a light or heavy chain of the basic four chain IgG structure. In one embodiment, the antibody comprises the heavy and light chain constant regions of a basic IgG molecule, where each N-terminus of an antibody chain has a scFv joined to it. The antibody typically comprises a Fc region that has been modified so that it does not bind FcyR. In one embodiment it comprises any of the modifications discussed herein that prevent or reduce FcyR binding. In one embodiment, the antibody has the format scFv-IgG1(N297A) as shown in Figure 1 but is not limited to the sequences originating from a particular clone.
iv. An antibody with a valency of six which is a multimer of three IgG antibody subunits, the Fc regions of each individual IgG subunit being unable to bind FcyR. All six antigen-binding sites may be thought of as "North" orientated. In one embodiment, the Fc regions are unable to bind receptors of the Fcgamma-Receptor group because they comprise a modification, such as any of those disclosed herein. In another embodiment, the Fc regions comprise the N297A modification. Any suitable way may be used to multimerise the individual IgG subunits. In a particularly preferred embodiment, the subunits associate through the use of a tenascin-C peptide (TNC) and in particular the presence of a peptide derived from TNC such as the peptide sequence of SEQ ID NO: 2. For example, in one embodiment, the heavy chains of the IgG subunits comprise or are linked to a tenascin peptide, with the association of the tenascin peptides leading to assembly of the multimer. In a preferred embodiment the peptides have the sequence of SEQ ID NO: 2 or a variant sequence thereof still able to multimerise. In one embodiment, the antibody has the format IgG1(N297A)-HC:TNC as shown in Figure 1, but is not limited to sequences from a particular clone.
v. An antibody with a valency of 12 representing a multimer of three subunits. The subunits in one embodiment comprising a IgG structure where the variable regions have each been replaced with a scFv specific for TNFR2, where all of the antigen binding sites may be thought of as "North" facing, e.g. scFv-IgG1(297A)-HC:TNC. The antibody is unable to bind Fc receptors, in one embodiment it does not bind FcyR because the Fc region has been modified to render it unable to do so, for example any of the modifications discussed herein have been employed. In a preferred embodiment the N297A modification is employed. Any suitable way may be used to bring together the three subunits and in one embodiment the TNC trimerization domain is employed. In a preferred embodiment, the C terminus of each heavy chain either comprises, or is linked to, a TNC trimerization domain to bring about multimerisation of several Fab2 or IgGs, such as that of SEQ ID NO: 2. In one embodiment the antibody has the format scFv:IgG1(N297A)-HC:TNC shown in Figure 1, but is not limited to sequences from a particular clone.
vi. A hexameric IgG antibody. Typically, all the antigen binding sites of the antibody are specific for TNFR2 so that the antibody has a valency of twelve. The Fc regions of each individual IgG subunit have a modification preventing binding to FcyR. Any of the modifications discussed herein may be employed, in one preferred embodiment the modification being N297A. Any suitable way to achieve hexamerisation either on or off target may be employed. In one particularly preferred embodiment each heavy chain of the individual IgG subunit comprises the RGY mutations to allow for hexamerisation. In one embodiment, the antibody carries the RGY mutation plus the N297A mutation (IgG1(N-RGY) shown in Figure 1) and so has both the mutation preventing Fc receptor binding and the mutations resulting in hexamerisation. Hence, in one embodiment the binding molecule has the format IgG1(N-RGY) as shown in Figure 1 but is not limited to sequences from a particular clone.

In one particularly preferred embodiment, the binding molecule is one of those set out in (iii) to (vi) above. In another preferred embodiment, the binding molecule is a tetravalent binding molecule, for example that set out in (iv). In a further preferred embodiment, the binding molecule is one with a valency of 12, for example selected from those set out in (v) and (vi) above.

In one embodiment of the invention, an antibody with both N-terminal and C-terminal placed binding sites on an antibody scaffold with parallel oriented chains (Fc, IgG, Fc-TNC, TNC-Fc, IgG-TNC) is employed. Hence, an antibody with "opposite" orientated binding sites is employed. Figure 7 of the present application shows illustrative examples of particularly preferred TNFR2 binding molecules which have binding sites which may be considered as in "opposing" orientations. In preferred embodiments, a binding molecule of the present invention is selected from the group set out below:
i. An antibody comprising a Fab region joined to a scFv, where the orientation of the binding sites of the Fab and the scFv for TNFR2 are in opposite orientation, thus the scFv domain is linked to the C-terminus of LC or the truncated HC. In a preferred embodiment, the scFv is joined to the C-terminus of the light chain of the Fab. Hence, in one embodiment an antibody of the present invention is in the format Fab-LC:scFv shown in Figure 7, but not limited to sequences from a particular clone.
ii. An antibody which is an IgG molecule which has scFv attached to the C-terminus of each heavy chain and an Fc region modified so that it does not bind to Fc receptors and in particular FcyR. The Fc region may comprise any of the modifications discussed herein that eliminate Fc binding and in particular the N297A heavy chain modification. In one embodiment, the antibody is of the format IgG1(N297A)-HC:scFv shown in Figure 7, but not limited to sequences from a particular clone.
iii. An antibody which is an IgG molecule which has scFv attached to the C-terminus of each light chain and an Fc region modified so that it does not bind to Fc receptors and in particular FcyR. The Fc region may comprise any of the modifications discussed herein that eliminate Fc binding and in particular the N297A heavy chain modification. In one embodiment, the antibody is of the format IgG1(N297A)-LC:scFv shown in Figure 7, but which is not limited to sequences from a particular clone.
iv. An antibody which is an IgG molecule which has scFv attached to the C-terminus of each light and heavy chain and an Fc region modified so that it does not bind to Fc receptors and in particular FcyR. The Fc region may comprise any of the modifications discussed herein that eliminate Fc binding and in particular the N297A heavy chain modification. In one embodiment, the antibody is of the format IgG1(N297A)-LC:scFv-HC:scFv shown in Figure 7.
v. An antibody which is an IgG molecule which has been modified so that the heavy and light chain variable regions have each been replaced with a TNFR2-specific scFv and where the heavy chains of the IgG molecule each have a TNFR2-specific scFv joined to them. Hence, such an antibody has a valency of six for TNFR2. The Fc region of the antibody having been modified so that it does not bind to Fc receptors and in particular FcyR. The Fc region may comprise any of the modifications discussed herein that eliminate Fc binding and in particular the N297A heavy chain modification. In one embodiment, the antibody is of the format LC:scFv-HC:scFv-IgG1(N297A)-HC:scFv shown in Figure 7, but not limited to sequences from a particular clone.
vi. An antibody which is an IgG molecule which has been modified so that the heavy and light chain variable regions have each been replaced with a scFv and where the light chains of the IgG molecule each have a scFv joined to them. Hence, such an antibody has a valency of six for TNFR2. The Fc region of the antibody having been modified so that it does not bind to Fc receptors and in particular FcyR. The Fc region may comprise any of the modifications discussed herein that eliminate Fc binding and in particular the N297A heavy chain modification. In one embodiment, the antibody is of the format LC:scFv-HC:scFv-IgG1(N297A)-LC:scFv shown in Figure 7, but not limited to sequences from a particular clone.
vii. An antibody which is an IgG molecule which has been modified so that the heavy and light chain variable regions have each been replaced with a scFv and where the light and heavy chains of the IgG molecule each have a scFv joined to them. Hence, such an antibody has a valency of eight for TNFR2. The Fc region of the antibody having been modified so that it does not bind to Fc receptors and in particular FcyR receptors. The Fc region may comprise any of the modifications discussed herein that eliminate Fc binding and in particular the N297A heavy chain modification. In one embodiment, the antibody is of the format LC:scFv-HC:scFv-IgG1(N297A)-LC:scFv-HC:scFv shown in Figure 7, but not limited to sequences from a particular clone.
viii. A multimer comprising three individual IgG antibody molecules that comprise at the C-terminus of the heavy chain a tenascin-C (TNC) trimerization peptide, with each tenascin peptide joined with its C-terminus to a scFv. The antibody molecule having a valency of twelve for TNFR2. The Fc region of the antibody having been modified so that it does not bind to Fc receptors and in particular FcyR. The Fc region may comprise any of the modifications discussed herein that eliminate Fc binding and in particular the N297A heavy chain modification. In one embodiment, the antibody is of the format IgG1(N297A)-HC:TNC-scFv shown in Figure 7, but not limited to sequences from a particular clone.

As discussed above, the formats discussed above shown in Figure 1 are provided where they are not limited to the sequences originating from a particular clone. In one embodiment, also provided are the specific formats where they do have the sequences from a specific clone provided in the Examples or variant sequences as discussed herein. In one embodiment for the formats with Fc regions, the heavy chains comprise heavy chain modifications as discussed herein, such as the DANA modifications discussed herein, and not just the N297A modification.

In one particularly preferred embodiment, the binding molecule of the present invention is an antibody selected from one of the following formats, with some of the advantages of the formats mentioned in brackets:
- Fab 1-scFv (high tissue penetrance, short-lived);
- IgG-HC:scFv (tested for a variety of antibodies, easy generation of bispecific variants);
- scFv-IgG (easy generation of bispecific variants);
- IgG-TNC (potentially useful in pathway discrimination); and
- scFv-IgG-TNC (highly active, easy generation of bispecific variants).

In one particularly preferred embodiment, where IgG is referred to above IgG1 is employed. Alternatively, in another embodiment, where IgG1 is referred to in a particular format, the same format is also provided where the IgG may be any IgG subtype.

In one embodiment, a binding molecule of the present invention is an antibody that:
(a) comprises a TNFR2 binding site at both the N-terminus and C-terminus of one or more heavy chain of the antibody; and/or
(b) comprises a TNFR2 binding site at both the N-terminus and C-terminus of one or more light chain of the antibody.

In one preferred embodiment, the binding molecule comprises a scFv at the C-terminal portion of heavy chains of the binding molecule, where the scFv binds TNFR2. In another embodiment, a binding molecule of the present invention comprises scFv at the C-terminal portion of the light chains in the molecule.

In one particularly preferred embodiment, a binding molecule of the present invention is an antibody that just comprises an Fc region which at the C-terminal portion of the heavy chains either has, or is linked to, or is conjugated to, ligands specific for TNFR2. In one embodiment, each heavy chain has three such ligands present at the C terminal portion of the heavy chains.

In one particularly preferred embodiment, a binding molecule of the present invention is an antibody or Fc region with a C-terminal TNC trimerization followed by a TNFR2-specific TNF mutant, in particular TNF80. In one embodiment, a polypeptide may comprise a single chain polypeptide comprising three TNF-alpha sequences that can tri-merise, such as three TNF80 units.

In one preferred embodiment, a binding molecule of the present invention is bivalent with spatial movable C- and N-terminal located antigen-binding sites.

In one embodiment, an antibody of the present invention is a bivalent antibody which comprises an Fab specific for TNFR2, where the heavy or light chain of the Fab portion of the antibody comprises an scFv specific for TNFR2. One advantage of such a small molecule is that it may show high tissue penetration. In one preferred embodiment, the antibody may comprise the scFv joined to the light chain of the Fab, for example with the two being part of the same polypepide. In one preferred embodiment, the antibody is in the format of Fab-LC:scFv as shown by Figure 7, but is not limited to sequences from the clones provided in the present Examples and may be any suitable sequences from an antibody for TNFR2. The Fab and scFv domains may, for instance, recognize in these embodiments the same or different TNFR2 epitopes.

In one preferred embodiment, the binding molecule, and in particular an antibody, of the present invention is tetravalent. In one embodiment, the binding molecule of the present invention is in the format Fab-HC-scFv, where the antibody comprises an Fc region modification eliminating or reducing Fc receptor binding, and where the antibody comprises a scFv at the C-terminal end of each heavy chain. In another preferred embodiment, the antibody is in the format Fab-HC-scFv where the antibody is bispecific in the sense that it has two different specificities for TNFR2. For example, in one preferred embodiment, the scFvs present bind a different epitope than the antigen-binding sites of the IgG. In an alternative embodiment, all of the TNFR2 binding sites recognize the same epitope of TNFR2. In a further preferred embodiment, an antibody of the present invention is in the format scFv:IgG, where the light and heavy chains of the antibody have been modified to include an scFv at the N-terminal portion, with the Fc region of the antibody modified so that it does not bind Fc receptors. Any of the modifications discussed herein that reduce or eliminate Fc binding may be employed. Such an antibody may be bispecific in the sense that the scFvs expressed on the heavy chain polypeptide may bind one epitope of TNFR2 and those of the light chains another specificity of TNFR2. In an alternative embodiment, all of the scFv regions may bind the same epitope of TNFR2.

In one embodiment, a binding molecule of the present invention is hexavalent. In one embodiment the antibody is in the format IgG-LC:scFv-HC:scFv and also has a modified Fc region so that it does not bind Fc receptors, for example having any of the Fc modifications discussed herein that eliminate or reduce binding to Fcγ receptors. In a preferred embodiment, the IgG is IgG1. In another preferred embodiment, the antibody is hexavalent, where it comprises an antibody Fc region, with each of the heavy chains linked to a trimer of TNF-α, or a variant thereof, at the C-terminal portion.

In another preferred embodiment, a binding molecule of the invention is 12-valent. In one preferred embodiment, a 12-valent binding molecule is a multimer of three individual antibody subunits where the heavy and light chain variable regions of the antibody are replaced with scFv molecules. In a preferred embodiment, the three antibody subunits are associated as the heavy chains for each antibody comprise a tenascin-C peptide at the C-terminus comprising the trimerization domain of TNC. The binding molecule will not bind Fc receptors and in a preferred embodiment comprises an Fc region with any of the modifications set out herein. In another embodiment, a binding molecule of the present invention may be a hexabody comprising six IgG molecules. For example, the binding molecule may be a hexabody of six IgG antibodies, where the Fc regions comprise RGY HexaBody mutations and also a modification that means they do not bind Fc receptors, such as any of the specific modifications set out herein. In a preferred embodiment the IgG is IgG1.

In one preferred embodiment, a binding molecule of the present invention is four or 12 valent for TNFR2. In one preferred embodiment a binding molecule of the present invention is an antibody selected from the format scFv-IgG(*)-HC-TNC and scFv-IgG(*), where * indicates an Fc region modification which eliminates or reduces Fc receptor binding. In a particularly preferred embodiment, the IgG portion of the binding molecule is IgG1. HC-TNC indicates that the heavy chains of the IgG comprise a tenascin peptide, for example that of SEQ ID NO:2 or a variant thereof. In another embodiment, a binding molecule of the present invention is an antibody selected from the format scFv-IgG(*)-HC-TNC, scFv-IgG(*), and IgG(*)-HC:RGY. For the IgG(*)-HC:RGY format again preferable the IgG portion of the molecule is an IgG1 antibody.

In an especially preferred embodiment, a binding molecule of the present invention will be able to bring about the formation of hexamers of TNFR2 molecules on the surface of the cell or at least hexamers. In one embodiment a binding molecule of the present invention may bind TNFR2 receptors in cis, that is bind a plurality of TNFR2 receptors on the surface of the same cell. In one embodiment, the binding molecule is able to at least bring about the formation of hexamers of TNFR2 on the surface of the same cell. In another embodiment, a binding molecule of the present invention is able to bind TNFR2 molecules in trans, that is the binding molecule binds at least one TNFR2 molecule on the surface of one cell and at least one TNFR2 molecule on the surface of another cell. In one embodiment, the binding molecule is able to bind cis and trans to TNFR2. For example, it may have the capability to do so and in another embodiment it may actually do so. In one embodiment a binding molecule of the present invention results in the formation of hexamers of TNFR2 on both cells when trans binding occurs. In one embodiment, the orientation of the binding sites may influence whether an antibody binds to TNFR2 in cis and/or trans fashion. In one embodiment, a binding molecule of the present invention is one that has at least two TNFR2 binding sites that have opposing orientation compared to each other preferably wherein the binding molecule has at least two such TNFR2 binding sites that are antibody antigen-binding sites. In one preferred embodiment, a binding molecule of the present invention is one that has at least two TNFR2 binding sites that are able to bind TNFR2 molecules on different cells allowing for trans-binding, preferably wherein the binding molecule has at least two such TNFR2 binding sites that are antibody antigen-binding sites. In another preferred embodiment a binding molecule of the present invention has a valency of at least four and is able to bind a plurality of different molecules of TNFR2 on the surface of the same cell, preferably where the binding molecule has a valency of at least six.

In another embodiment, a binding molecule of the present invention may be a diabody. A diabody is typically a small bivalent antigen-binding antibody portion, and there are a variety of diabody formats, any of which may be used, for example a diabody may comprise a heavy chain variable domain linked to a light chain variable domain on the same polypeptide chain linked by a peptide linker that is too short to allow pairing between the two domains on the same chain. This results in pairing with the complementary domains of another chain and in the assembly of a dimeric molecule with two antigen binding sites for TNFR2.

A wide variety of antibody formats exist and may be employed either as an antibody of the present invention or as part of it, for example Fab, Fv, scFv, (ScFv)2, (ScFv2), bispecific (scFv2), triabody, tetrabody, bispecific sc(Fv)2, tandem di-scFv, tandem tri-scFv, and tribodies all represent possible antibody formats of the present invention. As discussed above, a binding molecule of the present invention has a valency of at least two. In one preferred embodiment, a binding molecule of the present invention has a valency of at least two spatially movable potentially cell-cell connecting TNFR2 binding sites or at least six "parallel" or "unidirectionally" oriented cis-acting binding sites. In embodiments where formats mentioned herein have a valency of one they may be employed as part of an antibody of the present invention in such formats, rather than as the whole antibody. In another preferred embodiment, a binding molecule of the present may comprise VHH fragments. Organisms such as Camelids, sharks, and other cartilaginous fish produce heavy chain-only antibodies. The single-domain variable fragments of these heavy chain-only antibodies are termed VHHs or nanobodies or sdAb. VHHs retain the immunoglobulin fold shared by antibodies, using three hypervariable loops, CDR1, CDR2 and CDR3, to bind to their targets. A VHH fragment (e.g., NANOBODY^{®}) is a recombinant, antigen-specific, single-domain, variable fragment derived from camelid heavy chain antibodies. As a binding molecule of the present invention preferably has a valency of at least two for TNFR2, in one embodiment where the binding molecule comprises VHH fragments it will comprise at least two such VHH fragments. The individual VHH fragments may be joined together, for example through the use of a linker and hence, in one embodiment, may be expressed as a single polypeptide which overall comprises at least two VHH fragments which are each able to specifically bind TNFR2. VHH fragments do not have a Fc region and in one embodiment where VHH fragments are present as part of a binding molecule of the present invention, the binding molecule will lack an Fc region and so not bind Fc receptors for that reason. In one embodiment, a binding molecule of the present invention will comprise, or at least comprise, three, four, five, six or more VHH fragments specific for TNFR2. In another embodiment, a binding molecule of the present invention will comprise a single VHH domain antibody.

In one embodiment, rather than comprising one of the specific binding sites for TNFR2 set out herein, a binding molecule of the present invention may comprise a variant binding site for TNFR2. In one preferred embodiment, a variant binding molecule for TNFR2 will be defined by its ability to cross-block one of the specific binding molecules for TNFR2 set out herein binding to TNFR2. The ability to cross-block may be assessed using a binding assay for measuring binding of a binding molecule to TNFR2 and the ability of a further binding molecule to cross-block such binding to TNFR2. In one embodiment, the ability to cross-block is studied using a monovalent molecule that just comprises one copy of the binding site for TNFR2. For example, the ability of a scFv comprising the binding site to cross-block a scFv that only differs in having a binding site for TNFR2 from one of the specific binding molecules set out herein is studied. In another embodiment, the ability of the actual binding molecules to cross-block each other is studied without converting them into scFv molecules. In a further embodiment, a binding molecule of the present invention will be able to block, or reduce, the binding of TNFα to TNFR2. In another embodiment, a binding molecule of the present invention will be able to reduce, or block the binding of lymphotoxin-alpha to TNFR2

In one embodiment, the affinity of the binding domain for TNFR2 in a binding molecule, in particular an antibody, of the present invention, has a K_{D} which is about 400 nM or smaller, 200 nM or smaller such as about 100 nM, 50 nM, 20 nM, 10 nM, 1 nM, 500 pM, 250 pM, 200 pM, 100 pM or smaller. In one embodiment, the binding affinity is 50 pM or smaller. For example, in one embodiment an individual TNFR2 binding site in a binding molecule of the present invention will show such affinity for TNFR2, rather than that being the overall affinity for the binding molecule. In one embodiment, such affinity is the overall avidity of the antibody for TNFR2. In one embodiment, the affinity of an individual antigen-binding site of a binding molecule of the present invention may be less than 1 µM, less than 750 nM, less than 500 nM, less than 250 nM, less than 200 nM, less than 150 nM, less than 100 nM, less than 75 nM, less than 50 nM, less than 10 nM, less than 1 nM, less than 0.1 nM, less than 10 pM, less than 1 pM, or less than 0.1 pM. In some embodiments, the Kd is from about 0.1 pM to about 1 µM.

In one embodiment, an antibody of the present invention is a monoclonal antibody. In one embodiment, an antibody of the present invention is an IgG1, IgG2 or IgG4 antibody or comprises heavy chain constant region of that class of antibody. In one especially preferred embodiment, an antibody of the present invention is a IgG1 isotype antibody or comprises such an antibody. Without particular limitation, the term "antibody" encompasses antibodies from any appropriate source species, including chicken, sharks and mammalian such as mouse, hamster, rabbit, goat, cow, llama, non-human primate and human. In one preferred embodiment, an antibody of the present invention is a humanised antibody. In antoher preferred embodiment, the binding molecule, and in particular antibody, of the present invention is wholly human. Nonlimiting examples for methods to generate humanized antibodies are known in the art, e.g. from Riechmann et al. (Nature. 1988 Mar 24; 332(6162):323-7) or Jones et al. (Nature. 1986 May 29-Jun 4; 321 (6069) :522-5). In a further preferred embodiment, an antibody of the present invention is a human antibody. As discussed herein, a binding molecule of the present invention, and in particular an antibody, may comprise non-human sequences such as a tenascin-c peptide, but in one preferred embodiment all of the antibody based sequences of the antibody are human notwithstanding the presence of the tenascin peptide sequence. The term "antibody" encompasses monomeric antibodies (such as IgD, IgE, IgG) or oligomeric antibodies (such as IgA or IgM). The term "antibody" also encompasses - without particular limitations - isolated antibodies and modified antibodies such as genetically engineered antibodies, e.g. chimeric or humanized antibodies.

In one preferred embodiment a binding molecule of the present invention has only one specificity for TNFR2. Hence, in one preferred embodiment all of the antigen-binding sites of an antibody of the present invention which are specific for TNFR2 will be specific for the same epitope of TNFR2. In another embodiment, a binding molecule of the present invention will comprise more than one specificity for TNFR2. For example, that may be because the binding molecule that comprises both antibody antigen-binding sites, but also ligand based TNFR2 binding sites. In one preferred embodiment though, a binding molecule of the present invention is an antibody that comprises antigen binding sites that recognise more than one epitope of TNFR2. For example, in one preferred embodiment a binding molecule of the present invention, and in particular an antibody, is bispecific. In one preferred embodiment, a binding molecule of the present invention one specificity of the binding molecule for TNFR2 is provided by a pair of light and heavy chain variable regions whilst the other is provided by scFv fragments present as part of the overall binding molecule. In another embodiment, all of the TNFR2 binding sites of a binding molecule, and in particular antibody, are provided by scFvs with the binding molecule formed from two different polypeptides, each of which comprise a scFv with a different specificity.

In one embodiment, a binding molecule of the present invention may comprise an Fc region or an antibody molecule which has irrelevant specificity and the ability to bind to TNFR2 is provided by other portions of the binding molecule. For example, in one embodiment a binding molecule of the present invention comprises an Fc region where at the C-terminal portion of the heavy chains are TNF-alpha molecules and in particular mutant TNF-alpha molecules that are selective for TNFR2, such as the TNF80 mutant. In one embodiment, a binding molecule of the present invention may comprise an immunoglobulin of irrelevant specificity and at the C-terminal portion of the heavy chains may be TNF-alpha molecules, and in particular mutant TNF-alpha molecules such as TNF80. In one embodiment, the heavy chains of the Fc or irrelevant antibody may comprise as part of the same polypeptide a TNF-alpha and in particular a TNF80 mutant. In one embodiment, they may comprise a single chain polyptide comprising three TNF-alpha polypeptides and in particular three TNF80 polypeptides. In another embodiment, before the TNF-alpha portion of the polypeptides the heavy chains may comprise a TNC peptide or variant thereof. In a further preferred embodiment, a binding molecule of the present invention may comprise an Fc region that, rather than have variable regions has the two heavy chain portions of the Fc region as part of a single polypeptide.

An example of a preferred binding molecule comprising a TNF ligand is Fc(DANA)-TNC-TNF80 as depicted in Figure 24 where DANA indicates the heavy chains of the Fc regions comprising D265A and N297A mutantions and TNC indicates the tenascin trimerization domain peptide sequence. Hence, a preferred binding molecule of the present invention comprises three Fc regions where each heavy chain has the DANA modifications of D265A and N297A. In a further preferred embodiment, each of those heavy chain polypeptides comprises a TNC peptide. In a further preferred embodiment, each heavy chain polypeptide of each Fc region comprises a TNC peptide sequence followed by a TNF-alpha sequence and in particular a TNF80 sequence.

A further preferred binding molecule of the present invention is Fc(DANA)-TNF80 which comprises three Fc regions where each heavy chain polypeptide comprises the DANA modifications of D265A and N297A and a TNF-alpha, in particular a TNF80 sequence. The present invention also provides as binding molecules the Fc-TNC-TNF80 and Fc-TNF80 format molecules shown in Figure 24.

In another embodiment, a binding molecule is provided that specifically binds TNFR2, but not TNFR1, where the binding molecule is an FcyR independent agonist of TNFR2, has a valency of at least two for binding TNFR2, and comprises polypeptides comprising mutant TNF-alpha that bind TNFR2, but not TNFR1, and a tenascin peptide sequence that oligomerises the polypeptides. In one embodiment, such a binding molecule does not include antibody sequences. In one embodiment, the molecule comprises polypeptides that each comprise a mutant TNF-alpha sequence with a tenascin peptide, for example any of the tenascin polypeptides disclosed herein. In one preferred embodiment, the tenascin peptide sequences results in oligomerisation of such polypeptides. In one embodiment, the mutant TNF-alpha has 143N/145R modifications resulting in selective binding for TNFR2 over TNFR1. Such a TNF mutant may also be referred to as TNF80.

### Antibody constant regions and Fc region functions

In a preferred embodiment, a binding molecule of the present invention does not bind Fc receptors and in particular does not bind to FcyR receptors. In one preferred embodiment, the binding molecule of the present invention is an antibody and it does not bind to Fc rreceptors, either because it does not comprise an Fc region or alternatively as it is has an Fc region modified so that it does not bind Fc receptors. Fc domain as employed herein generally refers to -(CH₂CH₃)₂, unless the context clearly indicates otherwise, where CH2 is the heavy chain CH2 domain, CH3 is the heavy chain CH3 domain, and there are two CH₂CH₃ with one from each heavy chain. In one embodiment, an antibody of the present invention does not comprise a -CH2CH3 fragment. In one embodiment, an antibody of the present invention does not comprise a CH₂ domain. In one embodiment, an antibody of the present invention does not comprise a CH₃ domain.

In one embodiment, a binding molecule of the present invention binds to an Fc gamma receptor but to a substantially decreased extent relative to binding of an identical antibody comprising an unmodified Fc region to the FcgR (e.g., a decrease in binding to a FcyR by at least 70%, at least 80%, at least 90%, at least 95%, at least 98%, at least 99%, or 100% relative to binding of the identical antibody comprising an unmodified Fc region to the FcyR as measured). In a particularly preferred embodiment though the binding molecule has no detectable binding to an FcyR at all. Binding, including the presence or absence of binding, can be determined using a variety of techniques known in the art, for example but not limited to, equilibrium methods (e.g., enzyme-linked immunoabsorbent assay (ELISA); KinExA, Rathanaswami et al. Analytical Biochemistry, Vol. 373:52-60, 2008; or radioimmunoassay (RIA)), or by a surface plasmon resonance assay or other mechanism of kinetics-based assay (e.g., BIACORE^{™} analysis or Octet^{™} analysis (forteBIO)), and other methods such as indirect binding assays, competitive binding assays fluorescence resonance energy transfer (FRET), gel electrophoresis and chromatography (e.g. gel filtration).

In one embodiment, where an Fc region is present, the Fc region employed is mutated, in particular comprising a mutation described herein. In one embodiment the mutation is to remove binding to Fc receptors and in particular FcyR. In one preferred embodiment the antibody of the invention has been mutated so that it does not bind Fc receptors.

In one embodiment, an antibody of the present invention may comprise an aglycosyl IgG, for example to bring about reduced Fc function and in particular a nearly Fc-null phenotype. In one embodiment, an antibody of the invention has a modification at N297 and in particular N297A. In one embodiment an antibody of the invention has modifications at F243 and/or F244, in particular ones that mean that the antibody comprises an aglycosyl IgG. In one embodiment, an antibody of the present invention may comprise the F243A and/or F244A heavy chain modifications. In another embodiment, one or more of F241, F243, V262 and V264 may be modified and particularly to amino acids that influence glycosylation. In one embodiment, an antibody of the present invention may have modifications at F241A, F243A, V262E and V264E. Such modifications are discussed in Yu et al (2013) 135(26): 9723-9732, which is incorporated by reference in its entirety, particularly in relation to the modifications discussed therein. Such modifications provide a way to modulate, for example, Fc receptor binding. A modification which influences the glycosylation of the antibody may be present. Further, an antibody of the invention may be produced in a cell type that influences glycosylation as a further approach for sugar engineering. In one embodiment, the fucosylation, sialylation, galactosylation, and/or mannosylation of an antibody of the present invention may be altered either by sequence modifications and/or via the type of cell used to produce the antibody.

In one embodiment, an antibody of the present invention has modifications at position 297 and/or 299. For example, in one embodiment, an antibody of the present invention comprises a N297A modification in its heavy chains, preferably N297Q or mutation of Ser or Thr at 299 to other residues. In one embodiment it has both those modifications.

In one embodiment, a binding molecule of the present invention, and in particular an antibody, comprises two different heavy chains where the heavy chains comprise modifications that allow the different heavy chains to preferentially associate compared to heavy chains associating with identical heavy chains. Such an approach may be in particular employed where the antibody has more than one specificity for TNFR2, for instance where the antibody binds two different epitopes of TNFR2. In one embodiment, the two different heavy chains comprise knob-in-hole mutations. In certain aspects, the knob-in hole mutations are a T366W mutation in one heavy chain constant region and a T366S, L368A, and a Y407V mutation in the other domain. In certain aspects, the modifications comprise charge-pair mutations. In certain aspects, the charge-pair mutations are a T366K mutation in one of the heavy chain constant regions and a corresponding L351D mutation in the other domain. In an alternative embodiment, rather than have modifications that result in preferential pairing of different heavy chains, the heavy chains comprise modifications that mean a heterodimer comprising the two heavy chains can be purified preferentially from the homodimers only comprising one type of heavy chain. For example, the modifications may alter affinity for Protein A, with one heavy chain still able to bind Protein A, whilst the modified heavy chain does not do so, meaning that heterodimers of the two different heavy chains can be purified based on their affinity for Protein A.

In other embodiments, heavy and light chains may comprise modifications that change whether or not a disulphide bridge is formed between them. In a variety of embodiments, the modifications comprise mutations that generate engineered disulfide bridges between light and heavy chains. As described herein, "engineered disulfide bridges" are mutations that provide non-endogenous cysteine amino acids in two or more polypeptides such that a non-native disulfide bond forms when the two or more domains associate. Engineered disulfide bridges are described in greater detail in Merchant et al. (Nature Biotech (1998) 16:677-681), the entirety of which is hereby incorporated by reference. In a particular embodiment, the mutations that generate engineered disulfide bridges are a K392C mutation in one of a first or second CH3 domains, and a D399C in the other CH3 domain. In a preferred embodiment, the mutations that generate engineered disulfide bridges are a S354C mutation in one of a first or second CH3 domains, and a Y349C in the other CH3 domain. In another preferred embodiment, the mutations that generate engineered disulfide bridges are a 447C mutation in both the first and second CH3 domains that are provided by extension of the C-terminus of a CH3 domain incorporating a KSC tripeptide sequence.

In one embodiment, binding molecules of the present invention may comprise modifications that alter serum half-life of the binding molecule. Hence, in another embodiment, an antibody of the present invention has Fc region modification(s) that alter the half-life of the antibody. Such modifications may be present as well as those that alter Fc functions. In one preferred embodiment, an antibody of the present invention has modification(s) that alter the serum half-life of the antibody. In one particularly preferred embodiment, an antibody of the present invention has modification(s) that alter serum half-life of the antibody compared to an antibody lacking such modifications. In one embodiment, the modifications result in increased serum half-life. In another embodiment, they result in decreased serum half-life. In another preferred embodiment, an antibody of the present invention comprises one or more modifications that collectively both silence the Fc region and decrease the serum half-life of the antibody compared to an antibody lacking such modifications.

### Binding molecule generation and screening

In one embodiment, the binding molecules, in particular antibodies, of the present invention are mutated to provide improved affinity for a target antigen or antigens and in particular for TNFR2. Such variants can be obtained by a number of affinity maturation protocols including mutating the CDRs (Yang et al., J. Mol. Biol., 254, 392-403, 1995), chain shuffling (Marks et al., Bio/Technology, 10, 779-783, 1992), use of mutator strains of *E. coli* (Low et al J. Mol. Biol., 250, 359-368, 1996), DNA shuffling (Patten et al Curr. Opin. Biotechnol., 8, 724-733, 1997), phage display (Thompson et al., J. Mol. Biol., 256, 77-88, 1996) and sexual PCR (Crameri et al Nature, 391, 288-291, 1998). Vaughan *et al* (*supra*) discusses these methods of affinity maturation. Binding domains for use in the present invention may be generated by any suitable method known in the art, for example CDRs may be taken from non-human antibodies including commercially available antibodies and grafted into human frameworks or alternatively chimeric antibodies can be prepared with non-human variable regions and human constant regions etc. As the antibodies of the present invention may not be in a naturally occurring format, it may be that such screening is used to identify antibody antigen-binding sites with desirable properties for binding TNFR2 and then they are reformatted so that they are in one of the formats set out herein.

The skilled person may generate antibodies for use in the antibodies of the invention using any suitable method known in the art. Antigen polypeptides, for use in generating antibodies for example for use to immunize a host or for use in panning, such as in phage display, may be prepared by processes well known in the art from genetically engineered host cells comprising expression systems or they may be recovered from natural biological sources. In the present application, the term "polypeptides" includes peptides, polypeptides and proteins. These are used interchangeably unless otherwise specified. The antigen polypeptide may in some instances be part of a larger protein such as a fusion protein for example fused to an affinity tag or similar. In one embodiment, the host may be immunised with a cell transfected with TNFR2, for instance expressing TNFR2 on its surface.

Antibodies generated against an antigen polypeptide may be obtained, where immunisation of an animal is necessary, by administering the polypeptides to an animal, preferably a non-human animal, using well-known and routine protocols, see for example Handbook of Experimental Immunology, D. M. Weir (ed.), Vol 4, Blackwell Scientific Publishers, Oxford, England, 1986). Many warm-blooded animals, such as rabbits, mice, rats, sheep, cows, camels or pigs may be immunized. However, mice, rabbits, pigs and rats are generally most suitable. Monoclonal antibodies may be prepared by any method known in the art such as the hybridoma technique (Kohler & Milstein, 1975, Nature, 256:495-497), the trioma technique, the human B-cell hybridoma technique (Kozbor et al 1983, Immunology Today, 4:72) and the EBV-hybridoma technique (Cole et al Monoclonal Antibodies and Cancer Therapy, pp77-96, Alan R Liss, Inc., 1985).

Antibodies may also be generated using single lymphocyte antibody methods by cloning and expressing immunoglobulin variable region cDNAs generated from single lymphocytes selected for the production of specific antibodies by, for example, the methods described by Babcook, J. et al 1996, Proc. Natl. Acad. Sci. USA 93(15):7843-78481; WO92/02551; WO2004/051268 and WO2004/106377. The antibodies for use in the present invention can also be generated using various phage display methods known in the art and include those disclosed by Brinkman et al. (in J. Immunol. Methods, 1995, 182: 41-50), Ames et al. (J. Immunol. Methods, 1995, 184:177-186), Kettleborough et al. (Eur. J. Immunol. 1994, 24:952-958), Persic et al. (Gene, 1997 187 9-18), Burton et al. (Advances in Immunology, 1994, 57:191-280) and WO90/02809; WO91/10737; WO92/01047; WO92/18619; WO93/11236; WO95/15982; WO95/20401; and US 5,698,426; 5,223,409; 5,403,484; 5,580,717; 5,427,908; 5,750,753; 5,821,047; 5,571,698; 5,427,908; 5,516,637; 5,780,225; 5,658,727; 5,733,743; 5,969,108, and WO20011/30305.

In one example an antibody of the present invention is fully human, in particular one or more of the variable domains are fully human. Fully human molecules are those in which the variable regions and the constant regions (where present) of both the heavy and the light chains are all of human origin, or substantially identical to sequences of human origin, not necessarily from the same antibody. Examples of fully human antibodies may include antibodies produced, for example by the phage display methods described above and antibodies produced by mice in which the murine immunoglobulin variable and optionally the constant region genes have been replaced by their human counterparts e.g. as described in general terms in EP0546073, US5,545,806, US5,569,825, US5,625,126, US5,633,425, US5,661,016, US5,770,429, EP 0438474 and EP0463151 which are each incorporated by reference.

In one example, the antigen-binding sites, and in particular the variable regions, of the antibodies according to the invention are humanised. Humanised (which include CDR-grafted antibodies) as employed herein refers to molecules having one or more complementarity determining regions (CDRs) from a non-human species and a framework region from a human immunoglobulin molecule (see, e.g. US 5,585,089; WO91/09967 which are incorporated by reference). It will be appreciated that it may only be necessary to transfer the specificity determining residues of the CDRs rather than the entire CDR (see for example, Kashmiri et al., 2005, Methods, 36, 25-34). In a preferred embodiment though, the whole CDR or CDRs is/are transplanted. Humanised antibodies may optionally further comprise one or more framework residues derived from the non-human species from which the CDRs were derived. As used herein, the term "humanised antibody molecule" refers to an antibody molecule wherein the heavy and/or light chain contains one or more CDRs (including, if desired, one or more modified CDRs) from a donor antibody (e.g. a murine monoclonal antibody) grafted into a heavy and/or light chain variable region framework of an acceptor antibody (e.g. a human antibody). For a review, see Vaughan et al, Nature Biotechnology, 16, 535-539, 1998. In one embodiment, rather than the entire CDR being transferred, only one or more of the specificity determining residues from any one of the CDRs described herein above are transferred to the human antibody framework (see for example, Kashmiri et al., 2005, Methods, 36, 25-34). In one embodiment only the specificity determining residues from one or more of the CDRs described herein above are transferred to the human antibody framework. In another embodiment, only the specificity determining residues from each of the CDRs described herein above are transferred to the human antibody framework.

When the CDRs or specificity determining residues are grafted, any appropriate acceptor variable region framework sequence may be used having regard to the class/type of the donor antibody from which the CDRs are derived, including mouse, primate and human framework regions. Suitably, the humanised antibody according to the present invention has a variable domain comprising human acceptor framework regions as well as one or more of the CDRs provided herein. Examples of human frameworks which can be used in the present invention are KOL, NEWM, REI, EU, TUR, TEI, LAY and POM (Kabat *et al supra).* For example, KOL and NEWM can be used for the heavy chain, REI can be used for the light chain and EU, LAY and POM can be used for both the heavy chain and the light chain. Alternatively, human germline sequences may be used; these are available at:
http://www2.mrc-lmb.cam.ac.uk/vbase/list2.php.

In a humanised antibody molecule of the present invention, the acceptor heavy and light chains do not necessarily need to be derived from the same antibody and may, if desired, comprise composite chains having framework regions derived from different chains. The framework regions need not have exactly the same sequence as those of the acceptor antibody. For instance, unusual residues may be changed to more frequently-occurring residues for that acceptor chain class or type. Alternatively, selected residues in the acceptor framework regions may be changed so that they correspond to the residue found at the same position in the donor antibody (see Reichmann et al 1998, Nature, 332, 323-324). Such changes should be kept to the minimum necessary to recover the affinity of the donor antibody. A protocol for selecting residues in the acceptor framework regions which may need to be changed is set forth in WO 91/09967. Derivatives of frameworks may have 1, 2, 3, 4, 5, 6, 7, 8, 9, 10 or more amino acids replaced with an alternative amino acid, for example with a donor residue. Donor residues are residues from the donor antibody, i.e. the antibody from which the CDRs were originally derived, in particular the residue in a corresponding location from the donor sequence is adopted. Donor residues may be replaced by a suitable residue derived from a human receptor framework (acceptor residues).

The residues in antibody variable domains are conventionally numbered according to a system devised by Kabat *et al.* This system is set forth in Kabat el al., 1987, in Sequences of Proteins of Immunological Interest, US Department of Health and Human Services, NIH, USA (hereafter "Kabat *et al.* (*supra*)"). This numbering system is used in the present specification except where otherwise indicated. The Kabat residue designations do not always correspond directly with the linear numbering of the amino acid residues. The actual linear amino acid sequence may contain fewer or additional amino acids than in the strict Kabat numbering corresponding to a shortening of, or insertion into, a structural component, whether framework or complementarity determining region (CDR), of the basic variable domain structure. The correct Kabat numbering of residues may be determined for a given antibody by alignment of residues of homology in the sequence of the antibody with a "standard" Kabat numbered sequence. The CDRs of the heavy chain variable domain are located at residues 31-35 (CDR-H1), residues 50-65 (CDR-H2) and residues 95-102 (CDR-H3) according to the Kabat numbering system. However, according to Chothia (Chothia, C. and Lesk, A.M. J. Mol. Biol., 196, 901-917 (1987)), the loop equivalent to CDR-H1 extends from residue 26 to residue 32. Thus unless indicated otherwise 'CDR-H1' as employed herein is intended to refer to residues 26 to 35, as described by a combination of the Kabat numbering system and Chothia's topological loop definition. The CDRs of the light chain variable domain are located at residues 24-34 (CDR-L1), residues 50-56 (CDR-L2) and residues 89-97 (CDR-L3) according to the Kabat numbering system.

In one embodiment, the invention extends to an antibody sequence disclosed herein. In another it extends to humanized versions of such antibodies.

The skilled person is able to test variants of CDRs or humanised sequences in any suitable assay such as those described herein to confirm activity is maintained.

In one embodiment, variant binding molecules may be identified by identifying such binding molecules that are able to cross-block specific binding molecules set out herein. Cross-blocking antibodies can be identified using any suitable method in the art, for example by using competition ELISA or BIAcore assays where binding of the cross blocking antibody to antigen (TNFR2) prevents the binding of an antibody of the present invention or *vice versa.* Such cross blocking assays may use cells expressing TNFR2 as a target. In one embodiment, flow cytometry is used to assess binding to cells expressing TNFR2.

Variant binding molecules may be employed where they still retain the desired properties of binding molecules of the present invention. Hence, binding molecules and in particular antibodies with degrees of sequence identity to specific ones set out herein are also provided. The sequence identity may be over the entire length of a polypeptide or it may be in relation to specific portions such as just over the variable regions or just over the CDR sequences. Degrees of identity and similarity can be readily calculated (Computational Molecular Biology, Lesk, A.M., ed., Oxford University Press, New York, 1988; Biocomputing. Informatics and Genome Projects, Smith, D.W., ed., Academic Press, New York, 1993; Computer Analysis of Sequence Data, Part 1, Griffin, A.M., and Griffin, H.G., eds., Humana Press, New Jersey, 1994; Sequence Analysis in Molecular Biology, von Heinje, G., Academic Press, 1987, Sequence Analysis Primer, Gribskov, M. and Devereux, J., eds., M Stockton Press, New York, 1991, the BLAST^{™} software available from NCBI (Altschul, S.F. et al., 1990, J. Mol. Biol. 215:403-410; Gish, W. & States, D.J. 1993, Nature Genet. 3:266-272. Madden, T.L. et al., 1996, Meth. Enzymol. 266:131-141; Altschul, S.F. et al., 1997, Nucleic Acids Res. 25:3389-3402; Zhang, J. & Madden, T.L. 1997, Genome Res. 7:649-656). The present invention also extends to novel polypeptide sequences disclosed herein and sequences at least 80% similar or identical thereto, for example 85% or greater, such 90% or greater, in particular 95%, 96%, 97%, 98% or 99% or greater similarity or identity. In one embodiment a sequence may have at least 99% sequence identity to at least one of the specific sequences provided herein. "Identity", as used herein, indicates that at any particular position in the aligned sequences, the amino acid residue is identical between the sequences. "Similarity", as used herein, indicates that, at any particular position in the aligned sequences, the amino acid residue is of a similar type between the sequences. For example, leucine may be substituted for isoleucine or valine. Other amino acids which can often be substituted for one another include but are not limited to:
- phenylalanine, tyrosine and tryptophan (amino acids having aromatic side chains);
- lysine, arginine and histidine (amino acids having basic side chains);
- aspartate and glutamate (amino acids having acidic side chains);
- asparagine and glutamine (amino acids having amide side chains); and
- cysteine and methionine (amino acids having sulphur-containing side chains).

In one embodiment, a variant may have from one to ten, such as one, two, three, four, five or up to those values of amino acid sequence changes or at least those values, or up to those values, so long as the variant is still able to specifically bind TNFR2 and preferably act as an agonist of it in the formats described previously. In another embodiment, an antibody of the present invention may have at least five, six, seven, eight, nine, ten, eleven or twelve amino acid sequence changes compared to the CDRs of one of the specific antibodies set out herein, for example is may have that number of sequence changes in the six CDRs making up the antigen-binding site. An antibody of the present invention may have that number of sequence changes in the CDRs compared to the specific antibody set out herein. It may have up to that number of sequence changes. It may have at least that number of amino acid sequence changes. Such variant antibody molecules will typically retain the ability to specifically bind TNFR2. They may also retain out of the other functions set out herein.

It will be appreciated that this aspect of the invention also extends to variants of the specific binding molecules, and in particular antibodies, including humanised versions and modified versions, including those in which amino acids have been mutated in the CDRs to remove one or more isomerisation, deamidation, glycosylation site or cysteine residue as described herein above.

### Illustrative antibody and CDR sequences

The Examples of the present application include various specific antibody molecules, including molecules with TNF ligands as part of their structure to help illustrate the invention and its advantages. The invention is not limited to those sequences. In one embodiment a binding molecule, and in particular an antibody, of the present invention may comprise specific sequences from those illustrative antibodies, or variants of them. Variant antibodies are also provided, including variants as defined here, particularly those that specifically bind TNFR2, but not TNFR1, in an FcyR independent manner.

Table 3 of the present invention sets out the various pairs of expression constructs that were used to express a particular antibody. The Table also sets the polypeptides that are expressed by the pairs of vectors. In one embodiment, the present invention provides a binding molecule that comprises one of the pairwise combinations of polypeptides set out by Table 3. The original antibody clones used to generate antibodies in the Examples are mouse antibodies. In one embodiment, the present invention provides a binding molecule comprising one of the pairwise combinations of polypeptides set out in Table 3 where the mouse CDR sequences are retained but the rest of the antibody has been humanised. In another embodiment, a binding molecule of the present invention comprises one of the pairwise combinations of polypeptides set out in the Table except the whole of the mouse sequences have been swapped for those of a human antibody that is able to specifically bind TNFR2. In one embodiment, a binding molecule that cross-blocks the binding molecule that results from the pairwise combinations of polypeptides set out in Table 3 is employed. In another embodiment, a binding molecule that cross-reacts with a binding molecule that results from the pairwise combinations of polypeptides set out in Table 3 is employed. In one embodiment, a binding molecule of the present invention, and in particular an antibody, will comprise an antigen-binding site comprising a set of six CDRs from those of the pairwise combinations of heavy and light chains set out in Table 3 of the present application. In another embodiment, a binding molecule and in particular an antibody of the present invention will comprise a pair of light and heavy chain variable regions pair from those that are set out in Table 3.

Table 4 of the present applications sets out the various polypeptides expressed by the expression vectors used in the Examples of the present application. In one embodiment, a binding molecule of the present invention comprises at least one of those polypeptides. In another embodiment, a binding molecule of the present invention comprises at least one of those polypeptides, where the polypeptide has been humanised, with the mouse CDR sequences retained, but the rest of the mouse sequences replaced by human ones. In a preferred embodiment, the binding molecule may comprise two of those polypeptides, or humanized versions where all but the mouse CDRs have been replaced by the corresponding sequences. In one embodiment, a binding molecule of the present invention, and in particular an antibody, will comprise an antigen-binding site comprising a set of six CDRs from those of the pairwise combinations of heavy and light chains set out in Table 4 of the present application. In another embodiment, a binding molecule and in particular an antibody of the present invention will comprise a pair of light and heavy chain variable regions pair from those that are set out in Table 4.

Table 5 of the present application sets out the CDR sequences from the specific antibodies used as a starting point for the antibody formats set out in the present application. The three CDRs from a light chain are set out grouped together, as are the three CDRs from the heavy chains, with the CDRs from a light and heavy chains also grouped together. In one embodiment, a binding molecule of the present invention will comprise a set of three light chain CDRs as set out in Table 5. In another embodiment, it will comprise a set of three heavy chain CDRs as set out in Table 6, so both the light and heavy chain CDR sequences from one of the clones or functional variants of such sequences. In one embodiment, a binding molecule of the present invention comprises the CDRs for more than one specificity, so the binding molecule may, for instance, comprise two polypeptides that collectively comprise the six CDRs from one of the antibodies as set out in Table 5, but the binding molecule may comprise a further set of six CDRs for another of the antibodies, for example present as a scFv which is part of the binding molecule. In one embodiment, a binding molecule of the present invention is triparatopic for TNFR2 and so has three different specificities for TNFR2. In one embodiment, at least one of the specificities is conferred by a set of six CDRs from Table 5 or variants of those CDRs. In another embodiment, at least two of the specificities are conferred by sets of six CDRs from Table or variants thereof. In a further embodiment, all three of the specificities are. In another embodiment a binding molecule of the present invention may be tetraparatopic, for example with at least one, two, or three of the specifities being conferred by sets of six CDR set out in Table 5 or variants thereof. In one embodiment all four specificities may be conferred by sets of six CDRs from Table 5 or variants thereof.

In one preferred embodiment, a binding molecule of the present invention comprises an antigen binding site comprising a set of six CDRs from the C40 clone. In another preferred embodiment the binding molecule comprises an antigen binding site comprising a set of six CDRs that are from the C19 clone. In a further preferred embodiment an antibody has both an antigen binding site comprising a set of six CDRs from the C40 clone and also a further antigen-binding site comprising a set of six CDRs from the C19 clone. In one preferred embodiment, the CDRs are from the C4 clone. In one embodiment, all of the antigen-binding sites of an antibody of the present invention comprise the same set of six CDRs. In another embodiment, different antigen-binding sites of the antibody comprise a different set of six CDRs. In one embodiment, rather than the six CDRs from C40, C19, or C4 a variant set of six CDRs may be employed, such as any of the variants discussed herein.

In one embodiment any of the antibody formats discussed herein may be provided where the six CDRs of the antigen binding sites are those of C40. In another embodiment, they are those of C19. In another embodiment, they are those of C4. In another embodiment the CDRs are variants of those from C40, C19 or C4 which still retain the ability to bind to TNFR2.

The present invention also provides variants of the above binding molecules and specific binding molecules employed in the Example. For example, in one embodiment a variant may have from one, two, three, four, five, six or more amino acid sequence changes in the CDR sequences of an antigen-binding site compared to the six CDRs set out in Table 5 as a set, but still be able to act as an agonist of TNFR2. In another embodiment, it may have six or more such amino acid changes, for example it may have from six to fifteen such changes, for instance six, seven, eight, nine, ten or more such changes. In one embodiment, an antibody of the present invention may have at least the number of amino acid sequence changes specified. In another embodiment, it may have up to the value recited. In a further embodiment, it may have the recited number of amino acid sequence changes. In one embodiment, the sequence changes may be conservative amino acid sequence changes. In another embodiment, the sequence changes may include non-conservative sequence changes. Various specific modifications are discussed herein and they may be present in such variant molecules. In any of the embodiments set out herein where a specific antibody format is referred to in a further embodiment a binding molecule may be provided, or employed, which is the antibody of that format from the Examples of the present application, but which has been modified to replace all but the mouse CDR sequences with the human CDR sequences.

### Assays

In one embodiment, a functional assay may be employed to determine if a binding molecule of the present invention has a particular property or properties, for instance such as any of those mentioned herein. Hence, functional assays may be used in evaluating a binding molecule, an in particular an antibody, of the present invention. In one embodiment, one or more of the assays described in the Examples of the present application may be employed to assess a particular binding molecule and whether it has a desired property or properties. In a particularly preferred embodiment, one of the assays described in Example 1 may be employed.

A binding molecule of the present invention is able to bind TNFR2. The ability of a binding molecule of the present invention, or a candidate binding molecule, to bind TNFR2 may be assessed in a variety of ways. For example, in one embodiment the ability of the binding molecule to bind TNFR2 is assessed by employing TNFR2 protein, such as by using techniques like surface plasmon resonance using TNFR2, or a portion thereof, bound to a chip. In a particularly preferred embodiment the ability of a binding molecule to bind TNFR2 will be assessed using a cell expressing TNFR2 on its surface. In one embodiment, candidate molecules are labelled and then screened for their ability to bind cells expressing TNFR2, using techniques such as ELISA or flow cytometry. In another embodiment, candidate molecules may be incubated with cells expressing TNFR2 and then bound candidate molecules detected using secondary agents such as a labelled antibody specific for the species of the candidate molecules. In one embodiment, a binding molecule of present invention is labelled, for example using luciferase-tagged (e.g. Gaussia princeps luciferase (GpL)) variants of a binding molecule, an in particular antibody or the fusion proteins, for example as described in Kums et al., MAbs. 2017 Apr; 9(3):506-520). Such tagged binding molecules may also be used in competitive binding assays.

In one embodiment, a binding molecule of the present invention is able to: (a) oligomerise TNFR2 on the surface of a cell expressing TNFR2, preferably to form hexamers of TNFR2 or higher-order clusters of TNFR2 trimers on the cell surface; (b) trigger TNFR2 signalling; and/or (d) stimulate proliferation of leucocytes, preferably T cells, more preferably Treg cells. In one embodiment, a binding molecule of the present invention may bind pre-existing higher order complexes of TNFR2 receptors on the cell surface and in particular trimers of TNFR2 on the cell surface. For instance, in one embodiment a binding molecule of the present invention may bring about the formation of a super-cluster of TNFR2 receptors either on the same cell, or bridging two cells, or both.

In one embodiment, the ability of a candidate molecule to bring about oligomerisation of individual TNFR2 molecules on the surface of a cell expressing TNFR2 is measured. In one embodiment, a binding molecule of the present invention will be able to bring about such oligomerisation. For example, in one embodiment, a binding molecule of the present invention will be able to bring about the formation of hexamers or other oligomers of of TNFR2 trimers. In one embodiment, the formation of such oligomers may be detected using high resolution microscopy. In one embodiment the formation of oligomers of TNFR2 may be detected using a technique such as PALM, dSTQRM, chemical cross-linking, or FRET.

In one preferred embodiment, the ability of a binding molecule of the present invention to activate TNFR2 is assessed by measuring the downstream effects of TNFR2 activation, rather than directly measuring the binding itself. For example, in one embodiment, whether or not a binding molecule induces IL-8 production may be used as a way to establish if a given binding molecule can induce TNFR2 signaling. NEκB activation in response to TNFR2 signaling typically leads to the production of IL-8 and so that may be used as one way to assess whether a given binding molecule is able to activate TNFR2 signaling as in a desired embodiment, a binding molecule of the present invention is able to activate such signaling. In one embodiment a molecule known to activate TNFR2 signalling may be used as a positive control, such as a binding molecule of the present invention. An example of a preferred cell line that may be used to assess the ability of a binding molecule to bind to TNFR2 and stimulate IL-8 production is HT1080-Bcl2-TNFR2. In one embodiment, the functional assay comprises contacting a binding molecule being assessed to cells expressing TNFR2, such as for instance HT1080-Bcl2-TNFR2 cells, incubating the two overnight, then analyzing a sample of the supernatant for the presence of IL-8. In one embodiment, an ELISA is employed for such assessment, such as a BD OptEIA^{™} IL8 ELISA kit. In one embodiment, such a method may also comprise a positive control known to stimulate TNFR2 signalling. In one embodiment, such an assay may be used to screen one or more binding molecules for their ability to bind to and activate IL-8 production, such as to screen a library of potential binding molecules and to identify those activating IL-8 product. In one embodiment, such an assay may be used to check that a variant of a binding molecule of the present invention is still as active, or more active, than a specific binding molecule of the present application.

Another assay which may be used to assess a binding molecule of the present invention is a viability assay as TNFR2 can induce in some cell lines/cell types cell death by inhibition of survival proteins (TRAF2, cIAP1, cIAP2) and concomitant upregulation of endogenously produced TNF triggering TNFR1. In a preferred embodiment Kym-1 cells (Schneider et al., 1999) are used for such an assessment. For example, cells expressing TNFR2, in particular Kym-1 cells, seeded on a plate or in wells may be incubated with a test binding molecule, followed by crystal violet staining, then OD at 595 nm. A positive control may be employed that is known to induce cell killing, such as a "cell death"- such as a control mixture (an example of which is 200 ng/ml TNF, 200 ng/ml TRAIL, 200 ng/ml CD95L, 25 µg/ml CHX, 1 % (w/v) sodium azide). The values seen may be normalized compared to untreated cells and cells treated with the "death mixture". Again, such methods may be used to screen for binding molecules with the desired properties.

In one preferred embodiment, a binding molecule of the present invention will both induce IL-8 production, for example as assessed in the assay discussed above, and also be able to induce cell death, for example as assessed in the assay discussed above.

In one preferred embodiment, a binding molecule of the present invention will be able to bind to TNFR2 and initiate signaling through activation of the alternative NEκB pathway. In a particularly preferred embodiment, a binding molecule of the present invention will be able to activate the alternative NEκB pathway as shown by processing of p100 to p52. In one embodiment, cells expressing TNFR2 are contacted with the binding molecule being assessed, the cells then harvested, and a lysate of the cells assessed to measure p100 and p52 levels. In one embodiment, Western blotting is performed on the cell lysates using antibodies against p100 and p52 to study whether the former is processed to the latter. Any suitable cells may be used, but in a preferred emblodiment Kym-1 cells are used for the assessment. A positive control may be used, such as a binding molecule known to be active. A negative control may be also performed, such as cells that are incubated without a binding molecule and then assessed in the same way. In one preferred embodiment, a binding molecule of the present invention will be able to activate the classical NEκB pathway, for instance as measured by IL-8 production, and also be able to activate the alternative NEκB pathway, such as by assessing whether p100 is processed to p52. In another preferred embodiment, the binding molecule will also be able to activate cell killing, for example as assess using the cell viability assay as described above.

Typically, binding molecules of the present invention do not bind FcyR. In one preferred embodiment, a candidate binding molecule is assessed both for its ability to bind and activate TNFR2, but also for its ability not to bind to and activate FcyR. In one embodiment, the ability of a binding molecule of the present invention to bind Fc receptors and in particular FcyR is assessed. The lack of binding to Fc receptors may be assessed, for instance to determine whether or not CDC or ADCC activity is displayed and preferably neither will be by a binding molecule of the present invention.

In another embodiment, the ability of a binding molecule of the present invention to stimulate activation and/or expansion of cells will be assessed, for example to stimulate particular immune cells in that way, as a binding molecule of the present invention will be typically able to bring about activation and/or expansion of cells such as T cells. In one embodiment, the ability of a binding molecule of the present invention to stimulate Treg cells from PBMC is assessed. In one embodiment, the ability of a binding molecule to expand Tregs is assessed by a method comprising:
- isolating PBMC and then culturing the PBMC (for example at high density, such as at 10 million cells per ml);
- harvesting the cells and then seeding the PBMC (for example at a lower density of 1 million cells per ml);
- incubating the cells with a candidate binding molecule for four days; and
- performing analysis to determine the number of cells.

In a preferred embodiment, a negative control is performed where the cells are cultured without contacting with a candidate binding molecule. The cells may be assessed using flow cytometry in particular staining for CD4+ CD25+ FoxP3+ cells. In a preferred embodiment the number of FoxP3+ CD25+ cells within the CD3+ CD4+ cell population is measured. The cells may also be stained with antibodies specific for CD3 and/or CD8. In one preferred embodiment, a binding molecule of the present invention will give higher numbers of CD4+ CD25+ FoxP3+ cells compared to incubation without a binding molecule. In another embodiment, a candidate binding molecule may also be compared to a specific binding molecule of the present invention, for example to assess whether a variant binding molecule is also able to expand Tregs to the same or greater degree than the specific binding molecule of the present invention.

In another preferred embodiment, FoxP3-Luci mice are employed to study Treg cell expansion as the mice express luciferase under the control of the mouse FoxP3 promoter, which acts as a marker for Treg cells. For example, such mice may be injected with a candidate TNFR2 agonist then bioluminescence imaging is used to image Treg cells. A positive control with a known TNFR2 agonist may be performed, as may be a negative control. In one embodiment a variant or candidate binding molecule will be compared to a known TNFR2 agonist set out herein and if it results in an equivalent or greater level of Tregs as assessed by the bioluminescence imaging in a preferred embodiment it itself is also classified as a binding molecule of the present invention. Such assessment may also be combined with *ex vivo* assessment, for example by subsequently sacrificing the animal, isolating cells, and then looking at Treg numbers.

In another preferred embodiment, for testing of human-specific molecules, transgenic mice expressing human TNFR2, or in particular the extracellular domain of human TNFR2, for example obtained after random insertion of a human TNFR2 cDNA into the mouse genome or more specifically in the mouse TNFR2 gene locus, are employed to study Treg levels and in particular expansion. Such transgenic mice can be crossbred with FoxP3-Luci transgenic mice for in vivo imaging of Treg expansion. Upon sacrifice, separate tissues can also be processed via imaging for changed levels of Tregs, versus negative control animals. Treg expansion and Treg/Teff ratios can also be quantitated using flow cytometry, sourcing splenocytes, leukocytes in blood or other tissues. Alternatively, immunodeficient mice such as NSG mice can be injected with human PBMCs or human Tregs and the expansion of Tregs determined via flow cytometry.

The efficacy of a particular binding molecule may be assessed in an *in vivo* system such as in animal models. For example, various models of graft versus host disease (GvHD) may be employed, with a binding molecule given to such an animal model and then compared to a control animal which is the same animal model for GvHD but which has not been given the binding molecule. In one embodiment, a binding molecule of the present invention will present or reduce the GvHD in the animal model. Other animal models may be used in the same way, for example models of conditions such as inflammatory bowel disease (IBD), lupus, multiple sclerosis, type 1 diabetes, and atherosclerosis. In vivo studies may also be used to see whether a given binding molecule is able to expand Tregs *in vivo.*

### Conjugates, fusion proteins, and effector molecules

A binding molecule, an in particular an antibody, of the invention may be conjugated to an effector molecule. Hence, if desired a binding molecule, and in particular an antibody, for use in the present invention may be conjugated to one or more effector molecule(s). It will be appreciated that the effector molecule may comprise a single effector molecule or two or more such molecules so linked as to form a single moiety that can be attached to the binding molecules, an in particular antibodies, of the present invention. Where it is desired to obtain a binding molecule, and in particular an antibody, according to the present invention linked to an effector molecule, this may be prepared by standard chemical or recombinant DNA procedures in which the binding molecule, and in particular antibody, is linked either directly or via a coupling agent to the effector molecule. Techniques for conjugating such effector molecules to antibodies are well known in the art (see, Hellstrom et al., Controlled Drug Delivery, 2nd Ed., Robinson et al., eds., 1987, pp. 623-53; Thorpe et al., 1982, Immunol. Rev., 62:119-58 and Dubowchik et al., 1999, Pharmacology and Therapeutics, 83, 67-123). Particular chemical procedures include, for example, those described in WO 93/06231, WO 92/22583, WO 89/00195, WO 89/01476 and WO 03/031581. Alternatively, where the effector molecule is a protein or polypeptide the linkage may be achieved using recombinant DNA procedures, for example as described in WO 86/01533 and EP0392745. In one embodiment the binding molecules, in particular antibodies, of the present invention may comprise an effector molecule. The term effector molecule as used herein includes, for example, drugs, toxins, biologically active proteins, for example enzymes, antibody or antibody fragments, synthetic or naturally occurring polymers, nucleic acids and fragments thereof e.g. DNA, RNA and fragments thereof, radionuclides, particularly radioiodide, radioisotopes, chelated metals, nanoparticles and reporter groups such as fluorescent compounds or compounds which may be detected by NMR or ESR spectroscopy.

Examples of effector molecules may include cytotoxins or cytotoxic agents including any agent that is detrimental to (*e.g.* kills) cells. Examples include combrestatins, dolastatins, epothilones, staurosporin, maytansinoids, spongistatins, rhizoxin, halichondrins, roridins, hemiasterlins, taxol, cytochalasin B, gramicidin D, ethidium bromide, emetine, mitomycin, etoposide, tenoposide, vincristine, vinblastine, colchicin, doxorubicin, daunorubicin, dihydroxy anthracin dione, mitoxantrone, mithramycin, actinomycin D, 1-dehydrotestosterone, glucocorticoids, procaine, tetracaine, lidocaine, propranolol, and puromycin and analogs or homologs thereof. Effector molecules also include, but are not limited to, antimetabolites (e.g. methotrexate, 6-mercaptopurine, 6-thioguanine, cytarabine, 5-fluorouracil decarbazine), alkylating agents (e.g. mechlorethamine, thioepa chlorambucil, melphalan, carmustine (BSNU) and lomustine (CCNU), cyclothosphamide, busulfan, dibromomannitol, streptozotocin, mitomycin C, and cis-dichlorodiamine platinum (II) (DDP) cisplatin), anthracyclines (*e.g.* daunorubicin (formerly daunomycin) and doxorubicin), antibiotics (*e.g.* dactinomycin (formerly actinomycin), bleomycin, mithramycin, anthramycin (AMC), calicheamicins or duocarmycins), and anti-mitotic agents (e.g. vincristine and vinblastine).

Other effector molecules may include chelated radionuclides such as ¹¹¹In and ⁹⁰Y, Lu¹⁷⁷ Bismuth²¹³, Californium²⁵² Iridium¹⁹² and Tungsten¹⁸⁸/Rhenium¹⁸⁸; or drugs such as but not limited to, alkylphosphocholines, topoisomerase I inhibitors, taxoids and suramin. Other effector molecules include proteins, peptides and enzymes. Enzymes of interest include, but are not limited to, proteolytic enzymes, hydrolases, lyases, isomerases, transferases. Proteins, polypeptides and peptides of interest include, but are not limited to, immunoglobulins, toxins such as abrin, ricin A, pseudomonas exotoxin, or diphtheria toxin, a protein such as insulin, tumour necrosis factor, α-interferon, β-interferon, nerve growth factor, platelet derived growth factor or tissue plasminogen activator, a thrombotic agent or an anti-angiogenic agent, e.g. angiostatin or endostatin, or, a biological response modifier such as a lymphokine, interleukin-1 (IL-1), interleukin-2 (IL-2), granulocyte macrophage colony stimulating factor (GM-CSF), granulocyte colony stimulating factor (G-CSF), nerve growth factor (NGF) or other growth factor and immunoglobulins.

Other effector molecules may include detectable substances useful for example in diagnosis. Examples of detectable substances include various enzymes, prosthetic groups, fluorescent materials, luminescent materials, bioluminescent materials, radioactive nuclides, positron emitting metals (for use in positron emission tomography), and nonradioactive paramagnetic metal ions. See generally U.S. Patent No. 4,741,900 for metal ions which can be conjugated to antibodies for use as diagnostics. Suitable enzymes include horseradish peroxidase, alkaline phosphatase, beta-galactosidase, or acetylcholinesterase; suitable prosthetic groups include streptavidin, avidin and biotin; suitable fluorescent materials include umbelliferone, fluorescein, fluorescein isothiocyanate, rhodamine, dichlorotriazinylamine fluorescein, dansyl chloride and phycoerythrin; suitable luminescent materials include luminol; suitable bioluminescent materials include luciferase, luciferin, and aequorin; and suitable radioactive nuclides include ¹²⁵I, ¹³¹I, ¹¹¹In and ⁹⁹Tc.

In another embodiment, the effector molecule may increase or decrease the half-life of the binding molecule, in particular antibody, *in vivo,* and/or reduce immunogenicity and/or enhance delivery across an epithelial barrier to the immune system. Examples of suitable effector molecules of this type include polymers, albumin, albumin binding proteins or albumin binding compounds such as those described in WO 05/117984. Where the effector molecule is a polymer it may, in general, be a synthetic or a naturally occurring polymer, for example an optionally substituted straight or branched chain polyalkylene, polyalkenylene or polyoxyalkylene polymer or a branched or unbranched polysaccharide, e.g. a homo- or hetero- polysaccharide. Specific optional substituents which may be present on the above-mentioned synthetic polymers include one or more hydroxy, methyl or methoxy groups. Specific examples of synthetic polymers include optionally substituted straight or branched chain poly(ethyleneglycol), poly(propyleneglycol) poly(vinylalcohol) or derivatives thereof, especially optionally substituted poly(ethyleneglycol) such as methoxypoly(ethyleneglycol) or derivatives thereof.

A binding molecule, in particular an antibody, of the present invention may be conjugated to a molecule that modulates or alters serum half-life. A binding molecule, in particular an antibody, of the invention may bind to albumin, for example in order to modulate the serum half-life. In another embodiment, a binding molecule, in particular an antibody, of the invention may include a peptide linker which is an albumin binding peptide. Examples of albumin binding peptides are included in WO 2015/197772 and WO 2007/106120 the entirety of which are incorporated by reference.

In another embodiment, a binding molecule, in particular an antibody, of the invention is not conjugated to an effector molecule. In one embodiment, a binding molecule, in particular an antibody, of the invention is not conjugated to a toxin. In another embodiment, a binding molecule, in particular an antibody, of the invention is not conjugated to a radioisotope. In another embodiment, a binding molecule, in particular an antibody, of the invention is not conjugated to an agent for imaging.

As well as using conjugation to join an antibody molecule of the present invention it may be used to join different parts of the overall antibody or, for instance, to join ligand based TNFR2 binding sites to the antibody portion of the present molecule.

In one embodiment, a binding molecule of the present invention may be a fusion protein. A "fusion protein" as referred to herein is not limited to particular types of fusion proteins as long as the parts of the fusion protein are fused by covalent bonds. For example, the parts of the fusion protein can be fused by expression in one or more single polypeptide chain(s), by one or more disulfide linkages, by chemical conjugation (preferably by chemical conjugation using click chemistry) and/or by any other covalent linkage which is known in the art as a suitable link for proteins. Preferably, the parts of the fusion protein are fused by expression in one or more single polypeptide chain(s) and/or by one or more disulfide linkages. For example, a binding molecule, and in particular an antibody, may comprise a polyptide which comprises a heavy or light chain constant region that is fusion to the polypeptide sequence for an scFv. In another embodiment, the polypeptide may comprise a heavy chain constant region sequence which is fused to a tenascin peptide sequence, in particular with the tenascin peptide sequence at the C-terminus of the polypeptide. In other embodiments, fusion may be via by one or more disulfide linkages, by chemical conjugation (preferably by chemical conjugation using click chemistry) and/or by any other covalent linkage which is known in the art as a suitable link for proteins.

In one embodiment, a binding molecule of the present invention comprises a moiety that influences the serum half-life of the molecule. For example, a binding molecule of the present invention may comprise a Fc tail, serum albumin, and/or a moiety which is a binder of serum albumin, and PEG. In one preferred embodiment a binding molecule of the invention will either comprise, or be conjugated to, or have a binding site for serum albumin. In one embodiment, the binding molecule is conjugated to serum album or a variant thereof to alter the serum half-life of the binding molecule. In another embodiment, the binding molecule is an antibody that comprises an antigen-binding site for serum albumin. In another embodiment, the binding molecule, and in particular an antibody body, of the present invention may comprise a peptide sequence that binds to serum albumen.

### Pharmaceutical compositions

In one embodiment, the present invention provides a pharmaceutical composition comprising: (a) a binding molecule, and in particular antibody, of the present invention; and (b) a pharmaceutically acceptable carrier, diluent, and/or excipient. In one embodiment, the pharmaceutical composition comprises a binding molecule of the present invention which is an antibody. In one embodiment, a pharmaceutical composition of the present invention comprises a binding molecule of the present invention as well as a carrier, a stabilizer, an excipient, a diluent, a solubilizer, a surfactant, an emulsifier, a preservative and/or adjuvant. In one embodiment, a pharmaceutical composition of the present invention is in solid or liquid form. In one embodiment, the pharmaceutical composition may be in the form of a powder, a tablet, a solution or an aerosol. In one embodiment, a pharmaceutical composition of the present invention is provided in a frozen form.

A composition of the present invention will usually be supplied as a sterile, pharmaceutical composition. A pharmaceutical composition of the present invention may additionally comprise a pharmaceutically-acceptable adjuvant. In another embodiment, no such adjuvant is present in a pharmaceutical composition of the present invention. The present invention also provides a process for preparation of a pharmaceutical or medicament composition comprising adding and mixing a binding molecule, in particular antibody, of the present invention together with one or more of a pharmaceutically acceptable excipient, diluent or carrier.

Pharmaceutically acceptable carriers in therapeutic compositions may additionally contain liquids such as water, saline, glycerol and ethanol. Such carriers may be used, for example, so that the pharmaceutical compositions to be formulated as tablets, pills, dragees, capsules, liquids, gels, syrups, slurries and suspensions, for ingestion by the patient. The term "pharmaceutically acceptable excipient" as used herein typically refers to a pharmaceutically acceptable formulation carrier, solution or additive to enhance the desired characteristics of the compositions of the present invention. Excipients are well known in the art and include buffers (e.g., citrate buffer, phosphate buffer, acetate buffer and bicarbonate buffer), amino acids, urea, alcohols, ascorbic acid, phospholipids, proteins (e.g., serum albumin), EDTA, sodium chloride, liposomes, mannitol, sorbitol, and glycerol. Solutions or suspensions can be encapsulated in liposomes or biodegradable microspheres. Suitable carriers may be large, slowly metabolised macromolecules such as proteins, polypeptides, liposomes, polysaccharides, polylactic acids, polyglycolic acids, polymeric amino acids, amino acid copolymers and inactive virus particles. Pharmaceutically acceptable salts can be used, for example mineral acid salts, such as hydrochlorides, hydrobromides, phosphates and sulphates, or salts of organic acids, such as acetates, propionates, malonates and benzoates.

In certain embodiments, the pharmaceutical composition may contain formulation materials for the purpose of modifying, maintaining or preserving certain characteristics of the composition such as the pH, osmolarity, viscosity, clarity, color, isotonicity, odour, sterility, stability, rate of dissolution or release, adsorption or penetration. A thorough discussion of pharmaceutically acceptable carriers is available in Remington's Pharmaceutical Sciences (Mack Publishing Company, N.J. 1991). Additional pharmaceutical compositions include formulations involving the binding molecule of the present invention in sustained or controlled delivery formulations. Techniques for formulating a variety of sustained- or controlled-delivery means are known to those skilled in the art. A binding molecule of the present invention may also be entrapped in microcapsules prepared, for example, by coacervation techniques or by interfacial polymerization, in colloidal drug delivery systems, or in macroemulsions. Such techniques are also disclosed in Remington's Pharmaceutical Sciences.

A subject will be typically administered a therapeutically effective amount of a pharmaceutical composition and hence binding molecule of the present invention. The term "therapeutically effective amount" typically refers to an amount of a therapeutic agent needed to treat, ameliorate or prevent a targeted disease or condition, or to exhibit a detectable therapeutic or preventative effect. The precise therapeutically effective amount for a human subject will depend upon the severity of the disease state, the general health of the subject, the age, weight and gender of the subject, diet, time and frequency of administration, drug combination(s), reaction sensitivities and tolerance/response to therapy. This amount can be determined by routine experimentation and is within the judgement of the clinician. Generally, a therapeutically effective amount will be from 0.01 mg/kg to 50 mg/kg, for example 0.1 mg/kg to 20 mg/kg per day. Alternatively, the dose may be 1 to 500mg per day, such as 10 to 100, 200, 300 or 400mg per day. In one embodiment, the amount in a given dose is at least enough to bring about a particular function.

In one embodiment, a binding molecule of the present invention may be given in combination with another treatment for the condition being treated and in particular with a TNF-alpha blocker. In one embodiment, the second drug that a binding molecule of the present invention is used in conjunction with is an anti-inflammatory. For example, a binding molecule of the present invention may be provided simultaneously, sequentially, or separately with such a further agent. In another embodiment, a binding molecule of the present invention may be provided in the same pharmaceutical composition as a second therapeutic agent. Examples of drugs which may be provided in the same composition or used in conjunction with a binding molecule of the present invention include TNF-alpha blockers, rapamycin, ustekinumab, or agents which are used as standard-of-care in autoimmune or inflammatory diseases. Examples of TNF-alpha blockers which may be used in conjunction with a binding molecule of the present invention include, but are not limited to, infliximab, adalimumab, etanercept, golimumab, and certolizumab. In one embodiment the second drug is an antibody specific for TNF-alpha. In one embodiment, the second drug administered may be a steroid.

In one preferred embodiment, the therapeutic agent of the invention, when in a pharmaceutical preparation, may be present in unit dose forms. Suitable doses may be calculated for patients according to their weight, for example suitable doses may be in the range of 0.01 to 20mg/kg, for example 0.1 to 20mg/kg, for example 1 to 20mg/kg, for example 10 to 20mg/kg or for example 1 to 15mg/kg, for example 10 to 15mg/kg. To effectively treat conditions of use in the present invention in a human, suitable doses may be within the range of 0.01 to 1000 mg, for example 0.1 to 1000 mg, for example 0.1 to 500 mg, for example 500 mg, for example 0.1 to 100 mg, or 0.1 to 80 mg, or 0.1 to 60 mg, or 0.1 to 40 mg, or for example 1 to 100 mg, or 1 to 50 mg, of a dual targeting protein of this invention, which may be administered parenterally, for example subcutaneously, intravenously or intramuscularly. Such a dose may be, if necessary, repeated at appropriate time intervals selected as appropriate by a physician. A binding molecule of the present invention may be, for instance, lyophilized for storage and reconstituted in a suitable carrier prior to use. Lyophilization and reconstitution techniques can be employed.

The binding molecules and pharmaceutical compositions of this invention may be administered by any number of routes including, but not limited to, oral, intravenous, intramuscular, intra-arterial, intramedullary, intrathecal, intraventricular, transdermal, transcutaneous (for example, see WO 98/20734), subcutaneous, intraperitoneal, intranasal, enteral, topical, sublingual, intravaginal or rectal routes. Hyposprays may also be used to administer the pharmaceutical compositions of the invention. Direct delivery of the compositions will generally be accomplished by injection, subcutaneously, intraperitoneally, intravenously or intramuscularly, or delivered to the interstitial space of a tissue. In one preferred embodiment, administration is via intravenous administration, for example, in one preferred embodiment administration is via intravenous injection. In another preferred embodiment, administration is via subcutaneous administration, for example via subcutaneous injection. The compositions can also be administered into a specific tissue of interest. In some embodiments, a binding molecule of the present invention is administered via site-specific or targeted local delivery techniques. Examples of site-specific or targeted local delivery techniques include various implantable depot sources of the bnding molecule or local delivery catheters, such as infusion catheters, indwelling catheters, or needle catheters, synthetic grafts, adventitial wraps, shunts and stents or other implantable devices, site specific carriers, direct injection, or direct application.

Dosage treatment may be a single dose schedule or a multiple dose schedule. Where the product is for injection or infusion, it may take the form of a suspension, solution or emulsion in an oily or aqueous vehicle and it may contain formulatory agents, such as suspending, preservative, stabilising and/or dispersing agents. Alternatively, the binding molecule, in particular the antibody, may be in dry form, for reconstitution before use with an appropriate sterile liquid. In one embodiment, a pharmaceutical composition comprising a binding molecule of the present invention is provided in lyophilised form. If a composition is to be administered by a route using the gastrointestinal tract, the composition will need to contain agents which protect the binding protein from degradation but which release the binding molecule once it has been absorbed from the gastrointestinal tract. In another embodiment, a nebulisable formulation according to the present invention may be provided, for example, as single dose units (e.g., sealed plastic containers or vials) packed in foil envelopes. Each vial contains a unit dose in a volume, e.g., 2 ml, of solvent/solution buffer.

A pharmaceutical composition of the present invention may be provided in a receptacle that provides means for administration to a subject. In one embodiment, a pharmaceutical composition of the present invention may be provided in a prefilled syringe. The present invention therefore provides such a loaded syringe. It also provides an auto-injector loaded with a pharmaceutical composition of the present invention.

In one embodiment the formulation is provided as a formulation for topical administrations including inhalation. Suitable inhalable preparations include inhalable powders, metering aerosols containing propellant gases or inhalable solutions free from propellant gases. Inhalable powders according to the invention containing the active substance may consist solely of the abovementioned active substances or of a mixture of the abovementioned active substances with physiologically acceptable excipient. These inhalable powders may include monosaccharides (e.g. glucose or arabinose), disaccharides (e.g. lactose, saccharose, maltose), oligo- and polysaccharides (e.g. dextranes), polyalcohols (e.g. sorbitol, mannitol, xylitol), salts (e.g. sodium chloride, calcium carbonate) or mixtures of these with one another. Mono- or disaccharides are suitably used, the use of lactose or glucose, particularly but not exclusively in the form of their hydrates.

Particles for deposition in the lung require a particle size less than 10 microns, such as 1-9 microns for example from 1 to 5 µm. The particle size of the active ingredient (such as the antibody or fragment) is of primary importance. The propellant gases which can be used to prepare the inhalable aerosols are known in the art. Suitable propellant gases are selected from among hydrocarbons such as n-propane, n-butane or isobutane and halohydrocarbons such as chlorinated and/or fluorinated derivatives of methane, ethane, propane, butane, cyclopropane or cyclobutane. The above mentioned propellent gases may be used on their own or in mixtures thereof. Particularly suitable propellent gases are halogenated alkane derivatives selected from among TG 11, TG 12, TG 134a and TG227. Of the abovementioned halogenated hydrocarbons, TG134a (1,1,1,2-tetrafluoroethane) and TG227 (1,1,1,2,3,3,3-heptafluoropropane) and mixtures thereof are particularly suitable. The propellent-gas-containing inhalable aerosols may also contain other ingredients such as cosolvents, stabilisers, surface-active agents (surfactants), antioxidants, lubricants and means for adjusting the pH. All these ingredients are known in the art. The propellant-gas-containing inhalable aerosols according to the invention may contain up to 5 % by weight of active substance. Aerosols according to the invention contain, for example, 0.002 to 5 % by weight, 0.01 to 3 % by weight, 0.015 to 2 % by weight, 0.1 to 2 % by weight, 0.5 to 2 % by weight or 0.5 to 1 % by weight of active ingredient.

Alternatively topical administrations to the lung may also be by administration of a liquid solution or suspension formulation, for example employing a device such as a nebulizer, for example, a nebulizer connected to a compressor (e.g., the Pari LC-Jet Plus(R) nebulizer connected to a Pari Master(R) compressor manufactured by Pari Respiratory Equipment, Inc., Richmond, Va.).

Nebulizable formulation according to the present invention may be provided, for example, as single dose units (e.g., sealed plastic containers or vials) packed in foil envelopes. Each vial contains a unit dose in a volume, e.g., 2 mL, of solvent/solution buffer. The present invention also provides a syringe loaded with a composition comprising a binding molecule, in particular an antibody, of the invention. In one embodiment, a pre-filled syringe loaded with a unit dose of a binding molecule, in particular of an antibody of the invention, is provided. In another embodiment, an auto injector loaded with binding molecule, in particular an antibody, of the invention is provided. In a further embodiment, an IV bag loaded with binding molecule, in particular an antibody, of the invention is provided. Also provided, is the binding molecule, in particular antibody, of the invention in lyophilised form in a vial or similar receptacle in lyophilized form.

It is also envisaged that a binding molecule of the present invention may be administered by use of gene therapy. In order to achieve this, DNA sequences encoding the heavy and light chains of the antibody molecule under the control of appropriate DNA components are introduced into a patient such that the antibody chains are expressed from the DNA sequences and assembled in situ.

Once formulated, the compositions of the invention can be administered directly to the subject. The subjects to be treated can be animals. However, in one or more embodiments the compositions are adapted for administration to humans. In a particularly preferred embodiment the subject is human.

In one embodiment, where a binding molecule of the present invention is administered together with another agent, the two may be given simultaneously, sequentially or separately. In one embodiment, the two may be given in the same pharmaceutical composition.

In one embodiment, a pharmaceutical composition of the present invention is administered before, at the same time, and/or a transplant. For example, it may be so administered at the same time as the transplant of cells, tissues, or organs, such as any of those referred to herein. In another embodiment, it may be that the material transplanted into a subject is treated *ex vivo* prior to transplantation. In one embodiment, a binding molecule of the present invention is used to prevent, or reduce, rejection of transplanted material. In one embodiment, it is used to prevent, treat, or ameliorate an immune response against transplanted material. In one particularly preferred embodiment, it is used for that purpose in relation to graft versus host disease.

The present invention also extends to a kit, comprising a binding moleculeof the invention. In one embodiment a kit comprising any of the binding molecules of the invention is provided, optionally with instructions for administration. In yet another embodiment, the kit further comprises one or more reagents for performing one or more functional assays. In another embodiment, a kit containing single-chambered or multichambered pre-filled syringes is provided. The invention also provides a kit for a singledose administration unit.

### Pathological conditions, medical, and diagnostic uses

Also provided is the use of a binding molecule of the present invention for use as a medicament. In another embodiment a binding molecule of the present invention is provided for use in a method of therapy of the human or animal body. Please note that, in the various therapeutic uses set out herein where reference is made to a binding molecule, a pharmaceutical composition comprising a binding molecule may also be employed and *vice versa* unless stated otherwise. A binding molecule of the present invention may also be used in diagnosis, including in both *in vivo* diagnosis and also *in vitro* diagnosis, for example such diagnosis performed on a sample from a subject.

As discussed further below, a binding molecule of the present invention may be employed to treat a condition. As used herein, the terms "treat" or "treatment" refer to both therapeutic treatment and prophylactic or preventative measures, wherein the object is to prevent or slow down (lessen) an undesired physiological change or disorder. Beneficial or desired clinical results include, but are not limited to, alleviation of symptoms, diminishment of extent of disease, stabilized (i.e., not worsening) state of disease, delay or slowing of disease progression, amelioration or palliation of the disease state, and remission (whether partial or total), whether detectable or undetectable. "Treatment" can also mean prolonging survival as compared to expected survival if not receiving treatment. Those in need of treatment include those already with the condition or disorder as well as those prone to have the condition or disorder or those in which the condition or disorder is to be prevented.

In one particularly preferred embodiment, a binding molecule of the present invention may be used to modulate the immune system. For example, a binding molecule of the present invention may be used to stimulate cells of the immune system, for instance activating particular cells of the immune system. In one embodiment the cells may be stimulated to proliferate. In one preferred embodiment, a binding molecule of the present invention is used to so activate cells expressing TNFR2 on their surface. For example, the cells in question may be white blood cells and in particular T cells. In a particularly preferred embodiment, a binding molecule of the present invention is used to activate T reg cells. For example, a binding molecule of the present invention may be used to stimulate T reg cells which in turn suppress, reduce, or prevent an immune response.

The ability of the present invention to modulate the immune system means that it represents a particular good way to target, for example, an autoimmune disorder, or an inflammatory disorder. Hence, the present invention provides for a binding molecule or pharmaceutical composition of the present invention for use in a method of treating or preventing an autoimmune disorder, or an inflammatory disorder. The present invention provides a binding molecule or pharmaceutical composition for use in such a method wherein:
(a) the disorder is graft versus host disease (GvHD), preferably where the binding molecule is for use in a method where it is administered prior to, at the same time, or after a transplant of a cell, tissue, or organ; or
(b) the disorder is one involving dysfunction or unwanted proliferation of leukocytes, preferably of T cells, more preferably of Teff cells; such disorders may present with an imbalance of Tregs compared toTeff cells, for example due to increased numbers or activity of Teff which is not balance the the numbers and/or immunosuppressive properties of Tregs and in one embodiment the present invention promotes Tregs and in particular shifts the balance with effector cell to, or towards, normal.
(c) the disorder is selected from Inflammatory Bowel Disease (such as Ulcerative Colitis, Crohn's Disease, and Celiac Disease), multiple sclerosis, myasthenia gravis, skin autoimmune diseases such as pemphigus vulgaris or bullous pemphigoid, and Type 1 Diabetes, and atherosclerosis.

The present invention may be used in treating graft versus host disease (GVHD). In one embodiment, the present invention is employed to promote Treg activity prior to a cell, tissue or organ transplant. For example, in one embodiment the present invention is use to promote Treg activity before transplantation of cells, in particular prior to transplantation of stem cells, and preferably before the transplantation of hematopoietic stem cells. In another embodiment, rather than stimulate Tregs in the recipient prior to transplantation, the invention is used to expand Tregs in the cells, tissue, or organ that is to be transplanted to the host. In a further embodiment, they are used as part of the treatment for non-malignant hematopietic diseases.

The present invention may be used to reduce, prevent or treat an immune response against a transplant, for example against transplanted cells, tissue or an organ. Hence, the invention may be used to reduce, prevent or treat graft versus host disease (GvHD). In one embodiment, the present invention may be used in that way where what is transplanted are cells such as a cell population. In one embodiment, the transplanted material is, or comprises, haematopoietic stem cells (HSCs). In another embodiment, the transplanted material may be an organ or tisse, such as the transplant of a heart, lung, kidney, cornea, or other organ. In another embodiment, the transplanted material may be a graft, such as a skin graft. In one embodiment, the present invention provides a method comprise administering a binding molecule of the present invention to treat, prevent, or ameliorate an unwanted immune response against transplanted cells, tissues or organs. In one embodiment, the method may actually further comprise performing the transplant. In another embodiment, the binding molecule of the present invention is given to the subject before, during, and/or after the transplant. In a further embodiment, rather than administration of the binding molecule to the subject the method comprises treating the material to be transplanted *ex vivo* with the binding molecule before it is transplanted.

In one embodiment, rather than treat, prevent, or ameliorate the disease itself, the invention is employed to help ensure that the treatment for the disease, namely the transplanted cells, tissue, or organ, is effective by preventing or reducing the severity of GvHD. Hence, the present invention may be employed in a variety of embodiments where a disease is treated by transplanting cells, tissue or organ. In one preferred embodiment, the condition may be one treated via a stem cell transplant, for example a hematopoietic stem cell (HSC) transplant. In some embodiments, the subject has or is otherwise affected by a metabolic storage disorder which is to be treated by a transplant. The subject may suffer or otherwise be affected by a metabolic disorder selected from the group consisting of glycogen storage diseases, mucopolysaccharidoses, Gaucher's Disease, Hurlers Disease, sphingolipidoses, metachromatic leukodystrophy, or any other diseases or disorders which may benefit from the treatments and therapies disclosed herein and including, without limitation, severe combined immunodeficiency, Wiscott-Aldrich syndrome, hyper immunoglobulin M (IgM) syndrome, Chediak-Higashi disease, hereditary lymphohistiocytosis, osteopetrosis, osteogenesis imperfecta, storage diseases, thalassemia major, sickle cell disease, systemic sclerosis, systemic lupus erythematosus, multiple sclerosis, juvenile rheumatoid arthritis and those diseases, or disorders described in "Bone Marrow Transplantation for Non-Malignant Disease," ASH Education Book, 1 :319-338 (2000), the disclosure of which is incorporated herein by reference in its entirety as it pertains to pathologies that may be treated by administration of hematopoietic stem cell transplant therapy. In one embodiment, where the invention concerns transplantation, it may be that the transfer is of allogenic cells, tissues, or organs. In one embodiment, the transferred cells may be cells expressing a chimeric antigen receptor (CAR). In some embodiments, the subject is in need of chimeric antigen receptor T-cell (CART) therapy. For instance, such therapy may form part of a method of the present invention. In another preferred embodiment, the invention provides a method of promoting the engraftment of a cell population, tissue, or organ in a subject by treating, reducing, or preventing an immune response against said population, tissue, or organ.

The ability of a binding molecule of the present invention to modulate the immune system is also makes it a particularly valuable approach for targeting autoimmune disease. Hence, in another embodiment, the subject to be treated has an autoimmune disorder. In one particularly preferred embodiment, the autoimmune disorder is multiple sclerosis. In a further particular preferred embodiment, the subject has ulcerative colitis. In another particularly preferred embodiment, the condition is scleroderma. In one embodiment, the condition to be treated is lupus. Further examples of autoimmune diseases include scleroderma, Crohn's disease, Type 1 diabetes, or another autoimmune pathology described herein. In one embodiment, the autoimmune disease to be treated is selected from Ulcerative Colitis, Crohn's Disease, Celiac Disease, Inflammatory Bowel Disease, multiple sclerosis, lupus, Graves' disease and Type 1 Diabetes. In one embodiment, the subject has Type 1 Diabetes and that is treated. In one embodiment, the condition is atherosclerosis.

In one preferred embodiment, the condition treated is a condition involving unwanted inflammation. In one preferred embodiment, the condition is arthritis. For example, the present invention may be used to treat rheumatoid or osteoarthritis. Nonlimiting types of Examples of diseases which may be treated include rheumatoid arthritis, polyarticular juvenile idiopathic arthritis, psoriatic arthritis, and pediatric arthritis. In another preferred embodiment, the condition to be treated is selected from multiple sclerosis, ankylosing spondylitis, Crohn's disease, and ulcerative colitis.

In one embodiment, the ability of the invention to stimulate Treg cells is employed as a way to treat allergy. In another embodiment, the ability to stimulate Treg cells may be employed as a way to treat asthma.

### Detection and diagnosis

A binding molecule of the present invention may be used to detect TNFR2. For example the present invention provides a method comprising contacting a binding molecule of the present invention with a test sample and detecting any binding of the binding molecule. A binding molecule of the present invention may be labelled or linked to an enzyme which allows the detection of the binding molecule and hence that the binding molecule has bound. In one embodiment, such detections methods may be, for instance, ELISA assays or flow cytometry as a way to detect whether or not cells in a test sample express TNFR2 on their surface. A binding molecule of the present invention may be used in in vitro detection, it may also be used in detection of TNFR2 that is *in vivo.*

In one embodiment, the present invention provides an in vivo method for detecting TNFR2 that comprises administering a labelled binding molecule of the present invention and then detecting the location of the antibody in the body of a subject. In another embodiment, a binding molecule of the present invention may be used in the diagnosis of a condition, for example in identifying a reduction of cells expressing TNFR2, in particular a reduction of a particular subset of cells expressing TNFR2. In one preferred embodiment, the present invention provides a method of patient stratification comprising subdividing patients on the basis of the level of TNFR2, for example based on the level of a particular cell type expressing TNFR2.

The present invention also provides a kit for detecting TNFR2 comprising a binding molecule of the present invention and optionally instructions for employing the binding molecule in a method of detecting TNFR2.

In one embodiment, the present invention provides a binding molecule of the present invention as a companion diagnostic, for instance to determine whether or not to administer a drug to a subject based on detection of TNFR2, such as levels of TNFR2, for instance the number of particular cell types expressing TNFR2 or their location.

As discussed herein, the present invention may be used to treat a subject. By "subject" or "individual" or "animal" or "patient" or "mammal," is meant any subject, particularly a mammalian subject, for whom diagnosis, prognosis, or therapy is desired. Mammalian subjects include humans, domestic animals, farm animals, and zoo, sports, or pet animals such as dogs, cats, guinea pigs, rabbits, rats, mice, horses, cattle, cows, and so on. In an especially preferred embodiment, the subject is human.

The binding molecules of the present invention may also be used to stimulate T cell proliferation and in particular T reg cell proliferation. In one embodiment, such a method is performed *in vitro* or *ex vivo,* for example by contacting T reg cells with a binding molecule of the present invention. In another embodiment, the present invention may further comprise then transferring the cells to a subject, for example after the T reg population has been expanded or after contact with the binding molecule so that the proliferation occurs *in vivo* after the cells have been transferred to the subject.

### Further Embodiments

The present invention also provides a method of treating or preventing an autoimmune disorder, or an inflammatory disord of the present invention to a subject suffering from, or at risk of, the disorder.

The present invention also provides such a method wherein the method is for treating or preventing graft versus host disease (GvHD), with the method comprising administering the binding molecule to the subject and transplanting a cell, tissue, or organ to the subject, wherein the binding molecule is administered to the subject before, during, or after the transplant.

The present invention further provides such a method wherein:
(a) the disorder is one involving reduced numbers, dysfunction or unwanted proliferation of leukocytes, preferably of T cells, more preferably of T reg cells; or
(b) the disorder is one that can benefit from increasing numbers and/or function of Tregs
(b) the disorder is selected from Inflammatory Bowel Disease (such as Ulcerative Colitis, Crohn's Disease, and Celiac Disease), atherosclerosis, lupus, multiple sclerosis, Type 1 Diabetes, myasthenia gravis, pemphigus vulgaris, and bullous pemphigoid.

The present inventon also provides the use of a binding molecule of the present invention for manufacturing a medicament for use in a method of treating or preventing an autoimmune disorder, or an inflammatory disorder.

The present invention further provides such a use wherein the medicament is for use in a method of treating:
(a) the disorder is one involving dysfunction or unwanted proliferation of leukocytes, preferably of T cells, more preferably of Treg cells; or
(b) the disorder is selected from Inflammatory Bowel Disease (such as Ulcerative Colitis, Crohn's Disease, or Celiac Disease), lupus, multiple sclerosis, Type 1 Diabetes, atherosclerosis, myasthenia gravis, pemphigus vulgaris, and bullous pemphigoid.

The present invention also provides a diagnostic method comprising administering a binding molecule of the present invention to a subject and detecting binding of the binding molecule to TNFR2, preferably wherein the binding molecule is labelled and the binding of the binding molecule to TNFR2 is detected via the label.

All documents referred to herein are incorporated by reference. Reference herein to the singular, using terms such as "a" and "an" also encompasses the plural unless specifically stated otherwise. Where something is referred to herein as "comprising" in another embodiment what the invention may "consist essentially of' what is set out. In another embodiment, it may "consist of' what is set out. Unless defined otherwise, all technical and scientific terms used herein have the meaning commonly understood by a person skilled in the art to which this invention belongs.

### EXAMPLES

### Example 1 - Materials and Methods

### Introduction

The following provides a summary of the materials and methods that were employed when performing the experiments described in the subsequent experiments.

### Cell lines and cell culture conditions

HEK293T cells, HeLa cells, HeLa-TNFR2 (Weiss ***et*** al., 1997) cells, HT1080-TNFR2 (Gerspach et al., 2006, Cell Death Differ., 13(2): 273-284), and Kym-1 cells were cultured in RPMI 1640 media (Sigma-Aldrich, Irvine, United Kingdom) supplemented with 10 % FCS (GIBCO, EU Approved, South America). Cells were cultured at standard conditions (5 % CO₂, 37 °C).

### Antibody cloning, production, and purification

Heavy and light chain encoding cDNA fragments of in-house generated TNFR2-specific antibodies (Medler *et al.,* 2019) were generated, sequenced and subsequently cloned into the pCR3 expression vector (Invitrogen) using standard PCR and cloning techniques. Variants (mutants, fusion proteins) of antibody chains were generated using synthetic DNA fragments encoding the aa sequences of interest and standard cloning techniques. Expression plasmids for TNFR2-specific antibodies typically contain a FLAG-tag.

Antibodies and antibody variants were produced in HEK293T cells by use of the transfection reagent PEI (polyethylenimine; Polyscience Inc., Warrington, USA) as described for example in Medler and Wajant (2021). To quantify the amount of protein secreted into the supernatant, SDS-PAGE and anti-FLAG western blotting with an in house produced FLAG-tagged standard protein was performed (ANTI-FLAG M2 antibody, Sigma-Aldrich, St. Louis, USA).

The supernatant containing the produced TNFR2-specific antibodies were purified by anti-FLAG agarose affinity chromatography. After binding of the FLAG-tagged antibodies to anti-FLAG M2 affinity gel (Sigma-Aldrich, St. Louis, USA), the antibodies were eluated using an excess of FLAG-peptide (100 µg/ml) Sigma-Aldrich, St. Louis, USA) and fractionally collected. Purification was performed in a TBS-buffered system. To exchange TBS against PBS, proteins were afterwards dialyzed overnight at + 4 °C.

Purity and concentration of purified proteins/antibodies were determined by SDS-PAGE and silverstaining in comparison to Amersham's "Low Molecular Weight Calibration Kit for SDS Electrophoresis" (GE Healthcare UK Limited, Little Chalfont, UK) containing defined amounts of proteins of known molecular weigth. The silver staining kit was purchased from Thermo Scientific (Rockford, USA).

### Antibody formats

Figure 1 shows the antibody formats where the TNFR2 binding sites are unidirectionally orientated N-terminal, giving the nomenclature and domain architecture for each. The abbreviations used in the names given for the various antibody formats in the Figure are CI = clone identifier, e.g. C4, C19 etc.; N297A refers to the corresponding mutation in the heavy chain of human IgG1 reducing FcyR interaction; N-RGY refers to the N297A mutation plus mutations promoting IgG1 hexamerization; and TNC refers to a Tenascin-C trimeization domain peptide present at the C-terminus of the heavy chains act to allow three individual antibodies to be brought together in a trimeric form. The antibody formats and the number of N-terminal antigen bindings each is summarized in the Table below.

**Table 1 - Antibodies from Figure 1 and their valency**

| **Antibody format** | **Valency for TNFR2** |
|---|---|
| CI-Fab | 1 |
| CI-Fab2 | 2 |
| CI-IgG1(N297A) | 2 |
| ScFv:CI-IgG1(297A) | 4 |
| CI-IgG1(N297A)-HC:TNC | 6 |
| ScFv:CI-IgG1(N297A)-HC:TNC | 12 |
| CI-IgG1(N-RGY) | 12 |

Figure 7 shows the antibody formats where the TNFR2 binding sites are organized both N-terminal and C-terminal, with the nomenclature and domain architecture shown for each. The same abbreviations for clone identifier, the presence of a N297A modification, and the presence of tenascin trimerization peptide are used as in Figure 1.

**Table 2 - Antibodies from Figure 1 and their valency**

| **Antibody format** | **Number of N and C terminal antigen binding sites for TNFR2, as well as overall valency** |
|---|---|
| CI-Fab-LC:scFvCI | 2 (1 N terminal and 1 C Terminal) |
| CI-IgG1(N297A)-HC:scFvCI | 4 (2 N terminal and 2 C Terminal) |
| CI-IgG1(N297A)-LC:scFvCI | 4 (2 N terminal and 2 C Terminal) |
| CI-IgG1(N297A)-LC:scFvCI-HC:scFvCI | 6 (2 N terminal and 4 C Terminal) |
| LC:scFvCI-HC:scFvCI-IgG1(N297A)-HC:scFvCI | 6 (4 N terminal and 2 C Terminal) |
| LC:scFvCI-HC:scFvCI-IgG1 (N297A)-LC:scFvCI | 6 (4 N terminal and 2 C Terminal) |
| LC:scFvCI-HC:scFvCI-IgG1(N297A)-LC:scFvCI-HC:scFvCI | 8 (4 N terminal and 4 C Terminal) |
| CI-IgG1(297A)-HC:TNC-scFvCI | 12 (6 N terminal and 6 C Terminal) |

Table 3 below summarizes the binding molecules which are referred to in Figures 1 to 26 and discussed in the subsequent Examples, including the two expressions vectors used to express the polypeptides for each antibody.

**Table 3 - Proteins used in figure 1 to 26 Amino acid sequences of the light and heavy chains encoded by the listed pDualyx expression plasmids**

| Protein name | Expression plasmid(s) | | Polypeptides expressed |
|---|---|---|---|
| C4-IgG1(N297A) | pDualyx-010pre | pDualyx-011pre | SEQ ID NO: 3 & SEQ ID NO: 28 |
| C4-IgG1-LC:GpL | pDualyx-030pre | pDualyx-029pre | SEQ ID NO: 4 & SEQ ID NO: 29 |
| C4-IgG1(N297A)-HC:scFvC4 | pDualyx-010pre | pDualyx-012pre | SEQ ID NO: 3 & SEQ ID NO: 30 |
| C4-IgG1(N297A)-LC:scFvC4 | pDualyx-013pre | pDualyx-011pre | SEQ ID NO: 5 & SEQ ID NO: 28 |
| C4-IgG1(N297A)-LC:scFvC4-HC:scFvC4 | pDualyx-013pre | pDualyx-012pre | SEQ ID NO: 5 & SEQ ID NO: 30 |
| C4-Fab1 | pDualyx-010pre | pDualyx-019pre | SEQ ID NO: 3 & SEQ ID NO: 31 |
| C4-Fab1-LC:scFvC4 | pDualyx-013pre | pDualyx-019pre | SEQ ID NO: 5 & SEQ ID NO: 31 |
| C4-Fab2 | pDualyx-010pre | pDualyx-020pre | SEQ ID NO: 3 & SEQ ID NO: 32 |
| C4-Fab2-LC:scFvC4 | pDualyx-013pre | pDualyx-020pre | SEQ ID NO: 5 & SEQ ID NO: 32 |
| C4-IgG1 (N297A)-HC:TNC | pDualyx-010pre | pDualyx-044 | SEQ ID NO: 3 & SEQ ID NO: 33 |
| C4-IgG1 (N297A)-HC:RGY | pDualyx-010pre | pDualyx-118 | SEQ ID NO: 3 & SEQ ID NO: 34 |
| C4-IgG1 (N297A)-HC:TNC-scFvC4 | pDualyx-010pre | pDualyx-119 | SEQ ID NO: 3 & SEQ ID NO: 35 |
| C4(scFv)-IgG1 (N297A) | pDualyx-022 | pDualyx-021 | SEQ ID NO: 6 & SEQ ID NO: 36 |
| C4(scFv)-IgG1 (N297A)-LC:scFvC4 | pDualyx-023 | pDualyx-021 | SEQ ID NO: 7 & SEQ ID NO: 36 |
| C4(scFv)-IgG1 (N297A)-HC:scFvC4 | pDualyx-022 | pDualyx-024 | SEQ ID NO: 6 & SEQ ID NO: 37 |
| C4(scFv)-IgG1(N297A)-LC:scFvC4-HC:scFvC4 | pDualyx-023 | pDualyx-024 | SEQ ID NO: 7 & SEQ ID NO: 37 |
| C4(scFv)-IgG1(N297A)-HC:TNC | pDualyx-022 | pDualyx-051 | SEQ ID NO: 6 & SEQ ID NO: 38 |
| C4-IgG1(N297A)-HC:scFvC19 | pDualyx-010pre | pDualyx-080 | SEQ ID NO: 3 & SEQ ID NO: 39 |
| C4-IgG1 (N297A)-HC:scFvC27 | pDualyx-010pre | pDualyx-082 | SEQ ID NO: 3 & SEQ ID NO: 40 |
| C4-IgG1(N297A)-HC:scFvC40 | pDualyx-010pre | pDualyx-063 | SEQ ID NO: 3 & SEQ ID NO: 41 |
| C19-mulgG1(D265A) | pDualyx-005 | pDualyx-006 | SEQ ID NO: 8 & SEQ ID NO: 42 |
| C19-IgG1-LC:GpL | pDualyx-032 | pDualyx-031 | SEQ ID NO: 9 & SEQ ID NO: 43 |
| C27-IgG1(N297A) | pDualyx-092 | pDualyx-091 | SEQ ID NO: 10 & SEQ ID NO: 44 |
| C27-IgG1(N297A)-LC:GpL | pDualyx-093 | pDualyx-091 | SEQ ID NO: 11 & SEQ ID NO: 44 |
| C40-IgG1(N297A) | pDualyx-077 | pDualyx-075 | SEQ ID NO: 12 & SEQ ID NO: 45 |
| C40-IgG1 (N297A)-LC:GpL | pDualyx-076 | pDualyx-075 | SEQ ID NO: 13 & SEQ ID NO: 45 |
| C19-gG1 (N297A)-HC:scFvC19 | pDualyx-016pre | pDualyx-017pre | SEQ ID NO: 14 & SEQ ID NO: 46 |
| C27-IgG1(N297A)-HC:scFvC27 | pDualyx-092 | pDualyx-108 | SEQ ID NO: 10 & SEQ ID NO: 47 |
| C40-IgG1(N297A)-HC:scFvC40 | pDualyx-077 | pDualyx-111 | SEQ ID NO: 12 & SEQ ID NO: 48 |
| C1-IgG1-LC:GpL | pDualyx-028 | pDualyx-027 | SEQ ID NO: 15 & SEQ ID NO: 49 |
| C9-IgG1(N297A)-LC:GpL | pDualyx-087 | pDualyx-085 | SEQ ID NO: 16 & SEQ ID NO: 50 |
| C29-IgG1(N297A)-LC:GpL | pDualyx-090 | pDualyx-088 | SEQ ID NO: 17 & SEQ ID NO: 51 |
| C31-2-IgG1(N297A)-LC:GpL | pDualyx-071 | pDualyx-069 | SEQ ID NO: 18 & SEQ ID NO: 52 |
| 68/69-IgG1(N297A)-LC:GpL | pDualyx-040pre | pDualyx-041pre | SEQ ID NO: 19 & SEQ ID NO: 53 |
| C19-IgG1(N297A)-HC:TNC | pDualyx-016 | pDualyx-149 | SEQ ID NO: 14 & SEQ ID NO: 54 |
| C19(scFv)-IgG1(N297A) | pDualyx-151 | pDualyx-150 | SEQ ID NO: 25 & SEQ ID NO: 55 |
| C19(scFv)-IgG1(N297A)-HC:TNC | pDualyx-151 | pDualyx-152 | SEQ ID NO: 25 & SEQ ID NO: 56 |
| C19(scFv)-Fab2-HC:TNC | pDualyx-151 | pDualyx-153 | SEQ ID NO: 25 & SEQ ID NO: 57 |
| C40-IgG1(N297A)-HC:TNC | pDualyx-077 | pDualyx-159 | SEQ ID NO: 12 & SEQ ID NO: 62 |
| C40(scFv)-IgG1(N297A) | pDualyx-161 | pDualyx-160 | SEQ ID NO: 27 & SEQ ID NO: 63 |
| C40(scFv)-IgG1(N297A)-HC:TNC | pDualyx-161 | pDualyx-162 | SEQ ID NO: 27 & SEQ ID NO: 64 |
| C40(scFv)-Fab2-HC:TNC | pDualyx-161 | pDualyx-163 | SEQ ID NO: 27 & SEQ ID NO: 65 |
| C27-IgG1(N297A)-HC:TNC | pDualyx-092 | pDualyx-154 | SEQ ID NO: 10 & SEQ ID NO: 58 |
| C27(scFv)-IgG1(N297A) | pDualyx-156 | pDualyx-155 | SEQ ID NO: 26 & SEQ ID NO: 59 |
| C27(scFv)-IgG1(N297A)-HC:TNC | pDualyx-156 | pDualyx-157 | SEQ ID NO: 26 & SEQ ID NO: 60 |
| C27(scFv)-Fab2-HC:TNC | pDualyx-156 | pDualyx-158 | SEQ ID NO: 26 & SEQ ID NO: 61 |
| C4-IgG1(N297A)-LC:GpL-HC:scFvC4 | pDualyx-030 | pDualyx-012 | SEQ ID NO: 4 & SEQ ID NO: 30 |
| C4-Fab1-LC:scFvC9 | pDualyx-112 | pDualyx-019 | SEQ ID NO: 20 & SEQ ID NO: 31 |
| C4-Fab1-LC:scFvC27 | pDualyx-113 | pDualyx-019 | SEQ ID NO: 21 & SEQ ID NO: 31 |
| C4-Fab1-LC:scFvC29 | pDualyx-114 | pDualyx-019 | SEQ ID NO: 22 & SEQ ID NO: 31 |
| C4-Fab1-HC:scFvC40 | pDualyx-116 | pDualyx-019 | SEQ ID NO: 24 & SEQ ID NO: 31 |
| C4-Fab1-HC:scFv31.1 | pDualyx-115 | pDualyx-019 | SEQ ID NO: 23 & SEQ ID NO: 31 |

Table 4 below gives the amino acid sequences of the light and heavy chain polypeptides encoded by the expression constructs referred to in Table 3 above.

**Table 4 - Amino acid sequences of proteins encoded by the expression plasmids referred to in Table 3**

| **Expression plasmid** | **LC / HC** | **SEQ ID NO:** | **AA sequence (FASTA format)** |
|---|---|---|---|
| pDualyx-010pre | LC | SEQ ID NO: 3 | MNFGFSLIFLVLVLKGVQCEVKLVPRQLDYKDDDDK ELDIVMTQSHKFMSTSVGDRVSITCKASQDVDTAVA WYQQKPGQSPKLLIYWASTRHTGVPDRFTGSGSGTD YTLTISSVQAEDLARYYCQQYYSVPPTFGGGTKLGSEI KRTVAAPSVFIFPPSDEQLKSGTASVVCLLNNFYPREA KVQWKVDNALQSGNSQESVTEQDSKDSTYSLSSTLT LSKADYEKHKVYACEVTHQGLSSPVTKSFNRGEC- |
| pDualyx-030pre | LC | SEQ ID NO: 4 | MNFGFSLIFLVLVLKGVQCEVKLVPRQLDIVMTQSHK FMSTSVGDRVSITCKASQDVDTAVAWYQQKPGQSPK LLIYWASTRHTGVPDRFTGSGSGTDYTLTISSVQAEDL ARYYCQQYYSVPPTFGGGTKLGSEIKRTVAAPSVFIFP PSDEQLKSGTASVVCLLNNFYPREAKVQWKVDNALQ SGNSQESVTEQDSKDSTYSLSSTLTLSKADYEKHKVY ACEVTHQGLSSPVTKSFNRGECLEKPTENNEDFNIVA VASNFATTDLDADRGKLPGKKLPLEVLKEMEANARK AGCTRGCLICLSHIKCTPKMKKFIPGRCHTYEGDKES AQGGIGEAIVDIPEIPGFKDLEPMEQFIAQVDLCVDCT TGCLKGLANVQCSDLLKKWLPQRCATFASKIQGQVD KIKGAGGD- |
| pDualyx-013pre | LC | SEQ ID NO: 5 | MNFGFSLIFLVLVLKGVQCEVKLVPRQLDYKDDDDK ELDIVMTQSHKFMSTSVGDRVSITCKASQDVDTAVA WYQQKPGQSPKLLIYWASTRHTGVPDRFTGSGSGTD YTLTISSVQAEDLARYYCQQYYSVPPTFGGGTKLGSEI KRTVAAPSVFIFPPSDEQLKSGTASVVCLLNNFYPREA KVQWKVDNALQSGNSQESVTEQDSKDSTYSLSSTLT LSKADYEKHKVYACEVTHQGLSSPVTKSFNRGECLE QVQLLQSGPELVKPGASVKLSCKASGYSFTSYDINWV KQRPGQGLEWVGWIYPRDGDTKYNEKFKGKAILTVD TSSNTAYMNLHSLTSEDSAVYFCARLTGPYWYFDVW GTGTTVTVSSRSSTKGPKLEEGEFSEAQLDIVMTQSH KFMSTSVGDRVSITCKASQDVDTAVAWYQQKPGQSP KLLIYWASTRHTGVPDRFTGSGSGTDYTLTISSVQAE DLARYYCQQYYSVPPTFGGGTKLEIK- |
| pDualyx-022 | LC | SEQ ID NO: 6 | MNFGFSLIFLVLVLKGVQCEVKLVPRQLDYKDDDDK EFQVQLLQSGPELVKPGASVKLSCKASGYSFTSYDIN WVKQRPGQGLEWVGWIYPRDGDTKYNEKFKGKAIL TVDTSSNTAYMNLHSLTSEDSAVYFCARLTGPYWYF DVWGTGTTVTVSSRSSTKGPKLEEGEFSEAQLDIVMT QSHKFMSTSVGDRVSITCKASQDVDTAVAWYQQKPG QSPKLLIYWASTRHTGVPDRFTGSGSGTDYTLTISSVQ AEDLARYYCQQYYSVPPTFGGGTKLEIKGSEIKRTVA APSVFIFPPSDEQLKSGTASVVCLLNNFYPREAKVQW KVDNALQSGNSQESVTEQDSKDSTYSLSSTLTLSKAD YEKHKVYACEVTHQGLSSPVTKSFNRGEC- |
| pDualyx-023 | LC | SEQ ID NO: 7 | MNFGFSLIFLVLVLKGVQCEVKLVPRQLDYKDDDDK EFQVQLLQSGPELVKPGASVKLSCKASGYSFTSYDIN |
| | | | WVKQRPGQGLEWVGWIYPRDGDTKYNEKFKGKAIL TVDTSSNTAYMNLHSLTSEDSAVYFCARLTGPYWYF DVWGTGTTVTVSSRSSTKGPKLEEGEFSEAQLDIVMT QSHKFMSTSVGDRVSITCKASQDVDTAVAWYQQKPG QSPKLLIYWASTRHTGVPDRFTGSGSGTDYTLTISSVQ AEDLARYYCQQYYSVPPTFGGGTKLEIKGSEIKRTVA APSVFIFPPSDEQLKSGTASVVCLLNNFYPREAKVQW KVDNALQSGNSQESVTEQDSKDSTYSLSSTLTLSKAD YEKHKVYACEVTHQGLSSPVTKSFNRGECLEQVQLL QSGPELVKPGASVKLSCKASGYSFTSYDINWVKQRPG QGLEWVGWIYPRDGDTKYNEKFKGKAILTVDTSSNT AYMNLHSLTSEDSAVYFCARLTGPYWYFDVWGTGT TVTVSSRSSTKGPKLEEGEFSEAQLDIVMTQSHKFMS TSVGDRVSITCKASQDVDTAVAWYQQKPGQSPKLLI YWASTRHTGVPDRFTGSGSGTDYTLTISSVQAEDLAR YYCQQYYSVPPTFGGGTKLEIK- |
| pDualyx-005 | LC | SEQ ID NO: 8 | MNFGFSLIFLVLVLKGVQCEVKLVPRQLDYKDDDDK ELDIVMAQSQKFMSVSVGDRVSVTCKASQNVGTNVA WYQQRPGQSPKALIYSASYRYSGVPDRFTGSGSGTDF TLTISNVQSEDLAEYFCQQFDSHPLTFGAGTKLELKGS IKRADAAPTVSIFPPSSEQLTSGGASVVCFLNNFYPKDI NVKWKIDGSERQNGVLNSWTDQDSKDSTYSMSSTLT LTKDEYERHNSYTCEATHKTSTSPIVKSFNRNEC- |
| pDualyx-032 | LC | SEQ ID NO: 9 | MNFGFSLIFLVLVLKGVQCEVKLVPRQLDIVMAQSQK FMSVSVGDRVSVTCKASQNVGTNVAWYQQRPGQSP KALIYSASYRYSGVPDRFTGSGSGTDFTLTISNVQSED LAEYFCQQFDSHPLTFGAGTKLELKGSEIKRTVAAPS VFIFPPSDEQLKSGTASVVCLLNNFYPREAKVQWKVD NALQSGNSQESVTEQDSKDSTYSLSSTLTLSKADYEK HKVYACEVTHQGLSSPVTKSFNRGECLEKPTENNEDF NIVAVASNFATTDLDADRGKLPGKKLPLEVLKEMEA NARKAGCTRGCLICLSHIKCTPKMKKFIPGRCHTYEG DKESAQGGIGEAIVDIPEIPGFKDLEPMEQFIAQVDLC VDCTTGCLKGLANVQCSDLLKKWLPQRCATFASKIQ GQVDKIKGAGGD- |
| pDualyx-092 | LC | SEQ ID NO: 10 | MNFGFSLIFLVLVLKGVQCEVKLVPRQLDYKDDDDK ELNIVMTQSPKSLSMSVGERVTLNCKASENVKTYVS WYQQKPEQSPKLLIYGASNRYTGVPDRFTGSGSATDF TLTISSVQAEDLADYHCGQSYSYPFTFGAGTKLERKG SEIKRTVAAPSVFIFPPSDEQLKSGTASVVCLLNNFYP REAKVQWKVDNALQSGNSQESVTEQDSKDSTYSLSS TLTLSKADYEKHKVYACEVTHQGLSSPVTKSFNRGE C- |
| pDualyx-093 | LC | SEQ ID NO: 11 | MNFGFSLIFLVLVLKGVQCEVKLVPRQLDYKDDDDK ELNIVMTQSPKSLSMSVGERVTLNCKASENVKTYVS WYQQKPEQSPKLLIYGASNRYTGVPDRFTGSGSATDF TLTISSVQAEDLADYHCGQSYSYPFTFGAGTKLERKG SEIKRTVAAPSVFIFPPSDEQLKSGTASVVCLLNNFYP REAKVQWKVDNALQSGNSQESVTEQDSKDSTYSLSS TLTLSKADYEKHKVYACEVTHQGLSSPVTKSFNRGE CLEKPTENNEDFNIVAVASNFATTDLDADRGKLPGKK LPLEVLKEMEANARKAGCTRGCLICLSHIKCTPKMKK FIPGRCHTYEGDKESAQGGIGEAIVDIPEIPGFKDLEPM |
| | | | EQFIAQVDLCVDCTTGCLKGLANVQCSDLLKKWLPQ RCATFASKIQGQVDKIKGAGGD- |
| pDualyx-077 | LC | SEQ ID NO: 12 | MNFGFSLIFLVLVLKGVQCEVKLVPRQLDYKDDDDK ELDIQMTQSPSSLSASLGGKVTITCKASQDINKFIAWY QHKPGKGPRLLIHYTSTLQPGIPSRFSGSGSGRDYSFSI SNLEPEDIATYYCLQYDNLYTFGGGTKLEIKGSEIKRT VAAPSVFIFPPSDEQLKSGTASVVCLLNNFYPREAKV QWKVDNALQSGNSQESVTEQDSKDSTYSLSSTLTLSK ADYEKHKVYACEVTHQGLSSPVTKSFNRGEC- |
| pDualyx-076 | LC | SEQ ID NO: 13 | MNFGFSLIFLVLVLKGVQCEVKLVPRQLDYKDDDDK ELDIQMTQSPSSLSASLGGKVTITCKASQDINKFIAWY QHKPGKGPRLLIHYTSTLQPGIPSRFSGSGSGRDYSFSI SNLEPEDIATYYCLQYDNLYTFGGGTKLEIKGSEIKRT VAAPSVFIFPPSDEQLKSGTASVVCLLNNFYPREAKV QWKVDNALQSGNSQESVTEQDSKDSTYSLSSTLTLSK ADYEKHKVYACEVTHQGLSSPVTKSFNRGECLEKPT ENNEDFNIVAVASNFATTDLDADRGKLPGKKLPLEVL KEMEANARKAGCTRGCLICLSHIKCTPKMKKFIPGRC HTYEGDKESAQGGIGEAIVDIPEIPGFKDLEPMEQFIA QVDLCVDCTTGCLKGLANVQCSDLLKKWLPQRCATF ASKIQGQVDKIKGAGGD- |
| pDualyx-016pre | LC | SEQ ID NO: 14 | MNFGFSLIFLVLVLKGVQCEVKLVPRQLDYKDDDDK ELDIVMAQSQKFMSVSVGDRVSVTCKASQNVGTNVA WYQQRPGQSPKALIYSASYRYSGVPDRFTGSGSGTDF TLTISNVQSEDLAEYFCQQFDSHPLTFGAGTKLELKGS EIKRTVAAPSVFIFPPSDEQLKSGTASVVCLLNNFYPR EAKVQWKVDNALQSGNSQESVTEQDSKDSTYSLSST LTLSKADYEKHKVYACEVTHQGLSSPVTKSFNRGEC- |
| pDualyx-028 | LC | SEQ ID NO: 15 | MNFGFSLIFLVLVLKGVQCEVKLVPRQLQAVVTQESA LTTSPGETVTLTCRSSTGAVTTSNYANWIQEKPDHLF TGLIGGTNNRTPGVPARFSGSLIGDKAALTVTGAQTE DEAVYFCALWYSNHWVFGGGTKLTVLGRSEIKRTVA APSVFIFPPSDEQLKSGTASVVCLLNNFYPREAKVQW KVDNALQSGNSQESVTEQDSKDSTYSLSSTLTLSKAD YEKHKVYACEVTHQGLSSPVTKSFNRGECLEKPTEN NEDFNIVAVASNFATTDLDADRGKLPGKKLPLEVLKE MEANARKAGCTRGCLICLSHIKCTPKMKKFIPGRCHT YEGDKESAQGGIGEAIVDIPEIPGFKDLEPMEQFIAQV DLCVDCTTGCLKGLANVQCSDLLKKWLPQRCATFAS KIQGQVDKIKGAGGD- |
| pDualyx-087 | LC | SEQ ID NO: 16 | MNFGFSLIFLVLVLKGVQCEVKLVPRQLDYKDDDDK ELQIVLTQSPAIMSASPGEKVTITCSASSSVSYMHWFQ QKPGTSPKLWIYSTSNLASGVPARFSGSGSGTSYSLTI SRMEAEDAATYYCQQRSSYPPTFGGGTKLEIKGSEIK RTVAAPSVFIFPPSDEQLKSGTASVVCLLNNFYPREAK VQWKVDNALQSGNSQESVTEQDSKDSTYSLSSTLTLS KADYEKHKVYACEVTHQGLSSPVTKSFNRGECLEKP TENNEDFNIVAVASNFATTDLDADRGKLPGKKLPLEV LKEMEANARKAGCTRGCLICLSHIKCTPKMKKFIPGR CHTYEGDKESAQGGIGEAIVDIPEIPGFKDLEPMEQFI AQVDLCVDCTTGCLKGLANVQCSDLLKKWLPQRCA TFASKIQGQVDKIKGAGGD- |
| pDualyx-090 | LC | SEQ ID NO: 17 | MNFGFSLIFLVLVLKGVQCEVKLVPRQLDYKDDDDK ELDVVMTQTPLSLPVSLGDQASISCRASQSLVHSNGN TYLQWYLQKPGQSPKLLIYKVSNRFSGVPDRFSGSGS GTDFTLKISRVEAEDLGVFFCSQSTHVPFTFGSGTKLEI KGSEIKRTVAAPSVFIFPPSDEQLKSGTASVVCLLNNF YPREAKVQWKVDNALQSGNSQESVTEQDSKDSTYSL SSTLTLSKADYEKHKVYACEVTHQGLSSPVTKSFNRG ECLEKPTENNEDFNIVAVASNFATTDLDADRGKLPGK KLPLEVLKEMEANARKAGCTRGCLICLSHIKCTPKMK KFIPGRCHTYEGDKESAQGGIGEAIVDIPEIPGFKDLEP MEQFIAQVDLCVDCTTGCLKGLANVQCSDLLKKWLP QRCATFASKIQGQVDKIKGAGGD- |
| pDualyx-071 | LC | SEQ ID NO: 18 | MNFGFSLIFLVLVLKGVQCEVKLVPRQLDYKDDDDK ELQAVVTQESALTTSPGETVTLTCRSSTGAVTTSNYA NWVQEKPDHLFTGLIGGTNNRAPGVPARFSGSVIGDK AALTITGAQTEDEAIYFCALWYSNHWVFGGGTKLTV LGGSEIKRTVAAPSVFIFPPSDEQLKSGTASVVCLLNN FYPREAKVQWKVDNALQSGNSQESVTEQDSKDSTYS LSSTLTLSKADYEKHKVYACEVTHQGLSSPVTKSFNR GECLEKPTENNEDFNIVAVASNFATTDLDADRGKLPG KKLPLEVLKEMEANARKAGCTRGCLICLSHIKCTPKM KKFIPGRCHTYEGDKESAQGGIGEAIVDIPEIPGFKDLE PMEQFIAQVDLCVDCTTGCLKGLANVQCSDLLKKWL PQRCATFASKIQGQVDKIKGAGGD- |
| pDualyx-040pre | LC | SEQ ID NO: 19 | MNFGFSLIFLVLVLKGVQCEVKLVPRQLDYKDDDDK ELDVQMTQSPSSLSASVGDRVTITCKASQDINKFIAW YQQKPGKAPKLLIHYTSTLQPGIPSRFSGSGSGRDYTF TISSLQPEDIATYYCLQYGNLWTFGGGTKVEIKGSEIK RTVAAPSVFIFPPSDEQLKSGTASVVCLLNNFYPREAK VQWKVDNALQSGNSQESVTEQDSKDSTYSLSSTLTLS KADYEKHKVYACEVTHQGLSSPVTKSFNRGECLEKP TENNEDFNIVAVASNFATTDLDADRGKLPGKKLPLEV LKEMEANARKAGCTRGCLICLSHIKCTPKMKKFIPGR CHTYEGDKESAQGGIGEAIVDIPEIPGFKDLEPMEQFI AQVDLCVDCTTGCLKGLANVQCSDLLKKWLPQRCA TFASKIQGQVDKIKGAGGD- |
| pDualyx-112 | LC | SEQ ID NO: 20 | MNFGFSLIFLVLVLKGVQCEVKLVPRQLDYKDDDDK ELDIVMTQSHKFMSTSVGDRVSITCKASQDVDTAVA WYQQKPGQSPKLLIYWASTRHTGVPDRFTGSGSGTD YTLTISSVQAEDLARYYCQQYYSVPPTFGGGTKLGSEI KRTVAAPSVFIFPPSDEQLKSGTASVVCLLNNFYPREA KVQWKVDNALQSGNSQESVTEQDSKDSTYSLSSTLT LSKADYEKHKVYACEVTHQGLSSPVTKSFNRGECLE QIQLVQSGPELKKPGETVKISCKASGYTFTTAGMQWV QKMPGKGFKWIGWINTHSGEPKYAEDFKGRFAFSLE TSASTAYLQISNLKNEDTATYFCARWDGTGYWGQGT TLTVSSRSSTKGPKLEEGEFSEAQLQIVLTQSPAIMSAS PGEKVTITCSASSSVSYMHWFQQKPGTSPKLWIYSTS NLASGVPARFSGSGSGTSYSLTISRMEAEDAATYYCQ QRSSYPPTFGGGTKLEIK- |
| pDualyx-113 | LC | SEQ ID NO: 21 | MNFGFSLIFLVLVLKGVQCEVKLVPRQLDYKDDDDK ELDIVMTQSHKFMSTSVGDRVSITCKASQDVDTAVA WYQQKPGQSPKLLIYWASTRHTGVPDRFTGSGSGTD |
| | | | YTLTISSVQAEDLARYYCQQYYSVPPTFGGGTKLGSEI KRTVAAPSVFIFPPSDEQLKSGTASVVCLLNNFYPREA KVQWKVDNALQSGNSQESVTEQDSKDSTYSLSSTLT LSKADYEKHKVYACEVTHQGLSSPVTKSFNRGECLE EVKLLQSGGGLVQPGGSLKLSCEASGIDFSRYWMSW VRRAPGKGLEWIGESDPDSSTINYAPSLKDKFIISRDS ARNTLYLQMSKVRSEDTALYYCAREGYYDNEGYFD VWGTGTTVTVSSRSSTKGPKLEEGEFSEAQLNIVMTQ SPKSLSMSVGERVTLNCKASENVKTYVSWYQQKPEQ SPKLLIYGASNRYTGVPDRFTGSGSATDFTLTISSVQA EDLADYHCGQSYSYPFTFGAGTKLERK- |
| pDualyx-114 | LC | SEQ ID NO: 22 | MNFGFSLIFLVLVLKGVQCEVKLVPRQLDYKDDDDK ELDIVMTQSHKFMSTSVGDRVSITCKASQDVDTAVA WYQQKPGQSPKLLIYWASTRHTGVPDRFTGSGSGTD YTLTISSVQAEDLARYYCQQYYSVPPTFGGGTKLGSEI KRTVAAPSVFIFPPSDEQLKSGTASVVCLLNNFYPREA KVQWKVDNALQSGNSQESVTEQDSKDSTYSLSSTLT LSKADYEKHKVYACEVTHQGLSSPVTKSFNRGECLE EIQLQQSGTVLARPGASVKMSCKTSGYTFTNYWMH WLKQRPGQGLEWIGTIYPGNSETNYNPNFKGKAKLT AVSSASTAYMELSSLTNEDSAVYYCSRGEDYWGQGT SVTVSSRSSTKGPKLEEGEFSEAQLDVVMTQTPLSLPV SLGDQASISCRASQSLVHSNGNTYLQWYLQKPGQSPK LLIYKVSNRFSGVPDRFSGSGSGTDFTLKISRVEAEDL GVFFCSQSTHVPFTFGSGTKLEIK- |
| pDualyx-115 | LC | SEQ ID NO: 23 | MNFGFSLIFLVLVLKGVQCEVKLVPRQLDYKDDDDK ELDIVMTQSHKFMSTSVGDRVSITCKASQDVDTAVA WYQQKPGQSPKLLIYWASTRHTGVPDRFTGSGSGTD YTLTISSVQAEDLARYYCQQYYSVPPTFGGGTKLGSEI KRTVAAPSVFIFPPSDEQLKSGTASVVCLLNNFYPREA KVQWKVDNALQSGNSQESVTEQDSKDSTYSLSSTLT LSKADYEKHKVYACEVTHQGLSSPVTKSFNRGECLE DVQLQESGPGLVKPSQSLSLTCSVTGSSITSGYYWNW IRQFPGNKLEWMGYITNDGSNNYNPSLKNRISITRDTS ENQFFLKLNSVTTEDTATYYCARDGGWFHYFDYWG QGTTLTVSSRSSTKGPKLEEGEFSEAQLDVVMTQTPL SLPVSLGDQASISCRASQSLVHSNGNTYLQWYLQKPG QSPKLLIYKVSNRFSGVPDRFSGSGSGTDFTLKISRVE AEDLGVFFCSQSTHVPFTFGSGTKLEIK- |
| pDualyx-116 | LC | SEQ ID NO: 24 | MNFGFSLIFLVLVLKGVQCEVKLVPRQLDYKDDDDK ELDIVMTQSHKFMSTSVGDRVSITCKASQDVDTAVA WYQQKPGQSPKLLIYWASTRHTGVPDRFTGSGSGTD YTLTISSVQAEDLARYYCQQYYSVPPTFGGGTKLGSEI KRTVAAPSVFIFPPSDEQLKSGTASVVCLLNNFYPREA KVQWKVDNALQSGNSQESVTEQDSKDSTYSLSSTLT LSKADYEKHKVYACEVTHQGLSSPVTKSFNRGECLE QVTLKESGPGILQPSQTLSLTCSFSGFSLSTFGMGVGW IRQPSGKGLEWLAHIWWDDDKYYNPALKSRLTISKD TSENQVFLKIANVDTADTATYYCVRIAGTRYFDVWG TGTTVTVSSRSSTKGPKLEEGEFSEAQLDIQMTQSPSS LSASLGGKVTITCKASQDINKFIAWYQHKPGKGPRLLI HYTSTLQPGIPSRFSGSGSGRDYSFSISNLEPEDIATYY CLQYDNLYTFGGGTKLEIK- |
| pDualyx-151 | LC | SEQ ID NO: 25 | MNFGFSLIFLVLVLKGVQCEVKLVPRQLDYKDDDDK EFQIQLVQSGPEVKKPGETVKISCKASGYTFTIHGMS WVKQAPGKGLKWMGWINTYSGVPTYANDFKGRFAF SLETSASTAYLQINNLKNEDTATYFCARDEVRRGFGF AYWGQGTLVTVSARSSTKGPKLEEGEFSEAQLDIVM AQSQKFMSVSVGDRVSVTCKASQNVGTNVAWYQQR PGQSPKALIYSASYRYSGVPDRFTGSGSGTDFTLTISN VQSEDLAEYFCQQFDSHPLTFGAGTKLELKGSEIKRT VAAPSVFIFPPSDEQLKSGTASVVCLLNNFYPREAKV QWKVDNALQSGNSQESVTEQDSKDSTYSLSSTLTLSK ADYEKHKVYACEVTHQGLSSPVTKSFNRGEC- |
| pDualyx-156 | LC | SEQ ID NO: 26 | MNFGFSLIFLVLVLKGVQCEVKLVPRQLDYKDDDDK EFEVKLLQSGGGLVQPGGSLKLSCEASGIDFSRYWMS WVRRAPGKGLEWIGESDPDSSTINYAPSLKDKFIISRD SARNTLYLQMSKVRSEDTALYYCAREGYYDNEGYFD VWGTGTTVTVSSRSSTKGPKLEEGEFSEAQLNIVMTQ SPKSLSMSVGERVTLNCKASENVKTYVSWYQQKPEQ SPKLLIYGASNRYTGVPDRFTGSGSATDFTLTISSVQA EDLADYHCGQSYSYPFTFGAGTKLERKGSEIKRTVAA PSVFIFPPSDEQLKSGTASVVCLLNNFYPREAKVQWK VDNALQSGNSQESVTEQDSKDSTYSLSSTLTLSKADY EKHKVYACEVTHQGLSSPVTKSFNRGEC- |
| pDualyx-161 | LC | SEQ ID NO: 27 | MNFGFSLIFLVLVLKGVQCEVKLVPRQLDYKDDDDK EFQVTLKESGPGILQPSQTLSLTCSFSGFSLSTFGMGV GWIRQPSGKGLEWLAHIWWDDDKYYNPALKSRLTIS KDTSENQVFLKIANVDTADTATYYCVRIAGTRYFDV WGTGTTVTVSSRSSTKGPKLEEGEFSEAQLDIQMTQS PSSLSASLGGKVTITCKASQDINKFIAWYQHKPGKGP RLLIHYTSTLQPGIPSRFSGSGSGRDYSFSISNLEPEDIA TYYCLQYDNLYTFGGGTKLEIKGSEIKRTVAAPSVFIF PPSDEQLKSGTASVVCLLNNFYPREAKVQWKVDNAL QSGNSQESVTEQDSKDSTYSLSSTLTLSKADYEKHKV YACEVTHQGLSSPVTKSFNRGEC- |
| pDualyx-011pre | HC | SEQ ID NO: 28 | MNFGFSLIFLVLVLKGVQCEVKLVPRQLQVQLLQSGP ELVKPGASVKLSCKASGYSFTSYDINWVKQRPGQGL EWVGWIYPRDGDTKYNEKFKGKAILTVDTSSNTAYM NLHSLTSEDSAVYFCARLTGPYWYFDVWGTGTTVTV SSGSSSASTKGPSVFPLAPSSKSTSGGTAALGCLVKDY FPEPVTVSWNSGALTSGVHTFPAVLQSSGLYSLSSVV TVPSSSLGTQTYICNVNHKPSNTKVDKKVEPKSCDKT HTCPPCPAPELLGGPSVFLFPPKPKDTLMISRTPEVTC VVVDVSHEDPEVKFNWYVDGVEVHNAKTKPREEQY ASTYRVVSVLTVLHQDWLNGKEYKCKVSNKALPAPI EKTISKAKGQPREPQVYTLPPSRDELTKNQVSLTCLV KGFYPSDIAVEWESNGQPENNYKTTPPVLDSDGSFFL YSKLTVDKSRWQQGNVFSCSVMHEALHNHYTQKSL SLSPGK- |
| pDualyx-029pre | HC | SEQ ID NO: 29 | MNFGFSLIFLVLVLKGVQCEVKLVPRQLQVQLLQSGP ELVKPGASVKLSCKASGYSFTSYDINWVKQRPGQGL EWVGWIYPRDGDTKYNEKFKGKAILTVDTSSNTAYM NLHSLTSEDSAVYFCARLTGPYWYFDVWGTGTTVTV SSGSSSASTKGPSVFPLAPSSKSTSGGTAALGCLVKDY FPEPVTVSWNSGALTSGVHTFPAVLQSSGLYSLSSVV |
| | | | TVPSSSLGTQTYICNVNHKPSNTKVDKKVEPKSCDKT HTCPPCPAPELLGGPSVFLFPPKPKDTLMISRTPEVTC VVVDVSHEDPEVKFNWYVDGVEVHNAKTKPREEQY NSTYRVVSVLTVLHQDWLNGKEYKCKVSNKALPAPI EKTISKAKGQPREPQVYTLPPSRDELTKNQVSLTCLV KGFYPSDIAVEWESNGQPENNYKTTPPVLDSDGSFFL YSKLTVDKSRWQQGNVFSCSVMHEALHNHYTQKSL SLSPGK- |
| pDualyx-012pre | HC | SEQ ID NO: 30 | MNFGFSLIFLVLVLKGVQCEVKLVPRQLQVQLLQSGP ELVKPGASVKLSCKASGYSFTSYDINWVKQRPGQGL EWVGWIYPRDGDTKYNEKFKGKAILTVDTSSNTAYM NLHSLTSEDSAVYFCARLTGPYWYFDVWGTGTTVTV SSGSSSASTKGPSVFPLAPSSKSTSGGTAALGCLVKDY FPEPVTVSWNSGALTSGVHTFPAVLQSSGLYSLSSVV TVPSSSLGTQTYICNVNHKPSNTKVDKKVEPKSCDKT HTCPPCPAPELLGGPSVFLFPPKPKDTLMISRTPEVTC VVVDVSHEDPEVKFNWYVDGVEVHNAKTKPREEQY ASTYRVVSVLTVLHQDWLNGKEYKCKVSNKALPAPI EKTISKAKGQPREPQVYTLPPSRDELTKNQVSLTCLV KGFYPSDIAVEWESNGQPENNYKTTPPVLDSDGSFFL YSKLTVDKSRWQQGNVFSCSVMHEALHNHYTQKSL SLSPGKLEQVQLLQSGPELVKPGASVKLSCKASGYSF TSYDINWVKQRPGQGLEWVGWIYPRDGDTKYNEKF KGKAILTVDTSSNTAYMNLHSLTSEDSAVYFCARLTG PYWYFDVWGTGTTVTVSSRSSTKGPKLEEGEFSEAQL DIVMTQSHKFMSTSVGDRVSITCKASQDVDTAVAWY QQKPGQSPKLLIYWASTRHTGVPDRFTGSGSGTDYTL TISSVQAEDLARYYCQQYYSVPPTFGGGTKLEIK- |
| pDualyx-019pre | HC | SEQ ID NO: 31 | MNFGFSLIFLVLVLKGVQCEVKLVPRQLDYKDDDDK ELQVQLLQSGPELVKPGASVKLSCKASGYSFTSYDIN WVKQRPGQGLEWVGWIYPRDGDTKYNEKFKGKAIL TVDTSSNTAYMNLHSLTSEDSAVYFCARLTGPYWYF DVWGTGTTVTVSSGSSSASTKGPSVFPLAPSSKSTSGG TAALGCLVKDYFPEPVTVSWNSGALTSGVHTFPAVL QSSGLYSLSSVVTVPSSSLGTQTYICNVNHKPSNTKVD KKVEPKSCDKTHT- |
| pDualyx-020pre | HC | SEQ ID NO: 32 | MNFGFSLIFLVLVLKGVQCEVKLVPRQLQVQLLQSGP ELVKPGASVKLSCKASGYSFTSYDINWVKQRPGQGL EWVGWIYPRDGDTKYNEKFKGKAILTVDTSSNTAYM NLHSLTSEDSAVYFCARLTGPYWYFDVWGTGTTVTV SSGSSSASTKGPSVFPLAPSSKSTSGGTAALGCLVKDY FPEPVTVSWNSGALTSGVHTFPAVLQSSGLYSLSSVV TVPSSSLGTQTYICNVNHKPSNTKVDKKVEPKSCDKT HTCPPCPA- |
| pDualyx-044 | HC | SEQ ID NO: 33 | MNFGFSLIFLVLVLKGVQCEVKLVPRQLDYKDDDDK ELQVQLLQSGPELVKPGASVKLSCKASGYSFTSYDIN WVKQRPGQGLEWVGWIYPRDGDTKYNEKFKGKAIL TVDTSSNTAYMNLHSLTSEDSAVYFCARLTGPYWYF DVWGTGTTVTVSSGSSSASTKGPSVFPLAPSSKSTSGG TAALGCLVKDYFPEPVTVSWNSGALTSGVHTFPAVL QSSGLYSLSSVVTVPSSSLGTQTYICNVNHKPSNTKVD KKVEPKSCDKTHTCPPCPAPELLGGPSVFLFPPKPKDT LMISRTPEVTCVVVDVSHEDPEVKFNWYVDGVEVHN |
| | | | AKTKPREEQYASTYRVVSVLTVLHQDWLNGKEYKC KVSNKALPAPIEKTISKAKGQPREPQVYTLPPSRDELT KNQVSLTCLVKGFYPSDIAVEWESNGQPENNYKTTPP VLDSDGSFFLYSKLTVDKSRWQQGNVFSCSVMHEAL HNHYTQKSLSLSPGKEFLEDIACGCAAAPDIKDLLSRL EELEGLVSSLREQGTG- |
| pDualyx-118 | HC | SEQ ID NO: 34 | MNFGFSLIFLVLVLKGVQCEVKLVPRQLDYKDDDDK ELQVQLLQSGPELVKPGASVKLSCKASGYSFTSYDIN WVKQRPGQGLEWVGWIYPRDGDTKYNEKFKGKAIL TVDTSSNTAYMNLHSLTSEDSAVYFCARLTGPYWYF DVWGTGTTVTVSSGSSSASTKGPSVFPLAPSSKSTSGG TAALGCLVKDYFPEPVTVSWNSGALTSGVHTFPAVL QSSGLYSLSSVVTVPSSSLGTQTYICNVNHKPSNTKVD KKVEPKSCDKTHTCPPCPAPELLGGPSVFLFPPKPKDT LMISRTPEVTCVVVDVSHEDPEVKFNWYVDGVEVHN AKTKPREEQYASTYRVVSVLTVLHQDWLNGKEYKC KVSNKALPAPIEKTISKAKGQPRRPQVYTLPPSRDELT KNQVSLTCLVKGFYPSDIAVEWESNGQPENNYKTTPP VLDSDGSFFLYSKLTVDKSRWQQGNVFSCSVMHGAL HNHYTQKYLSLSPGK- |
| pDualyx-119 | HC | SEQ ID NO: 35 | MNFGFSLIFLVLVLKGVQCEVKLVPRQLDYKDDDDK ELQVQLLQSGPELVKPGASVKLSCKASGYSFTSYDIN WVKQRPGQGLEWVGWIYPRDGDTKYNEKFKGKAIL TVDTSSNTAYMNLHSLTSEDSAVYFCARLTGPYWYF DVWGTGTTVTVSSGSSSASTKGPSVFPLAPSSKSTSGG TAALGCLVKDYFPEPVTVSWNSGALTSGVHTFPAVL QSSGLYSLSSVVTVPSSSLGTQTYICNVNHKPSNTKVD KKVEPKSCDKTHTCPPCPAPELLGGPSVFLFPPKPKDT LMISRTPEVTCVVVDVSHEDPEVKFNWYVDGVEVHN AKTKPREEQYASTYRVVSVLTVLHQDWLNGKEYKC KVSNKALPAPIEKTISKAKGQPREPQVYTLPPSRDELT KNQVSLTCLVKGFYPSDIAVEWESNGQPENNYKTTPP VLDSDGSFFLYSKLTVDKSRWQQGNVFSCSVMHEAL HNHYTQKSLSLSPGKEFLEDIACGCAAAPDIKDLLSRL EELEGLVSSLREQGTGGGSVEQVQLLQSGPELVKPGA SVKLSCKASGYSFTSYDINWVKQRPGQGLEWVGWIY PRDGDTKYNEKFKGKAILTVDTSSNTAYMNLHSLTSE DSAVYFCARLTGPYWYFDVWGTGTTVTVSSRSSTKG PKLEEGEFSEAQLDIVMTQSHKFMSTSVGDRVSITCK ASQDVDTAVAWYQQKPGQSPKLLIYWASTRHTGVP DRFTGSGSGTDYTLTISSVQAEDLARYYCQQYYSVPP TFGGGTKLEIK- |
| pDualyx-021 | HC | SEQ ID NO: 36 | MNFGFSLIFLVLVLKGVQCEVKLVPRQLDYKDDDDK EFQVQLLQSGPELVKPGASVKLSCKASGYSFTSYDIN WVKQRPGQGLEWVGWIYPRDGDTKYNEKFKGKAIL TVDTSSNTAYMNLHSLTSEDSAVYFCARLTGPYWYF DVWGTGTTVTVSSRSSTKGPKLEEGEFSEAQLDIVMT QSHKFMSTSVGDRVSITCKASQDVDTAVAWYQQKPG QSPKLLIYWASTRHTGVPDRFTGSGSGTDYTLTISSVQ AEDLARYYCQQYYSVPPTFGGGTKLEIKGSSSASTKG PSVFPLAPSSKSTSGGTAALGCLVKDYFPEPVTVSWN SGALTSGVHTFPAVLQSSGLYSLSSVVTVPSSSLGTQT YICNVNHKPSNTKVDKKVEPKSCDKTHTCPPCPAPEL LGGPSVFLFPPKPKDTLMISRTPEVTCVVVDVSHEDPE |
| | | | VKFNWYVDGVEVHNAKTKPREEQYASTYRVVSVLT VLHQDWLNGKEYKCKVSNKALPAPIEKTISKAKGQP REPQVYTLPPSRDELTKNQVSLTCLVKGFYPSDIAVE WESNGQPENNYKTTPPVLDSDGSFFLYSKLTVDKSR WQQGNVFSCSVMHEALHNHYTQKSLSLSPGK- |
| pDualyx-024 | HC | SEQ ID NO: 37 | MNFGFSLIFLVLVLKGVQCEVKLVPRQLDYKDDDDK EFQVQLLQSGPELVKPGASVKLSCKASGYSFTSYDIN WVKQRPGQGLEWVGWIYPRDGDTKYNEKFKGKAIL TVDTSSNTAYMNLHSLTSEDSAVYFCARLTGPYWYF DVWGTGTTVTVSSRSSTKGPKLEEGEFSEAQLDIVMT QSHKFMSTSVGDRVSITCKASQDVDTAVAWYQQKPG QSPKLLIYWASTRHTGVPDRFTGSGSGTDYTLTISSVQ AEDLARYYCQQYYSVPPTFGGGTKLEIKGSSSASTKG PSVFPLAPSSKSTSGGTAALGCLVKDYFPEPVTVSWN SGALTSGVHTFPAVLQSSGLYSLSSVVTVPSSSLGTQT YICNVNHKPSNTKVDKKVEPKSCDKTHTCPPCPAPEL LGGPSVFLFPPKPKDTLMISRTPEVTCVVVDVSHEDPE VKFNWYVDGVEVHNAKTKPREEQYASTYRVVSVLT VLHQDWLNGKEYKCKVSNKALPAPIEKTISKAKGQP REPQVYTLPPSRDELTKNQVSLTCLVKGFYPSDIAVE WESNGQPENNYKTTPPVLDSDGSFFLYSKLTVDKSR WQQGNVFSCSVMHEALHNHYTQKSLSLSPGKLEQVQ LLQSGPELVKPGASVKLSCKASGYSFTSYDINWVKQR PGQGLEWVGWIYPRDGDTKYNEKFKGKAILTVDTSS NTAYMNLHSLTSEDSAVYFCARLTGPYWYFDVWGT GTTVTVSSRSSTKGPKLEEGEFSEAQLDIVMTQSHKF MSTSVGDRVSITCKASQDVDTAVAWYQQKPGQSPKL LIYWASTRHTGVPDRFTGSGSGTDYTLTISSVQAEDL ARYYCQQYYSVPPTFGGGTKLEIK- |
| pDualyx-051 | HC | SEQ ID NO: 38 | MNFGFSLIFLVLVLKGVQCEVKLVPRQLDYKDDDDK EFQVQLLQSGPELVKPGASVKLSCKASGYSFTSYDIN WVKQRPGQGLEWVGWIYPRDGDTKYNEKFKGKAIL TVDTSSNTAYMNLHSLTSEDSAVYFCARLTGPYWYF DVWGTGTTVTVSSRSSTKGPKLEEGEFSEAQLDIVMT QSHKFMSTSVGDRVSITCKASQDVDTAVAWYQQKPG QSPKLLIYWASTRHTGVPDRFTGSGSGTDYTLTISSVQ AEDLARYYCQQYYSVPPTFGGGTKLEIKGSSSASTKG PSVFPLAPSSKSTSGGTAALGCLVKDYFPEPVTVSWN SGALTSGVHTFPAVLQSSGLYSLSSVVTVPSSSLGTQT YICNVNHKPSNTKVDKKVEPKSCDKTHTCPPCPAPEL LGGPSVFLFPPKPKDTLMISRTPEVTCVVVDVSHEDPE VKFNWYVDGVEVHNAKTKPREEQYASTYRVVSVLT VLHQDWLNGKEYKCKVSNKALPAPIEKTISKAKGQP REPQVYTLPPSRDELTKNQVSLTCLVKGFYPSDIAVE WESNGQPENNYKTTPPVLDSDGSFFLYSKLTVDKSR WQQGNVFSCSVMHEALHNHYTQKSLSLSPGKLEDIA CGCAAAPDIKDLLSRLEELEGLVSSLREQGTG- |
| pDualyx-080 | HC | SEQ ID NO: 39 | MNFGFSLIFLVLVLKGVQCEVKLVPRQLDYKDDDDK ELQVQLLQSGPELVKPGASVKLSCKASGYSFTSYDIN WVKQRPGQGLEWVGWIYPRDGDTKYNEKFKGKAIL TVDTSSNTAYMNLHSLTSEDSAVYFCARLTGPYWYF DVWGTGTTVTVSSGSSSASTKGPSVFPLAPSSKSTSGG TAALGCLVKDYFPEPVTVSWNSGALTSGVHTFPAVL QSSGLYSLSSVVTVPSSSLGTQTYICNVNHKPSNTKVD |
| | | | KKVEPKSCDKTHTCPPCPAPELLGGPSVFLFPPKPKDT LMISRTPEVTCVVVDVSHEDPEVKFNWYVDGVEVHN AKTKPREEQYASTYRVVSVLTVLHQDWLNGKEYKC KVSNKALPAPIEKTISKAKGQPREPQVYTLPPSRDELT KNQVSLTCLVKGFYPSDIAVEWESNGQPENNYKTTPP VLDSDGSFFLYSKLTVDKSRWQQGNVFSCSVMHEAL HNHYTQKSLSLSPGKEFLEQIQLVQSGPEVKKPGETV KISCKASGYTFTIHGMSWVKQAPGKGLKWMGWINT YSGVPTYANDFKGRFAFSLETSASTAYLQINNLKNED TATYFCARDEVRRGFGFAYWGQGTLVTVSARSSTKG PKLEEGEFSEAQLDIVMAQSQKFMSVSVGDRVSVTC KASQNVGTNVAWYQQRPGQSPKALIYSASYRYSGVP DRFTGSGSGTDFTLTISNVQSEDLAEYFCQQFDSHPLT FGAGTKLELK- |
| pDualyx-082 | HC | SEQ ID NO: 40 | MNFGFSLIFLVLVLKGVQCEVKLVPRQLDYKDDDDK ELQVQLLQSGPELVKPGASVKLSCKASGYSFTSYDIN WVKQRPGQGLEWVGWIYPRDGDTKYNEKFKGKAIL TVDTSSNTAYMNLHSLTSEDSAVYFCARLTGPYWYF DVWGTGTTVTVSSGSSSASTKGPSVFPLAPSSKSTSGG TAALGCLVKDYFPEPVTVSWNSGALTSGVHTFPAVL QSSGLYSLSSVVTVPSSSLGTQTYICNVNHKPSNTKVD KKVEPKSCDKTHTCPPCPAPELLGGPSVFLFPPKPKDT LMISRTPEVTCVVVDVSHEDPEVKFNWYVDGVEVHN AKTKPREEQYASTYRVVSVLTVLHQDWLNGKEYKC KVSNKALPAPIEKTISKAKGQPREPQVYTLPPSRDELT KNQVSLTCLVKGFYPSDIAVEWESNGQPENNYKTTPP VLDSDGSFFLYSKLTVDKSRWQQGNVFSCSVMHEAL HNHYTQKSLSLSPGKEFLEEVKLLQSGGGLVQPGGSL KLSCEASGIDFSRYWMSWVRRAPGKGLEWIGESDPD SSTINYAPSLKDKFIISRDSARNTLYLQMSKVRSEDTA LYYCAREGYYDNEGYFDVWGTGTTVTVSSRSSTKGP KLEEGEFSEAQLNIVMTQSPKSLSMSVGERVTLNCKA SENVKTYVSWYQQKPEQSPKLLIYGASNRYTGVPDR FTGSGSATDFTLTISSVQAEDLADYHCGQSYSYPFTFG AGTKLERK- |
| pDualyx-063 | HC | SEQ ID NO: 41 | MNFGFSLIFLVLVLKGVQCEVKLVPRQLDYKDDDDK ELQVQLLQSGPELVKPGASVKLSCKASGYSFTSYDIN WVKQRPGQGLEWVGWIYPRDGDTKYNEKFKGKAIL TVDTSSNTAYMNLHSLTSEDSAVYFCARLTGPYWYF DVWGTGTTVTVSSGSSSASTKGPSVFPLAPSSKSTSGG TAALGCLVKDYFPEPVTVSWNSGALTSGVHTFPAVL QSSGLYSLSSVVTVPSSSLGTQTYICNVNHKPSNTKVD KKVEPKSCDKTHTCPPCPAPELLGGPSVFLFPPKPKDT LMISRTPEVTCVVVDVSHEDPEVKFNWYVDGVEVHN AKTKPREEQYASTYRVVSVLTVLHQDWLNGKEYKC KVSNKALPAPIEKTISKAKGQPREPQVYTLPPSRDELT KNQVSLTCLVKGFYPSDIAVEWESNGQPENNYKTTPP VLDSDGSFFLYSKLTVDKSRWQQGNVFSCSVMHEAL HNHYTQKSLSLSPGKEFLEQVTLKESGPGILQPSQTLS LTCSFSGFSLSTFGMGVGWIRQPSGKGLEWLAHIWW DDDKYYNPALKSRLTISKDTSENQVFLKIANVDTADT ATYYCVRIAGTRYFDVWGTGTTVTVSSRSSTKGPKLE EGEFSEAQLDIQMTQSPSSLSASLGGKVTITCKASQDI NKFIAWYQHKPGKGPRLLIHYTSTLQPGIPSRFSGSGS |
| | | | GRDYSFSISNLEPEDIATYYCLQYDNLYTFGGGTKLEI K- |
| pDualyx-006 | HC | SEQ ID NO: 42 | MNFGFSLIFLVLVLKGVQCEVKLVPRQLDYKDDDDK EFQIQLVQSGPEVKKPGETVKISCKASGYTFTIHGMS WVKQAPGKGLKWMGWINTYSGVPTYANDFKGRFAF SLETSASTAYLQINNLKNEDTATYFCARDEVRRGFGF AYWGQGTLVTVSARSSSAKTTPPSVYPLAPGSAAQT NSMVTLGCLVKGYFPEPVTVTWNSGSLSSGVHTFPA VLQSDLYTLSSSVTVPSSTWPSQTVTCNVAHPASSTK VDKKIVPRDCGCKPCICTVPEVSSVFIFPPKPKDVLTIT LTPKVTCVVVAISKDDPEVQFSWFVDDVEVHTAQTK PREEQINSTFRSVSELPIMHQDWLNGKEFKCRVNSAA FPAPIEKTISKTKGRPKAPQVYTIPPPKEQMAKDKVSL TCMITNFFPEDITVEWQWNGQPAENYKNTQPIMDTD GSYFVYSKLNVQKSNWEAGNTFTCSVLHEGLHNHHT EKSLSHSPGK- |
| pDualyx-031 | HC | SEQ ID NO: 43 | MNFGFSLIFLVLVLKGVQCEVKLVPRQLQIQLVQSGP EVKKPGETVKISCKASGYTFTIHGMSWVKQAPGKGL KWMGWINTYSGVPTYANDFKGRFAFSLETSASTAYL QINNLKNEDTATYFCARDEVRRGFGFAYWGQGTLVT VSAGSSSASTKGPSVFPLAPSSKSTSGGTAALGCLVKD YFPEPVTVSWNSGALTSGVHTFPAVLQSSGLYSLSSV VTVPSSSLGTQTYICNVNHKPSNTKVDKKVEPKSCDK THTCPPCPAPELLGGPSVFLFPPKPKDTLMISRTPEVTC VVVDVSHEDPEVKFNWYVDGVEVHNAKTKPREEQY NSTYRVVSVLTVLHQDWLNGKEYKCKVSNKALPAPI EKTISKAKGQPREPQVYTLPPSRDELTKNQVSLTCLV KGFYPSDIAVEWESNGQPENNYKTTPPVLDSDGSFFL YSKLTVDKSRWQQGNVFSCSVMHEALHNHYTQKSL SLSPGK- |
| pDualyx-091 | HC | SEQ ID NO: 44 | MNFGFSLIFLVLVLKGVQCEVKLVPRQLDYKDDDDK EFEVKLLQSGGGLVQPGGSLKLSCEASGIDFSRYWMS WVRRAPGKGLEWIGESDPDSSTINYAPSLKDKFIISRD SARNTLYLQMSKVRSEDTALYYCAREGYYDNEGYFD VWGTGTTVTVSSRSSSASTKGPSVFPLAPSSKSTSGGT AALGCLVKDYFPEPVTVSWNSGALTSGVHTFPAVLQ SSGLYSLSSVVTVPSSSLGTQTYICNVNHKPSNTKVDK KVEPKSCDKTHTCPPCPAPELLGGPSVFLFPPKPKDTL MISRTPEVTCVVVDVSHEDPEVKFNWYVDGVEVHNA KTKPREEQYASTYRVVSVLTVLHQDWLNGKEYKCK VSNKALPAPIEKTISKAKGQPREPQVYTLPPSRDELTK NQVSLTCLVKGFYPSDIAVEWESNGQPENNYKTTPPV LDSDGSFFLYSKLTVDKSRWQQGNVFSCSVMHEALH NHYTQKSLSLSPGK- |
| pDualyx-075 | HC | SEQ ID NO: 45 | MNFGFSLIFLVLVLKGVQCEVKLVPRQLDYKDDDDK EFQVTLKESGPGILQPSQTLSLTCSFSGFSLSTFGMGV GWIRQPSGKGLEWLAHIWWDDDKYYNPALKSRLTIS KDTSENQVFLKIANVDTADTATYYCVRIAGTRYFDV WGTGTTVTVSSRSSSASTKGPSVFPLAPSSKSTSGGTA ALGCLVKDYFPEPVTVSWNSGALTSGVHTFPAVLQSS GLYSLSSVVTVPSSSLGTQTYICNVNHKPSNTKVDKK VEPKSCDKTHTCPPCPAPELLGGPSVFLFPPKPKDTLM ISRTPEVTCVVVDVSHEDPEVKFNWYVDGVEVHNAK |
| | | | TKPREEQYASTYRVVSVLTVLHQDWLNGKEYKCKVS NKALPAPIEKTISKAKGQPREPQVYTLPPSRDELTKNQ VSLTCLVKGFYPSDIAVEWESNGQPENNYKTTPPVLD SDGSFFLYSKLTVDKSRWQQGNVFSCSVMHEALHNH YTQKSLSLSPGK- |
| pDualyx-017pre | HC | SEQ ID NO: 46 | MNFGFSLIFLVLVLKGVQCEVKLVPRQLQIQLVQSGP EVKKPGETVKISCKASGYTFTIHGMSWVKQAPGKGL KWMGWINTYSGVPTYANDFKGRFAFSLETSASTAYL QINNLKNEDTATYFCARDEVRRGFGFAYWGQGTLVT VSAGSSSASTKGPSVFPLAPSSKSTSGGTAALGCLVKD YFPEPVTVSWNSGALTSGVHTFPAVLQSSGLYSLSSV VTVPSSSLGTQTYICNVNHKPSNTKVDKKVEPKSCDK THTCPPCPAPELLGGPSVFLFPPKPKDTLMISRTPEVTC VVVDVSHEDPEVKFNWYVDGVEVHNAKTKPREEQY ASTYRVVSVLTVLHQDWLNGKEYKCKVSNKALPAPI EKTISKAKGQPREPQVYTLPPSRDELTKNQVSLTCLV KGFYPSDIAVEWESNGQPENNYKTTPPVLDSDGSFFL YSKLTVDKSRWQQGNVFSCSVMHEALHNHYTQKSL SLSPGKEFQIQLVQSGPEVKKPGETVKISCKASGYTFTI HGMSWVKQAPGKGLKWMGWINTYSGVPTYANDFK GRFAFSLETSASTAYLQINNLKNEDTATYFCARDEVR RGFGFAYWGQGTLVTVSARSSTKGPKLEEGEFSEAQL DIVMAQSQKFMSVSVGDRVSVTCKASQNVGTNVAW YQQRPGQSPKALIYSASYRYSGVPDRFTGSGSGTDFT LTISNVQSEDLAEYFCQQFDSHPLTFGAGTKLELK- |
| pDualyx-108 | HC | SEQ ID NO: 47 | MNFGFSLIFLVLVLKGVQCEVKLVPRQLDYKDDDDK EFEVKLLQSGGGLVQPGGSLKLSCEASGIDFSRYWMS WVRRAPGKGLEWIGESDPDSSTINYAPSLKDKFIISRD SARNTLYLQMSKVRSEDTALYYCAREGYYDNEGYFD VWGTGTTVTVSSRSSSASTKGPSVFPLAPSSKSTSGGT AALGCLVKDYFPEPVTVSWNSGALTSGVHTFPAVLQ SSGLYSLSSVVTVPSSSLGTQTYICNVNHKPSNTKVDK KVEPKSCDKTHTCPPCPAPELLGGPSVFLFPPKPKDTL MISRTPEVTCVVVDVSHEDPEVKFNWYVDGVEVHNA KTKPREEQYASTYRVVSVLTVLHQDWLNGKEYKCK VSNKALPAPIEKTISKAKGQPREPQVYTLPPSRDELTK NQVSLTCLVKGFYPSDIAVEWESNGQPENNYKTTPPV LDSDGSFFLYSKLTVDKSRWQQGNVFSCSVMHEALH NHYTQKSLSLSPGKLEEVKLLQSGGGLVQPGGSLKLS CEASGIDFSRYWMSWVRRAPGKGLEWIGESDPDSSTI NYAPSLKDKFIISRDSARNTLYLQMSKVRSEDTALYY CAREGYYDNEGYFDVWGTGTTVTVSSRSSTKGPKLE EGEFSEAQLNIVMTQSPKSLSMSVGERVTLNCKASEN VKTYVSWYQQKPEQSPKLLIYGASNRYTGVPDRFTG SGSATDFTLTISSVQAEDLADYHCGQSYSYPFTFGAG TKLERK- |
| pDualyx-111 | HC | SEQ ID NO: 48 | MNFGFSLIFLVLVLKGVQCEVKLVPRQLDYKDDDDK EFQVTLKESGPGILQPSQTLSLTCSFSGFSLSTFGMGV GWIRQPSGKGLEWLAHIWWDDDKYYNPALKSRLTIS KDTSENQVFLKIANVDTADTATYYCVRIAGTRYFDV WGTGTTVTVSSRSSSASTKGPSVFPLAPSSKSTSGGTA ALGCLVKDYFPEPVTVSWNSGALTSGVHTFPAVLQSS GLYSLSSVVTVPSSSLGTQTYICNVNHKPSNTKVDKK VEPKSCDKTHTCPPCPAPELLGGPSVFLFPPKPKDTLM |
| | | | ISRTPEVTCVVVDVSHEDPEVKFNWYVDGVEVHNAK TKPREEQYASTYRVVSVLTVLHQDWLNGKEYKCKVS NKALPAPIEKTISKAKGQPREPQVYTLPPSRDELTKNQ VSLTCLVKGFYPSDIAVEWESNGQPENNYKTTPPVLD SDGSFFLYSKLTVDKSRWQQGNVFSCSVMHEALHNH YTQKSLSLSPGKLEQVTLKESGPGILQPSQTLSLTCSFS GFSLSTFGMGVGWIRQPSGKGLEWLAHIWWDDDKY YNPALKSRLTISKDTSENQVFLKIANVDTADTATYYC VRIAGTRYFDVWGTGTTVTVSSRSSTKGPKLEEGEFS EAQLDIQMTQSPSSLSASLGGKVTITCKASQDINKFIA WYQHKPGKGPRLLIHYTSTLQPGIPSRFSGSGSGRDYS FSISNLEPEDIATYYCLQYDNLYTFGGGTKLEIK- |
| pDualyx-027 | HC | SEQ ID NO: 49 | MNFGFSLIFLVLVLKGVQCEVKLVPRQLEVQLQQSGA ELVRPGASVKLSCTASGFNIKDDYIHWVKQRPEQGLE WIGWIDPENSDTEYASKFQGKATITADTSSNTAYLQL SSLTSEDTAVYYCTTGLWLRTAIDYWGQGTSITVSSG SSSASTKGPSVFPLAPSSKSTSGGTAALGCLVKDYFPE PVTVSWNSGALTSGVHTFPAVLQSSGLYSLSSVVTVP SSSLGTQTYICNVNHKPSNTKVDKKVEPKSCDKTHTC PPCPAPELLGGPSVFLFPPKPKDTLMISRTPEVTCVVV DVSHEDPEVKFNWYVDGVEVHNAKTKPREEQYNST YRVVSVLTVLHQDWLNGKEYKCKVSNKALPAPIEKT ISKAKGQPREPQVYTLPPSRDELTKNQVSLTCLVKGF YPSDIAVEWESNGQPENNYKTTPPVLDSDGSFFLYSK LTVDKSRWQQGNVFSCSVMHEALHNHYTQKSLSLSP GK- |
| pDualyx-085 | HC | SEQ ID NO: 50 | MNFGFSLIFLVLVLKGVQCEVKLVPRQLDYKDDDDK EFQIQLVQSGPELKKPGETVKISCKASGYTFTTAGMQ WVQKMPGKGFKWIGWINTHSGEPKYAEDFKGRFAFS LETSASTAYLQISNLKNEDTATYFCARWDGTGYWGQ GTTLTVSSRSSSASTKGPSVFPLAPSSKSTSGGTAALG CLVKDYFPEPVTVSWNSGALTSGVHTFPAVLQSSGLY SLSSVVTVPSSSLGTQTYICNVNHKPSNTKVDKKVEP KSCDKTHTCPPCPAPELLGGPSVFLFPPKPKDTLMISR TPEVTCVVVDVSHEDPEVKFNWYVDGVEVHNAKTK PREEQYASTYRVVSVLTVLHQDWLNGKEYKCKVSN KALPAPIEKTISKAKGQPREPQVYTLPPSRDELTKNQV SLTCLVKGFYPSDIAVEWESNGQPENNYKTTPPVLDS DGSFFLYSKLTVDKSRWQQGNVFSCSVMHEALHNHY TQKSLSLSPGK- |
| pDualyx-088 | HC | SEQ ID NO: 51 | MNFGFSLIFLVLVLKGVQCEVKLVPRQLDYKDDDDK EFEIQLQQSGTVLARPGASVKMSCKTSGYTFTNYWM HWLKQRPGQGLEWIGTIYPGNSETNYNPNFKGKAKL TAVSSASTAYMELSSLTNEDSAVYYCSRGEDYWGQG TSVTVSSRSSSASTKGPSVFPLAPSSKSTSGGTAALGC LVKDYFPEPVTVSWNSGALTSGVHTFPAVLQSSGLYS LSSVVTVPSSSLGTQTYICNVNHKPSNTKVDKKVEPK SCDKTHTCPPCPAPELLGGPSVFLFPPKPKDTLMISRTP EVTCVVVDVSHEDPEVKFNWYVDGVEVHNAKTKPR EEQYASTYRVVSVLTVLHQDWLNGKEYKCKVSNKA LPAPIEKTISKAKGQPREPQVYTLPPSRDELTKNQVSL TCLVKGFYPSDIAVEWESNGQPENNYKTTPPVLDSDG SFFLYSKLTVDKSRWQQGNVFSCSVMHEALHNHYTQ |
| | | | KSLSLSPGK- |
| pDualyx-069 | HC | SEQ ID NO: 52 | MNFGFSLIFLVLVLKGVQCEVKLVPRQLDYKDDDDK EFDVQLQESGPGLVKPSQSLSLTCSVTGSSITSGYYWN WIRQFPGNKLEWMGYITNDGSNNYNPSLKNRISITRD TSENQFFLKLNSVTTEDTATYYCARDGGWFHYFDYW GQGTTLTVSSRSSSASTKGPSVFPLAPSSKSTSGGTAA LGCLVKDYFPEPVTVSWNSGALTSGVHTFPAVLQSSG LYSLSSVVTVPSSSLGTQTYICNVNHKPSNTKVDKKV EPKSCDKTHTCPPCPAPELLGGPSVFLFPPKPKDTLMI SRTPEVTCVVVDVSHEDPEVKFNWYVDGVEVHNAK TKPREEQYASTYRVVSVLTVLHQDWLNGKEYKCKVS NKALPAPIEKTISKAKGQPREPQVYTLPPSRDELTKNQ VSLTCLVKGFYPSDIAVEWESNGQPENNYKTTPPVLD SDGSFFLYSKLTVDKSRWQQGNVFSCSVMHEALHNH YTQKSLSLSPGK- |
| pDualyx-041pre | HC | SEQ ID NO: 53 | MNFGFSLIFLVLVLKGVQCEVKLVPRQLDYKDDDDK EFQVTLKESGPALVKPTQTLTLTCTFSGFSLSTSGMGV GWIRQPPGKALEWLAHIWWDDDKFYNPSLKSRLTIS KDTSKNQVVLTMTNMDPVDTATYYCARLTGTRYFD YWGQGTTVTVSSRSSSASTKGPSVFPLAPSSKSTSGGT AALGCLVKDYFPEPVTVSWNSGALTSGVHTFPAVLQ SSGLYSLSSVVTVPSSSLGTQTYICNVNHKPSNTKVDK KVEPKSCDKTHTCPPCPAPELLGGPSVFLFPPKPKDTL MISRTPEVTCVVVDVSHEDPEVKFNWYVDGVEVHNA KTKPREEQYASTYRVVSVLTVLHQDWLNGKEYKCK VSNKALPAPIEKTISKAKGQPREPQVYTLPPSRDELTK NQVSLTCLVKGFYPSDIAVEWESNGQPENNYKTTPPV LDSDGSFFLYSKLTVDKSRWQQGNVFSCSVMHEALH NHYTQKSLSLSPGK- |
| pDualyx-149 | HC | SEQ ID NO: 54 | MNFGFSLIFLVLVLKGVQCEVKLVPRQLDYKDDDDK EFQIQLVQSGPEVKKPGETVKISCKASGYTFTIHGMS WVKQAPGKGLKWMGWINTYSGVPTYANDFKGRFAF SLETSASTAYLQINNLKNEDTATYFCARDEVRRGFGF AYWGQGTLVTVSARSSSASTKGPSVFPLAPSSKSTSG GTAALGCLVKDYFPEPVTVSWNSGALTSGVHTFPAV LQSSGLYSLSSVVTVPSSSLGTQTYICNVNHKPSNTKV DKKVEPKSCDKTHTCPPCPAPELLGGPSVFLFPPKPKD TLMISRTPEVTCVVVDVSHEDPEVKFNWYVDGVEVH NAKTKPREEQYASTYRVVSVLTVLHQDWLNGKEYK CKVSNKALPAPIEKTISKAKGQPREPQVYTLPPSRDEL TKNQVSLTCLVKGFYPSDIAVEWESNGQPENNYKTTP PVLDSDGSFFLYSKLTVDKSRWQQGNVFSCSVMHEA LHNHYTQKSLSLSPGKLEDIACGCAAAPDIKDLLSRLE ELEGLVSSLREQGTG- |
| pDualyx-150 | HC | SEQ ID NO: 55 | MNFGFSLIFLVLVLKGVQCEVKLVPRQLDYKDDDDK EFQIQLVQSGPEVKKPGETVKISCKASGYTFTIHGMS WVKQAPGKGLKWMGWINTYSGVPTYANDFKGRFAF SLETSASTAYLQINNLKNEDTATYFCARDEVRRGFGF AYWGQGTLVTVSARSSTKGPKLEEGEFSEAQLDIVM AQSQKFMSVSVGDRVSVTCKASQNVGTNVAWYQQR PGQSPKALIYSASYRYSGVPDRFTGSGSGTDFTLTISN VQSEDLAEYFCQQFDSHPLTFGAGTKLELKGSSSAST KGPSVFPLAPSSKSTSGGTAALGCLVKDYFPEPVTVS |
| | | | WNSGALTSGVHTFPAVLQSSGLYSLSSVVTVPSSSLG TQTYICNVNHKPSNTKVDKKVEPKSCDKTHTCPPCPA PELLGGPSVFLFPPKPKDTLMISRTPEVTCVVVDVSHE DPEVKFNWYVDGVEVHNAKTKPREEQYASTYRVVS VLTVLHQDWLNGKEYKCKVSNKALPAPIEKTISKAK GQPREPQVYTLPPSRDELTKNQVSLTCLVKGFYPSDIA VEWESNGQPENNYKTTPPVLDSDGSFFLYSKLTVDKS RWQQGNVFSCSVMHEALHNHYTQKSLSLSPGK- |
| pDualyx-152 | HC | SEQ ID NO: 56 | MNFGFSLIFLVLVLKGVQCEVKLVPRQLDYKDDDDK EFQIQLVQSGPEVKKPGETVKISCKASGYTFTIHGMS WVKQAPGKGLKWMGWINTYSGVPTYANDFKGRFAF SLETSASTAYLQINNLKNEDTATYFCARDEVRRGFGF AYWGQGTLVTVSARSSTKGPKLEEGEFSEAQLDIVM AQSQKFMSVSVGDRVSVTCKASQNVGTNVAWYQQR PGQSPKALIYSASYRYSGVPDRFTGSGSGTDFTLTISN VQSEDLAEYFCQQFDSHPLTFGAGTKLELKGSSSAST KGPSVFPLAPSSKSTSGGTAALGCLVKDYFPEPVTVS WNSGALTSGVHTFPAVLQSSGLYSLSSVVTVPSSSLG TQTYICNVNHKPSNTKVDKKVEPKSCDKTHTCPPCPA PELLGGPSVFLFPPKPKDTLMISRTPEVTCVVVDVSHE DPEVKFNWYVDGVEVHNAKTKPREEQYASTYRVVS VLTVLHQDWLNGKEYKCKVSNKALPAPIEKTISKAK GQPREPQVYTLPPSRDELTKNQVSLTCLVKGFYPSDIA VEWESNGQPENNYKTTPPVLDSDGSFFLYSKLTVDKS RWQQGNVFSCSVMHEALHNHYTQKSLSLSPGKLEDI ACGCAAAPDIKDLLSRLEELEGLVSSLREQGTG- |
| pDualyx1-153 | HC | SEQ ID NO: 57 | MNFGFSLIFLVLVLKGVQCEVKLVPRQLDYKDDDDK EFQIQLVQSGPEVKKPGETVKISCKASGYTFTIHGMS WVKQAPGKGLKWMGWINTYSGVPTYANDFKGRFAF SLETSASTAYLQINNLKNEDTATYFCARDEVRRGFGF AYWGQGTLVTVSARSSTKGPKLEEGEFSEAQLDIVM AQSQKFMSVSVGDRVSVTCKASQNVGTNVAWYQQR PGQSPKALIYSASYRYSGVPDRFTGSGSGTDFTLTISN VQSEDLAEYFCQQFDSHPLTFGAGTKLELKGSSSAST KGPSVFPLAPSSKSTSGGTAALGCLVKDYFPEPVTVS WNSGALTSGVHTFPAVLQSSGLYSLSSVVTVPSSSLG TQTYICNVNHKPSNTKVDKKVEPKSCDKTHTCPPCPA LEDIACGCAAAPDIKDLLSRLEELEGLVSSLREQGTG- |
| pDualyx-154 | HC | SEQ ID NO: 58 | MNFGFSLIFLVLVLKGVQCEVKLVPRQLDYKDDDDK EFEVKLLQSGGGLVQPGGSLKLSCEASGIDFSRYWMS WVRRAPGKGLEWIGESDPDSSTINYAPSLKDKFIISRD SARNTLYLQMSKVRSEDTALYYCAREGYYDNEGYFD VWGTGTTVTVSSRSSSASTKGPSVFPLAPSSKSTSGGT AALGCLVKDYFPEPVTVSWNSGALTSGVHTFPAVLQ SSGLYSLSSVVTVPSSSLGTQTYICNVNHKPSNTKVDK KVEPKSCDKTHTCPPCPAPELLGGPSVFLFPPKPKDTL MISRTPEVTCVVVDVSHEDPEVKFNWYVDGVEVHNA KTKPREEQYASTYRVVSVLTVLHQDWLNGKEYKCK VSNKALPAPIEKTISKAKGQPREPQVYTLPPSRDELTK NQVSLTCLVKGFYPSDIAVEWESNGQPENNYKTTPPV LDSDGSFFLYSKLTVDKSRWQQGNVFSCSVMHEALH NHYTQKSLSLSPGKLEDIACGCAAAPDIKDLLSRLEEL EGLVSSLREQGTG- |
| pDualyx-155 | HC | SEQ ID NO: 59 | MNFGFSLIFLVLVLKGVQCEVKLVPRQLDYKDDDDK EFEVKLLQSGGGLVQPGGSLKLSCEASGIDFSRYWMS WVRRAPGKGLEWIGESDPDSSTINYAPSLKDKFIISRD SARNTLYLQMSKVRSEDTALYYCAREGYYDNEGYFD VWGTGTTVTVSSRSSTKGPKLEEGEFSEAQLNIVMTQ SPKSLSMSVGERVTLNCKASENVKTYVSWYQQKPEQ SPKLLIYGASNRYTGVPDRFTGSGSATDFTLTISSVQA EDLADYHCGQSYSYPFTFGAGTKLERKGSSSASTKGP SVFPLAPSSKSTSGGTAALGCLVKDYFPEPVTVSWNS GALTSGVHTFPAVLQSSGLYSLSSVVTVPSSSLGTQTY ICNVNHKPSNTKVDKKVEPKSCDKTHTCPPCPAPELL GGPSVFLFPPKPKDTLMISRTPEVTCVVVDVSHEDPEV KFNWYVDGVEVHNAKTKPREEQYASTYRVVSVLTV LHQDWLNGKEYKCKVSNKALPAPIEKTISKAKGQPR EPQVYTLPPSRDELTKNQVSLTCLVKGFYPSDIAVEW ESNGQPENNYKTTPPVLDSDGSFFLYSKLTVDKSRWQ QGNVFSCSVMHEALHNHYTQKSLSLSPGK- |
| pDualyx-157 | HC | SEQ ID NO: 60 | MNFGFSLIFLVLVLKGVQCEVKLVPRQLDYKDDDDK EFEVKLLQSGGGLVQPGGSLKLSCEASGIDFSRYWMS WVRRAPGKGLEWIGESDPDSSTINYAPSLKDKFIISRD SARNTLYLQMSKVRSEDTALYYCAREGYYDNEGYFD VWGTGTTVTVSSRSSTKGPKLEEGEFSEAQLNIVMTQ SPKSLSMSVGERVTLNCKASENVKTYVSWYQQKPEQ SPKLLIYGASNRYTGVPDRFTGSGSATDFTLTISSVQA EDLADYHCGQSYSYPFTFGAGTKLERKGSSSASTKGP SVFPLAPSSKSTSGGTAALGCLVKDYFPEPVTVSWNS GALTSGVHTFPAVLQSSGLYSLSSVVTVPSSSLGTQTY ICNVNHKPSNTKVDKKVEPKSCDKTHTCPPCPAPELL GGPSVFLFPPKPKDTLMISRTPEVTCVVVDVSHEDPEV KFNWYVDGVEVHNAKTKPREEQYASTYRVVSVLTV LHQDWLNGKEYKCKVSNKALPAPIEKTISKAKGQPR EPQVYTLPPSRDELTKNQVSLTCLVKGFYPSDIAVEW ESNGQPENNYKTTPPVLDSDGSFFLYSKLTVDKSRWQ QGNVFSCSVMHEALHNHYTQKSLSLSPGKLEDIACGC AAAPDIKDLLSRLEELEGLVSSLREQGTG- |
| pDualyx-158 | HC | SEQ ID NO: 61 | MNFGFSLIFLVLVLKGVQCEVKLVPRQLDYKDDDDK EFEVKLLQSGGGLVQPGGSLKLSCEASGIDFSRYWMS WVRRAPGKGLEWIGESDPDSSTINYAPSLKDKFIISRD SARNTLYLQMSKVRSEDTALYYCAREGYYDNEGYFD VWGTGTTVTVSSRSSTKGPKLEEGEFSEAQLNIVMTQ SPKSLSMSVGERVTLNCKASENVKTYVSWYQQKPEQ SPKLLIYGASNRYTGVPDRFTGSGSATDFTLTISSVQA EDLADYHCGQSYSYPFTFGAGTKLERKGSSSASTKGP SVFPLAPSSKSTSGGTAALGCLVKDYFPEPVTVSWNS GALTSGVHTFPAVLQSSGLYSLSSVVTVPSSSLGTQTY ICNVNHKPSNTKVDKKVEPKSCDKTHTCPPCPALEDI ACGCAAAPDIKDLLSRLEELEGLVSSLREQGTG- |
| pDualyx-159 | HC | SEQ ID NO: 62 | MNFGFSLIFLVLVLKGVQCEVKLVPRQLDYKDDDDK EFQVTLKESGPGILQPSQTLSLTCSFSGFSLSTFGMGV GWIRQPSGKGLEWLAHIWWDDDKYYNPALKSRLTIS KDTSENQVFLKIANVDTADTATYYCVRIAGTRYFDV WGTGTTVTVSSRSSSASTKGPSVFPLAPSSKSTSGGTA ALGCLVKDYFPEPVTVSWNSGALTSGVHTFPAVLQSS GLYSLSSVVTVPSSSLGTQTYICNVNHKPSNTKVDKK |
| | | | VEPKSCDKTHTCPPCPAPELLGGPSVFLFPPKPKDTLM ISRTPEVTCVVVDVSHEDPEVKFNWYVDGVEVHNAK TKPREEQYASTYRVVSVLTVLHQDWLNGKEYKCKVS NKALPAPIEKTISKAKGQPREPQVYTLPPSRDELTKNQ VSLTCLVKGFYPSDIAVEWESNGQPENNYKTTPPVLD SDGSFFLYSKLTVDKSRWQQGNVFSCSVMHEALHNH YTQKSLSLSPGKLEDIACGCAAAPDIKDLLSRLEELEG LVSSLREQGTG- |
| pDualyx-160 | HC | SEQ ID NO: 63 | MNFGFSLIFLVLVLKGVQCEVKLVPRQLDYKDDDDK EFQVTLKESGPGILQPSQTLSLTCSFSGFSLSTFGMGV GWIRQPSGKGLEWLAHIWWDDDKYYNPALKSRLTIS KDTSENQVFLKIANVDTADTATYYCVRIAGTRYFDV WGTGTTVTVSSRSSTKGPKLEEGEFSEAQLDIQMTQS PSSLSASLGGKVTITCKASQDINKFIAWYQHKPGKGP RLLIHYTSTLQPGIPSRFSGSGSGRDYSFSISNLEPEDIA TYYCLQYDNLYTFGGGTKLEIKGSSSASTKGPSVFPL APSSKSTSGGTAALGCLVKDYFPEPVTVSWNSGALTS GVHTFPAVLQSSGLYSLSSVVTVPSSSLGTQTYICNVN HKPSNTKVDKKVEPKSCDKTHTCPPCPAPELLGGPSV FLFPPKPKDTLMISRTPEVTCVVVDVSHEDPEVKFNW YVDGVEVHNAKTKPREEQYASTYRVVSVLTVLHQD WLNGKEYKCKVSNKALPAPIEKTISKAKGQPREPQVY TLPPSRDELTKNQVSLTCLVKGFYPSDIAVEWESNGQ PENNYKTTPPVLDSDGSFFLYSKLTVDKSRWQQGNV FSCSVMHEALHNHYTQKSLSLSPGK- |
| pDualyx-162 | HC | SEQ ID NO: 64 | MNFGFSLIFLVLVLKGVQCEVKLVPRQLDYKDDDDK EFQVTLKESGPGILQPSQTLSLTCSFSGFSLSTFGMGV GWIRQPSGKGLEWLAHIWWDDDKYYNPALKSRLTIS KDTSENQVFLKIANVDTADTATYYCVRIAGTRYFDV WGTGTTVTVSSRSSTKGPKLEEGEFSEAQLDIQMTQS PSSLSASLGGKVTITCKASQDINKFIAWYQHKPGKGP RLLIHYTSTLQPGIPSRFSGSGSGRDYSFSISNLEPEDIA TYYCLQYDNLYTFGGGTKLEIKGSSSASTKGPSVFPL APSSKSTSGGTAALGCLVKDYFPEPVTVSWNSGALTS GVHTFPAVLQSSGLYSLSSVVTVPSSSLGTQTYICNVN HKPSNTKVDKKVEPKSCDKTHTCPPCPAPELLGGPSV FLFPPKPKDTLMISRTPEVTCVVVDVSHEDPEVKFNW YVDGVEVHNAKTKPREEQYASTYRVVSVLTVLHQD WLNGKEYKCKVSNKALPAPIEKTISKAKGQPREPQVY TLPPSRDELTKNQVSLTCLVKGFYPSDIAVEWESNGQ PENNYKTTPPVLDSDGSFFLYSKLTVDKSRWQQGNV FSCSVMHEALHNHYTQKSLSLSPGKLEDIACGCAAAP DIKDLLSRLEELEGLVSSLREQGTG- |
| pDualyx-163 | HC | SEQ ID NO: 65 | MNFGFSLIFLVLVLKGVQCEVKLVPRQLDYKDDDDK EFQVTLKESGPGILQPSQTLSLTCSFSGFSLSTFGMGV GWIRQPSGKGLEWLAHIWWDDDKYYNPALKSRLTIS KDTSENQVFLKIANVDTADTATYYCVRIAGTRYFDV WGTGTTVTVSSRSSTKGPKLEEGEFSEAQLDIQMTQS PSSLSASLGGKVTITCKASQDINKFIAWYQHKPGKGP RLLIHYTSTLQPGIPSRFSGSGSGRDYSFSISNLEPEDIA TYYCLQYDNLYTFGGGTKLEIKGSSSASTKGPSVFPL APSSKSTSGGTAALGCLVKDYFPEPVTVSWNSGALTS GVHTFPAVLQSSGLYSLSSVVTVPSSSLGTQTYICNVN HKPSNTKVDKKVEPKSCDKTHTCPPCPALEDIACGCA |
| | | | AAPDIKDLLSRLEELEGLVSSLREQGTG- |

Table 5 below provides the CDR sequences of the specific antibody clones whose light and heavy chain variable regions and CDR sequences were used as a starting point for the antigen-binding sites of the various antibody formats employed in the present Examples. The shading is included simply to help indicate light and heavy chain CDRs from the same clone.

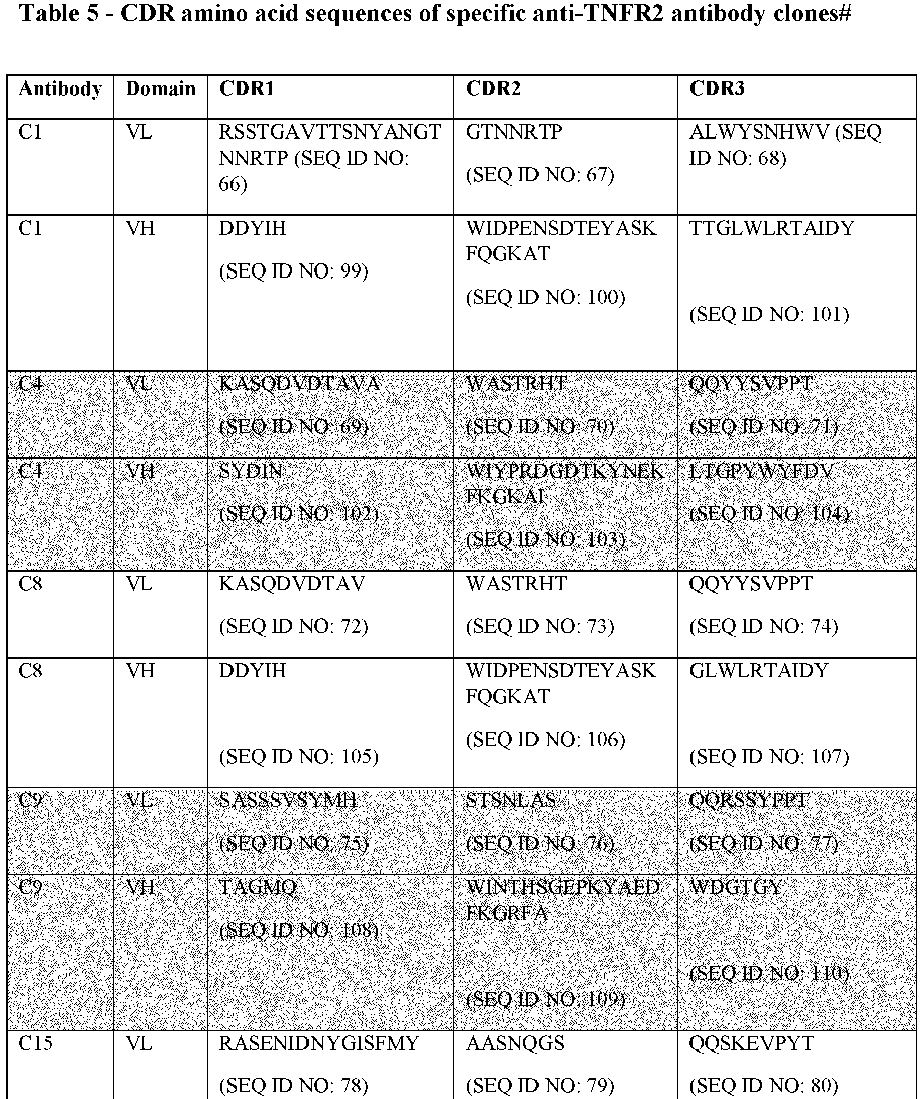

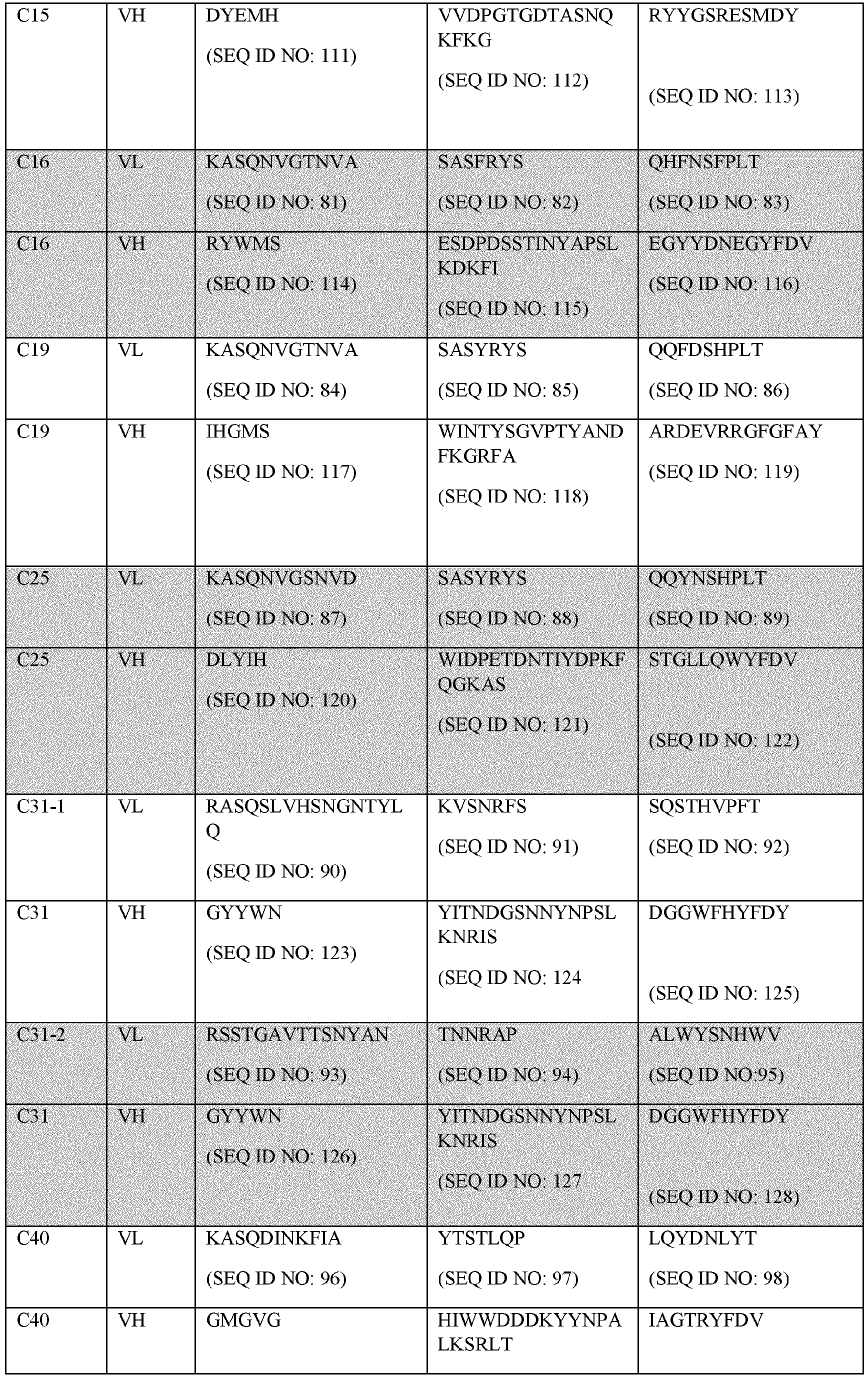

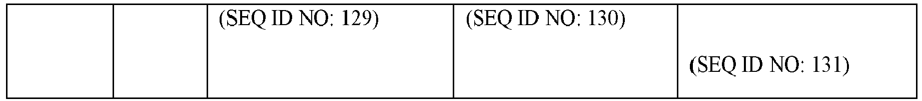

Table 6 provides further relevant amino acid sequences discussed herein, including the Accession number for them and the SEQ ID NO allocated to them.

**Table 6 - amino acid sequences of TNFR2, TNFα variants, LTα, and other protein**

| | |
|---|---|
| **SEQ ID NO:** | **Protein** |
| **SEQ ID NO: 1** | **Human TNFR2 - Accession No: NP_ 001057** |
| | 1 mapvavwaal avglelwaaa halpaqvaft pyapepgstc rlreyydqta qmccskcspg 61 qhakvfctkt sdtvcdsced stytqlwnwv peclscgsrc ssdqvetqac treqnrictc 121 rpgwycalsk qegcrlcapl rkcrpgfgva rpgtetsdvv ckpcapgtfs nttsstdicr 181 phqicnvvai pgnasmdavc tstsptrsma pgavhlpqpv strsqhtqpt pepstapsts 241 fllpmgpspp aegstgdfal pvglivgvta lglliigvvn cvimtqvkkk plclqreakv 301 phlpadkarg tqgpeqqhll itapssssss lessasaldr raptrnqpqa pgveasgage 361 arastgssds spgghgtqvn vtcivnvcss sdhssqcssq asstmgdtds spsespkdeq 421 vpfskeecaf rsqletpetl lgsteekplp lgvpdagmkp s |
| **SEQ ID NO: 2** | **Tenascin peptide - Amino acids 110 - 139 of chicken Tenascin:** |
| | ACGCAAAPDIKDLLSRLEELEGLVSSLREQ |
| **SEQ ID NO: 132** | **Human TNF-α - Accession No. NP_ 000585** |
| |   1 mstesmirdv elaeealpkk tggpqgsrrc lflslfsfli vagattlfcl lhfgvigpqr 61 eefprdlsli splaqavrss srtpsdkpva hvvanpqaeg qlqwlnrran allangvelr 121 dnqlvvpseg lyliysqvlf kgqgcpsthv llthtisria vsyqtkvnll saikspcqre 181 tpegaeakpw yepiylggvf qlekgdrlsa einrpdyldf aesgqvyfgi ial |
| **SEQ ID No: 133** | **Human Lymphotoxin-Alpha - Accession No: NP_ 000586** |
| | 1 mtpperlflp rvcgttlhll llglllvllp gaqglpgvgl tpsaaqtarq hpkmhlahst 61 lkpaahligd pskqnsllwr antdraflqd gfslsnnsll vptsgiyfvy sqvvfsgkay 121 spkatsspl y lahevqlfss qypfhvplls sqkmvypglq epwlhsmyhg aafqltqgdq 181 lsthtdgiph lvlspstvff gafal |
| **SEQ ID No: 134** | **TNC-sc(mu)TNF(143N/145R)** |
| | MNFGFSLIFLVLVLKGVQCEVKLVPRGSGSDYKDDDDKDIACGCAAAPDIKDLLSRLEELEGLVSSLREQGTGGG SGGRGEFEFTRDKPVAHVVANHQVEEQLEWLSQRANALLANGMDLKDNQLVVPADGLYLVYSQVLFKGQGCPDYV LLTHTVSRFAISYQEKVNLLSAVKSPCPKDTPEGAELKPWYEPIYLGGVFQLEKGDQLSAEVNLPKYLNFRESGQ VYFGVIALGGGSGGGSGGGSGGGSDKPVAHVVANHQVEEQLEWLSQRANALLANGMDLKDNQLVVPADGLYLVYS QVLFKGQGCPDYVLLTHTVSRFAISYQEKVNLLSAVKSPCPKDTPEGAELKPWYEPIYLGGVFQLEKGDQLSAEV NLPKYLNFRESGQVYFGVIALGGGSGGGSGGGSGGGSDKPVAHVVANHQVEEQLEWLSQRANALLANGMDLKDNQ LVVPADGLYLVYSQVLFKGQGCPDYVLLTHTVSRFAISYQEKVNLLSAVKSPCPKDTPEGAELKPWYEPIYLGGV FQLEKGDQLSAEVNLPKYLNFRESGQVYFGVIAL |
| **SEQ ID No: 135 & SEQ ID 136** | **Irr-IgG1(N297A)-HC:scFvsc(mu)TNF(143N/145R)*** |
| | **Heavy chain SEQ ID No: 135:** |
| | MNFGFSLIFLVLVLKGVQCEVKLVPRQLDYKDDDDKELQLQLQESGPGLVKPSETLSLTCTVSGASISANSYYGVW VRQSPGKGLEWVGSIAYRGNSNSGSTYYNPSLKSRATVSVDTSKNQVSLRLTSVTAADTALYYCARRQLLDDGTGY QWAAFDVWGQGTMVTVSSGSSSASTKGPSVFPLAPSSKSTSGGTAALGCLVKDYFPEPVTVSWNSGALTSGVHTFP AVLQSSGLYSLSSVVTVPSSSLGTQTYICNVNHKPSNTKVDKKVEPKSCDKTHTCPPCPAPELLGGPSVFLFPPKP KDTLMISRTPEVTCVVVDVSHEDPEVKFNWYVDGVEVHNAKTKPREEQYASTYRVVSVLTVLHQDWLNGKEYKCKV SNKALPAPIEKTISKAKGQPREPQVYTLPPSRDELTKNQVSLTCLVKGFYPSDIAVEWESNGQPENNYKTTPPVLD SDGSFFLYSKLTVDKSRWQQGNVFSCSVMHEALHNHYTQKSLSLSPGKEFEFTRDKPVAHVVANHQVEEQLEWLSQ RANALLANGMDLKDNQLVVPADGLYLVYSQVLFKGQGCPDYVLLTHTVSRFAISYQEKVNLLSAVKSPCPKDTPEG AELKPWYEPIYLGGVFQLEKGDQLSAEVNLPKYLNFRESGQVYFGVIALGGGSGGGSGGGSGGGSDKPVAHVVANH QVEEQLEWLSQRANALLANGMDLKDNQLVVPADGLYLVYSQVLFKGQGCPDYVLLTHTVSRFAISYQEKVNLLSAV KSPCPKDTPEGAELKPWYEPIYLGGVFQLEKGDQLSAEVNLPKYLNFRESGQVYFGVIALGGGSGGGSGGGSGGGS DKPVAHVVANHQVEEQLEWLSQRANALLANGMDLKDNQLVVPADGLYLVYSQVLFKGQGCPDYVLLTHTVSRFAIS YQEKVNLLSAVKSPCPKDTPEGAELKPWYEPIYLGGVFQLEKGDQLSAEVNLPKYLNFRESGQVYFGVIAL |
| | **Light chain SEQ ID No: 136:** |
| | MNFGFSLIFLVLVLKGVQCEVKLVPRQLDYKDDDDKELQSVLTQPPSVSEAPRQTVTISCSGNSFNIGRYPVNWYQQ LPGKAPKLLIYYNNLRFSGVSDRFSGSKSGTSASLAIRDLLSEDEADYYCSTWDDTLKGWVFGGGTKVTVLGGSEIK RTVAAPSVFIFPPSDEQLKSGTASVVCLLNNFYPREAKVQWKVDNALQSGNSQESVTEQDSKDSTYSLSSTLTLSKA DYEKHKVYACEVTHQGLSSPVTKSFNRGEC |
| **SEQ ID No: 137** | **TNFR2(mu)-pCMV-SPORT6 (Tnfrsf1b) (Origene) cDNA clone IMAGE:3710793; GenBank: BC039127.1** |
| | MAPAALWVALVFELQLWATGHTVPAQVVLTPYKPEPGYECQISQEYYDRKAQMCCAKCPPGQYVKHFCNKTSDTVCA DCEASMYTQVWNQFRTCLSCSSSCTTDQVEIRACTKQQNRVCACEAGRYCALKTHSGSCRQCMRLSKCGPGFGVASS RAPNGNVLCKACAPGTFSDTTSSTDVCRPHRICSILAIPGNASTDAVCAPESPTLSAIPRTLYVSQPEPTRSQPLDQ EPGPSQTPSILTSLGSTPIIEQSTKGGISLPIGLIVGVTSLGLLMLGLVNCIILVQRKKKPSCLQRDAKVPHVPDEK SQDAVGLEQQHLLTTAPSSSSSSLESSASAGDRRAPPGGHPQARVMAEAQGFQEARASSRISDSSHGSHGTHVNVTC IVNVCSSSDHSSQCSSQASATVGDPDAKPSASPKDEQVPFSQEECPSQSPCETTETLQSHEKPLPLGVPDMGMKPSQ AGWFDQIAVKVA |
| **SEQ ID No: 138** | **TNFR2-cyno; Macaca fascicularis; GenBank: EHH62644.1** |
| | MAVFPSSFQVAFTPYAPEPGGTCRLREYYDQTAQMCCSKCPPGQHAKVFCTKTSDTVCDSCEDSTYTQLWNWVPECL SCGSRCSSDQVETQACTREQNRICTCRPGWYCALSKQEGCRLCAQLRKCRPGFGVARPGTETSDVVCKPCAPGTFSN TTSSTDICRPHQICHVVAIPGNASMDAVCTSTSPTRSMAPGAVHLPQPVSTRSQHTQPTPAPSTAPGTSFLLPVGPS PPAEGSTGDIVLPVGLIVGVTALGLLIIGVVNCVIMTQVKKKPLCLQRETKVPHLPADKARGAQGPEQQHLLTTVPS SSSSSLESSASALDRRAPTRNQPQAPGAEKASGAGEARASTGSSDSSPGGHGTQVNVTCIVNVCSSSDHSSQCSSQA |
| | SSTMGDTDASPSGSPKDEQVPFSKEESAFRSQLETPETLLGSTEEKPLPLGVPDAGMKPS |
| **SEQ ID No: 139** | **Human Tenascin peptide - Amino acids 110 - 139 of human Tenascin** (NP_002151.2): |
| | Human ACGCAAAPDVKELLSRLEELENLVSSLREQ |

### Gel filtration

Purified antibody fusion proteins (50-200 µg) were analyzed for their native weight and potential protein aggregation by gel filtration on a MabPac SEC-1 column (Thermo Fisher) using the UltiMate 3000 HPLC system (Thermo Fisher) and the aqueous SEC-1 column performance check standard (#AL0-3042, Phenomenex, Torrance, CA, USA).

### Cellular binding studies

Affinity (K_{D}-values) of TNFR2-targeting antibodies to human, cynomolgus monkey, and murine TNFR2 was evaluated by cellular equilibrium binding studies. Therefore, HEK293T cells were transiently transfected with human, cynomolgus or mouse TNFR2 encoding expression plasmids or an empty vector control (EV) using the PEI method. Aliquots of HEK293T transfectants were treated with increasing concentration of the indicated GpL (*Gaussia princeps* luciferase)-tagged anti-TNFR2 antibodies, incubated for one hour at standard cell culture conditions and subsequently washed two times with ice-cold PBS. Cells were finally resuspended in 50 µl RPMI 1640 media supplemented with 0.5 % FCS and transferred into a 96-black well plate. Cell associated luciferase activity (RLU; relative light units) was detected with LUmo luminometer (anthos Mikrosysteme GmbH, Friesoythe, Germany) directly after adding 1.5 µM of the substrate coelenterazin (Carl Roth, Karlsruhe, Germany).

Affinity of TNFR2-specific antibodies and antibody variants were also evaluated with HeLa cells and HeLa cells stably expressing human TNFR2. HeLa and HeLa-TNFR2 cells were seeded out in 24-well plates. Next day, the cells were challenged with the indicated concentrations of GpL-labelled TNFR2 antibodies for 1 hour at 37 °C. To eliminate the unbound antibodies, the cells were washed 10 times with ice-cold PBS. The cells were then scratched and collected in 50 µl of medium (RPMI, 0.5% FCS) and transferred to black 96-well plates for measurement as mentioned above. The specific binding of different TNFR2-specific antibodies/antibody variants was calculated by subtracting the unspecific binding (values obtained from HeLa cells) from total binding (obtained from HeLa-TNFR2). The resulting values were fitted using the non-linear regression analysis option of GraphPad Prism 5.

### Analysis of IL8 induction

Cytokine induction is a well-established readout for NFκB activation in response to TNFR2 signaling. We analyzed the strength of NFκB activation by measuring the IL8 amount after TNFR2 stimulation with the help of a commercially available IL8 ELISA kit (IL8 ELISA Set, BD OptEIA^{™}, San Diego, USA).

HT1080-Bcl2-TNFR2 cells responding to TNFR2 stimulation with strong IL8 induction were seeded in 96-well plates (20.000 cells / well). The next day, cells were stimulated with the TNFR2-targeting reagents of interest and oligomerized TNF80 (= TNC-sc(mu)TNF(143N/145R) a highly potent TNFR2-selective TNF variant (Chopra *et al.,* 2015) as a positive control. After overnight incubation 50 µl of the supernatant was analyzed with the help of BD OptEIA^{™} IL8 ELISA kit as described in the manufacturer's manual.

### Viability assay

TNFR2 can induce in some cell lines/cell types cell death by inhibition of survival proteins (TRAF2, cIAP1, cIAP2) and concomitant upregulation of TNF triggering TNFR1. This type of response has been demonstrated among others in Kym-1 cells (Schneider *et al.,* 1999). To test whether the TNFR2-targeting constructs are able to trigger this TNFR2 response, Kym-1 cells were seeded in 96-well plates (20.000 cells/well) and were stimulated the next day with the TNFR2-targeting reagents of interest. To define complete cell killing an aliquot of cells were also treated with a "cell death"-control mixture ((200 ng/ml TNF, 200 ng/ml TRAIL, 200 ng/ml CD95L, 25 µg/ml CHX, 1 % (w/v) sodium azide)). Remaining plastic-attached cells were finally stained with 70 µl crystal-violet solution. After 20 minutes plates were washed once with dH₂0 and dried on air. After solving in methanol the OD at 595 nm was measured with a PHOmo photometer (anthos Mikrosysteme GmbH, Friesoythe, Germany) to quantify viability. Values were normalized according to untreated cells (100 % viability) and cells treated with the cytotoxic mixture (0 % viability).

### Activation of the alternative NFκB pathway

p100 processing to p52 is the main characteristic of the activity of the alternative NFκB pathway. To evaluate the ability of anti-TNFR2 antibodies to induce p100 processing, 1 x 10⁶ Kym-1 cells were seeded per well of a 6-well tissue culture plate. Next day, cells were stimulated with the TNFR2-targeting reagent of interest. After overnight incubation at standard cell culture conditions tota cell lysates were prepared (1 x wash PBS, scraping cells in 1 ml PBS, centrifugation (2 min, 12000 rpm, cell pellet lysed in 4x Laemmli sample buffer, sonicated for 25 sec with maximal amplitude (UP100H Ultrasonic Processor, Hielscher, Germany), heating for 5 min at 95 °C and removal of debris by centrifugation (2 min, 12000 rpm). For evaluation of p100 processing western blotting with standard techniques and standard equipment were perforemed using the anti-p100/p52 antibody of from Sigma Aldrich (Darmstadt, Germany).

### References for Materials & Methods

Chopra M, Biehl M, Steinfatt T, Brandl A, Kums J, Amich J, Vaeth M, Kuen J, Holtappels R, Podlech J, Mottok A, Kraus S, Jordan-Garrote AL, Bäuerlein CA, Brede C, Ribechini E, Fick A, Seher A, Polz J, Ottmüller KJ, Baker J, Nishikii H, Ritz M, Mattenheimer K, Schwinn S, Winter T, Schäfer V, Krappmann S, Einsele H, Müller TD, Reddehase MJ, Lutz MB, Männel DN, Berberich-Siebelt F, Wajant H, Beilhack A. Exogenous TNFR2 activation protects from acute GvHD via host T reg cell expansion. J Exp Med. 2016 Aug 22;213(9):1881-900.
Medler, J., Nelke, J., Weisenberger, D. et al. TNFRSF receptor-specific antibody fusion proteins with targeting controlled FcγR-independent agonistic activity. Cell Death Dis 10, 224 (2019). https://doi.org/10.1038/s41419-019-1456-x
Medler J, Wajant H. Analysis of FcγR-Dependent Agonism of Antibodies Specific for Receptors of the Tumor Necrosis Factor (TNF) Receptor Superfamily (TNFRSF). Methods Mol Biol. 2021;2248:81-90.
Schneider P, Schwenzer R, Haas E, Muhlenbeck F, Schubert G, Scheurich P et al. (1999). TWEAK can induce cell death via endogenous TNF and TNF receptor 1. Eur J Immunol 29: 1785-1792.
Weiss, T. et al. Enhancement of TNF receptor p60-mediated cytotoxicity by TNF receptorp80: requirement of the TNF receptor-associated factor-2 binding site. J. Immunol. 158, 2398-2404 (1997).

### Example 2: Western blot analysis of variants of the anti-TNFR2 antibody C4 with parallel organized (unidirectionally orientated) N-terminal TNFR2 binding sites

Flag-tagged variants of anti-TNFR2 antibody C4 with **unidirectionally orientated** (parallel organized) N-terminal TNFR2 binding sites were expressed in HEK293T cells as described in Example 1. The antibody formats assessed are those shown in Figure 1, with the specific antibodies employed having the heavy and light chain variable regions from the clone 4 TNFR2 antibody, so that the "Clone Identifier" indicated in the Figure is "C4". Plasmids encoding the chains (HC, LC) of each of the various C4 construct were transfected into the cells. Antibody variant containing supernatants along with various amounts of an antibody standard of known concentration were analyzed by WB and the concentration of the antibody variants in the supernatants were concluded from the standard antibody signals. A volume of supernant containing 200 ng for each antibody variant was then again subjected to Western blotting with anti-human IgG and anti-Flag antibodies. The results obtained are shown in Figure 2 with all of the antibody formats successfully expressed and found in the supernatant recovered from the cells.

### Example 3: TNFR2-mediated IL8 production after treatment with C4 variants with unidirectionally orientated (parallel organized) N-terminal TNFR2 binding sites

The ability of antibody variants with **unidirectionally orientated** (parallel organized) N-terminal TNFR2 binding sites was assessed using the antibody variants comprising the heavy and light chain variable regions from the original Clone 4 antibody specific for TNFR2. Again, the structure of the variants is that shown in Figure 1, but with the "Clone Identifier" being C4 denoting the origin of the antigen-binding sites. A positive control was performed using a highly active oligomeric form of the TNFR2-specific TNF mutant TNF(143N/145R).

In order to perform the assessment, HT1080-Bcl2-TNFR2 cells were stimulated with supernatants containing the concentrations of the various C4 constructs indicated in Figure 3. Next day, supernatants were harvested and analyzed by ELISA for their IL8 content. The results obtained are shown in Figure 3. The maximum response induced was with the highly active oligomeric form of the TNFR2-specific TNF mutant TNF(143N/145R) included as a positive control, with the results for that indicated by a dotted line in the graphs.

As can be seen from Figure 3, the antibody variants that were most active in the assay were those with multiple unidirectionally orientated (parallel) organized N-terminal TNFR2 binding sites. The lowest level of IL-8 production was seen with the C4-Fab, C4-Fab2, and C4-IgG1(N297A) variants which have two or less TNFR2 binding sites.

### Example 4: TNFR2-mediated cell death in Kym-1 cells after treatment with C4 variants with unidirectionally orientated (parallel organized) N-terminal TNFR2 binding sites

The ability of the antibody formats with N-terminal TNFR2 binding sites to trigger cell death in Kym-1 cells was then assessed, again using the Clone 4 light and heavy chain variable regions to confer specificity against TNFR2. The results obtained are shown in Figure 4 with the abbreviations used again those from Figure 1 apart from using "C4" to denote the origin of the antigen-binding sites.

Kym-1 cells were stimulated with supernatants containing the concentrations of the various C4 constructs indicated in Figure 4. Next day, cell viability was determined using crystal violet staining. The results obtained are shown in Figure 4. A highly active oligomeric form of the TNFR2-specific TNF mutant TNF(143N/145R) again served as a positive control for TNFR2 engagement. The most effective antibody variants in inducing apoptosis were the scFv:C4-IgG1(N297A) and scFv:C4-IgG1-(N297A)-HC:TNC variants the results for which are showing in the middle panel of Figure 4. Results for binding molecules based on the binding sites from the clones C19, C27, and C40 are given in Figure 6.

### Example 5: TNFR2-mediated p100 processing in Kym-1 cells after treatment with C4 variants with unidirectionally orientated (parallel) organized N-terminal TNFR2 binding sites

The ability of the C4 variants with unidirectionally orientated (parallel) organized N-terminal TNFR2 binding sites to trigger processing of p100 to p52 and hence indicate triggering of signal transduction through TNFR2 was assessed. Kym-1 cells were stimulated overnight with supernatants containing the concentrations of the various C4 variants indicated in Figure 5. The structures of the variants employed are shown in Figure 1 with "C4" denoting the "clone Identifier". Treatment was performed in the presence of 20 µM ZVAD to prevent TNFR2-induced cell death. A nonameric TNFR2-specific mutant of mouse TNF (oligo TNF80) was used as a positive control. Western blotting was performed using antibodies specific for the indicated proteins, p100, p52, and as a control β-actin. The results obtained are shown in Figure 5.

### Example 6: Western blot analysis of C4 variants with N-terminal and C-terminal organized TNFR2 binding sites

The experiments described in Example 2 for antibody variants with unidirectionally orientated (parallel organized) N-terminal TNFR2 binding sites were also performed for the C4 antibody variants depicted in Figure 7 that have both N-terminal and C-terminal TNFR2 binding sites. The variants included FLAG tags in the heavy and light chains, except that the heavy chain of the C4-IgG1(N297A)-HC:scFvC4 variant which did not contain a Flag tag.

Each C4 construct was transfected into the cells and the antibody variants purified from the supernatants of the cells. 200 ng of each antibody variant was used in Western blotting. The supernatants containing the Flag-tagged variants were analyzed by Western blotting with anti-human IgG and anti-Flag antibodies. The results obtained are shown in Figure 8 with all of the antibody formats successfully expressed and found in the supernatant recovered from the cells.

### Example 7: TNFR2-mediated IL8 production in HT1080-Bcl2-TNFR2 cells after treatment with C4 constructs with N-terminal and C-terminal organized TNFR2 binding sites

Equivalent experiments to those set out in Example 3 were also performed with the C4 variants with both N-terminal and C-terminal organized TNFR2 binding sites shown in Figure 7. In order to provide a comparison, a number of the antibody variants with only N-terminal TNFR2 binding sites were also tested side-by-side with those with both N-terminal and C-terminal TNFR2 binding sites.

HT1080-Bcl2-TNFR2 cells were stimulated with supernatants containing the concentrations of the various C4 constructs indicated in Figure 9. Next day, supernatants were harvested and analyzed by ELISA for their IL8 content. The results obtained are shown in Figure 9. The data shown are derived from parallel processed experiments which, for better resolution, are shown in three diagrams. The maximum response induced with a positive control (highly active oligomeric form of the TNFR2-specific TNF mutant TNF(143N/145R)) was indicated by a dotted line.

As shown in Figure 9, the antibody variants with both N- and C-terminal TNFR2 binding sites tended to give the most positive results, with valency though also having an impact. For instance, the results in the middle panel show such antibody formats triggering a higher level of IL-8 production than the C4-IgG1(N297A) variant with just N-terminal TNFR2 binding sites.

### Example 8: TNFR2-mediated cell death in Kym-1 cells after treatment with C4 constructs with N-terminal and C-terminal organized TNFR2 binding sites.

Equivalent experiments to those performed in Example 4 were also carried out for C4 antibody variants where the antibody has both N and C-terminal TNFR2 binding sites. Kym-1 cells were stimulated with supernatants containing the concentrations of the various C4 constructs indicated in Figure 10. Next day, cell viability was determined using the MTT assay. A highly active oligomeric form of the TNFR2-specific TNF mutant TNF(143N/145R) was used as a positive control. The results obtained are shown in Figure 10.

### Example 9: Binding properties of TNFR2-specific antibodies to HeLa cells expressing human TNFR2

The binding properties of a number of the TNFR2-specific antibodies derived from various clones was assessed using Hela cells expressing TNFR2. Equilibrium binding of the anti-TNFR2 antibody GpL fusion proteins to cells expressing TNFR2 at 37°C was assessed with the results obtained shown in Figure 11A. The level of specific, unspecific, and total binding is shown for each antibody. The first graph shows the results for a GpL-Flag-TNFwt positive control for binding to TNFR2.

A further experiment was performed where rather than GpL labelled antibody GpL labelled TNF was used. HeLa-TNFR2 cells were pretreated with the indicated amounts of anti-TNFR2 antibodies or remained untreated. 5 ng/ml GpL-TNF was added and binding was measured after 1 hour incubation at 37°C. Binding to untransfected HeLa cells was then analyzed to obtain unspecific binding values. The results obtained are shown in Figure 11B.

### Example 10: Competition of αTNFR2 antibodies

HeLa-TNFR2 cells were pretreated with the 3 - 10 µg of the anti-TNFR2 antibodies shown in Figure 12 or remained untreated. 50 ng/ml of the various αTNFR2-IgG1(N297A)-LC:GpL variants were added and binding was measured after 1 hour as discussed in the Materials & Methods Example 1. The results obtained are shown in Figure 12.

### Example 11: Tetravalent mAb occupied TNFR2 molecules trigger much stronger IL8 induction than TNFR2 molecules occupied by the conventional bivalent mAb variant

TNFR2-negative Hela cells and HeLa-TNFR2 cells were pairwise stimulated for 8 hours with the concentrations indicated in (A) and (B) of Figure 13 of the GpL fusion proteins of the bi- and tetravalent version of the TNFR2-specific mAb C4 (scheme see inset). Supernatants of HeLa-TNFR2 were then analyzed for TNFR2-induced IL8 production with the results shown in (B). Repeatedly washed cells (HeLa, and HeLa-TNFR2) were analyzed for cell-associated GpL activity. Specific binding to TNFR2, was calculated by subtraction of the unspecific binding values obtained from the HeLa cells from the total binding values derived of the HeLa-TNFR2 cells with the results obtained shown in (A). Please note, luciferase activity was normalized according to the ratio of the number of GpL reporter domains to the number of TNFR2-binding domains within the two mAb variants (1 versus 0.5).

IL8 production by the mAb variants were directly plotted as a function of their specific binding. The latter was transformed into "occupied receptors per cells" by help of the number of cells in the assay and the measured specific activity (RLU/molecule) of the GpL constructs. The results are shown in (C). Maximum binding of the two constructs obtained from A is indicated by dashed vertical lines. The dotted lines indicate linear regression of the IL8 production as function of receptor occupancy.

### Example 12: Binding properties of TNFR2-specific antibodies to HEK293T cells expressing human TNFR2

The binding properties of anti-TNFR2 antibodies from different clones were assessed. HEK293 cells were transiently transfected with human, murine and cynomolgus TNFR2 or empty vector. Equilibrium binding of the indicated anti-TNFR2 antibody GpL fusion proteins was then determined at 37°C. Binding to empty vector transfected cells was determined to obtain unspecific binding values. The results obtained are shown in Figure 14. The antibody binding specifically to all three of human, murine and cynomolgus TNFR2 was the C19-IgG1-LC-GpL antibody. The C4-IgG1-LC-GpL antibody bound to human TNFR2, but not monkey or murine TNFR2. The C40-IgG1-LC-GpL antibody bound to human and monkey TNFR2 but not to murine TNFR2.

Figure 15 shows the domain architecture of TNFR2, indicating the extracellular TNFR2 parts recognized by the anti-TNFR2 antibodies C4, C19, C27 and C40 and tetravalent CI-IgG1(N297A)-HC:scFvCI variants derived thereof.

### Example 13: Western blot analysis of mono- and bispecific TNFR2-specific CI-IgG-HC:scFvCI variants

A number of different antibody variants were expressed and analyzed by Western blotting in an equivalent manner to the approach used in Examples 2 and 6. The eleven variants analysed had TNFR2 binding sites from different clones, different heavy chain modifications, and a number of them also had C-terminal TNFR2 binding sites as well, with the variants studied indicated in Figure 16. The variants were expressed and analyzed in the equivalent way to that set out in Examples 2 and 6.

The top panel of Figure 16 showing the results of blotting with the anti-FLAG antibody against the FLAG tag that the antibodies were labelled with. Please note that: i) the heavy chains of C4-IgG1(N297A), C4-IgG1(N297A)-HC:scFvC4 and C4-IgG1(N297A)-HC:scFvC19 do not contain a Flag tag; and ii) the conventional C19 variant used was in the murine IgG1(D265A) isoform. The bottom panel of Figure 16 shows the results of the Western blot with anti-hlgG.

### Example 14: TNFR2-mediated IL8 production in HT1080-Bcl2-TNFR2 cells

Analysis of the ability of anti-TNFR2 antibodies to stimulate IL-8 production in HT1080-Bcl2-TNFR2 cells was performed in an equivalent manner to Examples 3 and 7. A number of monospecific antibody variants was assessed, as well as a number of bispecific antibodies with two different specificities for TNFR2. In order to perform the assessment, HT1080-Bcl2-TNFR2 cells were stimulated with supernatants containing the concentrations of the various constructs indicated in Figure 17. Next day, supernatants were harvested and analyzed by ELISA for their IL8 content. The results obtained are shown in Figure 17, with the top panel showing the results for the monospecific antibody and the bottom panel the results for the bispecific antibody.

The top panel shows that in each instance the antibody variant with both an N-terminal and C-terminal TNFR2 binding site stimulated a higher level of TNF production than the corresponding antibody variant with just N-terminal TNFR2 binding site. The results for the C40-IgG1(N297A)-HC:scFvC40 binding molecule were particularly positive in the results shown in the top panel.

### Example 15: TNFR2-mediated cell death in Kym-1 cells after treatment with mono-specific and bispecific TNFR2-specific CI-IgG-HC:scFvCI variants

The ability of monospecific and bispecific antibodies to induce cell death was assessed in an equivalent manner to the approach used in Examples 4 and 8. Kym-1 cells were stimulated with supernatants containing the concentrations of the various C4 constructs indicated in Figure 18. Next day, cell viability was determined using crystal violet staining. The results obtained are shown in Figure 18. The results for the monospecific antibodies are shown in the top panel, with those for the biparatopic antibodies shown in the bottom panel.

### Example 16: TNFR2-mediated p100 processing in Kym-1 cells after treatment with monospecific and bispecific TNFR2-specific CI-IgG-HC:scFvCI variants

The ability of monospecific and bispecific antibodies to bring about signal transduction through TNFR2 as assessed by looking at p100 processing in an equivalent manner to Examples 5 and 9. Kym-1 cells were stimulated overnight with supernatants containing the concentrations of the various C4 variants indicated in Figure 19. Treatment was performed in the presence of 20 µM ZVAD to prevent TNFR2-induced cell death. A nonameric TNFR2-specific mutant of mouse TNF (oligo. TNF80) was used as a positive control. Western blotting was performed using antibodies specific for the indicated proteins, p100, p52, and as a control β-actin. The results obtained are shown in Figure 19.

### Example 17: Purification and functional analysis of C4-IgG1(N297A)-HC:scFvC4, C4-IgG1(N297A)-LC:scFvC4 and C4-IgG1(N297A)-LC:scFvC4-HC:scFvC4

The constructs indicated in Figure 20 were assessed using the methods discussed in Example 1 and employed in the earlier Examples. The results obtained are shown in Figure 20. (A) Silver stained SDS-PAGE gel. (B) Gelfiltration analysis. (C) IL8 induction in HT1080-Bcl2-TNFR2 cells. (D) Cell death induction in Kym-1 cells. (E) Induction of plOO processing in Kym-1 cells. ZVAD was added to prevent cell death.

### Example 18: Activity

Table 7 below summarises the activity of various antibody formats employed in earlier Examples produced as set out in the Materials and Methods Section in Example 1. The antibody formats assessed were those having antigen-binding sites derived from the C4 clone "Bs" in the table indicates the number of "North" and "South" antigen binding sites of the antibody. The Table summarises the activity seen in the IL-8 assay (Activity I - classical NFκB pathway > IL8) or Activity II (TRAF2 depletion > alternative NFκB pathway (= p100 processing) and Kym-1 killing)).

**Table 7 - Summary of activity for C4 based antibody formats**

| **No.** | **Ab format** | **Bs** | **Activity I (IL8) ng/ml** | **Activity II (tox) ng/ml** |
|---|---|---|---|---|
| 1 | Fab1 | 1+0 | 0 | 0 |
| 2 | Fab1-scFv | 1+1 | 10 | 20 |
| 3 | Fab2 | 2+0 | 0 | 0 |
| 4 | Fab2-scFv | 2+2 | 50 | |
| 5 | IgG1 | 2 | 0 | 0 |
| 6 | irrIgG1-HC:scFv | 0+2 | 0 | |
| 7 | IgG1-HC:scFv | 2+2 | 5-20 | 10-20 |
| 8 | IgG1-LC:scFv | 2+2 | 5-20 | 10-20 |
| 9 | IgG1-LC:scFv-HC:scFv | 2+4 | 5-20 | 10-20 |
| 10 | IgG1-TNC | 6+0 | 100 | Very low |
| 11 | IgG1-RGY* | 12+0 | 20-100 | Low |
| 12 | LC:scFv-HC:scFv-IgG1 | 4**+0 | 10-20 | 20 |
| 13 | L/H:scFv-IgG1-LC:scFv | 4**+2 | 5-10 | 5-10 |
| 14 | L/H:scFv-IgG1-LC:scFv | 4**+2 | 5-10 | 5-10 |
| 15 | L/H:scFv-IgG1-L/HC:scFv | 4**+4 | 5-10 | 5-10 |
| 16 | LC:scFv-HC:scFv-IgG1-TNC | 12**+0 | 5-10 | 10 |
| 17 | scFv-Fc-TNC | 12+0 | 10 | |
| 18 | scFv-TNC-Fc | 12+0 | 5-20 | 20 |
| 19 | IgG1 - TNC-HC:scFv | 6+6 | 5-10 | 10-20 |
| | Star2 | 9+0 | 5-20 | 100-200 |

### Example 19 - Ex vivo assessment of Treg expansion

The ability of particular TNFR2 binding molecules to expand regulatory T cells was assessed. Tetravalent antibodies in the Fab-HC-scFv format, where the heavy chains of the Fc regions had the N297A amino acid mutation to eliminate Fc function, and the basic Fab with an IgG1 Fab. PBMCs were isolated from four donors, with the cells from each donor assessed separately. The PBMC were cultured for two days at a density of 10⁷ cells/ml. The PBMCs were then harvested and seeded into multi-well plates at a density of 10⁶ cells/ml, with 500,000 cells per well. The cells were then subdivided into: (a) untreated control cells; (b) cells which were treated with a STAR2 agonist molecule at 100 ng/ml; (c) cells which were treated with tetravalent antibodies with just one specificity (1000 - 0.3 ng/ml); and (d) cells which were treated with tetravalent antibodies with two specificities for TNFR2 (1000 - 0.3 ng/ml). Following culturing for four days the viability of the cells was assessed. The cells were stained with antibodies specific for CD3, CD8, CD4, CD25 and FoxP3. The cells were then assessed via flow cytometry. Figure 21 shows the results obtained in the top panel for Foxp3+ CD25+ cells within CD3+ CD4+ cells to measure Treg cells and their expansion. The bottom panel of Figure 21 shows Foxp3- cells within CD3+ CD4+ cells to measure Tcon cells. Both show percentage change compared to the number of cells prior to the four day incubation.

### Example 20: In vivo Treg expansion

The ability of the TNFR2 agonists provided to expand Treg cells was also assessed using syngeneic FoxP3-Luci mice. FoxP3-Luci mice are transgenic mice that express a fusion protein of FoxP3 and luciferase. This effectively means that it is possible to image the sites of Treg cells *in vivo.* The mice were given a TNFR2 agonist with daily bioluminescence imaging. After four days the mice were sacrificed and ex vivo analysis performed on cells isolated from the spleens of the mice, with flow cytometry used to assess Foxp3, CD3, and CD4. The time line for the experiment is summarized in Figure 22 in the top panel (A). The results of the ex vivo analysis are shown in Figure 22 in the bottom panel (B). The experiment was repeated with multiple mice per treatment group, as shown in Figure 23.

### Example 21 - Production and analysis of TNFR2-specific TNF constructs

The TNF-alpha based molecules shown in Figure 24, part (A) were generated. The molecules all comprised the TNF80 molecule which represents a mutated form of TNF-alpha that lost TNFR1 binding and is thus specific for TNFR2. The structures shown from left to right in part (A) are:
- **Fc-TNC-TNF80** - comprising three Fc regions where the C-terminal region of each polypeptide comprises a tenascin peptide (TNC) followed by a single TNF80 sequence (TNF80 corresponds to the TNFR2-specific mutant TNF(143N/145R). The tenascin peptides form trimers which in turns means that there are two trimers of the mutant TNF-alpha present.
- **Fc-TNF80** - comprising three Fc regions where the C-terminal region of each polypeptide comprises a single TNF80 sequence, which results in two trimers of the mutant TNF-alpha being present in the molecule.
- **Fc(DANA)-TNC-TNF80** - corresponding to Fc-TNC-TNF80 with the heavy chain modifications D265A and N297A (DANA).
- **TNF80** - corresponding to the basic TNF80 mutant in trimeric form.
- **TNC- scTNF80** - corresponding to single chain molecules comprising three TNF80 mutant molecules, where the chains further comprise a TNC peptide resulting in trimerisation of the three single chain polypeptides.

Murine soluble TNF80 based constructs for each of the above were used to express the molecules. The constructs were then purified by anti-Flag affinity purification utilizing a Flag tag contained in all constructs. They were then analyzed by SDS-PAGE and silver staining with the results obtained shown in Figure 24, part (B). Gel filtration analysis of the purified proteins shown in Figure 24, panel (B) was then performed and the results obtained are shown in Figure 24 panel (C). The gel filtration results show that the expressed binding molecules are not aggregated and represent a homogeneous protein species. The methodology used in this Example was as set out in the Materials & Methods in Example 1 above.

### Example 21 - Agonistic activity of different formats of soluble murine TNF80

The ability of the constructs discussed in Example 19 to act as agonists of TNFR2 was next assessed using Kym-1 cells to determine the ability of the binding molecules to induce cell death and HT1080-TNFR2 cells to determine the ability of the binding molecules to induce IL-8 release. The methodology set out above in the Materials & Methods of Example 1 was employed. The results obtained are shown in Figure 25.

The upper panel of Figure 25 shows the results for Kym-1 cells expressing TNFR2 stimulated with the indicated concentrations of the various muTNF80 variants and then assessed the next day using crystal violet staining to determine the ability of the constructs to induce cell death. As can be seen the TNC- sc(mu)TNF80 binding molecule gave the biggest drop in cell viability followed by the Fc(DANA)-TNC-TNF80 binding molecule.

The lower panel of Figure 25 shows the results for HT1080-Bcl2-TNFR2 cells. The HT1080-Bcl2-TNFR2 cells were stimulated with the indicated concentrations of the various TNF80 variants shown in figure 1. Next day, supernatants were harvested and analyzed by ELISA for their IL8 content. The data shown are derived from four independent experiments. As can be seen, the basic murine TNF80 gave the lowest level of induction of IL8 with the other binding molecules stimulating a higher level of IL-8 release.

### Example 22 - In vivo Treg expansion by Fc(DANA)-TNC-muTNF80

In order to show the ability of the binding molecules comprising TNF80 to act as agonists of TNFR2 *in vivo* experiments were next performed in mice using the Fc(DANA)-TNC-muTNF80 binding molecule, as well as using Newstar which comprises an irrelevant IgG molecule with scTNF80 trimers at the C-terminal portion of the heavy chains of the antibody. The results obtained are shown in Figure 26. Mice with luciferase under the control of the Foxp3 promoter were injected i.v with a single injection of the indicated amount of Fc(DANA)-TNC-muTNF80 and were analyzed daily by bioluminescence imaging (BLI) and were also assessed on day 4 for Treg frequency using FACs analysis. The results of the BLI analysis are shown in the upper panel of Figure 26. Day 0 and day 4 BLI pictures are shown in the middle panel of Figure 26. Results for the FACs analysis performed on day 4 are shown in the lower panel of Figure 26.

### Example 22 - Demonstration of specific binding for TNFR2

An experiment was performed to illustrate the ability of the binding molecules provided to bind TNFR2, but not TNFR1. HEK293 cells were transiently transfected with: (i) a TNFR2 expression plasmid; (ii) an expression plasmid encoding the extracellular domain of TNFR1 with a GPI anchor domain; or (iii) empty vector (ev). Next day various binding molecules with a GpL tag were assessed for their ability to bind TNFR2, but not significantly bind TNFR1. The results obtained are shown in Figure 27. The proteins indicated in Figure 27 were added at the indicated concentration to the HEK293 cells. After incubation for 1 hour at 37°C, followed by two washes with PBS, the remaining cell bound GpL activity was determined. GpL-TNF served as positive control binding to both TNF receptor types.

## Claims

1. A binding molecule that specifically binds TNFR2, but not TNFR1, where the binding molecule is an FcyR independent agonist of TNFR2 and has a valency of at least two for binding TNFR2.

2. The binding molecule of claim 1, wherein the binding molecule is able to:
(a) oligomerise TNFR2 on the surface of a cell expressing TNFR2, preferably to cluster six or more TNFR2 molecules on the cell surface;
(b) trigger TNFR2 signalling; and/or
(c) stimulate proliferation of leucocytes, preferably T cells, more preferably Treg cells.

3. The binding molecule of claim 1 or 2, wherein:
(a) the binding molecule comprises a moiety that alters serum half-life, preferably wherein the moiety is selected from the group consisting of a Fc tail, serum albumin, a moiety which is a binder of serum albumin, and PEG;
(a) the binding molecule is bivalent for TNFR2 binding;
(b) the binding molecule is trivalent for TNFR2 binding;
(c) the binding molecule is tetravalent for TNFR2 binding; or
(d) the binding molecule which has a valency of at least four for TNFR2 binding.

4. The binding molecule of any one the preceding claims wherein:
(a) the binding molecule is an antibody;
(b) the binding molecule is an antibody which comprises a ligand for TNFR2;
(c) the binding molecule comprises at least part of an antibody constant region, preferably an Fc region; or
(d) the binding molecule comprises a ligand for TNFR2, preferably wherein the ligand is derived from TNFα or lymphotoxin-alpha,
preferably where the binding molecule is:
(a) an antibody that comprises a TNFR2 binding site at both the N-terminus and C-terminus of one or more heavy chain of the antibody; or
(b) an antibody that comprises a TNFR2 binding site at both the N-terminus and C-terminus of one or more light chain of the antibody.

5. The binding molecule of claim 4, wherein:
(a) the antibody is a bivalent antibody;
(b) the antibody is a trivalent antibody;
(c) the antibody is tetravalent antibody;
(d) the antibody is a hexavalent antibody;
(e) the antibody is a heavy chain only antibody;
(f) the antibody is a single domain antibody, such as a single domain Ab (sdAb), VHH, or a Nanobody;
(g) the antibody is a multimer of at least two antibodies;
(h) the antibody comprises, or is linked to, a multimerizing moiety preferably tenascin-c peptide (TNC), preferably where the antibody is a multimer of at least two antibodies associated through the tenascin-c peptide sequence;
(i) the antibody is linked to one or more molecules derived from a ligand of TNFR2, preferably derived from TNF-α or lymphotoxin alpha.

6. The binding molecule of claim 4 or 5 wherein:
(a) the antibody lacks an Fc region; or
(b) the antibody comprises a modified Fc region that is unable to bind Fc receptors.

7. The binding molecule of any one of the preceding claims, wherein the binding molecule is an antibody in an antibody format selected from:
- IgG with an Fc modification eliminating Fc receptor binding;
- scFv:IgG where the IgG has an Fc modification eliminating Fc receptor binding;
- IgG-HC:TNC, where the IgG has a modification eliminating Fc receptor binding;
- scFv:IgG-HC:TNC where the IgG has a modification eliminating Fc receptor binding; and
- hexameric IgG;
wherein:
- HC represents a heavy chain of an IgG;
- LC represents a light chain of an IgG;
- scFv:IgG represents a scFv either linked to, or forming part of an IgG molecule; and
- HC:TNC indicates a heavy chain (HC) comprising, or linked to, tenascin-c trimerization domain peptide (TNC).

8. The binding molecule of any one of claims 1 to 7, wherein the binding molecule is an antibody in an antibody format selected from the group consisting of:
- Fab-LC:scFv;
- IgG-HC:scFv where the IgG has a modification eliminating Fc receptor binding, preferably N297A;
- IgG-LC:scFv where the IgG has a modification eliminating Fc receptor binding, preferably N297A;
- IgG-LC: scFv-HC: scFv;
- LC: scFv-HC: scFv-IgG-HC: scFv;
- LC: scFv-HC: scFv-IgG-LC: scFv;
- LC:scFv-HC:scFv-IgG-LC:scFv-HC:scFv; and
- IgG-HC-TNC-scFv;
- sdAb/VHH
wherein:
- HC represents a heavy chain of an IgG;
- LC represents a light chain of an IgG;
- LC:scFv indicates a scFv linked to, or forming part of, a light chain;
- HC:scFv indicates a scFv linked to, or forming part of, a heavy chain;
- HC:TNC indicates a heavy chain (HC) comprising, or linked to, tenascin (TNC);
- TNC-scFv indicating tenascin linked or bound to a scFv;
- sdAb indicates a single domain antibody; and
- VHH indicates the variable region of a camelid antibody,
preferably wherein the binding molecule is an antibody and is in an antibody format selected from:
- a Fab-scFv;
- IgG-HC:scFv;
- LC:scFv-HC:scFv-IgG1; and
- LC:scFv-HC:scFv-IgG1-TNC.

9. A binding molecule that specifically binds TNFR2, but not TNFR1, where the binding molecule is an FcyR independent agonist of TNFR2, has a valency of at least two for binding TNFR2, and comprises polypeptides comprising mutant TNF-alpha that bind TNFR2, but not TNFR1, and an Fc region, or a tenascin peptide sequence, or both, that oligomerises the polypeptides,
preferably wherein the binding molecule is one:
(a) which does not comprise antibody sequences;
(b) which comprises polypeptides comprising mutant TNF-alpha that binds TNFR2, but not TNFR1, and a tenascin peptide;
(c) wherein the TNF-alpha mutants are TNF80; and/or
(d) has a valency for TNFR2 of at least three, preferably having a valency for TNFR2 of six

10. The binding molecule of any one of the preceding claims for use as a medicament.

11. The binding molecule of any one of claims 1 to 9 for use in a method of treating or preventing an autoimmune disorder, or an inflammatory disorder,
preferably wherein:
(a) the disorder is graft versus host disease (GvHD), preferably where the binding molecule is for use in a method where it is administered prior to, at the same time, or after a transplant of a cell, tissue, or organ; or
(b) the disorder is one involving dysfunction or unwanted proliferation of leukocytes, preferably of T cells, more preferably of Treg cells; or
(c) the disorder is selected from Inflammatory Bowel Disease (such as Ulcerative Colitis, Crohn's Disease, or Celiac Disease), atherosclerosis, lupus, multiple sclerosis, Type 1 Diabetes, myasthenia gravis, pemphigus vulgaris, and bullous pemphigoid.

12. A method of stimulating cell proliferation comprising contacting a target cell expressing TNFR2 with a binding molecule according to any one of claims 1 to 9.

13. A pharmaceutical composition comprising a binding molecule according to any one of claims 1 to 9 and a pharmaceutically acceptable carrier.

14. A method of detecting TNFR2 comprising contacting a test sample with a binding molecule according to any one of claims 1 to 9 and detecting binding of the binding molecule to TNFR2,
preferably wherein the binding molecule is labelled and the binding of the binding molecule to TNFR2 is detected via the label.

15. A binding molecule according to any one of claims 1 to 9 for use in a method of diagnosis, the method comprising administering a binding molecule according to any one of claims 1 to 15 to a subject and detecting binding of the binding molecule to TNFR2,
preferably wherein the binding molecule is labelled and the binding of the binding molecule to TNFR2 is detected via the label.


SEQUENCE LISTING

<110>  JULIUS-MAXIMILIANS-UNIVERSITÄT WÜRZBURG

<120>  Novel TNFR2 binding molecules

<130>  N420734EP

<160>  139

<170>  PatentIn version 3.5

<210>  1
<211>  461
<212>  PRT
<213>  Homo sapiens

<400>  1

Met Ala Pro Val Ala Val Trp Ala Ala Leu Ala Val Gly Leu Glu Leu
1               5                   10                  15

Trp Ala Ala Ala His Ala Leu Pro Ala Gln Val Ala Phe Thr Pro Tyr
            20                  25                  30

Ala Pro Glu Pro Gly Ser Thr Cys Arg Leu Arg Glu Tyr Tyr Asp Gln
        35                  40                  45

Thr Ala Gln Met Cys Cys Ser Lys Cys Ser Pro Gly Gln His Ala Lys
    50                  55                  60

Val Phe Cys Thr Lys Thr Ser Asp Thr Val Cys Asp Ser Cys Glu Asp
65                  70                  75                  80

Ser Thr Tyr Thr Gln Leu Trp Asn Trp Val Pro Glu Cys Leu Ser Cys
                85                  90                  95

Gly Ser Arg Cys Ser Ser Asp Gln Val Glu Thr Gln Ala Cys Thr Arg
            100                 105                 110

Glu Gln Asn Arg Ile Cys Thr Cys Arg Pro Gly Trp Tyr Cys Ala Leu
        115                 120                 125

Ser Lys Gln Glu Gly Cys Arg Leu Cys Ala Pro Leu Arg Lys Cys Arg
    130                 135                 140

Pro Gly Phe Gly Val Ala Arg Pro Gly Thr Glu Thr Ser Asp Val Val
145                 150                 155                 160

Cys Lys Pro Cys Ala Pro Gly Thr Phe Ser Asn Thr Thr Ser Ser Thr
                165                 170                 175

Asp Ile Cys Arg Pro His Gln Ile Cys Asn Val Val Ala Ile Pro Gly
            180                 185                 190

Asn Ala Ser Met Asp Ala Val Cys Thr Ser Thr Ser Pro Thr Arg Ser
        195                 200                 205

Met Ala Pro Gly Ala Val His Leu Pro Gln Pro Val Ser Thr Arg Ser
    210                 215                 220

Gln His Thr Gln Pro Thr Pro Glu Pro Ser Thr Ala Pro Ser Thr Ser
225                 230                 235                 240

Phe Leu Leu Pro Met Gly Pro Ser Pro Pro Ala Glu Gly Ser Thr Gly
                245                 250                 255

Asp Phe Ala Leu Pro Val Gly Leu Ile Val Gly Val Thr Ala Leu Gly
            260                 265                 270

Leu Leu Ile Ile Gly Val Val Asn Cys Val Ile Met Thr Gln Val Lys
        275                 280                 285

Lys Lys Pro Leu Cys Leu Gln Arg Glu Ala Lys Val Pro His Leu Pro
    290                 295                 300

Ala Asp Lys Ala Arg Gly Thr Gln Gly Pro Glu Gln Gln His Leu Leu
305                 310                 315                 320

Ile Thr Ala Pro Ser Ser Ser Ser Ser Ser Leu Glu Ser Ser Ala Ser
                325                 330                 335

Ala Leu Asp Arg Arg Ala Pro Thr Arg Asn Gln Pro Gln Ala Pro Gly
            340                 345                 350

Val Glu Ala Ser Gly Ala Gly Glu Ala Arg Ala Ser Thr Gly Ser Ser
        355                 360                 365

Asp Ser Ser Pro Gly Gly His Gly Thr Gln Val Asn Val Thr Cys Ile
    370                 375                 380

Val Asn Val Cys Ser Ser Ser Asp His Ser Ser Gln Cys Ser Ser Gln
385                 390                 395                 400

Ala Ser Ser Thr Met Gly Asp Thr Asp Ser Ser Pro Ser Glu Ser Pro
                405                 410                 415

Lys Asp Glu Gln Val Pro Phe Ser Lys Glu Glu Cys Ala Phe Arg Ser
            420                 425                 430

Gln Leu Glu Thr Pro Glu Thr Leu Leu Gly Ser Thr Glu Glu Lys Pro
        435                 440                 445

Leu Pro Leu Gly Val Pro Asp Ala Gly Met Lys Pro Ser
    450                 455                 460

<210>  2
<211>  30
<212>  PRT
<213>  Gallus Gallus Domesticus

<400>  2

Ala Cys Gly Cys Ala Ala Ala Pro Asp Ile Lys Asp Leu Leu Ser Arg
1               5                   10                  15

Leu Glu Glu Leu Glu Gly Leu Val Ser Ser Leu Arg Glu Gln
            20                  25                  30

<210>  3
<211>  254
<212>  PRT
<213>  artificial sequence

<220>
<223>  pDualyx-010pre LC

<400>  3

Met Asn Phe Gly Phe Ser Leu Ile Phe Leu Val Leu Val Leu Lys Gly
1               5                   10                  15

Val Gln Cys Glu Val Lys Leu Val Pro Arg Gln Leu Asp Tyr Lys Asp
            20                  25                  30

Asp Asp Asp Lys Glu Leu Asp Ile Val Met Thr Gln Ser His Lys Phe
        35                  40                  45

Met Ser Thr Ser Val Gly Asp Arg Val Ser Ile Thr Cys Lys Ala Ser
    50                  55                  60

Gln Asp Val Asp Thr Ala Val Ala Trp Tyr Gln Gln Lys Pro Gly Gln
65                  70                  75                  80

Ser Pro Lys Leu Leu Ile Tyr Trp Ala Ser Thr Arg His Thr Gly Val
                85                  90                  95

Pro Asp Arg Phe Thr Gly Ser Gly Ser Gly Thr Asp Tyr Thr Leu Thr
            100                 105                 110

Ile Ser Ser Val Gln Ala Glu Asp Leu Ala Arg Tyr Tyr Cys Gln Gln

115                 120                 125

Tyr Tyr Ser Val Pro Pro Thr Phe Gly Gly Gly Thr Lys Leu Gly Ser
    130                 135                 140

Glu Ile Lys Arg Thr Val Ala Ala Pro Ser Val Phe Ile Phe Pro Pro
145                 150                 155                 160

Ser Asp Glu Gln Leu Lys Ser Gly Thr Ala Ser Val Val Cys Leu Leu
                165                 170                 175

Asn Asn Phe Tyr Pro Arg Glu Ala Lys Val Gln Trp Lys Val Asp Asn
            180                 185                 190

Ala Leu Gln Ser Gly Asn Ser Gln Glu Ser Val Thr Glu Gln Asp Ser
        195                 200                 205

Lys Asp Ser Thr Tyr Ser Leu Ser Ser Thr Leu Thr Leu Ser Lys Ala
    210                 215                 220

Asp Tyr Glu Lys His Lys Val Tyr Ala Cys Glu Val Thr His Gln Gly
225                 230                 235                 240

Leu Ser Ser Pro Val Thr Lys Ser Phe Asn Arg Gly Glu Cys
                245                 250

<210>  4
<211>  414
<212>  PRT
<213>  artificial sequence

<220>
<223>  pDualyx-030pre LC

<400>  4

Met Asn Phe Gly Phe Ser Leu Ile Phe Leu Val Leu Val Leu Lys Gly
1               5                   10                  15

Val Gln Cys Glu Val Lys Leu Val Pro Arg Gln Leu Asp Ile Val Met
            20                  25                  30

Thr Gln Ser His Lys Phe Met Ser Thr Ser Val Gly Asp Arg Val Ser
        35                  40                  45

Ile Thr Cys Lys Ala Ser Gln Asp Val Asp Thr Ala Val Ala Trp Tyr
    50                  55                  60

Gln Gln Lys Pro Gly Gln Ser Pro Lys Leu Leu Ile Tyr Trp Ala Ser
65                  70                  75                  80

Thr Arg His Thr Gly Val Pro Asp Arg Phe Thr Gly Ser Gly Ser Gly
                85                  90                  95

Thr Asp Tyr Thr Leu Thr Ile Ser Ser Val Gln Ala Glu Asp Leu Ala
            100                 105                 110

Arg Tyr Tyr Cys Gln Gln Tyr Tyr Ser Val Pro Pro Thr Phe Gly Gly
        115                 120                 125

Gly Thr Lys Leu Gly Ser Glu Ile Lys Arg Thr Val Ala Ala Pro Ser
    130                 135                 140

Val Phe Ile Phe Pro Pro Ser Asp Glu Gln Leu Lys Ser Gly Thr Ala
145                 150                 155                 160

Ser Val Val Cys Leu Leu Asn Asn Phe Tyr Pro Arg Glu Ala Lys Val
                165                 170                 175

Gln Trp Lys Val Asp Asn Ala Leu Gln Ser Gly Asn Ser Gln Glu Ser
            180                 185                 190

Val Thr Glu Gln Asp Ser Lys Asp Ser Thr Tyr Ser Leu Ser Ser Thr
        195                 200                 205

Leu Thr Leu Ser Lys Ala Asp Tyr Glu Lys His Lys Val Tyr Ala Cys
    210                 215                 220

Glu Val Thr His Gln Gly Leu Ser Ser Pro Val Thr Lys Ser Phe Asn
225                 230                 235                 240

Arg Gly Glu Cys Leu Glu Lys Pro Thr Glu Asn Asn Glu Asp Phe Asn
                245                 250                 255

Ile Val Ala Val Ala Ser Asn Phe Ala Thr Thr Asp Leu Asp Ala Asp
            260                 265                 270

Arg Gly Lys Leu Pro Gly Lys Lys Leu Pro Leu Glu Val Leu Lys Glu
        275                 280                 285

Met Glu Ala Asn Ala Arg Lys Ala Gly Cys Thr Arg Gly Cys Leu Ile
    290                 295                 300

Cys Leu Ser His Ile Lys Cys Thr Pro Lys Met Lys Lys Phe Ile Pro
305                 310                 315                 320

Gly Arg Cys His Thr Tyr Glu Gly Asp Lys Glu Ser Ala Gln Gly Gly

325                 330                 335

Ile Gly Glu Ala Ile Val Asp Ile Pro Glu Ile Pro Gly Phe Lys Asp
            340                 345                 350

Leu Glu Pro Met Glu Gln Phe Ile Ala Gln Val Asp Leu Cys Val Asp
        355                 360                 365

Cys Thr Thr Gly Cys Leu Lys Gly Leu Ala Asn Val Gln Cys Ser Asp
    370                 375                 380

Leu Leu Lys Lys Trp Leu Pro Gln Arg Cys Ala Thr Phe Ala Ser Lys
385                 390                 395                 400

Ile Gln Gly Gln Val Asp Lys Ile Lys Gly Ala Gly Gly Asp
                405                 410

<210>  5
<211>  501
<212>  PRT
<213>  artificial sequence

<220>
<223>  pDualyx-013pre LC

<400>  5

Met Asn Phe Gly Phe Ser Leu Ile Phe Leu Val Leu Val Leu Lys Gly
1               5                   10                  15

Val Gln Cys Glu Val Lys Leu Val Pro Arg Gln Leu Asp Tyr Lys Asp
            20                  25                  30

Asp Asp Asp Lys Glu Leu Asp Ile Val Met Thr Gln Ser His Lys Phe
        35                  40                  45

Met Ser Thr Ser Val Gly Asp Arg Val Ser Ile Thr Cys Lys Ala Ser
    50                  55                  60

Gln Asp Val Asp Thr Ala Val Ala Trp Tyr Gln Gln Lys Pro Gly Gln
65                  70                  75                  80

Ser Pro Lys Leu Leu Ile Tyr Trp Ala Ser Thr Arg His Thr Gly Val
                85                  90                  95

Pro Asp Arg Phe Thr Gly Ser Gly Ser Gly Thr Asp Tyr Thr Leu Thr
            100                 105                 110

Ile Ser Ser Val Gln Ala Glu Asp Leu Ala Arg Tyr Tyr Cys Gln Gln
        115                 120                 125

Tyr Tyr Ser Val Pro Pro Thr Phe Gly Gly Gly Thr Lys Leu Gly Ser
    130                 135                 140

Glu Ile Lys Arg Thr Val Ala Ala Pro Ser Val Phe Ile Phe Pro Pro
145                 150                 155                 160

Ser Asp Glu Gln Leu Lys Ser Gly Thr Ala Ser Val Val Cys Leu Leu
                165                 170                 175

Asn Asn Phe Tyr Pro Arg Glu Ala Lys Val Gln Trp Lys Val Asp Asn
            180                 185                 190

Ala Leu Gln Ser Gly Asn Ser Gln Glu Ser Val Thr Glu Gln Asp Ser
        195                 200                 205

Lys Asp Ser Thr Tyr Ser Leu Ser Ser Thr Leu Thr Leu Ser Lys Ala
    210                 215                 220

Asp Tyr Glu Lys His Lys Val Tyr Ala Cys Glu Val Thr His Gln Gly
225                 230                 235                 240

Leu Ser Ser Pro Val Thr Lys Ser Phe Asn Arg Gly Glu Cys Leu Glu
                245                 250                 255

Gln Val Gln Leu Leu Gln Ser Gly Pro Glu Leu Val Lys Pro Gly Ala
            260                 265                 270

Ser Val Lys Leu Ser Cys Lys Ala Ser Gly Tyr Ser Phe Thr Ser Tyr
        275                 280                 285

Asp Ile Asn Trp Val Lys Gln Arg Pro Gly Gln Gly Leu Glu Trp Val
    290                 295                 300

Gly Trp Ile Tyr Pro Arg Asp Gly Asp Thr Lys Tyr Asn Glu Lys Phe
305                 310                 315                 320

Lys Gly Lys Ala Ile Leu Thr Val Asp Thr Ser Ser Asn Thr Ala Tyr
                325                 330                 335

Met Asn Leu His Ser Leu Thr Ser Glu Asp Ser Ala Val Tyr Phe Cys
            340                 345                 350

Ala Arg Leu Thr Gly Pro Tyr Trp Tyr Phe Asp Val Trp Gly Thr Gly
        355                 360                 365

Thr Thr Val Thr Val Ser Ser Arg Ser Ser Thr Lys Gly Pro Lys Leu

370                 375                 380

Glu Glu Gly Glu Phe Ser Glu Ala Gln Leu Asp Ile Val Met Thr Gln
385                 390                 395                 400

Ser His Lys Phe Met Ser Thr Ser Val Gly Asp Arg Val Ser Ile Thr
                405                 410                 415

Cys Lys Ala Ser Gln Asp Val Asp Thr Ala Val Ala Trp Tyr Gln Gln
            420                 425                 430

Lys Pro Gly Gln Ser Pro Lys Leu Leu Ile Tyr Trp Ala Ser Thr Arg
        435                 440                 445

His Thr Gly Val Pro Asp Arg Phe Thr Gly Ser Gly Ser Gly Thr Asp
    450                 455                 460

Tyr Thr Leu Thr Ile Ser Ser Val Gln Ala Glu Asp Leu Ala Arg Tyr
465                 470                 475                 480

Tyr Cys Gln Gln Tyr Tyr Ser Val Pro Pro Thr Phe Gly Gly Gly Thr
                485                 490                 495

Lys Leu Glu Ile Lys
            500

<210>  6
<211>  395
<212>  PRT
<213>  artificial sequence

<220>
<223>  pDualyx-022 LC

<400>  6

Met Asn Phe Gly Phe Ser Leu Ile Phe Leu Val Leu Val Leu Lys Gly
1               5                   10                  15

Val Gln Cys Glu Val Lys Leu Val Pro Arg Gln Leu Asp Tyr Lys Asp
            20                  25                  30

Asp Asp Asp Lys Glu Phe Gln Val Gln Leu Leu Gln Ser Gly Pro Glu
        35                  40                  45

Leu Val Lys Pro Gly Ala Ser Val Lys Leu Ser Cys Lys Ala Ser Gly
    50                  55                  60

Tyr Ser Phe Thr Ser Tyr Asp Ile Asn Trp Val Lys Gln Arg Pro Gly
65                  70                  75                  80

Gln Gly Leu Glu Trp Val Gly Trp Ile Tyr Pro Arg Asp Gly Asp Thr
                85                  90                  95

Lys Tyr Asn Glu Lys Phe Lys Gly Lys Ala Ile Leu Thr Val Asp Thr
            100                 105                 110

Ser Ser Asn Thr Ala Tyr Met Asn Leu His Ser Leu Thr Ser Glu Asp
        115                 120                 125

Ser Ala Val Tyr Phe Cys Ala Arg Leu Thr Gly Pro Tyr Trp Tyr Phe
    130                 135                 140

Asp Val Trp Gly Thr Gly Thr Thr Val Thr Val Ser Ser Arg Ser Ser
145                 150                 155                 160

Thr Lys Gly Pro Lys Leu Glu Glu Gly Glu Phe Ser Glu Ala Gln Leu
                165                 170                 175

Asp Ile Val Met Thr Gln Ser His Lys Phe Met Ser Thr Ser Val Gly
            180                 185                 190

Asp Arg Val Ser Ile Thr Cys Lys Ala Ser Gln Asp Val Asp Thr Ala
        195                 200                 205

Val Ala Trp Tyr Gln Gln Lys Pro Gly Gln Ser Pro Lys Leu Leu Ile
    210                 215                 220

Tyr Trp Ala Ser Thr Arg His Thr Gly Val Pro Asp Arg Phe Thr Gly
225                 230                 235                 240

Ser Gly Ser Gly Thr Asp Tyr Thr Leu Thr Ile Ser Ser Val Gln Ala
                245                 250                 255

Glu Asp Leu Ala Arg Tyr Tyr Cys Gln Gln Tyr Tyr Ser Val Pro Pro
            260                 265                 270

Thr Phe Gly Gly Gly Thr Lys Leu Glu Ile Lys Gly Ser Glu Ile Lys
        275                 280                 285

Arg Thr Val Ala Ala Pro Ser Val Phe Ile Phe Pro Pro Ser Asp Glu
    290                 295                 300

Gln Leu Lys Ser Gly Thr Ala Ser Val Val Cys Leu Leu Asn Asn Phe
305                 310                 315                 320

Tyr Pro Arg Glu Ala Lys Val Gln Trp Lys Val Asp Asn Ala Leu Gln

325                 330                 335

Ser Gly Asn Ser Gln Glu Ser Val Thr Glu Gln Asp Ser Lys Asp Ser
            340                 345                 350

Thr Tyr Ser Leu Ser Ser Thr Leu Thr Leu Ser Lys Ala Asp Tyr Glu
        355                 360                 365

Lys His Lys Val Tyr Ala Cys Glu Val Thr His Gln Gly Leu Ser Ser
    370                 375                 380

Pro Val Thr Lys Ser Phe Asn Arg Gly Glu Cys
385                 390                 395

<210>  7
<211>  642
<212>  PRT
<213>  artificial sequence

<220>
<223>  pDualyx-023 LC

<400>  7

Met Asn Phe Gly Phe Ser Leu Ile Phe Leu Val Leu Val Leu Lys Gly
1               5                   10                  15

Val Gln Cys Glu Val Lys Leu Val Pro Arg Gln Leu Asp Tyr Lys Asp
            20                  25                  30

Asp Asp Asp Lys Glu Phe Gln Val Gln Leu Leu Gln Ser Gly Pro Glu
        35                  40                  45

Leu Val Lys Pro Gly Ala Ser Val Lys Leu Ser Cys Lys Ala Ser Gly
    50                  55                  60

Tyr Ser Phe Thr Ser Tyr Asp Ile Asn Trp Val Lys Gln Arg Pro Gly
65                  70                  75                  80

Gln Gly Leu Glu Trp Val Gly Trp Ile Tyr Pro Arg Asp Gly Asp Thr
                85                  90                  95

Lys Tyr Asn Glu Lys Phe Lys Gly Lys Ala Ile Leu Thr Val Asp Thr
            100                 105                 110

Ser Ser Asn Thr Ala Tyr Met Asn Leu His Ser Leu Thr Ser Glu Asp
        115                 120                 125

Ser Ala Val Tyr Phe Cys Ala Arg Leu Thr Gly Pro Tyr Trp Tyr Phe
    130                 135                 140

Asp Val Trp Gly Thr Gly Thr Thr Val Thr Val Ser Ser Arg Ser Ser
145                 150                 155                 160

Thr Lys Gly Pro Lys Leu Glu Glu Gly Glu Phe Ser Glu Ala Gln Leu
                165                 170                 175

Asp Ile Val Met Thr Gln Ser His Lys Phe Met Ser Thr Ser Val Gly
            180                 185                 190

Asp Arg Val Ser Ile Thr Cys Lys Ala Ser Gln Asp Val Asp Thr Ala
        195                 200                 205

Val Ala Trp Tyr Gln Gln Lys Pro Gly Gln Ser Pro Lys Leu Leu Ile
    210                 215                 220

Tyr Trp Ala Ser Thr Arg His Thr Gly Val Pro Asp Arg Phe Thr Gly
225                 230                 235                 240

Ser Gly Ser Gly Thr Asp Tyr Thr Leu Thr Ile Ser Ser Val Gln Ala
                245                 250                 255

Glu Asp Leu Ala Arg Tyr Tyr Cys Gln Gln Tyr Tyr Ser Val Pro Pro
            260                 265                 270

Thr Phe Gly Gly Gly Thr Lys Leu Glu Ile Lys Gly Ser Glu Ile Lys
        275                 280                 285

Arg Thr Val Ala Ala Pro Ser Val Phe Ile Phe Pro Pro Ser Asp Glu
    290                 295                 300

Gln Leu Lys Ser Gly Thr Ala Ser Val Val Cys Leu Leu Asn Asn Phe
305                 310                 315                 320

Tyr Pro Arg Glu Ala Lys Val Gln Trp Lys Val Asp Asn Ala Leu Gln
                325                 330                 335

Ser Gly Asn Ser Gln Glu Ser Val Thr Glu Gln Asp Ser Lys Asp Ser
            340                 345                 350

Thr Tyr Ser Leu Ser Ser Thr Leu Thr Leu Ser Lys Ala Asp Tyr Glu
        355                 360                 365

Lys His Lys Val Tyr Ala Cys Glu Val Thr His Gln Gly Leu Ser Ser
    370                 375                 380

Pro Val Thr Lys Ser Phe Asn Arg Gly Glu Cys Leu Glu Gln Val Gln

385                 390                 395                 400

Leu Leu Gln Ser Gly Pro Glu Leu Val Lys Pro Gly Ala Ser Val Lys
                405                 410                 415

Leu Ser Cys Lys Ala Ser Gly Tyr Ser Phe Thr Ser Tyr Asp Ile Asn
            420                 425                 430

Trp Val Lys Gln Arg Pro Gly Gln Gly Leu Glu Trp Val Gly Trp Ile
        435                 440                 445

Tyr Pro Arg Asp Gly Asp Thr Lys Tyr Asn Glu Lys Phe Lys Gly Lys
    450                 455                 460

Ala Ile Leu Thr Val Asp Thr Ser Ser Asn Thr Ala Tyr Met Asn Leu
465                 470                 475                 480

His Ser Leu Thr Ser Glu Asp Ser Ala Val Tyr Phe Cys Ala Arg Leu
                485                 490                 495

Thr Gly Pro Tyr Trp Tyr Phe Asp Val Trp Gly Thr Gly Thr Thr Val
            500                 505                 510

Thr Val Ser Ser Arg Ser Ser Thr Lys Gly Pro Lys Leu Glu Glu Gly
        515                 520                 525

Glu Phe Ser Glu Ala Gln Leu Asp Ile Val Met Thr Gln Ser His Lys
    530                 535                 540

Phe Met Ser Thr Ser Val Gly Asp Arg Val Ser Ile Thr Cys Lys Ala
545                 550                 555                 560

Ser Gln Asp Val Asp Thr Ala Val Ala Trp Tyr Gln Gln Lys Pro Gly
                565                 570                 575

Gln Ser Pro Lys Leu Leu Ile Tyr Trp Ala Ser Thr Arg His Thr Gly
            580                 585                 590

Val Pro Asp Arg Phe Thr Gly Ser Gly Ser Gly Thr Asp Tyr Thr Leu
        595                 600                 605

Thr Ile Ser Ser Val Gln Ala Glu Asp Leu Ala Arg Tyr Tyr Cys Gln
    610                 615                 620

Gln Tyr Tyr Ser Val Pro Pro Thr Phe Gly Gly Gly Thr Lys Leu Glu
625                 630                 635                 640

Ile Lys

<210> 8
<211> 256
<212> PRT
<213> artificial sequence

<220>
<223> pDualyx-005 LC

<400> 8

Met Asn Phe Gly Phe Ser Leu Ile Phe Leu Val Leu Val Leu Lys Gly
1               5                   10                  15

Val Gln Cys Glu Val Lys Leu Val Pro Arg Gln Leu Asp Tyr Lys Asp
            20                  25                  30

Asp Asp Asp Lys Glu Leu Asp Ile Val Met Ala Gln Ser Gln Lys Phe
        35                  40                  45

Met Ser Val Ser Val Gly Asp Arg Val Ser Val Thr Cys Lys Ala Ser
    50                  55                  60

Gln Asn Val Gly Thr Asn Val Ala Trp Tyr Gln Gln Arg Pro Gly Gln
65                  70                  75                  80

Ser Pro Lys Ala Leu Ile Tyr Ser Ala Ser Tyr Arg Tyr Ser Gly Val
                85                  90                  95

Pro Asp Arg Phe Thr Gly Ser Gly Ser Gly Thr Asp Phe Thr Leu Thr
            100                 105                 110

Ile Ser Asn Val Gln Ser Glu Asp Leu Ala Glu Tyr Phe Cys Gln Gln
        115                 120                 125

Phe Asp Ser His Pro Leu Thr Phe Gly Ala Gly Thr Lys Leu Glu Leu
    130                 135                 140

Lys Gly Ser Ile Lys Arg Ala Asp Ala Ala Pro Thr Val Ser Ile Phe
145                 150                 155                 160

Pro Pro Ser Ser Glu Gln Leu Thr Ser Gly Gly Ala Ser Val Val Cys
                165                 170                 175

Phe Leu Asn Asn Phe Tyr Pro Lys Asp Ile Asn Val Lys Trp Lys Ile
            180                 185                 190

Asp Gly Ser Glu Arg Gln Asn Gly Val Leu Asn Ser Trp Thr Asp Gln

195                 200                 205

Asp Ser Lys Asp Ser Thr Tyr Ser Met Ser Ser Thr Leu Thr Leu Thr
    210                 215                 220

Lys Asp Glu Tyr Glu Arg His Asn Ser Tyr Thr Cys Glu Ala Thr His
225                 230                 235                 240

Lys Thr Ser Thr Ser Pro Ile Val Lys Ser Phe Asn Arg Asn Glu Cys
                245                 250                 255

<210>  9
<211>  417
<212>  PRT
<213>  artificial sequence

<220>
<223>  pDualyx-032 LC

<400>  9

Met Asn Phe Gly Phe Ser Leu Ile Phe Leu Val Leu Val Leu Lys Gly
1               5                   10                  15

Val Gln Cys Glu Val Lys Leu Val Pro Arg Gln Leu Asp Ile Val Met
            20                  25                  30

Ala Gln Ser Gln Lys Phe Met Ser Val Ser Val Gly Asp Arg Val Ser
        35                  40                  45

Val Thr Cys Lys Ala Ser Gln Asn Val Gly Thr Asn Val Ala Trp Tyr
    50                  55                  60

Gln Gln Arg Pro Gly Gln Ser Pro Lys Ala Leu Ile Tyr Ser Ala Ser
65                  70                  75                  80

Tyr Arg Tyr Ser Gly Val Pro Asp Arg Phe Thr Gly Ser Gly Ser Gly
                85                  90                  95

Thr Asp Phe Thr Leu Thr Ile Ser Asn Val Gln Ser Glu Asp Leu Ala
            100                 105                 110

Glu Tyr Phe Cys Gln Gln Phe Asp Ser His Pro Leu Thr Phe Gly Ala
        115                 120                 125

Gly Thr Lys Leu Glu Leu Lys Gly Ser Glu Ile Lys Arg Thr Val Ala
    130                 135                 140

Ala Pro Ser Val Phe Ile Phe Pro Pro Ser Asp Glu Gln Leu Lys Ser
145                 150                 155                 160

Gly Thr Ala Ser Val Val Cys Leu Leu Asn Asn Phe Tyr Pro Arg Glu
                165                 170                 175

Ala Lys Val Gln Trp Lys Val Asp Asn Ala Leu Gln Ser Gly Asn Ser
            180                 185                 190

Gln Glu Ser Val Thr Glu Gln Asp Ser Lys Asp Ser Thr Tyr Ser Leu
        195                 200                 205

Ser Ser Thr Leu Thr Leu Ser Lys Ala Asp Tyr Glu Lys His Lys Val
    210                 215                 220

Tyr Ala Cys Glu Val Thr His Gln Gly Leu Ser Ser Pro Val Thr Lys
225                 230                 235                 240

Ser Phe Asn Arg Gly Glu Cys Leu Glu Lys Pro Thr Glu Asn Asn Glu
                245                 250                 255

Asp Phe Asn Ile Val Ala Val Ala Ser Asn Phe Ala Thr Thr Asp Leu
            260                 265                 270

Asp Ala Asp Arg Gly Lys Leu Pro Gly Lys Lys Leu Pro Leu Glu Val
        275                 280                 285

Leu Lys Glu Met Glu Ala Asn Ala Arg Lys Ala Gly Cys Thr Arg Gly
    290                 295                 300

Cys Leu Ile Cys Leu Ser His Ile Lys Cys Thr Pro Lys Met Lys Lys
305                 310                 315                 320

Phe Ile Pro Gly Arg Cys His Thr Tyr Glu Gly Asp Lys Glu Ser Ala
                325                 330                 335

Gln Gly Gly Ile Gly Glu Ala Ile Val Asp Ile Pro Glu Ile Pro Gly
            340                 345                 350

Phe Lys Asp Leu Glu Pro Met Glu Gln Phe Ile Ala Gln Val Asp Leu
        355                 360                 365

Cys Val Asp Cys Thr Thr Gly Cys Leu Lys Gly Leu Ala Asn Val Gln
    370                 375                 380

Cys Ser Asp Leu Leu Lys Lys Trp Leu Pro Gln Arg Cys Ala Thr Phe
385                 390                 395                 400

Ala Ser Lys Ile Gln Gly Gln Val Asp Lys Ile Lys Gly Ala Gly Gly

405                 410                 415

Asp

<210>  10
<211>  257
<212>  PRT
<213>  artificial sequence

<220>
<223>  pDualyx-092 LC

<400>  10

Met Asn Phe Gly Phe Ser Leu Ile Phe Leu Val Leu Val Leu Lys Gly
1               5                   10                  15

Val Gln Cys Glu Val Lys Leu Val Pro Arg Gln Leu Asp Tyr Lys Asp
            20                  25                  30

Asp Asp Asp Lys Glu Leu Asn Ile Val Met Thr Gln Ser Pro Lys Ser
        35                  40                  45

Leu Ser Met Ser Val Gly Glu Arg Val Thr Leu Asn Cys Lys Ala Ser
    50                  55                  60

Glu Asn Val Lys Thr Tyr Val Ser Trp Tyr Gln Gln Lys Pro Glu Gln
65                  70                  75                  80

Ser Pro Lys Leu Leu Ile Tyr Gly Ala Ser Asn Arg Tyr Thr Gly Val
                85                  90                  95

Pro Asp Arg Phe Thr Gly Ser Gly Ser Ala Thr Asp Phe Thr Leu Thr
            100                 105                 110

Ile Ser Ser Val Gln Ala Glu Asp Leu Ala Asp Tyr His Cys Gly Gln
        115                 120                 125

Ser Tyr Ser Tyr Pro Phe Thr Phe Gly Ala Gly Thr Lys Leu Glu Arg
    130                 135                 140

Lys Gly Ser Glu Ile Lys Arg Thr Val Ala Ala Pro Ser Val Phe Ile
145                 150                 155                 160

Phe Pro Pro Ser Asp Glu Gln Leu Lys Ser Gly Thr Ala Ser Val Val
                165                 170                 175

Cys Leu Leu Asn Asn Phe Tyr Pro Arg Glu Ala Lys Val Gln Trp Lys
            180                 185                 190

Val Asp Asn Ala Leu Gln Ser Gly Asn Ser Gln Glu Ser Val Thr Glu
        195                 200                 205

Gln Asp Ser Lys Asp Ser Thr Tyr Ser Leu Ser Ser Thr Leu Thr Leu
    210                 215                 220

Ser Lys Ala Asp Tyr Glu Lys His Lys Val Tyr Ala Cys Glu Val Thr
225                 230                 235                 240

His Gln Gly Leu Ser Ser Pro Val Thr Lys Ser Phe Asn Arg Gly Glu
                245                 250                 255

Cys

<210>  11
<211>  427
<212>  PRT
<213>  artificial sequence

<220>
<223>  pDualyx-093 LC

<400>  11

Met Asn Phe Gly Phe Ser Leu Ile Phe Leu Val Leu Val Leu Lys Gly
1               5                   10                  15

Val Gln Cys Glu Val Lys Leu Val Pro Arg Gln Leu Asp Tyr Lys Asp
            20                  25                  30

Asp Asp Asp Lys Glu Leu Asn Ile Val Met Thr Gln Ser Pro Lys Ser
        35                  40                  45

Leu Ser Met Ser Val Gly Glu Arg Val Thr Leu Asn Cys Lys Ala Ser
    50                  55                  60

Glu Asn Val Lys Thr Tyr Val Ser Trp Tyr Gln Gln Lys Pro Glu Gln
65                  70                  75                  80

Ser Pro Lys Leu Leu Ile Tyr Gly Ala Ser Asn Arg Tyr Thr Gly Val
                85                  90                  95

Pro Asp Arg Phe Thr Gly Ser Gly Ser Ala Thr Asp Phe Thr Leu Thr
            100                 105                 110

Ile Ser Ser Val Gln Ala Glu Asp Leu Ala Asp Tyr His Cys Gly Gln
        115                 120                 125

Ser Tyr Ser Tyr Pro Phe Thr Phe Gly Ala Gly Thr Lys Leu Glu Arg
    130                 135                 140

Lys Gly Ser Glu Ile Lys Arg Thr Val Ala Ala Pro Ser Val Phe Ile
145                 150                 155                 160

Phe Pro Pro Ser Asp Glu Gln Leu Lys Ser Gly Thr Ala Ser Val Val
                165                 170                 175

Cys Leu Leu Asn Asn Phe Tyr Pro Arg Glu Ala Lys Val Gln Trp Lys
            180                 185                 190

Val Asp Asn Ala Leu Gln Ser Gly Asn Ser Gln Glu Ser Val Thr Glu
        195                 200                 205

Gln Asp Ser Lys Asp Ser Thr Tyr Ser Leu Ser Ser Thr Leu Thr Leu
    210                 215                 220

Ser Lys Ala Asp Tyr Glu Lys His Lys Val Tyr Ala Cys Glu Val Thr
225                 230                 235                 240

His Gln Gly Leu Ser Ser Pro Val Thr Lys Ser Phe Asn Arg Gly Glu
                245                 250                 255

Cys Leu Glu Lys Pro Thr Glu Asn Asn Glu Asp Phe Asn Ile Val Ala
            260                 265                 270

Val Ala Ser Asn Phe Ala Thr Thr Asp Leu Asp Ala Asp Arg Gly Lys
        275                 280                 285

Leu Pro Gly Lys Lys Leu Pro Leu Glu Val Leu Lys Glu Met Glu Ala
    290                 295                 300

Asn Ala Arg Lys Ala Gly Cys Thr Arg Gly Cys Leu Ile Cys Leu Ser
305                 310                 315                 320

His Ile Lys Cys Thr Pro Lys Met Lys Lys Phe Ile Pro Gly Arg Cys
                325                 330                 335

His Thr Tyr Glu Gly Asp Lys Glu Ser Ala Gln Gly Gly Ile Gly Glu
            340                 345                 350

Ala Ile Val Asp Ile Pro Glu Ile Pro Gly Phe Lys Asp Leu Glu Pro
        355                 360                 365

Met Glu Gln Phe Ile Ala Gln Val Asp Leu Cys Val Asp Cys Thr Thr
    370                 375                 380

Gly Cys Leu Lys Gly Leu Ala Asn Val Gln Cys Ser Asp Leu Leu Lys
385                 390                 395                 400

Lys Trp Leu Pro Gln Arg Cys Ala Thr Phe Ala Ser Lys Ile Gln Gly
                405                 410                 415

Gln Val Asp Lys Ile Lys Gly Ala Gly Gly Asp
            420                 425

<210>  12
<211>  256
<212>  PRT
<213>  artificial sequence

<220>
<223>  pDualyx-077 LC

<400>  12

Met Asn Phe Gly Phe Ser Leu Ile Phe Leu Val Leu Val Leu Lys Gly
1               5                   10                  15

Val Gln Cys Glu Val Lys Leu Val Pro Arg Gln Leu Asp Tyr Lys Asp
            20                  25                  30

Asp Asp Asp Lys Glu Leu Asp Ile Gln Met Thr Gln Ser Pro Ser Ser
        35                  40                  45

Leu Ser Ala Ser Leu Gly Gly Lys Val Thr Ile Thr Cys Lys Ala Ser
    50                  55                  60

Gln Asp Ile Asn Lys Phe Ile Ala Trp Tyr Gln His Lys Pro Gly Lys
65                  70                  75                  80

Gly Pro Arg Leu Leu Ile His Tyr Thr Ser Thr Leu Gln Pro Gly Ile
                85                  90                  95

Pro Ser Arg Phe Ser Gly Ser Gly Ser Gly Arg Asp Tyr Ser Phe Ser
            100                 105                 110

Ile Ser Asn Leu Glu Pro Glu Asp Ile Ala Thr Tyr Tyr Cys Leu Gln
        115                 120                 125

Tyr Asp Asn Leu Tyr Thr Phe Gly Gly Gly Thr Lys Leu Glu Ile Lys
    130                 135                 140

Gly Ser Glu Ile Lys Arg Thr Val Ala Ala Pro Ser Val Phe Ile Phe
145                 150                 155                 160

Pro Pro Ser Asp Glu Gln Leu Lys Ser Gly Thr Ala Ser Val Val Cys
                165                 170                 175

Leu Leu Asn Asn Phe Tyr Pro Arg Glu Ala Lys Val Gln Trp Lys Val
            180                 185                 190

Asp Asn Ala Leu Gln Ser Gly Asn Ser Gln Glu Ser Val Thr Glu Gln
        195                 200                 205

Asp Ser Lys Asp Ser Thr Tyr Ser Leu Ser Ser Thr Leu Thr Leu Ser
    210                 215                 220

Lys Ala Asp Tyr Glu Lys His Lys Val Tyr Ala Cys Glu Val Thr His
225                 230                 235                 240

Gln Gly Leu Ser Ser Pro Val Thr Lys Ser Phe Asn Arg Gly Glu Cys
                245                 250                 255

<210>  13
<211>  426
<212>  PRT
<213>  artificial sequence

<220>
<223>  pDualyx-076 LC

<400>  13

Met Asn Phe Gly Phe Ser Leu Ile Phe Leu Val Leu Val Leu Lys Gly
1               5                   10                  15

Val Gln Cys Glu Val Lys Leu Val Pro Arg Gln Leu Asp Tyr Lys Asp
            20                  25                  30

Asp Asp Asp Lys Glu Leu Asp Ile Gln Met Thr Gln Ser Pro Ser Ser
        35                  40                  45

Leu Ser Ala Ser Leu Gly Gly Lys Val Thr Ile Thr Cys Lys Ala Ser
    50                  55                  60

Gln Asp Ile Asn Lys Phe Ile Ala Trp Tyr Gln His Lys Pro Gly Lys
65                  70                  75                  80

Gly Pro Arg Leu Leu Ile His Tyr Thr Ser Thr Leu Gln Pro Gly Ile
                85                  90                  95

Pro Ser Arg Phe Ser Gly Ser Gly Ser Gly Arg Asp Tyr Ser Phe Ser
            100                 105                 110

Ile Ser Asn Leu Glu Pro Glu Asp Ile Ala Thr Tyr Tyr Cys Leu Gln
        115                 120                 125

Tyr Asp Asn Leu Tyr Thr Phe Gly Gly Gly Thr Lys Leu Glu Ile Lys
    130                 135                 140

Gly Ser Glu Ile Lys Arg Thr Val Ala Ala Pro Ser Val Phe Ile Phe
145                 150                 155                 160

Pro Pro Ser Asp Glu Gln Leu Lys Ser Gly Thr Ala Ser Val Val Cys
                165                 170                 175

Leu Leu Asn Asn Phe Tyr Pro Arg Glu Ala Lys Val Gln Trp Lys Val
            180                 185                 190

Asp Asn Ala Leu Gln Ser Gly Asn Ser Gln Glu Ser Val Thr Glu Gln
        195                 200                 205

Asp Ser Lys Asp Ser Thr Tyr Ser Leu Ser Ser Thr Leu Thr Leu Ser
    210                 215                 220

Lys Ala Asp Tyr Glu Lys His Lys Val Tyr Ala Cys Glu Val Thr His
225                 230                 235                 240

Gln Gly Leu Ser Ser Pro Val Thr Lys Ser Phe Asn Arg Gly Glu Cys
                245                 250                 255

Leu Glu Lys Pro Thr Glu Asn Asn Glu Asp Phe Asn Ile Val Ala Val
            260                 265                 270

Ala Ser Asn Phe Ala Thr Thr Asp Leu Asp Ala Asp Arg Gly Lys Leu
        275                 280                 285

Pro Gly Lys Lys Leu Pro Leu Glu Val Leu Lys Glu Met Glu Ala Asn
    290                 295                 300

Ala Arg Lys Ala Gly Cys Thr Arg Gly Cys Leu Ile Cys Leu Ser His
305                 310                 315                 320

Ile Lys Cys Thr Pro Lys Met Lys Lys Phe Ile Pro Gly Arg Cys His
                325                 330                 335

Thr Tyr Glu Gly Asp Lys Glu Ser Ala Gln Gly Gly Ile Gly Glu Ala
            340                 345                 350

Ile Val Asp Ile Pro Glu Ile Pro Gly Phe Lys Asp Leu Glu Pro Met
        355                 360                 365

Glu Gln Phe Ile Ala Gln Val Asp Leu Cys Val Asp Cys Thr Thr Gly
    370                 375                 380

Cys Leu Lys Gly Leu Ala Asn Val Gln Cys Ser Asp Leu Leu Lys Lys
385                 390                 395                 400

Trp Leu Pro Gln Arg Cys Ala Thr Phe Ala Ser Lys Ile Gln Gly Gln
                405                 410                 415

Val Asp Lys Ile Lys Gly Ala Gly Gly Asp
            420                 425

<210>  14
<211>  257
<212>  PRT
<213>  artificial sequence

<220>
<223>  pDualyx-016pre LC

<400>  14

Met Asn Phe Gly Phe Ser Leu Ile Phe Leu Val Leu Val Leu Lys Gly
1               5                   10                  15

Val Gln Cys Glu Val Lys Leu Val Pro Arg Gln Leu Asp Tyr Lys Asp
            20                  25                  30

Asp Asp Asp Lys Glu Leu Asp Ile Val Met Ala Gln Ser Gln Lys Phe
        35                  40                  45

Met Ser Val Ser Val Gly Asp Arg Val Ser Val Thr Cys Lys Ala Ser
    50                  55                  60

Gln Asn Val Gly Thr Asn Val Ala Trp Tyr Gln Gln Arg Pro Gly Gln
65                  70                  75                  80

Ser Pro Lys Ala Leu Ile Tyr Ser Ala Ser Tyr Arg Tyr Ser Gly Val
                85                  90                  95

Pro Asp Arg Phe Thr Gly Ser Gly Ser Gly Thr Asp Phe Thr Leu Thr
            100                 105                 110

Ile Ser Asn Val Gln Ser Glu Asp Leu Ala Glu Tyr Phe Cys Gln Gln
        115                 120                 125

Phe Asp Ser His Pro Leu Thr Phe Gly Ala Gly Thr Lys Leu Glu Leu
    130                 135                 140

Lys Gly Ser Glu Ile Lys Arg Thr Val Ala Ala Pro Ser Val Phe Ile
145                 150                 155                 160

Phe Pro Pro Ser Asp Glu Gln Leu Lys Ser Gly Thr Ala Ser Val Val
                165                 170                 175

Cys Leu Leu Asn Asn Phe Tyr Pro Arg Glu Ala Lys Val Gln Trp Lys
            180                 185                 190

Val Asp Asn Ala Leu Gln Ser Gly Asn Ser Gln Glu Ser Val Thr Glu
        195                 200                 205

Gln Asp Ser Lys Asp Ser Thr Tyr Ser Leu Ser Ser Thr Leu Thr Leu
    210                 215                 220

Ser Lys Ala Asp Tyr Glu Lys His Lys Val Tyr Ala Cys Glu Val Thr
225                 230                 235                 240

His Gln Gly Leu Ser Ser Pro Val Thr Lys Ser Phe Asn Arg Gly Glu
                245                 250                 255

Cys

<210>  15
<211>  420
<212>  PRT
<213>  artificial sequence

<220>
<223>  pDualyx-028 LC

<400>  15

Met Asn Phe Gly Phe Ser Leu Ile Phe Leu Val Leu Val Leu Lys Gly
1               5                   10                  15

Val Gln Cys Glu Val Lys Leu Val Pro Arg Gln Leu Gln Ala Val Val
            20                  25                  30

Thr Gln Glu Ser Ala Leu Thr Thr Ser Pro Gly Glu Thr Val Thr Leu
        35                  40                  45

Thr Cys Arg Ser Ser Thr Gly Ala Val Thr Thr Ser Asn Tyr Ala Asn
    50                  55                  60

Trp Ile Gln Glu Lys Pro Asp His Leu Phe Thr Gly Leu Ile Gly Gly
65                  70                  75                  80

Thr Asn Asn Arg Thr Pro Gly Val Pro Ala Arg Phe Ser Gly Ser Leu

                85                  90                  95

Ile Gly Asp Lys Ala Ala Leu Thr Val Thr Gly Ala Gln Thr Glu Asp
            100                 105                 110

Glu Ala Val Tyr Phe Cys Ala Leu Trp Tyr Ser Asn His Trp Val Phe
        115                 120                 125

Gly Gly Gly Thr Lys Leu Thr Val Leu Gly Arg Ser Glu Ile Lys Arg
    130                 135                 140

Thr Val Ala Ala Pro Ser Val Phe Ile Phe Pro Pro Ser Asp Glu Gln
145                 150                 155                 160

Leu Lys Ser Gly Thr Ala Ser Val Val Cys Leu Leu Asn Asn Phe Tyr
                165                 170                 175

Pro Arg Glu Ala Lys Val Gln Trp Lys Val Asp Asn Ala Leu Gln Ser
            180                 185                 190

Gly Asn Ser Gln Glu Ser Val Thr Glu Gln Asp Ser Lys Asp Ser Thr
        195                 200                 205

Tyr Ser Leu Ser Ser Thr Leu Thr Leu Ser Lys Ala Asp Tyr Glu Lys
    210                 215                 220

His Lys Val Tyr Ala Cys Glu Val Thr His Gln Gly Leu Ser Ser Pro
225                 230                 235                 240

Val Thr Lys Ser Phe Asn Arg Gly Glu Cys Leu Glu Lys Pro Thr Glu
                245                 250                 255

Asn Asn Glu Asp Phe Asn Ile Val Ala Val Ala Ser Asn Phe Ala Thr
            260                 265                 270

Thr Asp Leu Asp Ala Asp Arg Gly Lys Leu Pro Gly Lys Lys Leu Pro
        275                 280                 285

Leu Glu Val Leu Lys Glu Met Glu Ala Asn Ala Arg Lys Ala Gly Cys
    290                 295                 300

Thr Arg Gly Cys Leu Ile Cys Leu Ser His Ile Lys Cys Thr Pro Lys
305                 310                 315                 320

Met Lys Lys Phe Ile Pro Gly Arg Cys His Thr Tyr Glu Gly Asp Lys
                325                 330                 335

Glu Ser Ala Gln Gly Gly Ile Gly Glu Ala Ile Val Asp Ile Pro Glu
            340                 345                 350

Ile Pro Gly Phe Lys Asp Leu Glu Pro Met Glu Gln Phe Ile Ala Gln
        355                 360                 365

Val Asp Leu Cys Val Asp Cys Thr Thr Gly Cys Leu Lys Gly Leu Ala
    370                 375                 380

Asn Val Gln Cys Ser Asp Leu Leu Lys Lys Trp Leu Pro Gln Arg Cys
385                 390                 395                 400

Ala Thr Phe Ala Ser Lys Ile Gln Gly Gln Val Asp Lys Ile Lys Gly
                405                 410                 415

Ala Gly Gly Asp
            420

<210>  16
<211>  426
<212>  PRT
<213>  artificial sequence

<220>
<223>  pDualyx-087 LC

<400>  16

Met Asn Phe Gly Phe Ser Leu Ile Phe Leu Val Leu Val Leu Lys Gly
1               5                   10                  15

Val Gln Cys Glu Val Lys Leu Val Pro Arg Gln Leu Asp Tyr Lys Asp
            20                  25                  30

Asp Asp Asp Lys Glu Leu Gln Ile Val Leu Thr Gln Ser Pro Ala Ile
        35                  40                  45

Met Ser Ala Ser Pro Gly Glu Lys Val Thr Ile Thr Cys Ser Ala Ser
    50                  55                  60

Ser Ser Val Ser Tyr Met His Trp Phe Gln Gln Lys Pro Gly Thr Ser
65                  70                  75                  80

Pro Lys Leu Trp Ile Tyr Ser Thr Ser Asn Leu Ala Ser Gly Val Pro
                85                  90                  95

Ala Arg Phe Ser Gly Ser Gly Ser Gly Thr Ser Tyr Ser Leu Thr Ile
            100                 105                 110

Ser Arg Met Glu Ala Glu Asp Ala Ala Thr Tyr Tyr Cys Gln Gln Arg

115                 120                 125

Ser Ser Tyr Pro Pro Thr Phe Gly Gly Gly Thr Lys Leu Glu Ile Lys
    130                 135                 140

Gly Ser Glu Ile Lys Arg Thr Val Ala Ala Pro Ser Val Phe Ile Phe
145                 150                 155                 160

Pro Pro Ser Asp Glu Gln Leu Lys Ser Gly Thr Ala Ser Val Val Cys
                165                 170                 175

Leu Leu Asn Asn Phe Tyr Pro Arg Glu Ala Lys Val Gln Trp Lys Val
            180                 185                 190

Asp Asn Ala Leu Gln Ser Gly Asn Ser Gln Glu Ser Val Thr Glu Gln
        195                 200                 205

Asp Ser Lys Asp Ser Thr Tyr Ser Leu Ser Ser Thr Leu Thr Leu Ser
    210                 215                 220

Lys Ala Asp Tyr Glu Lys His Lys Val Tyr Ala Cys Glu Val Thr His
225                 230                 235                 240

Gln Gly Leu Ser Ser Pro Val Thr Lys Ser Phe Asn Arg Gly Glu Cys
                245                 250                 255

Leu Glu Lys Pro Thr Glu Asn Asn Glu Asp Phe Asn Ile Val Ala Val
            260                 265                 270

Ala Ser Asn Phe Ala Thr Thr Asp Leu Asp Ala Asp Arg Gly Lys Leu
        275                 280                 285

Pro Gly Lys Lys Leu Pro Leu Glu Val Leu Lys Glu Met Glu Ala Asn
    290                 295                 300

Ala Arg Lys Ala Gly Cys Thr Arg Gly Cys Leu Ile Cys Leu Ser His
305                 310                 315                 320

Ile Lys Cys Thr Pro Lys Met Lys Lys Phe Ile Pro Gly Arg Cys His
                325                 330                 335

Thr Tyr Glu Gly Asp Lys Glu Ser Ala Gln Gly Gly Ile Gly Glu Ala
            340                 345                 350

Ile Val Asp Ile Pro Glu Ile Pro Gly Phe Lys Asp Leu Glu Pro Met
        355                 360                 365

Glu Gln Phe Ile Ala Gln Val Asp Leu Cys Val Asp Cys Thr Thr Gly
    370                 375                 380

Cys Leu Lys Gly Leu Ala Asn Val Gln Cys Ser Asp Leu Leu Lys Lys
385                 390                 395                 400

Trp Leu Pro Gln Arg Cys Ala Thr Phe Ala Ser Lys Ile Gln Gly Gln
                405                 410                 415

Val Asp Lys Ile Lys Gly Ala Gly Gly Asp
            420                 425

<210>  17
<211>  432
<212>  PRT
<213>  artificial sequence

<220>
<223>  pDualyx-090 LC

<400>  17

Met Asn Phe Gly Phe Ser Leu Ile Phe Leu Val Leu Val Leu Lys Gly
1               5                   10                  15

Val Gln Cys Glu Val Lys Leu Val Pro Arg Gln Leu Asp Tyr Lys Asp
            20                  25                  30

Asp Asp Asp Lys Glu Leu Asp Val Val Met Thr Gln Thr Pro Leu Ser
        35                  40                  45

Leu Pro Val Ser Leu Gly Asp Gln Ala Ser Ile Ser Cys Arg Ala Ser
    50                  55                  60

Gln Ser Leu Val His Ser Asn Gly Asn Thr Tyr Leu Gln Trp Tyr Leu
65                  70                  75                  80

Gln Lys Pro Gly Gln Ser Pro Lys Leu Leu Ile Tyr Lys Val Ser Asn
                85                  90                  95

Arg Phe Ser Gly Val Pro Asp Arg Phe Ser Gly Ser Gly Ser Gly Thr
            100                 105                 110

Asp Phe Thr Leu Lys Ile Ser Arg Val Glu Ala Glu Asp Leu Gly Val
        115                 120                 125

Phe Phe Cys Ser Gln Ser Thr His Val Pro Phe Thr Phe Gly Ser Gly
    130                 135                 140

Thr Lys Leu Glu Ile Lys Gly Ser Glu Ile Lys Arg Thr Val Ala Ala

145                 150                 155                 160

Pro Ser Val Phe Ile Phe Pro Pro Ser Asp Glu Gln Leu Lys Ser Gly
                165                 170                 175

Thr Ala Ser Val Val Cys Leu Leu Asn Asn Phe Tyr Pro Arg Glu Ala
            180                 185                 190

Lys Val Gln Trp Lys Val Asp Asn Ala Leu Gln Ser Gly Asn Ser Gln
        195                 200                 205

Glu Ser Val Thr Glu Gln Asp Ser Lys Asp Ser Thr Tyr Ser Leu Ser
    210                 215                 220

Ser Thr Leu Thr Leu Ser Lys Ala Asp Tyr Glu Lys His Lys Val Tyr
225                 230                 235                 240

Ala Cys Glu Val Thr His Gln Gly Leu Ser Ser Pro Val Thr Lys Ser
                245                 250                 255

Phe Asn Arg Gly Glu Cys Leu Glu Lys Pro Thr Glu Asn Asn Glu Asp
            260                 265                 270

Phe Asn Ile Val Ala Val Ala Ser Asn Phe Ala Thr Thr Asp Leu Asp
        275                 280                 285

Ala Asp Arg Gly Lys Leu Pro Gly Lys Lys Leu Pro Leu Glu Val Leu
    290                 295                 300

Lys Glu Met Glu Ala Asn Ala Arg Lys Ala Gly Cys Thr Arg Gly Cys
305                 310                 315                 320

Leu Ile Cys Leu Ser His Ile Lys Cys Thr Pro Lys Met Lys Lys Phe
                325                 330                 335

Ile Pro Gly Arg Cys His Thr Tyr Glu Gly Asp Lys Glu Ser Ala Gln
            340                 345                 350

Gly Gly Ile Gly Glu Ala Ile Val Asp Ile Pro Glu Ile Pro Gly Phe
        355                 360                 365

Lys Asp Leu Glu Pro Met Glu Gln Phe Ile Ala Gln Val Asp Leu Cys
    370                 375                 380

Val Asp Cys Thr Thr Gly Cys Leu Lys Gly Leu Ala Asn Val Gln Cys
385                 390                 395                 400

Ser Asp Leu Leu Lys Lys Trp Leu Pro Gln Arg Cys Ala Thr Phe Ala
                405                 410                 415

Ser Lys Ile Gln Gly Gln Val Asp Lys Ile Lys Gly Ala Gly Gly Asp
            420                 425                 430

<210>  18
<211>  430
<212>  PRT
<213>  artificial sequence

<220>
<223>  pDualyx-071 LC

<400>  18

Met Asn Phe Gly Phe Ser Leu Ile Phe Leu Val Leu Val Leu Lys Gly
1               5                   10                  15

Val Gln Cys Glu Val Lys Leu Val Pro Arg Gln Leu Asp Tyr Lys Asp
            20                  25                  30

Asp Asp Asp Lys Glu Leu Gln Ala Val Val Thr Gln Glu Ser Ala Leu
        35                  40                  45

Thr Thr Ser Pro Gly Glu Thr Val Thr Leu Thr Cys Arg Ser Ser Thr
    50                  55                  60

Gly Ala Val Thr Thr Ser Asn Tyr Ala Asn Trp Val Gln Glu Lys Pro
65                  70                  75                  80

Asp His Leu Phe Thr Gly Leu Ile Gly Gly Thr Asn Asn Arg Ala Pro
                85                  90                  95

Gly Val Pro Ala Arg Phe Ser Gly Ser Val Ile Gly Asp Lys Ala Ala
            100                 105                 110

Leu Thr Ile Thr Gly Ala Gln Thr Glu Asp Glu Ala Ile Tyr Phe Cys
        115                 120                 125

Ala Leu Trp Tyr Ser Asn His Trp Val Phe Gly Gly Gly Thr Lys Leu
    130                 135                 140

Thr Val Leu Gly Gly Ser Glu Ile Lys Arg Thr Val Ala Ala Pro Ser
145                 150                 155                 160

Val Phe Ile Phe Pro Pro Ser Asp Glu Gln Leu Lys Ser Gly Thr Ala
                165                 170                 175

Ser Val Val Cys Leu Leu Asn Asn Phe Tyr Pro Arg Glu Ala Lys Val

180                 185                 190

Gln Trp Lys Val Asp Asn Ala Leu Gln Ser Gly Asn Ser Gln Glu Ser
    195                 200                 205

Val Thr Glu Gln Asp Ser Lys Asp Ser Thr Tyr Ser Leu Ser Ser Thr
    210                 215                 220

Leu Thr Leu Ser Lys Ala Asp Tyr Glu Lys His Lys Val Tyr Ala Cys
225                 230                 235                 240

Glu Val Thr His Gln Gly Leu Ser Ser Pro Val Thr Lys Ser Phe Asn
                245                 250                 255

Arg Gly Glu Cys Leu Glu Lys Pro Thr Glu Asn Asn Glu Asp Phe Asn
            260                 265                 270

Ile Val Ala Val Ala Ser Asn Phe Ala Thr Thr Asp Leu Asp Ala Asp
        275                 280                 285

Arg Gly Lys Leu Pro Gly Lys Lys Leu Pro Leu Glu Val Leu Lys Glu
    290                 295                 300

Met Glu Ala Asn Ala Arg Lys Ala Gly Cys Thr Arg Gly Cys Leu Ile
305                 310                 315                 320

Cys Leu Ser His Ile Lys Cys Thr Pro Lys Met Lys Lys Phe Ile Pro
                325                 330                 335

Gly Arg Cys His Thr Tyr Glu Gly Asp Lys Glu Ser Ala Gln Gly Gly
            340                 345                 350

Ile Gly Glu Ala Ile Val Asp Ile Pro Glu Ile Pro Gly Phe Lys Asp
        355                 360                 365

Leu Glu Pro Met Glu Gln Phe Ile Ala Gln Val Asp Leu Cys Val Asp
    370                 375                 380

Cys Thr Thr Gly Cys Leu Lys Gly Leu Ala Asn Val Gln Cys Ser Asp
385                 390                 395                 400

Leu Leu Lys Lys Trp Leu Pro Gln Arg Cys Ala Thr Phe Ala Ser Lys
                405                 410                 415

Ile Gln Gly Gln Val Asp Lys Ile Lys Gly Ala Gly Gly Asp
            420                 425                 430

<210>  19
<211>  426
<212>  PRT
<213>  artificial sequence

<220>
<223>  pDualyx-040pre LC

<400>  19

Met Asn Phe Gly Phe Ser Leu Ile Phe Leu Val Leu Val Leu Lys Gly
1               5                   10                  15

Val Gln Cys Glu Val Lys Leu Val Pro Arg Gln Leu Asp Tyr Lys Asp
            20                  25                  30

Asp Asp Asp Lys Glu Leu Asp Val Gln Met Thr Gln Ser Pro Ser Ser
        35                  40                  45

Leu Ser Ala Ser Val Gly Asp Arg Val Thr Ile Thr Cys Lys Ala Ser
    50                  55                  60

Gln Asp Ile Asn Lys Phe Ile Ala Trp Tyr Gln Gln Lys Pro Gly Lys
65                  70                  75                  80

Ala Pro Lys Leu Leu Ile His Tyr Thr Ser Thr Leu Gln Pro Gly Ile
                85                  90                  95

Pro Ser Arg Phe Ser Gly Ser Gly Ser Gly Arg Asp Tyr Thr Phe Thr
            100                 105                 110

Ile Ser Ser Leu Gln Pro Glu Asp Ile Ala Thr Tyr Tyr Cys Leu Gln
        115                 120                 125

Tyr Gly Asn Leu Trp Thr Phe Gly Gly Gly Thr Lys Val Glu Ile Lys
    130                 135                 140

Gly Ser Glu Ile Lys Arg Thr Val Ala Ala Pro Ser Val Phe Ile Phe
145                 150                 155                 160

Pro Pro Ser Asp Glu Gln Leu Lys Ser Gly Thr Ala Ser Val Val Cys
                165                 170                 175

Leu Leu Asn Asn Phe Tyr Pro Arg Glu Ala Lys Val Gln Trp Lys Val
            180                 185                 190

Asp Asn Ala Leu Gln Ser Gly Asn Ser Gln Glu Ser Val Thr Glu Gln
        195                 200                 205

Asp Ser Lys Asp Ser Thr Tyr Ser Leu Ser Ser Thr Leu Thr Leu Ser

210                 215                 220

Lys Ala Asp Tyr Glu Lys His Lys Val Tyr Ala Cys Glu Val Thr His
225                 230                 235                 240

Gln Gly Leu Ser Ser Pro Val Thr Lys Ser Phe Asn Arg Gly Glu Cys
                245                 250                 255

Leu Glu Lys Pro Thr Glu Asn Asn Glu Asp Phe Asn Ile Val Ala Val
            260                 265                 270

Ala Ser Asn Phe Ala Thr Thr Asp Leu Asp Ala Asp Arg Gly Lys Leu
        275                 280                 285

Pro Gly Lys Lys Leu Pro Leu Glu Val Leu Lys Glu Met Glu Ala Asn
    290                 295                 300

Ala Arg Lys Ala Gly Cys Thr Arg Gly Cys Leu Ile Cys Leu Ser His
305                 310                 315                 320

Ile Lys Cys Thr Pro Lys Met Lys Lys Phe Ile Pro Gly Arg Cys His
                325                 330                 335

Thr Tyr Glu Gly Asp Lys Glu Ser Ala Gln Gly Gly Ile Gly Glu Ala
            340                 345                 350

Ile Val Asp Ile Pro Glu Ile Pro Gly Phe Lys Asp Leu Glu Pro Met
        355                 360                 365

Glu Gln Phe Ile Ala Gln Val Asp Leu Cys Val Asp Cys Thr Thr Gly
    370                 375                 380

Cys Leu Lys Gly Leu Ala Asn Val Gln Cys Ser Asp Leu Leu Lys Lys
385                 390                 395                 400

Trp Leu Pro Gln Arg Cys Ala Thr Phe Ala Ser Lys Ile Gln Gly Gln
                405                 410                 415

Val Asp Lys Ile Lys Gly Ala Gly Gly Asp
            420                 425

<210>  20
<211>  496
<212>  PRT
<213>  artificial sequence

<220>
<223>  pDualyx-112 LC

<400>  20

Met Asn Phe Gly Phe Ser Leu Ile Phe Leu Val Leu Val Leu Lys Gly
1               5                   10                  15

Val Gln Cys Glu Val Lys Leu Val Pro Arg Gln Leu Asp Tyr Lys Asp
            20                  25                  30

Asp Asp Asp Lys Glu Leu Asp Ile Val Met Thr Gln Ser His Lys Phe
        35                  40                  45

Met Ser Thr Ser Val Gly Asp Arg Val Ser Ile Thr Cys Lys Ala Ser
    50                  55                  60

Gln Asp Val Asp Thr Ala Val Ala Trp Tyr Gln Gln Lys Pro Gly Gln
65                  70                  75                  80

Ser Pro Lys Leu Leu Ile Tyr Trp Ala Ser Thr Arg His Thr Gly Val
                85                  90                  95

Pro Asp Arg Phe Thr Gly Ser Gly Ser Gly Thr Asp Tyr Thr Leu Thr
            100                 105                 110

Ile Ser Ser Val Gln Ala Glu Asp Leu Ala Arg Tyr Tyr Cys Gln Gln
        115                 120                 125

Tyr Tyr Ser Val Pro Pro Thr Phe Gly Gly Gly Thr Lys Leu Gly Ser
    130                 135                 140

Glu Ile Lys Arg Thr Val Ala Ala Pro Ser Val Phe Ile Phe Pro Pro
145                 150                 155                 160

Ser Asp Glu Gln Leu Lys Ser Gly Thr Ala Ser Val Val Cys Leu Leu
                165                 170                 175

Asn Asn Phe Tyr Pro Arg Glu Ala Lys Val Gln Trp Lys Val Asp Asn
            180                 185                 190

Ala Leu Gln Ser Gly Asn Ser Gln Glu Ser Val Thr Glu Gln Asp Ser
        195                 200                 205

Lys Asp Ser Thr Tyr Ser Leu Ser Ser Thr Leu Thr Leu Ser Lys Ala
    210                 215                 220

Asp Tyr Glu Lys His Lys Val Tyr Ala Cys Glu Val Thr His Gln Gly
225                 230                 235                 240

Leu Ser Ser Pro Val Thr Lys Ser Phe Asn Arg Gly Glu Cys Leu Glu

245                 250                 255

Gln Ile Gln Leu Val Gln Ser Gly Pro Glu Leu Lys Lys Pro Gly Glu
            260                 265                 270

Thr Val Lys Ile Ser Cys Lys Ala Ser Gly Tyr Thr Phe Thr Thr Ala
        275                 280                 285

Gly Met Gln Trp Val Gln Lys Met Pro Gly Lys Gly Phe Lys Trp Ile
    290                 295                 300

Gly Trp Ile Asn Thr His Ser Gly Glu Pro Lys Tyr Ala Glu Asp Phe
305                 310                 315                 320

Lys Gly Arg Phe Ala Phe Ser Leu Glu Thr Ser Ala Ser Thr Ala Tyr
                325                 330                 335

Leu Gln Ile Ser Asn Leu Lys Asn Glu Asp Thr Ala Thr Tyr Phe Cys
            340                 345                 350

Ala Arg Trp Asp Gly Thr Gly Tyr Trp Gly Gln Gly Thr Thr Leu Thr
        355                 360                 365

Val Ser Ser Arg Ser Ser Thr Lys Gly Pro Lys Leu Glu Glu Gly Glu
    370                 375                 380

Phe Ser Glu Ala Gln Leu Gln Ile Val Leu Thr Gln Ser Pro Ala Ile
385                 390                 395                 400

Met Ser Ala Ser Pro Gly Glu Lys Val Thr Ile Thr Cys Ser Ala Ser
                405                 410                 415

Ser Ser Val Ser Tyr Met His Trp Phe Gln Gln Lys Pro Gly Thr Ser
            420                 425                 430

Pro Lys Leu Trp Ile Tyr Ser Thr Ser Asn Leu Ala Ser Gly Val Pro
        435                 440                 445

Ala Arg Phe Ser Gly Ser Gly Ser Gly Thr Ser Tyr Ser Leu Thr Ile
    450                 455                 460

Ser Arg Met Glu Ala Glu Asp Ala Ala Thr Tyr Tyr Cys Gln Gln Arg
465                 470                 475                 480

Ser Ser Tyr Pro Pro Thr Phe Gly Gly Gly Thr Lys Leu Glu Ile Lys
                485                 490                 495

<210>  21
<211>  503
<212>  PRT
<213>  artificial sequence

<220>
<223>  pDualyx-113 LC

<400>  21

Met Asn Phe Gly Phe Ser Leu Ile Phe Leu Val Leu Val Leu Lys Gly
1               5                   10                  15

Val Gln Cys Glu Val Lys Leu Val Pro Arg Gln Leu Asp Tyr Lys Asp
            20                  25                  30

Asp Asp Asp Lys Glu Leu Asp Ile Val Met Thr Gln Ser His Lys Phe
        35                  40                  45

Met Ser Thr Ser Val Gly Asp Arg Val Ser Ile Thr Cys Lys Ala Ser
    50                  55                  60

Gln Asp Val Asp Thr Ala Val Ala Trp Tyr Gln Gln Lys Pro Gly Gln
65                  70                  75                  80

Ser Pro Lys Leu Leu Ile Tyr Trp Ala Ser Thr Arg His Thr Gly Val
                85                  90                  95

Pro Asp Arg Phe Thr Gly Ser Gly Ser Gly Thr Asp Tyr Thr Leu Thr
            100                 105                 110

Ile Ser Ser Val Gln Ala Glu Asp Leu Ala Arg Tyr Tyr Cys Gln Gln
        115                 120                 125

Tyr Tyr Ser Val Pro Pro Thr Phe Gly Gly Gly Thr Lys Leu Gly Ser
    130                 135                 140

Glu Ile Lys Arg Thr Val Ala Ala Pro Ser Val Phe Ile Phe Pro Pro
145                 150                 155                 160

Ser Asp Glu Gln Leu Lys Ser Gly Thr Ala Ser Val Val Cys Leu Leu
                165                 170                 175

Asn Asn Phe Tyr Pro Arg Glu Ala Lys Val Gln Trp Lys Val Asp Asn
            180                 185                 190

Ala Leu Gln Ser Gly Asn Ser Gln Glu Ser Val Thr Glu Gln Asp Ser
        195                 200                 205

Lys Asp Ser Thr Tyr Ser Leu Ser Ser Thr Leu Thr Leu Ser Lys Ala

210                 215                 220

Asp Tyr Glu Lys His Lys Val Tyr Ala Cys Glu Val Thr His Gln Gly
225                 230                 235                 240

Leu Ser Ser Pro Val Thr Lys Ser Phe Asn Arg Gly Glu Cys Leu Glu
                245                 250                 255

Glu Val Lys Leu Leu Gln Ser Gly Gly Gly Leu Val Gln Pro Gly Gly
            260                 265                 270

Ser Leu Lys Leu Ser Cys Glu Ala Ser Gly Ile Asp Phe Ser Arg Tyr
        275                 280                 285

Trp Met Ser Trp Val Arg Arg Ala Pro Gly Lys Gly Leu Glu Trp Ile
    290                 295                 300

Gly Glu Ser Asp Pro Asp Ser Ser Thr Ile Asn Tyr Ala Pro Ser Leu
305                 310                 315                 320

Lys Asp Lys Phe Ile Ile Ser Arg Asp Ser Ala Arg Asn Thr Leu Tyr
                325                 330                 335

Leu Gln Met Ser Lys Val Arg Ser Glu Asp Thr Ala Leu Tyr Tyr Cys
            340                 345                 350

Ala Arg Glu Gly Tyr Tyr Asp Asn Glu Gly Tyr Phe Asp Val Trp Gly
        355                 360                 365

Thr Gly Thr Thr Val Thr Val Ser Ser Arg Ser Ser Thr Lys Gly Pro
    370                 375                 380

Lys Leu Glu Glu Gly Glu Phe Ser Glu Ala Gln Leu Asn Ile Val Met
385                 390                 395                 400

Thr Gln Ser Pro Lys Ser Leu Ser Met Ser Val Gly Glu Arg Val Thr
                405                 410                 415

Leu Asn Cys Lys Ala Ser Glu Asn Val Lys Thr Tyr Val Ser Trp Tyr
            420                 425                 430

Gln Gln Lys Pro Glu Gln Ser Pro Lys Leu Leu Ile Tyr Gly Ala Ser
        435                 440                 445

Asn Arg Tyr Thr Gly Val Pro Asp Arg Phe Thr Gly Ser Gly Ser Ala
    450                 455                 460

Thr Asp Phe Thr Leu Thr Ile Ser Ser Val Gln Ala Glu Asp Leu Ala
465                 470                 475                 480

Asp Tyr His Cys Gly Gln Ser Tyr Ser Tyr Pro Phe Thr Phe Gly Ala
                485                 490                 495

Gly Thr Lys Leu Glu Arg Lys
            500

<210>  22
<211>  500
<212>  PRT
<213>  artificial sequence

<220>
<223>  pDualyx-114 LC

<400>  22

Met Asn Phe Gly Phe Ser Leu Ile Phe Leu Val Leu Val Leu Lys Gly
1               5                   10                  15

Val Gln Cys Glu Val Lys Leu Val Pro Arg Gln Leu Asp Tyr Lys Asp
            20                  25                  30

Asp Asp Asp Lys Glu Leu Asp Ile Val Met Thr Gln Ser His Lys Phe
        35                  40                  45

Met Ser Thr Ser Val Gly Asp Arg Val Ser Ile Thr Cys Lys Ala Ser
    50                  55                  60

Gln Asp Val Asp Thr Ala Val Ala Trp Tyr Gln Gln Lys Pro Gly Gln
65                  70                  75                  80

Ser Pro Lys Leu Leu Ile Tyr Trp Ala Ser Thr Arg His Thr Gly Val
                85                  90                  95

Pro Asp Arg Phe Thr Gly Ser Gly Ser Gly Thr Asp Tyr Thr Leu Thr
            100                 105                 110

Ile Ser Ser Val Gln Ala Glu Asp Leu Ala Arg Tyr Tyr Cys Gln Gln
        115                 120                 125

Tyr Tyr Ser Val Pro Pro Thr Phe Gly Gly Gly Thr Lys Leu Gly Ser
    130                 135                 140

Glu Ile Lys Arg Thr Val Ala Ala Pro Ser Val Phe Ile Phe Pro Pro
145                 150                 155                 160

Ser Asp Glu Gln Leu Lys Ser Gly Thr Ala Ser Val Val Cys Leu Leu

                165                 170                 175

Asn Asn Phe Tyr Pro Arg Glu Ala Lys Val Gln Trp Lys Val Asp Asn
            180                 185                 190

Ala Leu Gln Ser Gly Asn Ser Gln Glu Ser Val Thr Glu Gln Asp Ser
        195                 200                 205

Lys Asp Ser Thr Tyr Ser Leu Ser Ser Thr Leu Thr Leu Ser Lys Ala
    210                 215                 220

Asp Tyr Glu Lys His Lys Val Tyr Ala Cys Glu Val Thr His Gln Gly
225                 230                 235                 240

Leu Ser Ser Pro Val Thr Lys Ser Phe Asn Arg Gly Glu Cys Leu Glu
                245                 250                 255

Glu Ile Gln Leu Gln Gln Ser Gly Thr Val Leu Ala Arg Pro Gly Ala
            260                 265                 270

Ser Val Lys Met Ser Cys Lys Thr Ser Gly Tyr Thr Phe Thr Asn Tyr
        275                 280                 285

Trp Met His Trp Leu Lys Gln Arg Pro Gly Gln Gly Leu Glu Trp Ile
    290                 295                 300

Gly Thr Ile Tyr Pro Gly Asn Ser Glu Thr Asn Tyr Asn Pro Asn Phe
305                 310                 315                 320

Lys Gly Lys Ala Lys Leu Thr Ala Val Ser Ser Ala Ser Thr Ala Tyr
                325                 330                 335

Met Glu Leu Ser Ser Leu Thr Asn Glu Asp Ser Ala Val Tyr Tyr Cys
            340                 345                 350

Ser Arg Gly Glu Asp Tyr Trp Gly Gln Gly Thr Ser Val Thr Val Ser
        355                 360                 365

Ser Arg Ser Ser Thr Lys Gly Pro Lys Leu Glu Glu Gly Glu Phe Ser
    370                 375                 380

Glu Ala Gln Leu Asp Val Val Met Thr Gln Thr Pro Leu Ser Leu Pro
385                 390                 395                 400

Val Ser Leu Gly Asp Gln Ala Ser Ile Ser Cys Arg Ala Ser Gln Ser
                405                 410                 415

Leu Val His Ser Asn Gly Asn Thr Tyr Leu Gln Trp Tyr Leu Gln Lys
            420                 425                 430

Pro Gly Gln Ser Pro Lys Leu Leu Ile Tyr Lys Val Ser Asn Arg Phe
        435                 440                 445

Ser Gly Val Pro Asp Arg Phe Ser Gly Ser Gly Ser Gly Thr Asp Phe
    450                 455                 460

Thr Leu Lys Ile Ser Arg Val Glu Ala Glu Asp Leu Gly Val Phe Phe
465                 470                 475                 480

Cys Ser Gln Ser Thr His Val Pro Phe Thr Phe Gly Ser Gly Thr Lys
                485                 490                 495

Leu Glu Ile Lys
            500

<210>  23
<211>  506
<212>  PRT
<213>  Artificial Sequence

<220>
<223>  pDualyx-115 LC

<400>  23

Met Asn Phe Gly Phe Ser Leu Ile Phe Leu Val Leu Val Leu Lys Gly
1               5                   10                  15

Val Gln Cys Glu Val Lys Leu Val Pro Arg Gln Leu Asp Tyr Lys Asp
            20                  25                  30

Asp Asp Asp Lys Glu Leu Asp Ile Val Met Thr Gln Ser His Lys Phe
        35                  40                  45

Met Ser Thr Ser Val Gly Asp Arg Val Ser Ile Thr Cys Lys Ala Ser
    50                  55                  60

Gln Asp Val Asp Thr Ala Val Ala Trp Tyr Gln Gln Lys Pro Gly Gln
65                  70                  75                  80

Ser Pro Lys Leu Leu Ile Tyr Trp Ala Ser Thr Arg His Thr Gly Val
                85                  90                  95

Pro Asp Arg Phe Thr Gly Ser Gly Ser Gly Thr Asp Tyr Thr Leu Thr
            100                 105                 110

Ile Ser Ser Val Gln Ala Glu Asp Leu Ala Arg Tyr Tyr Cys Gln Gln

115                 120                 125

Tyr Tyr Ser Val Pro Pro Thr Phe Gly Gly Gly Thr Lys Leu Gly Ser
    130                 135                 140

Glu Ile Lys Arg Thr Val Ala Ala Pro Ser Val Phe Ile Phe Pro Pro
145                 150                 155                 160

Ser Asp Glu Gln Leu Lys Ser Gly Thr Ala Ser Val Val Cys Leu Leu
                165                 170                 175

Asn Asn Phe Tyr Pro Arg Glu Ala Lys Val Gln Trp Lys Val Asp Asn
            180                 185                 190

Ala Leu Gln Ser Gly Asn Ser Gln Glu Ser Val Thr Glu Gln Asp Ser
        195                 200                 205

Lys Asp Ser Thr Tyr Ser Leu Ser Ser Thr Leu Thr Leu Ser Lys Ala
    210                 215                 220

Asp Tyr Glu Lys His Lys Val Tyr Ala Cys Glu Val Thr His Gln Gly
225                 230                 235                 240

Leu Ser Ser Pro Val Thr Lys Ser Phe Asn Arg Gly Glu Cys Leu Glu
                245                 250                 255

Asp Val Gln Leu Gln Glu Ser Gly Pro Gly Leu Val Lys Pro Ser Gln
            260                 265                 270

Ser Leu Ser Leu Thr Cys Ser Val Thr Gly Ser Ser Ile Thr Ser Gly
        275                 280                 285

Tyr Tyr Trp Asn Trp Ile Arg Gln Phe Pro Gly Asn Lys Leu Glu Trp
    290                 295                 300

Met Gly Tyr Ile Thr Asn Asp Gly Ser Asn Asn Tyr Asn Pro Ser Leu
305                 310                 315                 320

Lys Asn Arg Ile Ser Ile Thr Arg Asp Thr Ser Glu Asn Gln Phe Phe
                325                 330                 335

Leu Lys Leu Asn Ser Val Thr Thr Glu Asp Thr Ala Thr Tyr Tyr Cys
            340                 345                 350

Ala Arg Asp Gly Gly Trp Phe His Tyr Phe Asp Tyr Trp Gly Gln Gly
        355                 360                 365

Thr Thr Leu Thr Val Ser Ser Arg Ser Ser Thr Lys Gly Pro Lys Leu
    370                 375                 380

Glu Glu Gly Glu Phe Ser Glu Ala Gln Leu Asp Val Val Met Thr Gln
385                 390                 395                 400

Thr Pro Leu Ser Leu Pro Val Ser Leu Gly Asp Gln Ala Ser Ile Ser
                405                 410                 415

Cys Arg Ala Ser Gln Ser Leu Val His Ser Asn Gly Asn Thr Tyr Leu
            420                 425                 430

Gln Trp Tyr Leu Gln Lys Pro Gly Gln Ser Pro Lys Leu Leu Ile Tyr
        435                 440                 445

Lys Val Ser Asn Arg Phe Ser Gly Val Pro Asp Arg Phe Ser Gly Ser
    450                 455                 460

Gly Ser Gly Thr Asp Phe Thr Leu Lys Ile Ser Arg Val Glu Ala Glu
465                 470                 475                 480

Asp Leu Gly Val Phe Phe Cys Ser Gln Ser Thr His Val Pro Phe Thr
                485                 490                 495

Phe Gly Ser Gly Thr Lys Leu Glu Ile Lys
            500                 505

<210>  24
<211>  500
<212>  PRT
<213>  artificial sequence

<220>
<223>  pDualyx-116 LC

<400>  24

Met Asn Phe Gly Phe Ser Leu Ile Phe Leu Val Leu Val Leu Lys Gly
1               5                   10                  15

Val Gln Cys Glu Val Lys Leu Val Pro Arg Gln Leu Asp Tyr Lys Asp
            20                  25                  30

Asp Asp Asp Lys Glu Leu Asp Ile Val Met Thr Gln Ser His Lys Phe
        35                  40                  45

Met Ser Thr Ser Val Gly Asp Arg Val Ser Ile Thr Cys Lys Ala Ser
    50                  55                  60

Gln Asp Val Asp Thr Ala Val Ala Trp Tyr Gln Gln Lys Pro Gly Gln

65                  70                  75                  80

Ser Pro Lys Leu Leu Ile Tyr Trp Ala Ser Thr Arg His Thr Gly Val
                85                  90                  95

Pro Asp Arg Phe Thr Gly Ser Gly Ser Gly Thr Asp Tyr Thr Leu Thr
            100                 105                 110

Ile Ser Ser Val Gln Ala Glu Asp Leu Ala Arg Tyr Tyr Cys Gln Gln
        115                 120                 125

Tyr Tyr Ser Val Pro Pro Thr Phe Gly Gly Gly Thr Lys Leu Gly Ser
    130                 135                 140

Glu Ile Lys Arg Thr Val Ala Ala Pro Ser Val Phe Ile Phe Pro Pro
145                 150                 155                 160

Ser Asp Glu Gln Leu Lys Ser Gly Thr Ala Ser Val Val Cys Leu Leu
                165                 170                 175

Asn Asn Phe Tyr Pro Arg Glu Ala Lys Val Gln Trp Lys Val Asp Asn
            180                 185                 190

Ala Leu Gln Ser Gly Asn Ser Gln Glu Ser Val Thr Glu Gln Asp Ser
        195                 200                 205

Lys Asp Ser Thr Tyr Ser Leu Ser Ser Thr Leu Thr Leu Ser Lys Ala
    210                 215                 220

Asp Tyr Glu Lys His Lys Val Tyr Ala Cys Glu Val Thr His Gln Gly
225                 230                 235                 240

Leu Ser Ser Pro Val Thr Lys Ser Phe Asn Arg Gly Glu Cys Leu Glu
                245                 250                 255

Gln Val Thr Leu Lys Glu Ser Gly Pro Gly Ile Leu Gln Pro Ser Gln
            260                 265                 270

Thr Leu Ser Leu Thr Cys Ser Phe Ser Gly Phe Ser Leu Ser Thr Phe
        275                 280                 285

Gly Met Gly Val Gly Trp Ile Arg Gln Pro Ser Gly Lys Gly Leu Glu
    290                 295                 300

Trp Leu Ala His Ile Trp Trp Asp Asp Asp Lys Tyr Tyr Asn Pro Ala
305                 310                 315                 320

Leu Lys Ser Arg Leu Thr Ile Ser Lys Asp Thr Ser Glu Asn Gln Val
                325                 330                 335

Phe Leu Lys Ile Ala Asn Val Asp Thr Ala Asp Thr Ala Thr Tyr Tyr
            340                 345                 350

Cys Val Arg Ile Ala Gly Thr Arg Tyr Phe Asp Val Trp Gly Thr Gly
        355                 360                 365

Thr Thr Val Thr Val Ser Ser Arg Ser Ser Thr Lys Gly Pro Lys Leu
    370                 375                 380

Glu Glu Gly Glu Phe Ser Glu Ala Gln Leu Asp Ile Gln Met Thr Gln
385                 390                 395                 400

Ser Pro Ser Ser Leu Ser Ala Ser Leu Gly Gly Lys Val Thr Ile Thr
                405                 410                 415

Cys Lys Ala Ser Gln Asp Ile Asn Lys Phe Ile Ala Trp Tyr Gln His
            420                 425                 430

Lys Pro Gly Lys Gly Pro Arg Leu Leu Ile His Tyr Thr Ser Thr Leu
        435                 440                 445

Gln Pro Gly Ile Pro Ser Arg Phe Ser Gly Ser Gly Ser Gly Arg Asp
    450                 455                 460

Tyr Ser Phe Ser Ile Ser Asn Leu Glu Pro Glu Asp Ile Ala Thr Tyr
465                 470                 475                 480

Tyr Cys Leu Gln Tyr Asp Asn Leu Tyr Thr Phe Gly Gly Gly Thr Lys
                485                 490                 495

Leu Glu Ile Lys
            500

<210>  25
<211>  396
<212>  PRT
<213>  artificial sequence

<220>
<223>  pDualyx-151 LC

<400>  25

Met Asn Phe Gly Phe Ser Leu Ile Phe Leu Val Leu Val Leu Lys Gly
1               5                   10                  15

Val Gln Cys Glu Val Lys Leu Val Pro Arg Gln Leu Asp Tyr Lys Asp

20                  25                  30

Asp Asp Asp Lys Glu Phe Gln Ile Gln Leu Val Gln Ser Gly Pro Glu
        35                  40                  45

Val Lys Lys Pro Gly Glu Thr Val Lys Ile Ser Cys Lys Ala Ser Gly
    50                  55                  60

Tyr Thr Phe Thr Ile His Gly Met Ser Trp Val Lys Gln Ala Pro Gly
65                  70                  75                  80

Lys Gly Leu Lys Trp Met Gly Trp Ile Asn Thr Tyr Ser Gly Val Pro
                85                  90                  95

Thr Tyr Ala Asn Asp Phe Lys Gly Arg Phe Ala Phe Ser Leu Glu Thr
            100                 105                 110

Ser Ala Ser Thr Ala Tyr Leu Gln Ile Asn Asn Leu Lys Asn Glu Asp
        115                 120                 125

Thr Ala Thr Tyr Phe Cys Ala Arg Asp Glu Val Arg Arg Gly Phe Gly
    130                 135                 140

Phe Ala Tyr Trp Gly Gln Gly Thr Leu Val Thr Val Ser Ala Arg Ser
145                 150                 155                 160

Ser Thr Lys Gly Pro Lys Leu Glu Glu Gly Glu Phe Ser Glu Ala Gln
                165                 170                 175

Leu Asp Ile Val Met Ala Gln Ser Gln Lys Phe Met Ser Val Ser Val
            180                 185                 190

Gly Asp Arg Val Ser Val Thr Cys Lys Ala Ser Gln Asn Val Gly Thr
        195                 200                 205

Asn Val Ala Trp Tyr Gln Gln Arg Pro Gly Gln Ser Pro Lys Ala Leu
    210                 215                 220

Ile Tyr Ser Ala Ser Tyr Arg Tyr Ser Gly Val Pro Asp Arg Phe Thr
225                 230                 235                 240

Gly Ser Gly Ser Gly Thr Asp Phe Thr Leu Thr Ile Ser Asn Val Gln
                245                 250                 255

Ser Glu Asp Leu Ala Glu Tyr Phe Cys Gln Gln Phe Asp Ser His Pro
            260                 265                 270

Leu Thr Phe Gly Ala Gly Thr Lys Leu Glu Leu Lys Gly Ser Glu Ile
        275                 280                 285

Lys Arg Thr Val Ala Ala Pro Ser Val Phe Ile Phe Pro Pro Ser Asp
    290                 295                 300

Glu Gln Leu Lys Ser Gly Thr Ala Ser Val Val Cys Leu Leu Asn Asn
305                 310                 315                 320

Phe Tyr Pro Arg Glu Ala Lys Val Gln Trp Lys Val Asp Asn Ala Leu
                325                 330                 335

Gln Ser Gly Asn Ser Gln Glu Ser Val Thr Glu Gln Asp Ser Lys Asp
            340                 345                 350

Ser Thr Tyr Ser Leu Ser Ser Thr Leu Thr Leu Ser Lys Ala Asp Tyr
        355                 360                 365

Glu Lys His Lys Val Tyr Ala Cys Glu Val Thr His Gln Gly Leu Ser
    370                 375                 380

Ser Pro Val Thr Lys Ser Phe Asn Arg Gly Glu Cys
385                 390                 395

<210>  26
<211>  397
<212>  PRT
<213>  artificial sequence

<220>
<223>  pDualyx-156 LC

<400>  26

Met Asn Phe Gly Phe Ser Leu Ile Phe Leu Val Leu Val Leu Lys Gly
1               5                   10                  15

Val Gln Cys Glu Val Lys Leu Val Pro Arg Gln Leu Asp Tyr Lys Asp
            20                  25                  30

Asp Asp Asp Lys Glu Phe Glu Val Lys Leu Leu Gln Ser Gly Gly Gly
        35                  40                  45

Leu Val Gln Pro Gly Gly Ser Leu Lys Leu Ser Cys Glu Ala Ser Gly
    50                  55                  60

Ile Asp Phe Ser Arg Tyr Trp Met Ser Trp Val Arg Arg Ala Pro Gly
65                  70                  75                  80

Lys Gly Leu Glu Trp Ile Gly Glu Ser Asp Pro Asp Ser Ser Thr Ile

85                  90                  95

Asn Tyr Ala Pro Ser Leu Lys Asp Lys Phe Ile Ile Ser Arg Asp Ser
            100                 105                 110

Ala Arg Asn Thr Leu Tyr Leu Gln Met Ser Lys Val Arg Ser Glu Asp
        115                 120                 125

Thr Ala Leu Tyr Tyr Cys Ala Arg Glu Gly Tyr Tyr Asp Asn Glu Gly
    130                 135                 140

Tyr Phe Asp Val Trp Gly Thr Gly Thr Thr Val Thr Val Ser Ser Arg
145                 150                 155                 160

Ser Ser Thr Lys Gly Pro Lys Leu Glu Glu Gly Glu Phe Ser Glu Ala
                165                 170                 175

Gln Leu Asn Ile Val Met Thr Gln Ser Pro Lys Ser Leu Ser Met Ser
            180                 185                 190

Val Gly Glu Arg Val Thr Leu Asn Cys Lys Ala Ser Glu Asn Val Lys
        195                 200                 205

Thr Tyr Val Ser Trp Tyr Gln Gln Lys Pro Glu Gln Ser Pro Lys Leu
    210                 215                 220

Leu Ile Tyr Gly Ala Ser Asn Arg Tyr Thr Gly Val Pro Asp Arg Phe
225                 230                 235                 240

Thr Gly Ser Gly Ser Ala Thr Asp Phe Thr Leu Thr Ile Ser Ser Val
                245                 250                 255

Gln Ala Glu Asp Leu Ala Asp Tyr His Cys Gly Gln Ser Tyr Ser Tyr
            260                 265                 270

Pro Phe Thr Phe Gly Ala Gly Thr Lys Leu Glu Arg Lys Gly Ser Glu
        275                 280                 285

Ile Lys Arg Thr Val Ala Ala Pro Ser Val Phe Ile Phe Pro Pro Ser
    290                 295                 300

Asp Glu Gln Leu Lys Ser Gly Thr Ala Ser Val Val Cys Leu Leu Asn
305                 310                 315                 320

Asn Phe Tyr Pro Arg Glu Ala Lys Val Gln Trp Lys Val Asp Asn Ala
                325                 330                 335

Leu Gln Ser Gly Asn Ser Gln Glu Ser Val Thr Glu Gln Asp Ser Lys
            340                 345                 350

Asp Ser Thr Tyr Ser Leu Ser Ser Thr Leu Thr Leu Ser Lys Ala Asp
        355                 360                 365

Tyr Glu Lys His Lys Val Tyr Ala Cys Glu Val Thr His Gln Gly Leu
    370                 375                 380

Ser Ser Pro Val Thr Lys Ser Phe Asn Arg Gly Glu Cys
385                 390                 395

<210>  27
<211>  394
<212>  PRT
<213>  artificial sequence

<220>
<223>  pDualyx-161 LC

<400>  27

Met Asn Phe Gly Phe Ser Leu Ile Phe Leu Val Leu Val Leu Lys Gly
1               5                   10                  15

Val Gln Cys Glu Val Lys Leu Val Pro Arg Gln Leu Asp Tyr Lys Asp
            20                  25                  30

Asp Asp Asp Lys Glu Phe Gln Val Thr Leu Lys Glu Ser Gly Pro Gly
        35                  40                  45

Ile Leu Gln Pro Ser Gln Thr Leu Ser Leu Thr Cys Ser Phe Ser Gly
    50                  55                  60

Phe Ser Leu Ser Thr Phe Gly Met Gly Val Gly Trp Ile Arg Gln Pro
65                  70                  75                  80

Ser Gly Lys Gly Leu Glu Trp Leu Ala His Ile Trp Trp Asp Asp Asp
                85                  90                  95

Lys Tyr Tyr Asn Pro Ala Leu Lys Ser Arg Leu Thr Ile Ser Lys Asp
            100                 105                 110

Thr Ser Glu Asn Gln Val Phe Leu Lys Ile Ala Asn Val Asp Thr Ala
        115                 120                 125

Asp Thr Ala Thr Tyr Tyr Cys Val Arg Ile Ala Gly Thr Arg Tyr Phe
    130                 135                 140

Asp Val Trp Gly Thr Gly Thr Thr Val Thr Val Ser Ser Arg Ser Ser

145                 150                 155                 160

Thr Lys Gly Pro Lys Leu Glu Glu Gly Glu Phe Ser Glu Ala Gln Leu
                165                 170                 175

Asp Ile Gln Met Thr Gln Ser Pro Ser Ser Leu Ser Ala Ser Leu Gly
            180                 185                 190

Gly Lys Val Thr Ile Thr Cys Lys Ala Ser Gln Asp Ile Asn Lys Phe
        195                 200                 205

Ile Ala Trp Tyr Gln His Lys Pro Gly Lys Gly Pro Arg Leu Leu Ile
    210                 215                 220

His Tyr Thr Ser Thr Leu Gln Pro Gly Ile Pro Ser Arg Phe Ser Gly
225                 230                 235                 240

Ser Gly Ser Gly Arg Asp Tyr Ser Phe Ser Ile Ser Asn Leu Glu Pro
                245                 250                 255

Glu Asp Ile Ala Thr Tyr Tyr Cys Leu Gln Tyr Asp Asn Leu Tyr Thr
            260                 265                 270

Phe Gly Gly Gly Thr Lys Leu Glu Ile Lys Gly Ser Glu Ile Lys Arg
        275                 280                 285

Thr Val Ala Ala Pro Ser Val Phe Ile Phe Pro Pro Ser Asp Glu Gln
    290                 295                 300

Leu Lys Ser Gly Thr Ala Ser Val Val Cys Leu Leu Asn Asn Phe Tyr
305                 310                 315                 320

Pro Arg Glu Ala Lys Val Gln Trp Lys Val Asp Asn Ala Leu Gln Ser
                325                 330                 335

Gly Asn Ser Gln Glu Ser Val Thr Glu Gln Asp Ser Lys Asp Ser Thr
            340                 345                 350

Tyr Ser Leu Ser Ser Thr Leu Thr Leu Ser Lys Ala Asp Tyr Glu Lys
        355                 360                 365

His Lys Val Tyr Ala Cys Glu Val Thr His Gln Gly Leu Ser Ser Pro
    370                 375                 380

Val Thr Lys Ser Phe Asn Arg Gly Glu Cys
385                 390

<210>  28
<211>  481
<212>  PRT
<213>  artificial sequence

<220>
<223>  pDualyx-011pre HC

<400>  28

Met Asn Phe Gly Phe Ser Leu Ile Phe Leu Val Leu Val Leu Lys Gly
1               5                   10                  15

Val Gln Cys Glu Val Lys Leu Val Pro Arg Gln Leu Gln Val Gln Leu
            20                  25                  30

Leu Gln Ser Gly Pro Glu Leu Val Lys Pro Gly Ala Ser Val Lys Leu
        35                  40                  45

Ser Cys Lys Ala Ser Gly Tyr Ser Phe Thr Ser Tyr Asp Ile Asn Trp
    50                  55                  60

Val Lys Gln Arg Pro Gly Gln Gly Leu Glu Trp Val Gly Trp Ile Tyr
65                  70                  75                  80

Pro Arg Asp Gly Asp Thr Lys Tyr Asn Glu Lys Phe Lys Gly Lys Ala
                85                  90                  95

Ile Leu Thr Val Asp Thr Ser Ser Asn Thr Ala Tyr Met Asn Leu His
            100                 105                 110

Ser Leu Thr Ser Glu Asp Ser Ala Val Tyr Phe Cys Ala Arg Leu Thr
        115                 120                 125

Gly Pro Tyr Trp Tyr Phe Asp Val Trp Gly Thr Gly Thr Thr Val Thr
    130                 135                 140

Val Ser Ser Gly Ser Ser Ser Ala Ser Thr Lys Gly Pro Ser Val Phe
145                 150                 155                 160

Pro Leu Ala Pro Ser Ser Lys Ser Thr Ser Gly Gly Thr Ala Ala Leu
                165                 170                 175

Gly Cys Leu Val Lys Asp Tyr Phe Pro Glu Pro Val Thr Val Ser Trp
            180                 185                 190

Asn Ser Gly Ala Leu Thr Ser Gly Val His Thr Phe Pro Ala Val Leu
        195                 200                 205

Gln Ser Ser Gly Leu Tyr Ser Leu Ser Ser Val Val Thr Val Pro Ser

210                 215                 220

Ser Ser Leu Gly Thr Gln Thr Tyr Ile Cys Asn Val Asn His Lys Pro
225                 230                 235                 240

Ser Asn Thr Lys Val Asp Lys Lys Val Glu Pro Lys Ser Cys Asp Lys
                245                 250                 255

Thr His Thr Cys Pro Pro Cys Pro Ala Pro Glu Leu Leu Gly Gly Pro
            260                 265                 270

Ser Val Phe Leu Phe Pro Pro Lys Pro Lys Asp Thr Leu Met Ile Ser
        275                 280                 285

Arg Thr Pro Glu Val Thr Cys Val Val Val Asp Val Ser His Glu Asp
    290                 295                 300

Pro Glu Val Lys Phe Asn Trp Tyr Val Asp Gly Val Glu Val His Asn
305                 310                 315                 320

Ala Lys Thr Lys Pro Arg Glu Glu Gln Tyr Ala Ser Thr Tyr Arg Val
                325                 330                 335

Val Ser Val Leu Thr Val Leu His Gln Asp Trp Leu Asn Gly Lys Glu
            340                 345                 350

Tyr Lys Cys Lys Val Ser Asn Lys Ala Leu Pro Ala Pro Ile Glu Lys
        355                 360                 365

Thr Ile Ser Lys Ala Lys Gly Gln Pro Arg Glu Pro Gln Val Tyr Thr
    370                 375                 380

Leu Pro Pro Ser Arg Asp Glu Leu Thr Lys Asn Gln Val Ser Leu Thr
385                 390                 395                 400

Cys Leu Val Lys Gly Phe Tyr Pro Ser Asp Ile Ala Val Glu Trp Glu
                405                 410                 415

Ser Asn Gly Gln Pro Glu Asn Asn Tyr Lys Thr Thr Pro Pro Val Leu
            420                 425                 430

Asp Ser Asp Gly Ser Phe Phe Leu Tyr Ser Lys Leu Thr Val Asp Lys
        435                 440                 445

Ser Arg Trp Gln Gln Gly Asn Val Phe Ser Cys Ser Val Met His Glu
    450                 455                 460

Ala Leu His Asn His Tyr Thr Gln Lys Ser Leu Ser Leu Ser Pro Gly
465                 470                 475                 480

Lys

<210>  29
<211>  481
<212>  PRT
<213>  artificial sequence

<220>
<223>  pDualyx-029pre HC

<400>  29

Met Asn Phe Gly Phe Ser Leu Ile Phe Leu Val Leu Val Leu Lys Gly
1               5                   10                  15

Val Gln Cys Glu Val Lys Leu Val Pro Arg Gln Leu Gln Val Gln Leu
            20                  25                  30

Leu Gln Ser Gly Pro Glu Leu Val Lys Pro Gly Ala Ser Val Lys Leu
        35                  40                  45

Ser Cys Lys Ala Ser Gly Tyr Ser Phe Thr Ser Tyr Asp Ile Asn Trp
    50                  55                  60

Val Lys Gln Arg Pro Gly Gln Gly Leu Glu Trp Val Gly Trp Ile Tyr
65                  70                  75                  80

Pro Arg Asp Gly Asp Thr Lys Tyr Asn Glu Lys Phe Lys Gly Lys Ala
                85                  90                  95

Ile Leu Thr Val Asp Thr Ser Ser Asn Thr Ala Tyr Met Asn Leu His
            100                 105                 110

Ser Leu Thr Ser Glu Asp Ser Ala Val Tyr Phe Cys Ala Arg Leu Thr
        115                 120                 125

Gly Pro Tyr Trp Tyr Phe Asp Val Trp Gly Thr Gly Thr Thr Val Thr
    130                 135                 140

Val Ser Ser Gly Ser Ser Ser Ala Ser Thr Lys Gly Pro Ser Val Phe
145                 150                 155                 160

Pro Leu Ala Pro Ser Ser Lys Ser Thr Ser Gly Gly Thr Ala Ala Leu
                165                 170                 175

Gly Cys Leu Val Lys Asp Tyr Phe Pro Glu Pro Val Thr Val Ser Trp

            180                 185                 190

Asn Ser Gly Ala Leu Thr Ser Gly Val His Thr Phe Pro Ala Val Leu
        195                 200                 205

Gln Ser Ser Gly Leu Tyr Ser Leu Ser Ser Val Val Thr Val Pro Ser
    210                 215                 220

Ser Ser Leu Gly Thr Gln Thr Tyr Ile Cys Asn Val Asn His Lys Pro
225                 230                 235                 240

Ser Asn Thr Lys Val Asp Lys Lys Val Glu Pro Lys Ser Cys Asp Lys
                245                 250                 255

Thr His Thr Cys Pro Pro Cys Pro Ala Pro Glu Leu Leu Gly Gly Pro
            260                 265                 270

Ser Val Phe Leu Phe Pro Pro Lys Pro Lys Asp Thr Leu Met Ile Ser
        275                 280                 285

Arg Thr Pro Glu Val Thr Cys Val Val Val Asp Val Ser His Glu Asp
    290                 295                 300

Pro Glu Val Lys Phe Asn Trp Tyr Val Asp Gly Val Glu Val His Asn
305                 310                 315                 320

Ala Lys Thr Lys Pro Arg Glu Glu Gln Tyr Asn Ser Thr Tyr Arg Val
                325                 330                 335

Val Ser Val Leu Thr Val Leu His Gln Asp Trp Leu Asn Gly Lys Glu
            340                 345                 350

Tyr Lys Cys Lys Val Ser Asn Lys Ala Leu Pro Ala Pro Ile Glu Lys
        355                 360                 365

Thr Ile Ser Lys Ala Lys Gly Gln Pro Arg Glu Pro Gln Val Tyr Thr
    370                 375                 380

Leu Pro Pro Ser Arg Asp Glu Leu Thr Lys Asn Gln Val Ser Leu Thr
385                 390                 395                 400

Cys Leu Val Lys Gly Phe Tyr Pro Ser Asp Ile Ala Val Glu Trp Glu
                405                 410                 415

Ser Asn Gly Gln Pro Glu Asn Asn Tyr Lys Thr Thr Pro Pro Val Leu
            420                 425                 430

Asp Ser Asp Gly Ser Phe Phe Leu Tyr Ser Lys Leu Thr Val Asp Lys
        435                 440                 445

Ser Arg Trp Gln Gln Gly Asn Val Phe Ser Cys Ser Val Met His Glu
    450                 455                 460

Ala Leu His Asn His Tyr Thr Gln Lys Ser Leu Ser Leu Ser Pro Gly
465                 470                 475                 480

Lys

<210>  30
<211>  728
<212>  PRT
<213>  artificial sequence

<220>
<223>  pDualyx-012pre HC

<400>  30

Met Asn Phe Gly Phe Ser Leu Ile Phe Leu Val Leu Val Leu Lys Gly
1               5                   10                  15

Val Gln Cys Glu Val Lys Leu Val Pro Arg Gln Leu Gln Val Gln Leu
            20                  25                  30

Leu Gln Ser Gly Pro Glu Leu Val Lys Pro Gly Ala Ser Val Lys Leu
        35                  40                  45

Ser Cys Lys Ala Ser Gly Tyr Ser Phe Thr Ser Tyr Asp Ile Asn Trp
    50                  55                  60

Val Lys Gln Arg Pro Gly Gln Gly Leu Glu Trp Val Gly Trp Ile Tyr
65                  70                  75                  80

Pro Arg Asp Gly Asp Thr Lys Tyr Asn Glu Lys Phe Lys Gly Lys Ala
                85                  90                  95

Ile Leu Thr Val Asp Thr Ser Ser Asn Thr Ala Tyr Met Asn Leu His
            100                 105                 110

Ser Leu Thr Ser Glu Asp Ser Ala Val Tyr Phe Cys Ala Arg Leu Thr
        115                 120                 125

Gly Pro Tyr Trp Tyr Phe Asp Val Trp Gly Thr Gly Thr Thr Val Thr
    130                 135                 140

Val Ser Ser Gly Ser Ser Ser Ala Ser Thr Lys Gly Pro Ser Val Phe

145                 150                 155                 160

Pro Leu Ala Pro Ser Ser Lys Ser Thr Ser Gly Gly Thr Ala Ala Leu
                165                 170                 175

Gly Cys Leu Val Lys Asp Tyr Phe Pro Glu Pro Val Thr Val Ser Trp
            180                 185                 190

Asn Ser Gly Ala Leu Thr Ser Gly Val His Thr Phe Pro Ala Val Leu
        195                 200                 205

Gln Ser Ser Gly Leu Tyr Ser Leu Ser Ser Val Val Thr Val Pro Ser
    210                 215                 220

Ser Ser Leu Gly Thr Gln Thr Tyr Ile Cys Asn Val Asn His Lys Pro
225                 230                 235                 240

Ser Asn Thr Lys Val Asp Lys Lys Val Glu Pro Lys Ser Cys Asp Lys
                245                 250                 255

Thr His Thr Cys Pro Pro Cys Pro Ala Pro Glu Leu Leu Gly Gly Pro
            260                 265                 270

Ser Val Phe Leu Phe Pro Pro Lys Pro Lys Asp Thr Leu Met Ile Ser
        275                 280                 285

Arg Thr Pro Glu Val Thr Cys Val Val Val Asp Val Ser His Glu Asp
    290                 295                 300

Pro Glu Val Lys Phe Asn Trp Tyr Val Asp Gly Val Glu Val His Asn
305                 310                 315                 320

Ala Lys Thr Lys Pro Arg Glu Glu Gln Tyr Ala Ser Thr Tyr Arg Val
                325                 330                 335

Val Ser Val Leu Thr Val Leu His Gln Asp Trp Leu Asn Gly Lys Glu
            340                 345                 350

Tyr Lys Cys Lys Val Ser Asn Lys Ala Leu Pro Ala Pro Ile Glu Lys
        355                 360                 365

Thr Ile Ser Lys Ala Lys Gly Gln Pro Arg Glu Pro Gln Val Tyr Thr
    370                 375                 380

Leu Pro Pro Ser Arg Asp Glu Leu Thr Lys Asn Gln Val Ser Leu Thr
385                 390                 395                 400

Cys Leu Val Lys Gly Phe Tyr Pro Ser Asp Ile Ala Val Glu Trp Glu
                405                 410                 415

Ser Asn Gly Gln Pro Glu Asn Asn Tyr Lys Thr Thr Pro Pro Val Leu
            420                 425                 430

Asp Ser Asp Gly Ser Phe Phe Leu Tyr Ser Lys Leu Thr Val Asp Lys
        435                 440                 445

Ser Arg Trp Gln Gln Gly Asn Val Phe Ser Cys Ser Val Met His Glu
    450                 455                 460

Ala Leu His Asn His Tyr Thr Gln Lys Ser Leu Ser Leu Ser Pro Gly
465                 470                 475                 480

Lys Leu Glu Gln Val Gln Leu Leu Gln Ser Gly Pro Glu Leu Val Lys
                485                 490                 495

Pro Gly Ala Ser Val Lys Leu Ser Cys Lys Ala Ser Gly Tyr Ser Phe
            500                 505                 510

Thr Ser Tyr Asp Ile Asn Trp Val Lys Gln Arg Pro Gly Gln Gly Leu
        515                 520                 525

Glu Trp Val Gly Trp Ile Tyr Pro Arg Asp Gly Asp Thr Lys Tyr Asn
    530                 535                 540

Glu Lys Phe Lys Gly Lys Ala Ile Leu Thr Val Asp Thr Ser Ser Asn
545                 550                 555                 560

Thr Ala Tyr Met Asn Leu His Ser Leu Thr Ser Glu Asp Ser Ala Val
                565                 570                 575

Tyr Phe Cys Ala Arg Leu Thr Gly Pro Tyr Trp Tyr Phe Asp Val Trp
            580                 585                 590

Gly Thr Gly Thr Thr Val Thr Val Ser Ser Arg Ser Ser Thr Lys Gly
        595                 600                 605

Pro Lys Leu Glu Glu Gly Glu Phe Ser Glu Ala Gln Leu Asp Ile Val
    610                 615                 620

Met Thr Gln Ser His Lys Phe Met Ser Thr Ser Val Gly Asp Arg Val
625                 630                 635                 640

Ser Ile Thr Cys Lys Ala Ser Gln Asp Val Asp Thr Ala Val Ala Trp
                645                 650                 655

Tyr Gln Gln Lys Pro Gly Gln Ser Pro Lys Leu Leu Ile Tyr Trp Ala
            660                 665                 670

Ser Thr Arg His Thr Gly Val Pro Asp Arg Phe Thr Gly Ser Gly Ser
        675                 680                 685

Gly Thr Asp Tyr Thr Leu Thr Ile Ser Ser Val Gln Ala Glu Asp Leu
    690                 695                 700

Ala Arg Tyr Tyr Cys Gln Gln Tyr Tyr Ser Val Pro Pro Thr Phe Gly
705                 710                 715                 720

Gly Gly Thr Lys Leu Glu Ile Lys
                725

<210> 31
<211> 269
<212> PRT
<213> artificial sequence

<220>
<223> pDualyx-019pre HC

<400> 31

Met Asn Phe Gly Phe Ser Leu Ile Phe Leu Val Leu Val Leu Lys Gly
1               5                   10                  15

Val Gln Cys Glu Val Lys Leu Val Pro Arg Gln Leu Asp Tyr Lys Asp
            20                  25                  30

Asp Asp Asp Lys Glu Leu Gln Val Gln Leu Leu Gln Ser Gly Pro Glu
        35                  40                  45

Leu Val Lys Pro Gly Ala Ser Val Lys Leu Ser Cys Lys Ala Ser Gly
    50                  55                  60

Tyr Ser Phe Thr Ser Tyr Asp Ile Asn Trp Val Lys Gln Arg Pro Gly
65                  70                  75                  80

Gln Gly Leu Glu Trp Val Gly Trp Ile Tyr Pro Arg Asp Gly Asp Thr
                85                  90                  95

Lys Tyr Asn Glu Lys Phe Lys Gly Lys Ala Ile Leu Thr Val Asp Thr
            100                 105                 110

Ser Ser Asn Thr Ala Tyr Met Asn Leu His Ser Leu Thr Ser Glu Asp
        115                 120                 125

Ser Ala Val Tyr Phe Cys Ala Arg Leu Thr Gly Pro Tyr Trp Tyr Phe
    130                 135                 140

Asp Val Trp Gly Thr Gly Thr Thr Val Thr Val Ser Ser Gly Ser Ser
145                 150                 155                 160

Ser Ala Ser Thr Lys Gly Pro Ser Val Phe Pro Leu Ala Pro Ser Ser
                165                 170                 175

Lys Ser Thr Ser Gly Gly Thr Ala Ala Leu Gly Cys Leu Val Lys Asp
            180                 185                 190

Tyr Phe Pro Glu Pro Val Thr Val Ser Trp Asn Ser Gly Ala Leu Thr
        195                 200                 205

Ser Gly Val His Thr Phe Pro Ala Val Leu Gln Ser Ser Gly Leu Tyr
    210                 215                 220

Ser Leu Ser Ser Val Val Thr Val Pro Ser Ser Ser Leu Gly Thr Gln
225                 230                 235                 240

Thr Tyr Ile Cys Asn Val Asn His Lys Pro Ser Asn Thr Lys Val Asp
                245                 250                 255

Lys Lys Val Glu Pro Lys Ser Cys Asp Lys Thr His Thr
            260                 265

<210>  32
<211>  265
<212>  PRT
<213>  artificial sequence

<220>
<223>  pDualyx-020pre HC

<400>  32

Met Asn Phe Gly Phe Ser Leu Ile Phe Leu Val Leu Val Leu Lys Gly
1               5                   10                  15

Val Gln Cys Glu Val Lys Leu Val Pro Arg Gln Leu Gln Val Gln Leu
            20                  25                  30

Leu Gln Ser Gly Pro Glu Leu Val Lys Pro Gly Ala Ser Val Lys Leu
        35                  40                  45

Ser Cys Lys Ala Ser Gly Tyr Ser Phe Thr Ser Tyr Asp Ile Asn Trp
    50                  55                  60

Val Lys Gln Arg Pro Gly Gln Gly Leu Glu Trp Val Gly Trp Ile Tyr

65                  70                  75                  80

Pro Arg Asp Gly Asp Thr Lys Tyr Asn Glu Lys Phe Lys Gly Lys Ala
                85                  90                  95

Ile Leu Thr Val Asp Thr Ser Ser Asn Thr Ala Tyr Met Asn Leu His
            100                 105                 110

Ser Leu Thr Ser Glu Asp Ser Ala Val Tyr Phe Cys Ala Arg Leu Thr
        115                 120                 125

Gly Pro Tyr Trp Tyr Phe Asp Val Trp Gly Thr Gly Thr Thr Val Thr
    130                 135                 140

Val Ser Ser Gly Ser Ser Ser Ala Ser Thr Lys Gly Pro Ser Val Phe
145                 150                 155                 160

Pro Leu Ala Pro Ser Ser Lys Ser Thr Ser Gly Gly Thr Ala Ala Leu
                165                 170                 175

Gly Cys Leu Val Lys Asp Tyr Phe Pro Glu Pro Val Thr Val Ser Trp
            180                 185                 190

Asn Ser Gly Ala Leu Thr Ser Gly Val His Thr Phe Pro Ala Val Leu
        195                 200                 205

Gln Ser Ser Gly Leu Tyr Ser Leu Ser Ser Val Val Thr Val Pro Ser
    210                 215                 220

Ser Ser Leu Gly Thr Gln Thr Tyr Ile Cys Asn Val Asn His Lys Pro
225                 230                 235                 240

Ser Asn Thr Lys Val Asp Lys Lys Val Glu Pro Lys Ser Cys Asp Lys
                245                 250                 255

Thr His Thr Cys Pro Pro Cys Pro Ala
            260                 265

<210>  33
<211>  530
<212>  PRT
<213>  artificial sequence

<220>
<223>  pDualyx-044 HC

<400>  33

Met Asn Phe Gly Phe Ser Leu Ile Phe Leu Val Leu Val Leu Lys Gly
1               5                   10                  15

Val Gln Cys Glu Val Lys Leu Val Pro Arg Gln Leu Asp Tyr Lys Asp
            20                  25                  30

Asp Asp Asp Lys Glu Leu Gln Val Gln Leu Leu Gln Ser Gly Pro Glu
        35                  40                  45

Leu Val Lys Pro Gly Ala Ser Val Lys Leu Ser Cys Lys Ala Ser Gly
    50                  55                  60

Tyr Ser Phe Thr Ser Tyr Asp Ile Asn Trp Val Lys Gln Arg Pro Gly
65                  70                  75                  80

Gln Gly Leu Glu Trp Val Gly Trp Ile Tyr Pro Arg Asp Gly Asp Thr
                85                  90                  95

Lys Tyr Asn Glu Lys Phe Lys Gly Lys Ala Ile Leu Thr Val Asp Thr
            100                 105                 110

Ser Ser Asn Thr Ala Tyr Met Asn Leu His Ser Leu Thr Ser Glu Asp
        115                 120                 125

Ser Ala Val Tyr Phe Cys Ala Arg Leu Thr Gly Pro Tyr Trp Tyr Phe
    130                 135                 140

Asp Val Trp Gly Thr Gly Thr Thr Val Thr Val Ser Ser Gly Ser Ser
145                 150                 155                 160

Ser Ala Ser Thr Lys Gly Pro Ser Val Phe Pro Leu Ala Pro Ser Ser
                165                 170                 175

Lys Ser Thr Ser Gly Gly Thr Ala Ala Leu Gly Cys Leu Val Lys Asp
            180                 185                 190

Tyr Phe Pro Glu Pro Val Thr Val Ser Trp Asn Ser Gly Ala Leu Thr
        195                 200                 205

Ser Gly Val His Thr Phe Pro Ala Val Leu Gln Ser Ser Gly Leu Tyr
    210                 215                 220

Ser Leu Ser Ser Val Val Thr Val Pro Ser Ser Ser Leu Gly Thr Gln
225                 230                 235                 240

Thr Tyr Ile Cys Asn Val Asn His Lys Pro Ser Asn Thr Lys Val Asp
                245                 250                 255

Lys Lys Val Glu Pro Lys Ser Cys Asp Lys Thr His Thr Cys Pro Pro

            260                 265                 270

Cys Pro Ala Pro Glu Leu Leu Gly Gly Pro Ser Val Phe Leu Phe Pro
        275                 280                 285

Pro Lys Pro Lys Asp Thr Leu Met Ile Ser Arg Thr Pro Glu Val Thr
    290                 295                 300

Cys Val Val Val Asp Val Ser His Glu Asp Pro Glu Val Lys Phe Asn
305                 310                 315                 320

Trp Tyr Val Asp Gly Val Glu Val His Asn Ala Lys Thr Lys Pro Arg
                325                 330                 335

Glu Glu Gln Tyr Ala Ser Thr Tyr Arg Val Val Ser Val Leu Thr Val
            340                 345                 350

Leu His Gln Asp Trp Leu Asn Gly Lys Glu Tyr Lys Cys Lys Val Ser
        355                 360                 365

Asn Lys Ala Leu Pro Ala Pro Ile Glu Lys Thr Ile Ser Lys Ala Lys
    370                 375                 380

Gly Gln Pro Arg Glu Pro Gln Val Tyr Thr Leu Pro Pro Ser Arg Asp
385                 390                 395                 400

Glu Leu Thr Lys Asn Gln Val Ser Leu Thr Cys Leu Val Lys Gly Phe
                405                 410                 415

Tyr Pro Ser Asp Ile Ala Val Glu Trp Glu Ser Asn Gly Gln Pro Glu
            420                 425                 430

Asn Asn Tyr Lys Thr Thr Pro Pro Val Leu Asp Ser Asp Gly Ser Phe
        435                 440                 445

Phe Leu Tyr Ser Lys Leu Thr Val Asp Lys Ser Arg Trp Gln Gln Gly
    450                 455                 460

Asn Val Phe Ser Cys Ser Val Met His Glu Ala Leu His Asn His Tyr
465                 470                 475                 480

Thr Gln Lys Ser Leu Ser Leu Ser Pro Gly Lys Glu Phe Leu Glu Asp
                485                 490                 495

Ile Ala Cys Gly Cys Ala Ala Ala Pro Asp Ile Lys Asp Leu Leu Ser
            500                 505                 510

Arg Leu Glu Glu Leu Glu Gly Leu Val Ser Ser Leu Arg Glu Gln Gly
        515                 520                 525

Thr Gly
    530

<210>  34
<211>  491
<212>  PRT
<213>  artificial sequence

<220>
<223>  pDualyx-118 HC

<400>  34

Met Asn Phe Gly Phe Ser Leu Ile Phe Leu Val Leu Val Leu Lys Gly
1               5                   10                  15

Val Gln Cys Glu Val Lys Leu Val Pro Arg Gln Leu Asp Tyr Lys Asp
            20                  25                  30

Asp Asp Asp Lys Glu Leu Gln Val Gln Leu Leu Gln Ser Gly Pro Glu
        35                  40                  45

Leu Val Lys Pro Gly Ala Ser Val Lys Leu Ser Cys Lys Ala Ser Gly
    50                  55                  60

Tyr Ser Phe Thr Ser Tyr Asp Ile Asn Trp Val Lys Gln Arg Pro Gly
65                  70                  75                  80

Gln Gly Leu Glu Trp Val Gly Trp Ile Tyr Pro Arg Asp Gly Asp Thr
                85                  90                  95

Lys Tyr Asn Glu Lys Phe Lys Gly Lys Ala Ile Leu Thr Val Asp Thr
            100                 105                 110

Ser Ser Asn Thr Ala Tyr Met Asn Leu His Ser Leu Thr Ser Glu Asp
        115                 120                 125

Ser Ala Val Tyr Phe Cys Ala Arg Leu Thr Gly Pro Tyr Trp Tyr Phe
    130                 135                 140

Asp Val Trp Gly Thr Gly Thr Thr Val Thr Val Ser Ser Gly Ser Ser
145                 150                 155                 160

Ser Ala Ser Thr Lys Gly Pro Ser Val Phe Pro Leu Ala Pro Ser Ser
                165                 170                 175

Lys Ser Thr Ser Gly Gly Thr Ala Ala Leu Gly Cys Leu Val Lys Asp

180                 185                 190

Tyr Phe Pro Glu Pro Val Thr Val Ser Trp Asn Ser Gly Ala Leu Thr
    195                 200                 205

Ser Gly Val His Thr Phe Pro Ala Val Leu Gln Ser Ser Gly Leu Tyr
    210                 215                 220

Ser Leu Ser Ser Val Val Thr Val Pro Ser Ser Ser Leu Gly Thr Gln
225                 230                 235                 240

Thr Tyr Ile Cys Asn Val Asn His Lys Pro Ser Asn Thr Lys Val Asp
                245                 250                 255

Lys Lys Val Glu Pro Lys Ser Cys Asp Lys Thr His Thr Cys Pro Pro
            260                 265                 270

Cys Pro Ala Pro Glu Leu Leu Gly Gly Pro Ser Val Phe Leu Phe Pro
    275                 280                 285

Pro Lys Pro Lys Asp Thr Leu Met Ile Ser Arg Thr Pro Glu Val Thr
    290                 295                 300

Cys Val Val Val Asp Val Ser His Glu Asp Pro Glu Val Lys Phe Asn
305                 310                 315                 320

Trp Tyr Val Asp Gly Val Glu Val His Asn Ala Lys Thr Lys Pro Arg
                325                 330                 335

Glu Glu Gln Tyr Ala Ser Thr Tyr Arg Val Val Ser Val Leu Thr Val
            340                 345                 350

Leu His Gln Asp Trp Leu Asn Gly Lys Glu Tyr Lys Cys Lys Val Ser
    355                 360                 365

Asn Lys Ala Leu Pro Ala Pro Ile Glu Lys Thr Ile Ser Lys Ala Lys
    370                 375                 380

Gly Gln Pro Arg Arg Pro Gln Val Tyr Thr Leu Pro Pro Ser Arg Asp
385                 390                 395                 400

Glu Leu Thr Lys Asn Gln Val Ser Leu Thr Cys Leu Val Lys Gly Phe
                405                 410                 415

Tyr Pro Ser Asp Ile Ala Val Glu Trp Glu Ser Asn Gly Gln Pro Glu
            420                 425                 430

Asn Asn Tyr Lys Thr Thr Pro Pro Val Leu Asp Ser Asp Gly Ser Phe
        435                 440                 445

Phe Leu Tyr Ser Lys Leu Thr Val Asp Lys Ser Arg Trp Gln Gln Gly
    450                 455                 460

Asn Val Phe Ser Cys Ser Val Met His Gly Ala Leu His Asn His Tyr
465                 470                 475                 480

Thr Gln Lys Tyr Leu Ser Leu Ser Pro Gly Lys
                485                 490

<210>  35
<211>  780
<212>  PRT
<213>  artificial sequence

<220>
<223>  pDualyx-119 HC

<400>  35

Met Asn Phe Gly Phe Ser Leu Ile Phe Leu Val Leu Val Leu Lys Gly
1               5                   10                  15

Val Gln Cys Glu Val Lys Leu Val Pro Arg Gln Leu Asp Tyr Lys Asp
            20                  25                  30

Asp Asp Asp Lys Glu Leu Gln Val Gln Leu Leu Gln Ser Gly Pro Glu
        35                  40                  45

Leu Val Lys Pro Gly Ala Ser Val Lys Leu Ser Cys Lys Ala Ser Gly
    50                  55                  60

Tyr Ser Phe Thr Ser Tyr Asp Ile Asn Trp Val Lys Gln Arg Pro Gly
65                  70                  75                  80

Gln Gly Leu Glu Trp Val Gly Trp Ile Tyr Pro Arg Asp Gly Asp Thr
                85                  90                  95

Lys Tyr Asn Glu Lys Phe Lys Gly Lys Ala Ile Leu Thr Val Asp Thr
            100                 105                 110

Ser Ser Asn Thr Ala Tyr Met Asn Leu His Ser Leu Thr Ser Glu Asp
        115                 120                 125

Ser Ala Val Tyr Phe Cys Ala Arg Leu Thr Gly Pro Tyr Trp Tyr Phe
    130                 135                 140

Asp Val Trp Gly Thr Gly Thr Thr Val Thr Val Ser Ser Gly Ser Ser

145                 150                 155                 160

Ser Ala Ser Thr Lys Gly Pro Ser Val Phe Pro Leu Ala Pro Ser Ser
                165                 170                 175

Lys Ser Thr Ser Gly Gly Thr Ala Ala Leu Gly Cys Leu Val Lys Asp
            180                 185                 190

Tyr Phe Pro Glu Pro Val Thr Val Ser Trp Asn Ser Gly Ala Leu Thr
        195                 200                 205

Ser Gly Val His Thr Phe Pro Ala Val Leu Gln Ser Ser Gly Leu Tyr
    210                 215                 220

Ser Leu Ser Ser Val Val Thr Val Pro Ser Ser Ser Leu Gly Thr Gln
225                 230                 235                 240

Thr Tyr Ile Cys Asn Val Asn His Lys Pro Ser Asn Thr Lys Val Asp
                245                 250                 255

Lys Lys Val Glu Pro Lys Ser Cys Asp Lys Thr His Thr Cys Pro Pro
            260                 265                 270

Cys Pro Ala Pro Glu Leu Leu Gly Gly Pro Ser Val Phe Leu Phe Pro
        275                 280                 285

Pro Lys Pro Lys Asp Thr Leu Met Ile Ser Arg Thr Pro Glu Val Thr
    290                 295                 300

Cys Val Val Val Asp Val Ser His Glu Asp Pro Glu Val Lys Phe Asn
305                 310                 315                 320

Trp Tyr Val Asp Gly Val Glu Val His Asn Ala Lys Thr Lys Pro Arg
                325                 330                 335

Glu Glu Gln Tyr Ala Ser Thr Tyr Arg Val Val Ser Val Leu Thr Val
            340                 345                 350

Leu His Gln Asp Trp Leu Asn Gly Lys Glu Tyr Lys Cys Lys Val Ser
        355                 360                 365

Asn Lys Ala Leu Pro Ala Pro Ile Glu Lys Thr Ile Ser Lys Ala Lys
    370                 375                 380

Gly Gln Pro Arg Glu Pro Gln Val Tyr Thr Leu Pro Pro Ser Arg Asp
385                 390                 395                 400

Glu Leu Thr Lys Asn Gln Val Ser Leu Thr Cys Leu Val Lys Gly Phe
                405                 410                 415

Tyr Pro Ser Asp Ile Ala Val Glu Trp Glu Ser Asn Gly Gln Pro Glu
            420                 425                 430

Asn Asn Tyr Lys Thr Thr Pro Pro Val Leu Asp Ser Asp Gly Ser Phe
        435                 440                 445

Phe Leu Tyr Ser Lys Leu Thr Val Asp Lys Ser Arg Trp Gln Gln Gly
    450                 455                 460

Asn Val Phe Ser Cys Ser Val Met His Glu Ala Leu His Asn His Tyr
465                 470                 475                 480

Thr Gln Lys Ser Leu Ser Leu Ser Pro Gly Lys Glu Phe Leu Glu Asp
                485                 490                 495

Ile Ala Cys Gly Cys Ala Ala Ala Pro Asp Ile Lys Asp Leu Leu Ser
            500                 505                 510

Arg Leu Glu Glu Leu Glu Gly Leu Val Ser Ser Leu Arg Glu Gln Gly
        515                 520                 525

Thr Gly Gly Gly Ser Val Glu Gln Val Gln Leu Leu Gln Ser Gly Pro
    530                 535                 540

Glu Leu Val Lys Pro Gly Ala Ser Val Lys Leu Ser Cys Lys Ala Ser
545                 550                 555                 560

Gly Tyr Ser Phe Thr Ser Tyr Asp Ile Asn Trp Val Lys Gln Arg Pro
                565                 570                 575

Gly Gln Gly Leu Glu Trp Val Gly Trp Ile Tyr Pro Arg Asp Gly Asp
            580                 585                 590

Thr Lys Tyr Asn Glu Lys Phe Lys Gly Lys Ala Ile Leu Thr Val Asp
        595                 600                 605

Thr Ser Ser Asn Thr Ala Tyr Met Asn Leu His Ser Leu Thr Ser Glu
    610                 615                 620

Asp Ser Ala Val Tyr Phe Cys Ala Arg Leu Thr Gly Pro Tyr Trp Tyr
625                 630                 635                 640

Phe Asp Val Trp Gly Thr Gly Thr Thr Val Thr Val Ser Ser Arg Ser
                645                 650                 655

Ser Thr Lys Gly Pro Lys Leu Glu Glu Gly Glu Phe Ser Glu Ala Gln
            660                 665                 670

Leu Asp Ile Val Met Thr Gln Ser His Lys Phe Met Ser Thr Ser Val
        675                 680                 685

Gly Asp Arg Val Ser Ile Thr Cys Lys Ala Ser Gln Asp Val Asp Thr
    690                 695                 700

Ala Val Ala Trp Tyr Gln Gln Lys Pro Gly Gln Ser Pro Lys Leu Leu
705                 710                 715                 720

Ile Tyr Trp Ala Ser Thr Arg His Thr Gly Val Pro Asp Arg Phe Thr
                725                 730                 735

Gly Ser Gly Ser Gly Thr Asp Tyr Thr Leu Thr Ile Ser Ser Val Gln
            740                 745                 750

Ala Glu Asp Leu Ala Arg Tyr Tyr Cys Gln Gln Tyr Tyr Ser Val Pro
        755                 760                 765

Pro Thr Phe Gly Gly Gly Thr Lys Leu Glu Ile Lys
    770                 775                 780

<210>  36
<211>  617
<212>  PRT
<213>  artificial sequence

<220>
<223>  pDualyx-021 HC

<400>  36

Met Asn Phe Gly Phe Ser Leu Ile Phe Leu Val Leu Val Leu Lys Gly
1               5                   10                  15

Val Gln Cys Glu Val Lys Leu Val Pro Arg Gln Leu Asp Tyr Lys Asp
            20                  25                  30

Asp Asp Asp Lys Glu Phe Gln Val Gln Leu Leu Gln Ser Gly Pro Glu
        35                  40                  45

Leu Val Lys Pro Gly Ala Ser Val Lys Leu Ser Cys Lys Ala Ser Gly
    50                  55                  60

Tyr Ser Phe Thr Ser Tyr Asp Ile Asn Trp Val Lys Gln Arg Pro Gly
65                  70                  75                  80

Gln Gly Leu Glu Trp Val Gly Trp Ile Tyr Pro Arg Asp Gly Asp Thr
                85                  90                  95

Lys Tyr Asn Glu Lys Phe Lys Gly Lys Ala Ile Leu Thr Val Asp Thr
            100                 105                 110

Ser Ser Asn Thr Ala Tyr Met Asn Leu His Ser Leu Thr Ser Glu Asp
        115                 120                 125

Ser Ala Val Tyr Phe Cys Ala Arg Leu Thr Gly Pro Tyr Trp Tyr Phe
    130                 135                 140

Asp Val Trp Gly Thr Gly Thr Thr Val Thr Val Ser Ser Arg Ser Ser
145                 150                 155                 160

Thr Lys Gly Pro Lys Leu Glu Glu Gly Glu Phe Ser Glu Ala Gln Leu
                165                 170                 175

Asp Ile Val Met Thr Gln Ser His Lys Phe Met Ser Thr Ser Val Gly
            180                 185                 190

Asp Arg Val Ser Ile Thr Cys Lys Ala Ser Gln Asp Val Asp Thr Ala
        195                 200                 205

Val Ala Trp Tyr Gln Gln Lys Pro Gly Gln Ser Pro Lys Leu Leu Ile
    210                 215                 220

Tyr Trp Ala Ser Thr Arg His Thr Gly Val Pro Asp Arg Phe Thr Gly
225                 230                 235                 240

Ser Gly Ser Gly Thr Asp Tyr Thr Leu Thr Ile Ser Ser Val Gln Ala
                245                 250                 255

Glu Asp Leu Ala Arg Tyr Tyr Cys Gln Gln Tyr Tyr Ser Val Pro Pro
            260                 265                 270

Thr Phe Gly Gly Gly Thr Lys Leu Glu Ile Lys Gly Ser Ser Ser Ala
        275                 280                 285

Ser Thr Lys Gly Pro Ser Val Phe Pro Leu Ala Pro Ser Ser Lys Ser
    290                 295                 300

Thr Ser Gly Gly Thr Ala Ala Leu Gly Cys Leu Val Lys Asp Tyr Phe
305                 310                 315                 320

Pro Glu Pro Val Thr Val Ser Trp Asn Ser Gly Ala Leu Thr Ser Gly
                325                 330                 335

Val His Thr Phe Pro Ala Val Leu Gln Ser Ser Gly Leu Tyr Ser Leu
        340                 345                 350

Ser Ser Val Val Thr Val Pro Ser Ser Ser Leu Gly Thr Gln Thr Tyr
        355                 360                 365

Ile Cys Asn Val Asn His Lys Pro Ser Asn Thr Lys Val Asp Lys Lys
    370                 375                 380

Val Glu Pro Lys Ser Cys Asp Lys Thr His Thr Cys Pro Pro Cys Pro
385                 390                 395                 400

Ala Pro Glu Leu Leu Gly Gly Pro Ser Val Phe Leu Phe Pro Pro Lys
                405                 410                 415

Pro Lys Asp Thr Leu Met Ile Ser Arg Thr Pro Glu Val Thr Cys Val
            420                 425                 430

Val Val Asp Val Ser His Glu Asp Pro Glu Val Lys Phe Asn Trp Tyr
        435                 440                 445

Val Asp Gly Val Glu Val His Asn Ala Lys Thr Lys Pro Arg Glu Glu
    450                 455                 460

Gln Tyr Ala Ser Thr Tyr Arg Val Val Ser Val Leu Thr Val Leu His
465                 470                 475                 480

Gln Asp Trp Leu Asn Gly Lys Glu Tyr Lys Cys Lys Val Ser Asn Lys
                485                 490                 495

Ala Leu Pro Ala Pro Ile Glu Lys Thr Ile Ser Lys Ala Lys Gly Gln
            500                 505                 510

Pro Arg Glu Pro Gln Val Tyr Thr Leu Pro Pro Ser Arg Asp Glu Leu
        515                 520                 525

Thr Lys Asn Gln Val Ser Leu Thr Cys Leu Val Lys Gly Phe Tyr Pro
    530                 535                 540

Ser Asp Ile Ala Val Glu Trp Glu Ser Asn Gly Gln Pro Glu Asn Asn
545                 550                 555                 560

Tyr Lys Thr Thr Pro Pro Val Leu Asp Ser Asp Gly Ser Phe Phe Leu
                565                 570                 575

Tyr Ser Lys Leu Thr Val Asp Lys Ser Arg Trp Gln Gln Gly Asn Val
            580                 585                 590

Phe Ser Cys Ser Val Met His Glu Ala Leu His Asn His Tyr Thr Gln
        595                 600                 605

Lys Ser Leu Ser Leu Ser Pro Gly Lys
    610                 615

<210>  37
<211>  864
<212>  PRT
<213>  artificial sequence

<220>
<223>  pDualyx-024 HC

<400>  37

Met Asn Phe Gly Phe Ser Leu Ile Phe Leu Val Leu Val Leu Lys Gly
1               5                   10                  15

Val Gln Cys Glu Val Lys Leu Val Pro Arg Gln Leu Asp Tyr Lys Asp
            20                  25                  30

Asp Asp Asp Lys Glu Phe Gln Val Gln Leu Leu Gln Ser Gly Pro Glu
        35                  40                  45

Leu Val Lys Pro Gly Ala Ser Val Lys Leu Ser Cys Lys Ala Ser Gly
    50                  55                  60

Tyr Ser Phe Thr Ser Tyr Asp Ile Asn Trp Val Lys Gln Arg Pro Gly
65                  70                  75                  80

Gln Gly Leu Glu Trp Val Gly Trp Ile Tyr Pro Arg Asp Gly Asp Thr
                85                  90                  95

Lys Tyr Asn Glu Lys Phe Lys Gly Lys Ala Ile Leu Thr Val Asp Thr
            100                 105                 110

Ser Ser Asn Thr Ala Tyr Met Asn Leu His Ser Leu Thr Ser Glu Asp
        115                 120                 125

Ser Ala Val Tyr Phe Cys Ala Arg Leu Thr Gly Pro Tyr Trp Tyr Phe
    130                 135                 140

Asp Val Trp Gly Thr Gly Thr Thr Val Thr Val Ser Ser Arg Ser Ser
145                 150                 155                 160

Thr Lys Gly Pro Lys Leu Glu Glu Gly Glu Phe Ser Glu Ala Gln Leu
                165                 170                 175

Asp Ile Val Met Thr Gln Ser His Lys Phe Met Ser Thr Ser Val Gly
            180                 185                 190

Asp Arg Val Ser Ile Thr Cys Lys Ala Ser Gln Asp Val Asp Thr Ala
        195                 200                 205

Val Ala Trp Tyr Gln Gln Lys Pro Gly Gln Ser Pro Lys Leu Leu Ile
    210                 215                 220

Tyr Trp Ala Ser Thr Arg His Thr Gly Val Pro Asp Arg Phe Thr Gly
225                 230                 235                 240

Ser Gly Ser Gly Thr Asp Tyr Thr Leu Thr Ile Ser Ser Val Gln Ala
                245                 250                 255

Glu Asp Leu Ala Arg Tyr Tyr Cys Gln Gln Tyr Tyr Ser Val Pro Pro
            260                 265                 270

Thr Phe Gly Gly Gly Thr Lys Leu Glu Ile Lys Gly Ser Ser Ser Ala
        275                 280                 285

Ser Thr Lys Gly Pro Ser Val Phe Pro Leu Ala Pro Ser Ser Lys Ser
    290                 295                 300

Thr Ser Gly Gly Thr Ala Ala Leu Gly Cys Leu Val Lys Asp Tyr Phe
305                 310                 315                 320

Pro Glu Pro Val Thr Val Ser Trp Asn Ser Gly Ala Leu Thr Ser Gly
                325                 330                 335

Val His Thr Phe Pro Ala Val Leu Gln Ser Ser Gly Leu Tyr Ser Leu
            340                 345                 350

Ser Ser Val Val Thr Val Pro Ser Ser Ser Leu Gly Thr Gln Thr Tyr
        355                 360                 365

Ile Cys Asn Val Asn His Lys Pro Ser Asn Thr Lys Val Asp Lys Lys
    370                 375                 380

Val Glu Pro Lys Ser Cys Asp Lys Thr His Thr Cys Pro Pro Cys Pro
385                 390                 395                 400

Ala Pro Glu Leu Leu Gly Gly Pro Ser Val Phe Leu Phe Pro Pro Lys
                405                 410                 415

Pro Lys Asp Thr Leu Met Ile Ser Arg Thr Pro Glu Val Thr Cys Val
            420                 425                 430

Val Val Asp Val Ser His Glu Asp Pro Glu Val Lys Phe Asn Trp Tyr
        435                 440                 445

Val Asp Gly Val Glu Val His Asn Ala Lys Thr Lys Pro Arg Glu Glu
    450                 455                 460

Gln Tyr Ala Ser Thr Tyr Arg Val Val Ser Val Leu Thr Val Leu His
465                 470                 475                 480

Gln Asp Trp Leu Asn Gly Lys Glu Tyr Lys Cys Lys Val Ser Asn Lys
                485                 490                 495

Ala Leu Pro Ala Pro Ile Glu Lys Thr Ile Ser Lys Ala Lys Gly Gln
            500                 505                 510

Pro Arg Glu Pro Gln Val Tyr Thr Leu Pro Pro Ser Arg Asp Glu Leu
        515                 520                 525

Thr Lys Asn Gln Val Ser Leu Thr Cys Leu Val Lys Gly Phe Tyr Pro
    530                 535                 540

Ser Asp Ile Ala Val Glu Trp Glu Ser Asn Gly Gln Pro Glu Asn Asn
545                 550                 555                 560

Tyr Lys Thr Thr Pro Pro Val Leu Asp Ser Asp Gly Ser Phe Phe Leu
                565                 570                 575

Tyr Ser Lys Leu Thr Val Asp Lys Ser Arg Trp Gln Gln Gly Asn Val
            580                 585                 590

Phe Ser Cys Ser Val Met His Glu Ala Leu His Asn His Tyr Thr Gln
        595                 600                 605

Lys Ser Leu Ser Leu Ser Pro Gly Lys Leu Glu Gln Val Gln Leu Leu
    610                 615                 620

Gln Ser Gly Pro Glu Leu Val Lys Pro Gly Ala Ser Val Lys Leu Ser
625                 630                 635                 640

Cys Lys Ala Ser Gly Tyr Ser Phe Thr Ser Tyr Asp Ile Asn Trp Val
                645                 650                 655

Lys Gln Arg Pro Gly Gln Gly Leu Glu Trp Val Gly Trp Ile Tyr Pro
            660                 665                 670

Arg Asp Gly Asp Thr Lys Tyr Asn Glu Lys Phe Lys Gly Lys Ala Ile

675                 680                 685

Leu Thr Val Asp Thr Ser Ser Asn Thr Ala Tyr Met Asn Leu His Ser
    690                 695                 700

Leu Thr Ser Glu Asp Ser Ala Val Tyr Phe Cys Ala Arg Leu Thr Gly
705                 710                 715                 720

Pro Tyr Trp Tyr Phe Asp Val Trp Gly Thr Gly Thr Thr Val Thr Val
                725                 730                 735

Ser Ser Arg Ser Ser Thr Lys Gly Pro Lys Leu Glu Glu Gly Glu Phe
            740                 745                 750

Ser Glu Ala Gln Leu Asp Ile Val Met Thr Gln Ser His Lys Phe Met
        755                 760                 765

Ser Thr Ser Val Gly Asp Arg Val Ser Ile Thr Cys Lys Ala Ser Gln
    770                 775                 780

Asp Val Asp Thr Ala Val Ala Trp Tyr Gln Gln Lys Pro Gly Gln Ser
785                 790                 795                 800

Pro Lys Leu Leu Ile Tyr Trp Ala Ser Thr Arg His Thr Gly Val Pro
                805                 810                 815

Asp Arg Phe Thr Gly Ser Gly Ser Gly Thr Asp Tyr Thr Leu Thr Ile
            820                 825                 830

Ser Ser Val Gln Ala Glu Asp Leu Ala Arg Tyr Tyr Cys Gln Gln Tyr
        835                 840                 845

Tyr Ser Val Pro Pro Thr Phe Gly Gly Gly Thr Lys Leu Glu Ile Lys
    850                 855                 860

<210>  38
<211>  654
<212>  PRT
<213>  artificial sequence

<220>
<223>  pDualyx-051 HC

<400>  38

Met Asn Phe Gly Phe Ser Leu Ile Phe Leu Val Leu Val Leu Lys Gly
1               5                   10                  15

Val Gln Cys Glu Val Lys Leu Val Pro Arg Gln Leu Asp Tyr Lys Asp
            20                  25                  30

Asp Asp Asp Lys Glu Phe Gln Val Gln Leu Leu Gln Ser Gly Pro Glu
        35                  40                  45

Leu Val Lys Pro Gly Ala Ser Val Lys Leu Ser Cys Lys Ala Ser Gly
    50                  55                  60

Tyr Ser Phe Thr Ser Tyr Asp Ile Asn Trp Val Lys Gln Arg Pro Gly
65                  70                  75                  80

Gln Gly Leu Glu Trp Val Gly Trp Ile Tyr Pro Arg Asp Gly Asp Thr
                85                  90                  95

Lys Tyr Asn Glu Lys Phe Lys Gly Lys Ala Ile Leu Thr Val Asp Thr
            100                 105                 110

Ser Ser Asn Thr Ala Tyr Met Asn Leu His Ser Leu Thr Ser Glu Asp
        115                 120                 125

Ser Ala Val Tyr Phe Cys Ala Arg Leu Thr Gly Pro Tyr Trp Tyr Phe
    130                 135                 140

Asp Val Trp Gly Thr Gly Thr Thr Val Thr Val Ser Ser Arg Ser Ser
145                 150                 155                 160

Thr Lys Gly Pro Lys Leu Glu Glu Gly Glu Phe Ser Glu Ala Gln Leu
                165                 170                 175

Asp Ile Val Met Thr Gln Ser His Lys Phe Met Ser Thr Ser Val Gly
            180                 185                 190

Asp Arg Val Ser Ile Thr Cys Lys Ala Ser Gln Asp Val Asp Thr Ala
        195                 200                 205

Val Ala Trp Tyr Gln Gln Lys Pro Gly Gln Ser Pro Lys Leu Leu Ile
    210                 215                 220

Tyr Trp Ala Ser Thr Arg His Thr Gly Val Pro Asp Arg Phe Thr Gly
225                 230                 235                 240

Ser Gly Ser Gly Thr Asp Tyr Thr Leu Thr Ile Ser Ser Val Gln Ala
                245                 250                 255

Glu Asp Leu Ala Arg Tyr Tyr Cys Gln Gln Tyr Tyr Ser Val Pro Pro
            260                 265                 270

Thr Phe Gly Gly Gly Thr Lys Leu Glu Ile Lys Gly Ser Ser Ser Ala

        275                 280                 285

Ser Thr Lys Gly Pro Ser Val Phe Pro Leu Ala Pro Ser Ser Lys Ser
    290                 295                 300

Thr Ser Gly Gly Thr Ala Ala Leu Gly Cys Leu Val Lys Asp Tyr Phe
305                 310                 315                 320

Pro Glu Pro Val Thr Val Ser Trp Asn Ser Gly Ala Leu Thr Ser Gly
                325                 330                 335

Val His Thr Phe Pro Ala Val Leu Gln Ser Ser Gly Leu Tyr Ser Leu
            340                 345                 350

Ser Ser Val Val Thr Val Pro Ser Ser Ser Leu Gly Thr Gln Thr Tyr
        355                 360                 365

Ile Cys Asn Val Asn His Lys Pro Ser Asn Thr Lys Val Asp Lys Lys
    370                 375                 380

Val Glu Pro Lys Ser Cys Asp Lys Thr His Thr Cys Pro Pro Cys Pro
385                 390                 395                 400

Ala Pro Glu Leu Leu Gly Gly Pro Ser Val Phe Leu Phe Pro Pro Lys
                405                 410                 415

Pro Lys Asp Thr Leu Met Ile Ser Arg Thr Pro Glu Val Thr Cys Val
            420                 425                 430

Val Val Asp Val Ser His Glu Asp Pro Glu Val Lys Phe Asn Trp Tyr
        435                 440                 445

Val Asp Gly Val Glu Val His Asn Ala Lys Thr Lys Pro Arg Glu Glu
    450                 455                 460

Gln Tyr Ala Ser Thr Tyr Arg Val Val Ser Val Leu Thr Val Leu His
465                 470                 475                 480

Gln Asp Trp Leu Asn Gly Lys Glu Tyr Lys Cys Lys Val Ser Asn Lys
                485                 490                 495

Ala Leu Pro Ala Pro Ile Glu Lys Thr Ile Ser Lys Ala Lys Gly Gln
            500                 505                 510

Pro Arg Glu Pro Gln Val Tyr Thr Leu Pro Pro Ser Arg Asp Glu Leu
        515                 520                 525

Thr Lys Asn Gln Val Ser Leu Thr Cys Leu Val Lys Gly Phe Tyr Pro
    530                 535                 540

Ser Asp Ile Ala Val Glu Trp Glu Ser Asn Gly Gln Pro Glu Asn Asn
545                 550                 555                 560

Tyr Lys Thr Thr Pro Pro Val Leu Asp Ser Asp Gly Ser Phe Phe Leu
                565                 570                 575

Tyr Ser Lys Leu Thr Val Asp Lys Ser Arg Trp Gln Gln Gly Asn Val
            580                 585                 590

Phe Ser Cys Ser Val Met His Glu Ala Leu His Asn His Tyr Thr Gln
        595                 600                 605

Lys Ser Leu Ser Leu Ser Pro Gly Lys Leu Glu Asp Ile Ala Cys Gly
    610                 615                 620

Cys Ala Ala Ala Pro Asp Ile Lys Asp Leu Leu Ser Arg Leu Glu Glu
625                 630                 635                 640

Leu Glu Gly Leu Val Ser Ser Leu Arg Glu Gln Gly Thr Gly
                645                 650

<210>  39
<211>  741
<212>  PRT
<213>  artificial sequence

<220>
<223>  pDualyx-080 HC

<400>  39

Met Asn Phe Gly Phe Ser Leu Ile Phe Leu Val Leu Val Leu Lys Gly
1               5                   10                  15

Val Gln Cys Glu Val Lys Leu Val Pro Arg Gln Leu Asp Tyr Lys Asp
            20                  25                  30

Asp Asp Asp Lys Glu Leu Gln Val Gln Leu Leu Gln Ser Gly Pro Glu
        35                  40                  45

Leu Val Lys Pro Gly Ala Ser Val Lys Leu Ser Cys Lys Ala Ser Gly
    50                  55                  60

Tyr Ser Phe Thr Ser Tyr Asp Ile Asn Trp Val Lys Gln Arg Pro Gly
65                  70                  75                  80

Gln Gly Leu Glu Trp Val Gly Trp Ile Tyr Pro Arg Asp Gly Asp Thr

85                  90                  95

Lys Tyr Asn Glu Lys Phe Lys Gly Lys Ala Ile Leu Thr Val Asp Thr
        100                 105                 110

Ser Ser Asn Thr Ala Tyr Met Asn Leu His Ser Leu Thr Ser Glu Asp
    115                 120                 125

Ser Ala Val Tyr Phe Cys Ala Arg Leu Thr Gly Pro Tyr Trp Tyr Phe
    130                 135                 140

Asp Val Trp Gly Thr Gly Thr Thr Val Thr Val Ser Ser Gly Ser Ser
145                 150                 155                 160

Ser Ala Ser Thr Lys Gly Pro Ser Val Phe Pro Leu Ala Pro Ser Ser
                165                 170                 175

Lys Ser Thr Ser Gly Gly Thr Ala Ala Leu Gly Cys Leu Val Lys Asp
        180                 185                 190

Tyr Phe Pro Glu Pro Val Thr Val Ser Trp Asn Ser Gly Ala Leu Thr
    195                 200                 205

Ser Gly Val His Thr Phe Pro Ala Val Leu Gln Ser Ser Gly Leu Tyr
    210                 215                 220

Ser Leu Ser Ser Val Val Thr Val Pro Ser Ser Ser Leu Gly Thr Gln
225                 230                 235                 240

Thr Tyr Ile Cys Asn Val Asn His Lys Pro Ser Asn Thr Lys Val Asp
                245                 250                 255

Lys Lys Val Glu Pro Lys Ser Cys Asp Lys Thr His Thr Cys Pro Pro
        260                 265                 270

Cys Pro Ala Pro Glu Leu Leu Gly Gly Pro Ser Val Phe Leu Phe Pro
    275                 280                 285

Pro Lys Pro Lys Asp Thr Leu Met Ile Ser Arg Thr Pro Glu Val Thr
    290                 295                 300

Cys Val Val Val Asp Val Ser His Glu Asp Pro Glu Val Lys Phe Asn
305                 310                 315                 320

Trp Tyr Val Asp Gly Val Glu Val His Asn Ala Lys Thr Lys Pro Arg
                325                 330                 335

Glu Glu Gln Tyr Ala Ser Thr Tyr Arg Val Val Ser Val Leu Thr Val
            340                 345                 350

Leu His Gln Asp Trp Leu Asn Gly Lys Glu Tyr Lys Cys Lys Val Ser
        355                 360                 365

Asn Lys Ala Leu Pro Ala Pro Ile Glu Lys Thr Ile Ser Lys Ala Lys
    370                 375                 380

Gly Gln Pro Arg Glu Pro Gln Val Tyr Thr Leu Pro Pro Ser Arg Asp
385                 390                 395                 400

Glu Leu Thr Lys Asn Gln Val Ser Leu Thr Cys Leu Val Lys Gly Phe
                405                 410                 415

Tyr Pro Ser Asp Ile Ala Val Glu Trp Glu Ser Asn Gly Gln Pro Glu
            420                 425                 430

Asn Asn Tyr Lys Thr Thr Pro Pro Val Leu Asp Ser Asp Gly Ser Phe
        435                 440                 445

Phe Leu Tyr Ser Lys Leu Thr Val Asp Lys Ser Arg Trp Gln Gln Gly
    450                 455                 460

Asn Val Phe Ser Cys Ser Val Met His Glu Ala Leu His Asn His Tyr
465                 470                 475                 480

Thr Gln Lys Ser Leu Ser Leu Ser Pro Gly Lys Glu Phe Leu Glu Gln
                485                 490                 495

Ile Gln Leu Val Gln Ser Gly Pro Glu Val Lys Lys Pro Gly Glu Thr
            500                 505                 510

Val Lys Ile Ser Cys Lys Ala Ser Gly Tyr Thr Phe Thr Ile His Gly
        515                 520                 525

Met Ser Trp Val Lys Gln Ala Pro Gly Lys Gly Leu Lys Trp Met Gly
    530                 535                 540

Trp Ile Asn Thr Tyr Ser Gly Val Pro Thr Tyr Ala Asn Asp Phe Lys
545                 550                 555                 560

Gly Arg Phe Ala Phe Ser Leu Glu Thr Ser Ala Ser Thr Ala Tyr Leu
                565                 570                 575

Gln Ile Asn Asn Leu Lys Asn Glu Asp Thr Ala Thr Tyr Phe Cys Ala
            580                 585                 590

Arg Asp Glu Val Arg Arg Gly Phe Gly Phe Ala Tyr Trp Gly Gln Gly
        595                 600                 605

Thr Leu Val Thr Val Ser Ala Arg Ser Ser Thr Lys Gly Pro Lys Leu
    610                 615                 620

Glu Glu Gly Glu Phe Ser Glu Ala Gln Leu Asp Ile Val Met Ala Gln
625                 630                 635                 640

Ser Gln Lys Phe Met Ser Val Ser Val Gly Asp Arg Val Ser Val Thr
                645                 650                 655

Cys Lys Ala Ser Gln Asn Val Gly Thr Asn Val Ala Trp Tyr Gln Gln
            660                 665                 670

Arg Pro Gly Gln Ser Pro Lys Ala Leu Ile Tyr Ser Ala Ser Tyr Arg
        675                 680                 685

Tyr Ser Gly Val Pro Asp Arg Phe Thr Gly Ser Gly Ser Gly Thr Asp
    690                 695                 700

Phe Thr Leu Thr Ile Ser Asn Val Gln Ser Glu Asp Leu Ala Glu Tyr
705                 710                 715                 720

Phe Cys Gln Gln Phe Asp Ser His Pro Leu Thr Phe Gly Ala Gly Thr
                725                 730                 735

Lys Leu Glu Leu Lys
            740

<210> 40
<211> 742
<212> PRT
<213> artificial sequence

<220>
<223> pDualyx-082 HC

<400> 40

Met Asn Phe Gly Phe Ser Leu Ile Phe Leu Val Leu Val Leu Lys Gly
1               5                   10                  15

Val Gln Cys Glu Val Lys Leu Val Pro Arg Gln Leu Asp Tyr Lys Asp
            20                  25                  30

Asp Asp Asp Lys Glu Leu Gln Val Gln Leu Leu Gln Ser Gly Pro Glu
        35                  40                  45

Leu Val Lys Pro Gly Ala Ser Val Lys Leu Ser Cys Lys Ala Ser Gly
    50                  55                  60

Tyr Ser Phe Thr Ser Tyr Asp Ile Asn Trp Val Lys Gln Arg Pro Gly
65                  70                  75                  80

Gln Gly Leu Glu Trp Val Gly Trp Ile Tyr Pro Arg Asp Gly Asp Thr
                85                  90                  95

Lys Tyr Asn Glu Lys Phe Lys Gly Lys Ala Ile Leu Thr Val Asp Thr
            100                 105                 110

Ser Ser Asn Thr Ala Tyr Met Asn Leu His Ser Leu Thr Ser Glu Asp
        115                 120                 125

Ser Ala Val Tyr Phe Cys Ala Arg Leu Thr Gly Pro Tyr Trp Tyr Phe
    130                 135                 140

Asp Val Trp Gly Thr Gly Thr Thr Val Thr Val Ser Ser Gly Ser Ser
145                 150                 155                 160

Ser Ala Ser Thr Lys Gly Pro Ser Val Phe Pro Leu Ala Pro Ser Ser
                165                 170                 175

Lys Ser Thr Ser Gly Gly Thr Ala Ala Leu Gly Cys Leu Val Lys Asp
            180                 185                 190

Tyr Phe Pro Glu Pro Val Thr Val Ser Trp Asn Ser Gly Ala Leu Thr
        195                 200                 205

Ser Gly Val His Thr Phe Pro Ala Val Leu Gln Ser Ser Gly Leu Tyr
    210                 215                 220

Ser Leu Ser Ser Val Val Thr Val Pro Ser Ser Ser Leu Gly Thr Gln
225                 230                 235                 240

Thr Tyr Ile Cys Asn Val Asn His Lys Pro Ser Asn Thr Lys Val Asp
                245                 250                 255

Lys Lys Val Glu Pro Lys Ser Cys Asp Lys Thr His Thr Cys Pro Pro
            260                 265                 270

Cys Pro Ala Pro Glu Leu Leu Gly Gly Pro Ser Val Phe Leu Phe Pro
        275                 280                 285

Pro Lys Pro Lys Asp Thr Leu Met Ile Ser Arg Thr Pro Glu Val Thr
    290                 295                 300

Cys Val Val Val Asp Val Ser His Glu Asp Pro Glu Val Lys Phe Asn
305                 310                 315                 320

Trp Tyr Val Asp Gly Val Glu Val His Asn Ala Lys Thr Lys Pro Arg
                325                 330                 335

Glu Glu Gln Tyr Ala Ser Thr Tyr Arg Val Val Ser Val Leu Thr Val
            340                 345                 350

Leu His Gln Asp Trp Leu Asn Gly Lys Glu Tyr Lys Cys Lys Val Ser
        355                 360                 365

Asn Lys Ala Leu Pro Ala Pro Ile Glu Lys Thr Ile Ser Lys Ala Lys
    370                 375                 380

Gly Gln Pro Arg Glu Pro Gln Val Tyr Thr Leu Pro Pro Ser Arg Asp
385                 390                 395                 400

Glu Leu Thr Lys Asn Gln Val Ser Leu Thr Cys Leu Val Lys Gly Phe
                405                 410                 415

Tyr Pro Ser Asp Ile Ala Val Glu Trp Glu Ser Asn Gly Gln Pro Glu
            420                 425                 430

Asn Asn Tyr Lys Thr Thr Pro Pro Val Leu Asp Ser Asp Gly Ser Phe
        435                 440                 445

Phe Leu Tyr Ser Lys Leu Thr Val Asp Lys Ser Arg Trp Gln Gln Gly
    450                 455                 460

Asn Val Phe Ser Cys Ser Val Met His Glu Ala Leu His Asn His Tyr
465                 470                 475                 480

Thr Gln Lys Ser Leu Ser Leu Ser Pro Gly Lys Glu Phe Leu Glu Glu
                485                 490                 495

Val Lys Leu Leu Gln Ser Gly Gly Gly Leu Val Gln Pro Gly Gly Ser
            500                 505                 510

Leu Lys Leu Ser Cys Glu Ala Ser Gly Ile Asp Phe Ser Arg Tyr Trp
        515                 520                 525

Met Ser Trp Val Arg Arg Ala Pro Gly Lys Gly Leu Glu Trp Ile Gly
    530                 535                 540

Glu Ser Asp Pro Asp Ser Ser Thr Ile Asn Tyr Ala Pro Ser Leu Lys
545                 550                 555                 560

Asp Lys Phe Ile Ile Ser Arg Asp Ser Ala Arg Asn Thr Leu Tyr Leu
                565                 570                 575

Gln Met Ser Lys Val Arg Ser Glu Asp Thr Ala Leu Tyr Tyr Cys Ala
            580                 585                 590

Arg Glu Gly Tyr Tyr Asp Asn Glu Gly Tyr Phe Asp Val Trp Gly Thr
        595                 600                 605

Gly Thr Thr Val Thr Val Ser Ser Arg Ser Ser Thr Lys Gly Pro Lys
    610                 615                 620

Leu Glu Glu Gly Glu Phe Ser Glu Ala Gln Leu Asn Ile Val Met Thr
625                 630                 635                 640

Gln Ser Pro Lys Ser Leu Ser Met Ser Val Gly Glu Arg Val Thr Leu
                645                 650                 655

Asn Cys Lys Ala Ser Glu Asn Val Lys Thr Tyr Val Ser Trp Tyr Gln
            660                 665                 670

Gln Lys Pro Glu Gln Ser Pro Lys Leu Leu Ile Tyr Gly Ala Ser Asn
        675                 680                 685

Arg Tyr Thr Gly Val Pro Asp Arg Phe Thr Gly Ser Gly Ser Ala Thr
    690                 695                 700

Asp Phe Thr Leu Thr Ile Ser Ser Val Gln Ala Glu Asp Leu Ala Asp
705                 710                 715                 720

Tyr His Cys Gly Gln Ser Tyr Ser Tyr Pro Phe Thr Phe Gly Ala Gly
                725                 730                 735

Thr Lys Leu Glu Arg Lys
            740

<210>  41
<211>  739
<212>  PRT
<213>  artificial sequence

<220>
<223>  pDualyx-063 HC

<400>  41

Met Asn Phe Gly Phe Ser Leu Ile Phe Leu Val Leu Val Leu Lys Gly
1               5                   10                  15

Val Gln Cys Glu Val Lys Leu Val Pro Arg Gln Leu Asp Tyr Lys Asp
            20                  25                  30

Asp Asp Asp Lys Glu Leu Gln Val Gln Leu Leu Gln Ser Gly Pro Glu
        35                  40                  45

Leu Val Lys Pro Gly Ala Ser Val Lys Leu Ser Cys Lys Ala Ser Gly
    50                  55                  60

Tyr Ser Phe Thr Ser Tyr Asp Ile Asn Trp Val Lys Gln Arg Pro Gly
65                  70                  75                  80

Gln Gly Leu Glu Trp Val Gly Trp Ile Tyr Pro Arg Asp Gly Asp Thr
                85                  90                  95

Lys Tyr Asn Glu Lys Phe Lys Gly Lys Ala Ile Leu Thr Val Asp Thr
            100                 105                 110

Ser Ser Asn Thr Ala Tyr Met Asn Leu His Ser Leu Thr Ser Glu Asp
        115                 120                 125

Ser Ala Val Tyr Phe Cys Ala Arg Leu Thr Gly Pro Tyr Trp Tyr Phe
    130                 135                 140

Asp Val Trp Gly Thr Gly Thr Thr Val Thr Val Ser Ser Gly Ser Ser
145                 150                 155                 160

Ser Ala Ser Thr Lys Gly Pro Ser Val Phe Pro Leu Ala Pro Ser Ser
                165                 170                 175

Lys Ser Thr Ser Gly Gly Thr Ala Ala Leu Gly Cys Leu Val Lys Asp
            180                 185                 190

Tyr Phe Pro Glu Pro Val Thr Val Ser Trp Asn Ser Gly Ala Leu Thr
        195                 200                 205

Ser Gly Val His Thr Phe Pro Ala Val Leu Gln Ser Ser Gly Leu Tyr
    210                 215                 220

Ser Leu Ser Ser Val Val Thr Val Pro Ser Ser Ser Leu Gly Thr Gln
225                 230                 235                 240

Thr Tyr Ile Cys Asn Val Asn His Lys Pro Ser Asn Thr Lys Val Asp
                245                 250                 255

Lys Lys Val Glu Pro Lys Ser Cys Asp Lys Thr His Thr Cys Pro Pro
            260                 265                 270

Cys Pro Ala Pro Glu Leu Leu Gly Gly Pro Ser Val Phe Leu Phe Pro
        275                 280                 285

Pro Lys Pro Lys Asp Thr Leu Met Ile Ser Arg Thr Pro Glu Val Thr
    290                 295                 300

Cys Val Val Val Asp Val Ser His Glu Asp Pro Glu Val Lys Phe Asn
305                 310                 315                 320

Trp Tyr Val Asp Gly Val Glu Val His Asn Ala Lys Thr Lys Pro Arg
                325                 330                 335

Glu Glu Gln Tyr Ala Ser Thr Tyr Arg Val Val Ser Val Leu Thr Val
            340                 345                 350

Leu His Gln Asp Trp Leu Asn Gly Lys Glu Tyr Lys Cys Lys Val Ser
        355                 360                 365

Asn Lys Ala Leu Pro Ala Pro Ile Glu Lys Thr Ile Ser Lys Ala Lys
    370                 375                 380

Gly Gln Pro Arg Glu Pro Gln Val Tyr Thr Leu Pro Pro Ser Arg Asp
385                 390                 395                 400

Glu Leu Thr Lys Asn Gln Val Ser Leu Thr Cys Leu Val Lys Gly Phe
                405                 410                 415

Tyr Pro Ser Asp Ile Ala Val Glu Trp Glu Ser Asn Gly Gln Pro Glu
            420                 425                 430

Asn Asn Tyr Lys Thr Thr Pro Pro Val Leu Asp Ser Asp Gly Ser Phe
        435                 440                 445

Phe Leu Tyr Ser Lys Leu Thr Val Asp Lys Ser Arg Trp Gln Gln Gly
    450                 455                 460

Asn Val Phe Ser Cys Ser Val Met His Glu Ala Leu His Asn His Tyr
465                 470                 475                 480

Thr Gln Lys Ser Leu Ser Leu Ser Pro Gly Lys Glu Phe Leu Glu Gln
                485                 490                 495

Val Thr Leu Lys Glu Ser Gly Pro Gly Ile Leu Gln Pro Ser Gln Thr
            500                 505                 510

Leu Ser Leu Thr Cys Ser Phe Ser Gly Phe Ser Leu Ser Thr Phe Gly

515                 520                 525

Met Gly Val Gly Trp Ile Arg Gln Pro Ser Gly Lys Gly Leu Glu Trp
    530                 535                 540

Leu Ala His Ile Trp Trp Asp Asp Asp Lys Tyr Tyr Asn Pro Ala Leu
545                 550                 555                 560

Lys Ser Arg Leu Thr Ile Ser Lys Asp Thr Ser Glu Asn Gln Val Phe
                565                 570                 575

Leu Lys Ile Ala Asn Val Asp Thr Ala Asp Thr Ala Thr Tyr Tyr Cys
            580                 585                 590

Val Arg Ile Ala Gly Thr Arg Tyr Phe Asp Val Trp Gly Thr Gly Thr
        595                 600                 605

Thr Val Thr Val Ser Ser Arg Ser Ser Thr Lys Gly Pro Lys Leu Glu
    610                 615                 620

Glu Gly Glu Phe Ser Glu Ala Gln Leu Asp Ile Gln Met Thr Gln Ser
625                 630                 635                 640

Pro Ser Ser Leu Ser Ala Ser Leu Gly Gly Lys Val Thr Ile Thr Cys
                645                 650                 655

Lys Ala Ser Gln Asp Ile Asn Lys Phe Ile Ala Trp Tyr Gln His Lys
            660                 665                 670

Pro Gly Lys Gly Pro Arg Leu Leu Ile His Tyr Thr Ser Thr Leu Gln
        675                 680                 685

Pro Gly Ile Pro Ser Arg Phe Ser Gly Ser Gly Ser Gly Arg Asp Tyr
    690                 695                 700

Ser Phe Ser Ile Ser Asn Leu Glu Pro Glu Asp Ile Ala Thr Tyr Tyr
705                 710                 715                 720

Cys Leu Gln Tyr Asp Asn Leu Tyr Thr Phe Gly Gly Gly Thr Lys Leu
                725                 730                 735

Glu Ile Lys

<210>  42
<211>  486
<212>  PRT
<213>  artificial sequence

<220>
<223>  pDualyx-006 HC

<400>  42

Met Asn Phe Gly Phe Ser Leu Ile Phe Leu Val Leu Val Leu Lys Gly
1               5                   10                  15

Val Gln Cys Glu Val Lys Leu Val Pro Arg Gln Leu Asp Tyr Lys Asp
            20                  25                  30

Asp Asp Asp Lys Glu Phe Gln Ile Gln Leu Val Gln Ser Gly Pro Glu
        35                  40                  45

Val Lys Lys Pro Gly Glu Thr Val Lys Ile Ser Cys Lys Ala Ser Gly
    50                  55                  60

Tyr Thr Phe Thr Ile His Gly Met Ser Trp Val Lys Gln Ala Pro Gly
65                  70                  75                  80

Lys Gly Leu Lys Trp Met Gly Trp Ile Asn Thr Tyr Ser Gly Val Pro
                85                  90                  95

Thr Tyr Ala Asn Asp Phe Lys Gly Arg Phe Ala Phe Ser Leu Glu Thr
            100                 105                 110

Ser Ala Ser Thr Ala Tyr Leu Gln Ile Asn Asn Leu Lys Asn Glu Asp
        115                 120                 125

Thr Ala Thr Tyr Phe Cys Ala Arg Asp Glu Val Arg Arg Gly Phe Gly
    130                 135                 140

Phe Ala Tyr Trp Gly Gln Gly Thr Leu Val Thr Val Ser Ala Arg Ser
145                 150                 155                 160

Ser Ser Ala Lys Thr Thr Pro Pro Ser Val Tyr Pro Leu Ala Pro Gly
                165                 170                 175

Ser Ala Ala Gln Thr Asn Ser Met Val Thr Leu Gly Cys Leu Val Lys
            180                 185                 190

Gly Tyr Phe Pro Glu Pro Val Thr Val Thr Trp Asn Ser Gly Ser Leu
        195                 200                 205

Ser Ser Gly Val His Thr Phe Pro Ala Val Leu Gln Ser Asp Leu Tyr
    210                 215                 220

Thr Leu Ser Ser Ser Val Thr Val Pro Ser Ser Thr Trp Pro Ser Gln

225                 230                 235                 240

Thr Val Thr Cys Asn Val Ala His Pro Ala Ser Ser Thr Lys Val Asp
                245                 250                 255

Lys Lys Ile Val Pro Arg Asp Cys Gly Cys Lys Pro Cys Ile Cys Thr
            260                 265                 270

Val Pro Glu Val Ser Ser Val Phe Ile Phe Pro Pro Lys Pro Lys Asp
        275                 280                 285

Val Leu Thr Ile Thr Leu Thr Pro Lys Val Thr Cys Val Val Val Ala
    290                 295                 300

Ile Ser Lys Asp Asp Pro Glu Val Gln Phe Ser Trp Phe Val Asp Asp
305                 310                 315                 320

Val Glu Val His Thr Ala Gln Thr Lys Pro Arg Glu Glu Gln Ile Asn
                325                 330                 335

Ser Thr Phe Arg Ser Val Ser Glu Leu Pro Ile Met His Gln Asp Trp
            340                 345                 350

Leu Asn Gly Lys Glu Phe Lys Cys Arg Val Asn Ser Ala Ala Phe Pro
        355                 360                 365

Ala Pro Ile Glu Lys Thr Ile Ser Lys Thr Lys Gly Arg Pro Lys Ala
    370                 375                 380

Pro Gln Val Tyr Thr Ile Pro Pro Pro Lys Glu Gln Met Ala Lys Asp
385                 390                 395                 400

Lys Val Ser Leu Thr Cys Met Ile Thr Asn Phe Phe Pro Glu Asp Ile
                405                 410                 415

Thr Val Glu Trp Gln Trp Asn Gly Gln Pro Ala Glu Asn Tyr Lys Asn
            420                 425                 430

Thr Gln Pro Ile Met Asp Thr Asp Gly Ser Tyr Phe Val Tyr Ser Lys
        435                 440                 445

Leu Asn Val Gln Lys Ser Asn Trp Glu Ala Gly Asn Thr Phe Thr Cys
    450                 455                 460

Ser Val Leu His Glu Gly Leu His Asn His His Thr Glu Lys Ser Leu
465                 470                 475                 480

Ser His Ser Pro Gly Lys
                485

<210>  43
<211>  482
<212>  PRT
<213>  artificial sequence

<220>
<223>  pDualyx-031 HC

<400>  43

Met Asn Phe Gly Phe Ser Leu Ile Phe Leu Val Leu Val Leu Lys Gly
1               5                   10                  15

Val Gln Cys Glu Val Lys Leu Val Pro Arg Gln Leu Gln Ile Gln Leu
            20                  25                  30

Val Gln Ser Gly Pro Glu Val Lys Lys Pro Gly Glu Thr Val Lys Ile
        35                  40                  45

Ser Cys Lys Ala Ser Gly Tyr Thr Phe Thr Ile His Gly Met Ser Trp
    50                  55                  60

Val Lys Gln Ala Pro Gly Lys Gly Leu Lys Trp Met Gly Trp Ile Asn
65                  70                  75                  80

Thr Tyr Ser Gly Val Pro Thr Tyr Ala Asn Asp Phe Lys Gly Arg Phe
                85                  90                  95

Ala Phe Ser Leu Glu Thr Ser Ala Ser Thr Ala Tyr Leu Gln Ile Asn
            100                 105                 110

Asn Leu Lys Asn Glu Asp Thr Ala Thr Tyr Phe Cys Ala Arg Asp Glu
        115                 120                 125

Val Arg Arg Gly Phe Gly Phe Ala Tyr Trp Gly Gln Gly Thr Leu Val
    130                 135                 140

Thr Val Ser Ala Gly Ser Ser Ser Ala Ser Thr Lys Gly Pro Ser Val
145                 150                 155                 160

Phe Pro Leu Ala Pro Ser Ser Lys Ser Thr Ser Gly Gly Thr Ala Ala
                165                 170                 175

Leu Gly Cys Leu Val Lys Asp Tyr Phe Pro Glu Pro Val Thr Val Ser
            180                 185                 190

Trp Asn Ser Gly Ala Leu Thr Ser Gly Val His Thr Phe Pro Ala Val

195                 200                 205

Leu Gln Ser Ser Gly Leu Tyr Ser Leu Ser Ser Val Val Thr Val Pro
    210                 215                 220

Ser Ser Ser Leu Gly Thr Gln Thr Tyr Ile Cys Asn Val Asn His Lys
225                 230                 235                 240

Pro Ser Asn Thr Lys Val Asp Lys Lys Val Glu Pro Lys Ser Cys Asp
                245                 250                 255

Lys Thr His Thr Cys Pro Pro Cys Pro Ala Pro Glu Leu Leu Gly Gly
            260                 265                 270

Pro Ser Val Phe Leu Phe Pro Pro Lys Pro Lys Asp Thr Leu Met Ile
        275                 280                 285

Ser Arg Thr Pro Glu Val Thr Cys Val Val Val Asp Val Ser His Glu
    290                 295                 300

Asp Pro Glu Val Lys Phe Asn Trp Tyr Val Asp Gly Val Glu Val His
305                 310                 315                 320

Asn Ala Lys Thr Lys Pro Arg Glu Glu Gln Tyr Asn Ser Thr Tyr Arg
                325                 330                 335

Val Val Ser Val Leu Thr Val Leu His Gln Asp Trp Leu Asn Gly Lys
            340                 345                 350

Glu Tyr Lys Cys Lys Val Ser Asn Lys Ala Leu Pro Ala Pro Ile Glu
        355                 360                 365

Lys Thr Ile Ser Lys Ala Lys Gly Gln Pro Arg Glu Pro Gln Val Tyr
    370                 375                 380

Thr Leu Pro Pro Ser Arg Asp Glu Leu Thr Lys Asn Gln Val Ser Leu
385                 390                 395                 400

Thr Cys Leu Val Lys Gly Phe Tyr Pro Ser Asp Ile Ala Val Glu Trp
                405                 410                 415

Glu Ser Asn Gly Gln Pro Glu Asn Asn Tyr Lys Thr Thr Pro Pro Val
            420                 425                 430

Leu Asp Ser Asp Gly Ser Phe Phe Leu Tyr Ser Lys Leu Thr Val Asp
        435                 440                 445

Lys Ser Arg Trp Gln Gln Gly Asn Val Phe Ser Cys Ser Val Met His
    450                 455                 460

Glu Ala Leu His Asn His Tyr Thr Gln Lys Ser Leu Ser Leu Ser Pro
465                 470                 475                 480

Gly Lys

<210>  44
<211>  493
<212>  PRT
<213>  artificial sequence

<220>
<223>  pDualyx-091 HC

<400>  44

Met Asn Phe Gly Phe Ser Leu Ile Phe Leu Val Leu Val Leu Lys Gly
1               5                   10                  15

Val Gln Cys Glu Val Lys Leu Val Pro Arg Gln Leu Asp Tyr Lys Asp
            20                  25                  30

Asp Asp Asp Lys Glu Phe Glu Val Lys Leu Leu Gln Ser Gly Gly Gly
        35                  40                  45

Leu Val Gln Pro Gly Gly Ser Leu Lys Leu Ser Cys Glu Ala Ser Gly
    50                  55                  60

Ile Asp Phe Ser Arg Tyr Trp Met Ser Trp Val Arg Arg Ala Pro Gly
65                  70                  75                  80

Lys Gly Leu Glu Trp Ile Gly Glu Ser Asp Pro Asp Ser Ser Thr Ile
                85                  90                  95

Asn Tyr Ala Pro Ser Leu Lys Asp Lys Phe Ile Ile Ser Arg Asp Ser
            100                 105                 110

Ala Arg Asn Thr Leu Tyr Leu Gln Met Ser Lys Val Arg Ser Glu Asp
        115                 120                 125

Thr Ala Leu Tyr Tyr Cys Ala Arg Glu Gly Tyr Tyr Asp Asn Glu Gly
    130                 135                 140

Tyr Phe Asp Val Trp Gly Thr Gly Thr Thr Val Thr Val Ser Ser Arg
145                 150                 155                 160

Ser Ser Ser Ala Ser Thr Lys Gly Pro Ser Val Phe Pro Leu Ala Pro

165                 170                 175

Ser Ser Lys Ser Thr Ser Gly Gly Thr Ala Ala Leu Gly Cys Leu Val
            180                 185                 190

Lys Asp Tyr Phe Pro Glu Pro Val Thr Val Ser Trp Asn Ser Gly Ala
        195                 200                 205

Leu Thr Ser Gly Val His Thr Phe Pro Ala Val Leu Gln Ser Ser Gly
    210                 215                 220

Leu Tyr Ser Leu Ser Ser Val Val Thr Val Pro Ser Ser Ser Leu Gly
225                 230                 235                 240

Thr Gln Thr Tyr Ile Cys Asn Val Asn His Lys Pro Ser Asn Thr Lys
                245                 250                 255

Val Asp Lys Lys Val Glu Pro Lys Ser Cys Asp Lys Thr His Thr Cys
            260                 265                 270

Pro Pro Cys Pro Ala Pro Glu Leu Leu Gly Gly Pro Ser Val Phe Leu
        275                 280                 285

Phe Pro Pro Lys Pro Lys Asp Thr Leu Met Ile Ser Arg Thr Pro Glu
    290                 295                 300

Val Thr Cys Val Val Val Asp Val Ser His Glu Asp Pro Glu Val Lys
305                 310                 315                 320

Phe Asn Trp Tyr Val Asp Gly Val Glu Val His Asn Ala Lys Thr Lys
                325                 330                 335

Pro Arg Glu Glu Gln Tyr Ala Ser Thr Tyr Arg Val Val Ser Val Leu
            340                 345                 350

Thr Val Leu His Gln Asp Trp Leu Asn Gly Lys Glu Tyr Lys Cys Lys
        355                 360                 365

Val Ser Asn Lys Ala Leu Pro Ala Pro Ile Glu Lys Thr Ile Ser Lys
    370                 375                 380

Ala Lys Gly Gln Pro Arg Glu Pro Gln Val Tyr Thr Leu Pro Pro Ser
385                 390                 395                 400

Arg Asp Glu Leu Thr Lys Asn Gln Val Ser Leu Thr Cys Leu Val Lys
                405                 410                 415

Gly Phe Tyr Pro Ser Asp Ile Ala Val Glu Trp Glu Ser Asn Gly Gln
            420                 425                 430

Pro Glu Asn Asn Tyr Lys Thr Thr Pro Pro Val Leu Asp Ser Asp Gly
        435                 440                 445

Ser Phe Phe Leu Tyr Ser Lys Leu Thr Val Asp Lys Ser Arg Trp Gln
    450                 455                 460

Gln Gly Asn Val Phe Ser Cys Ser Val Met His Glu Ala Leu His Asn
465                 470                 475                 480

His Tyr Thr Gln Lys Ser Leu Ser Leu Ser Pro Gly Lys
                485                 490

<210>  45
<211>  491
<212>  PRT
<213>  artificial sequence

<220>
<223>  pDualyx-075 HC

<400>  45

Met Asn Phe Gly Phe Ser Leu Ile Phe Leu Val Leu Val Leu Lys Gly
1               5                   10                  15

Val Gln Cys Glu Val Lys Leu Val Pro Arg Gln Leu Asp Tyr Lys Asp
            20                  25                  30

Asp Asp Asp Lys Glu Phe Gln Val Thr Leu Lys Glu Ser Gly Pro Gly
        35                  40                  45

Ile Leu Gln Pro Ser Gln Thr Leu Ser Leu Thr Cys Ser Phe Ser Gly
    50                  55                  60

Phe Ser Leu Ser Thr Phe Gly Met Gly Val Gly Trp Ile Arg Gln Pro
65                  70                  75                  80

Ser Gly Lys Gly Leu Glu Trp Leu Ala His Ile Trp Trp Asp Asp Asp
                85                  90                  95

Lys Tyr Tyr Asn Pro Ala Leu Lys Ser Arg Leu Thr Ile Ser Lys Asp
            100                 105                 110

Thr Ser Glu Asn Gln Val Phe Leu Lys Ile Ala Asn Val Asp Thr Ala
        115                 120                 125

Asp Thr Ala Thr Tyr Tyr Cys Val Arg Ile Ala Gly Thr Arg Tyr Phe

130                 135                 140

Asp Val Trp Gly Thr Gly Thr Thr Val Thr Val Ser Ser Arg Ser Ser
145                 150                 155                 160

Ser Ala Ser Thr Lys Gly Pro Ser Val Phe Pro Leu Ala Pro Ser Ser
                165                 170                 175

Lys Ser Thr Ser Gly Gly Thr Ala Ala Leu Gly Cys Leu Val Lys Asp
            180                 185                 190

Tyr Phe Pro Glu Pro Val Thr Val Ser Trp Asn Ser Gly Ala Leu Thr
        195                 200                 205

Ser Gly Val His Thr Phe Pro Ala Val Leu Gln Ser Ser Gly Leu Tyr
    210                 215                 220

Ser Leu Ser Ser Val Val Thr Val Pro Ser Ser Ser Leu Gly Thr Gln
225                 230                 235                 240

Thr Tyr Ile Cys Asn Val Asn His Lys Pro Ser Asn Thr Lys Val Asp
                245                 250                 255

Lys Lys Val Glu Pro Lys Ser Cys Asp Lys Thr His Thr Cys Pro Pro
            260                 265                 270

Cys Pro Ala Pro Glu Leu Leu Gly Gly Pro Ser Val Phe Leu Phe Pro
        275                 280                 285

Pro Lys Pro Lys Asp Thr Leu Met Ile Ser Arg Thr Pro Glu Val Thr
    290                 295                 300

Cys Val Val Val Asp Val Ser His Glu Asp Pro Glu Val Lys Phe Asn
305                 310                 315                 320

Trp Tyr Val Asp Gly Val Glu Val His Asn Ala Lys Thr Lys Pro Arg
                325                 330                 335

Glu Glu Gln Tyr Ala Ser Thr Tyr Arg Val Val Ser Val Leu Thr Val
            340                 345                 350

Leu His Gln Asp Trp Leu Asn Gly Lys Glu Tyr Lys Cys Lys Val Ser
        355                 360                 365

Asn Lys Ala Leu Pro Ala Pro Ile Glu Lys Thr Ile Ser Lys Ala Lys
    370                 375                 380

Gly Gln Pro Arg Glu Pro Gln Val Tyr Thr Leu Pro Pro Ser Arg Asp
385                 390                 395                 400

Glu Leu Thr Lys Asn Gln Val Ser Leu Thr Cys Leu Val Lys Gly Phe
                405                 410                 415

Tyr Pro Ser Asp Ile Ala Val Glu Trp Glu Ser Asn Gly Gln Pro Glu
            420                 425                 430

Asn Asn Tyr Lys Thr Thr Pro Pro Val Leu Asp Ser Asp Gly Ser Phe
        435                 440                 445

Phe Leu Tyr Ser Lys Leu Thr Val Asp Lys Ser Arg Trp Gln Gln Gly
    450                 455                 460

Asn Val Phe Ser Cys Ser Val Met His Glu Ala Leu His Asn His Tyr
465                 470                 475                 480

Thr Gln Lys Ser Leu Ser Leu Ser Pro Gly Lys
                485                 490

<210>  46
<211>  730
<212>  PRT
<213>  artificial sequence

<220>
<223>  pDualyx-017pre HC

<400>  46

Met Asn Phe Gly Phe Ser Leu Ile Phe Leu Val Leu Val Leu Lys Gly
1               5                   10                  15

Val Gln Cys Glu Val Lys Leu Val Pro Arg Gln Leu Gln Ile Gln Leu
            20                  25                  30

Val Gln Ser Gly Pro Glu Val Lys Lys Pro Gly Glu Thr Val Lys Ile
        35                  40                  45

Ser Cys Lys Ala Ser Gly Tyr Thr Phe Thr Ile His Gly Met Ser Trp
    50                  55                  60

Val Lys Gln Ala Pro Gly Lys Gly Leu Lys Trp Met Gly Trp Ile Asn
65                  70                  75                  80

Thr Tyr Ser Gly Val Pro Thr Tyr Ala Asn Asp Phe Lys Gly Arg Phe
                85                  90                  95

Ala Phe Ser Leu Glu Thr Ser Ala Ser Thr Ala Tyr Leu Gln Ile Asn

            100                 105                 110

Asn Leu Lys Asn Glu Asp Thr Ala Thr Tyr Phe Cys Ala Arg Asp Glu
        115                 120                 125

Val Arg Arg Gly Phe Gly Phe Ala Tyr Trp Gly Gln Gly Thr Leu Val
    130                 135                 140

Thr Val Ser Ala Gly Ser Ser Ser Ala Ser Thr Lys Gly Pro Ser Val
145                 150                 155                 160

Phe Pro Leu Ala Pro Ser Ser Lys Ser Thr Ser Gly Gly Thr Ala Ala
                165                 170                 175

Leu Gly Cys Leu Val Lys Asp Tyr Phe Pro Glu Pro Val Thr Val Ser
            180                 185                 190

Trp Asn Ser Gly Ala Leu Thr Ser Gly Val His Thr Phe Pro Ala Val
        195                 200                 205

Leu Gln Ser Ser Gly Leu Tyr Ser Leu Ser Ser Val Val Thr Val Pro
    210                 215                 220

Ser Ser Ser Leu Gly Thr Gln Thr Tyr Ile Cys Asn Val Asn His Lys
225                 230                 235                 240

Pro Ser Asn Thr Lys Val Asp Lys Lys Val Glu Pro Lys Ser Cys Asp
                245                 250                 255

Lys Thr His Thr Cys Pro Pro Cys Pro Ala Pro Glu Leu Leu Gly Gly
            260                 265                 270

Pro Ser Val Phe Leu Phe Pro Pro Lys Pro Lys Asp Thr Leu Met Ile
        275                 280                 285

Ser Arg Thr Pro Glu Val Thr Cys Val Val Val Asp Val Ser His Glu
    290                 295                 300

Asp Pro Glu Val Lys Phe Asn Trp Tyr Val Asp Gly Val Glu Val His
305                 310                 315                 320

Asn Ala Lys Thr Lys Pro Arg Glu Glu Gln Tyr Ala Ser Thr Tyr Arg
                325                 330                 335

Val Val Ser Val Leu Thr Val Leu His Gln Asp Trp Leu Asn Gly Lys
            340                 345                 350

Glu Tyr Lys Cys Lys Val Ser Asn Lys Ala Leu Pro Ala Pro Ile Glu
        355                 360                 365

Lys Thr Ile Ser Lys Ala Lys Gly Gln Pro Arg Glu Pro Gln Val Tyr
    370                 375                 380

Thr Leu Pro Pro Ser Arg Asp Glu Leu Thr Lys Asn Gln Val Ser Leu
385                 390                 395                 400

Thr Cys Leu Val Lys Gly Phe Tyr Pro Ser Asp Ile Ala Val Glu Trp
                405                 410                 415

Glu Ser Asn Gly Gln Pro Glu Asn Asn Tyr Lys Thr Thr Pro Pro Val
            420                 425                 430

Leu Asp Ser Asp Gly Ser Phe Phe Leu Tyr Ser Lys Leu Thr Val Asp
        435                 440                 445

Lys Ser Arg Trp Gln Gln Gly Asn Val Phe Ser Cys Ser Val Met His
    450                 455                 460

Glu Ala Leu His Asn His Tyr Thr Gln Lys Ser Leu Ser Leu Ser Pro
465                 470                 475                 480

Gly Lys Glu Phe Gln Ile Gln Leu Val Gln Ser Gly Pro Glu Val Lys
                485                 490                 495

Lys Pro Gly Glu Thr Val Lys Ile Ser Cys Lys Ala Ser Gly Tyr Thr
            500                 505                 510

Phe Thr Ile His Gly Met Ser Trp Val Lys Gln Ala Pro Gly Lys Gly
        515                 520                 525

Leu Lys Trp Met Gly Trp Ile Asn Thr Tyr Ser Gly Val Pro Thr Tyr
    530                 535                 540

Ala Asn Asp Phe Lys Gly Arg Phe Ala Phe Ser Leu Glu Thr Ser Ala
545                 550                 555                 560

Ser Thr Ala Tyr Leu Gln Ile Asn Asn Leu Lys Asn Glu Asp Thr Ala
                565                 570                 575

Thr Tyr Phe Cys Ala Arg Asp Glu Val Arg Arg Gly Phe Gly Phe Ala
            580                 585                 590

Tyr Trp Gly Gln Gly Thr Leu Val Thr Val Ser Ala Arg Ser Ser Thr
        595                 600                 605

Lys Gly Pro Lys Leu Glu Glu Gly Glu Phe Ser Glu Ala Gln Leu Asp
    610                 615                 620

Ile Val Met Ala Gln Ser Gln Lys Phe Met Ser Val Ser Val Gly Asp
625                 630                 635                 640

Arg Val Ser Val Thr Cys Lys Ala Ser Gln Asn Val Gly Thr Asn Val
                645                 650                 655

Ala Trp Tyr Gln Gln Arg Pro Gly Gln Ser Pro Lys Ala Leu Ile Tyr
            660                 665                 670

Ser Ala Ser Tyr Arg Tyr Ser Gly Val Pro Asp Arg Phe Thr Gly Ser
        675                 680                 685

Gly Ser Gly Thr Asp Phe Thr Leu Thr Ile Ser Asn Val Gln Ser Glu
    690                 695                 700

Asp Leu Ala Glu Tyr Phe Cys Gln Gln Phe Asp Ser His Pro Leu Thr
705                 710                 715                 720

Phe Gly Ala Gly Thr Lys Leu Glu Leu Lys
                725                 730

<210>  47
<211>  742
<212>  PRT
<213>  artificial sequence

<220>
<223>  pDualyx-108 HC

<400>  47

Met Asn Phe Gly Phe Ser Leu Ile Phe Leu Val Leu Val Leu Lys Gly
1               5                   10                  15

Val Gln Cys Glu Val Lys Leu Val Pro Arg Gln Leu Asp Tyr Lys Asp
            20                  25                  30

Asp Asp Asp Lys Glu Phe Glu Val Lys Leu Leu Gln Ser Gly Gly Gly
        35                  40                  45

Leu Val Gln Pro Gly Gly Ser Leu Lys Leu Ser Cys Glu Ala Ser Gly
    50                  55                  60

Ile Asp Phe Ser Arg Tyr Trp Met Ser Trp Val Arg Arg Ala Pro Gly
65                  70                  75                  80

Lys Gly Leu Glu Trp Ile Gly Glu Ser Asp Pro Asp Ser Ser Thr Ile
                85                  90                  95

Asn Tyr Ala Pro Ser Leu Lys Asp Lys Phe Ile Ile Ser Arg Asp Ser
            100                 105                 110

Ala Arg Asn Thr Leu Tyr Leu Gln Met Ser Lys Val Arg Ser Glu Asp
        115                 120                 125

Thr Ala Leu Tyr Tyr Cys Ala Arg Glu Gly Tyr Tyr Asp Asn Glu Gly
    130                 135                 140

Tyr Phe Asp Val Trp Gly Thr Gly Thr Thr Val Thr Val Ser Ser Arg
145                 150                 155                 160

Ser Ser Ser Ala Ser Thr Lys Gly Pro Ser Val Phe Pro Leu Ala Pro
                165                 170                 175

Ser Ser Lys Ser Thr Ser Gly Gly Thr Ala Ala Leu Gly Cys Leu Val
            180                 185                 190

Lys Asp Tyr Phe Pro Glu Pro Val Thr Val Ser Trp Asn Ser Gly Ala
        195                 200                 205

Leu Thr Ser Gly Val His Thr Phe Pro Ala Val Leu Gln Ser Ser Gly
    210                 215                 220

Leu Tyr Ser Leu Ser Ser Val Val Thr Val Pro Ser Ser Ser Leu Gly
225                 230                 235                 240

Thr Gln Thr Tyr Ile Cys Asn Val Asn His Lys Pro Ser Asn Thr Lys
                245                 250                 255

Val Asp Lys Lys Val Glu Pro Lys Ser Cys Asp Lys Thr His Thr Cys
            260                 265                 270

Pro Pro Cys Pro Ala Pro Glu Leu Leu Gly Gly Pro Ser Val Phe Leu
        275                 280                 285

Phe Pro Pro Lys Pro Lys Asp Thr Leu Met Ile Ser Arg Thr Pro Glu
    290                 295                 300

Val Thr Cys Val Val Val Asp Val Ser His Glu Asp Pro Glu Val Lys
305                 310                 315                 320

Phe Asn Trp Tyr Val Asp Gly Val Glu Val His Asn Ala Lys Thr Lys
                325                 330                 335

Pro Arg Glu Glu Gln Tyr Ala Ser Thr Tyr Arg Val Val Ser Val Leu
        340                 345                 350

Thr Val Leu His Gln Asp Trp Leu Asn Gly Lys Glu Tyr Lys Cys Lys
    355                 360                 365

Val Ser Asn Lys Ala Leu Pro Ala Pro Ile Glu Lys Thr Ile Ser Lys
370                 375                 380

Ala Lys Gly Gln Pro Arg Glu Pro Gln Val Tyr Thr Leu Pro Pro Ser
385                 390                 395                 400

Arg Asp Glu Leu Thr Lys Asn Gln Val Ser Leu Thr Cys Leu Val Lys
                405                 410                 415

Gly Phe Tyr Pro Ser Asp Ile Ala Val Glu Trp Glu Ser Asn Gly Gln
            420                 425                 430

Pro Glu Asn Asn Tyr Lys Thr Thr Pro Pro Val Leu Asp Ser Asp Gly
        435                 440                 445

Ser Phe Phe Leu Tyr Ser Lys Leu Thr Val Asp Lys Ser Arg Trp Gln
    450                 455                 460

Gln Gly Asn Val Phe Ser Cys Ser Val Met His Glu Ala Leu His Asn
465                 470                 475                 480

His Tyr Thr Gln Lys Ser Leu Ser Leu Ser Pro Gly Lys Leu Glu Glu
                485                 490                 495

Val Lys Leu Leu Gln Ser Gly Gly Gly Leu Val Gln Pro Gly Gly Ser
            500                 505                 510

Leu Lys Leu Ser Cys Glu Ala Ser Gly Ile Asp Phe Ser Arg Tyr Trp
        515                 520                 525

Met Ser Trp Val Arg Arg Ala Pro Gly Lys Gly Leu Glu Trp Ile Gly
    530                 535                 540

Glu Ser Asp Pro Asp Ser Ser Thr Ile Asn Tyr Ala Pro Ser Leu Lys
545                 550                 555                 560

Asp Lys Phe Ile Ile Ser Arg Asp Ser Ala Arg Asn Thr Leu Tyr Leu
                565                 570                 575

Gln Met Ser Lys Val Arg Ser Glu Asp Thr Ala Leu Tyr Tyr Cys Ala
            580                 585                 590

Arg Glu Gly Tyr Tyr Asp Asn Glu Gly Tyr Phe Asp Val Trp Gly Thr
        595                 600                 605

Gly Thr Thr Val Thr Val Ser Ser Arg Ser Ser Thr Lys Gly Pro Lys
    610                 615                 620

Leu Glu Glu Gly Glu Phe Ser Glu Ala Gln Leu Asn Ile Val Met Thr
625                 630                 635                 640

Gln Ser Pro Lys Ser Leu Ser Met Ser Val Gly Glu Arg Val Thr Leu
                645                 650                 655

Asn Cys Lys Ala Ser Glu Asn Val Lys Thr Tyr Val Ser Trp Tyr Gln
            660                 665                 670

Gln Lys Pro Glu Gln Ser Pro Lys Leu Leu Ile Tyr Gly Ala Ser Asn
        675                 680                 685

Arg Tyr Thr Gly Val Pro Asp Arg Phe Thr Gly Ser Gly Ser Ala Thr
    690                 695                 700

Asp Phe Thr Leu Thr Ile Ser Ser Val Gln Ala Glu Asp Leu Ala Asp
705                 710                 715                 720

Tyr His Cys Gly Gln Ser Tyr Ser Tyr Pro Phe Thr Phe Gly Ala Gly
                725                 730                 735

Thr Lys Leu Glu Arg Lys
            740

<210>  48
<211>  737
<212>  PRT
<213>  artificial sequence

<220>
<223>  pDualyx-111 HC

<400>  48

Met Asn Phe Gly Phe Ser Leu Ile Phe Leu Val Leu Val Leu Lys Gly
1               5                   10                  15

Val Gln Cys Glu Val Lys Leu Val Pro Arg Gln Leu Asp Tyr Lys Asp
            20                  25                  30

Asp Asp Asp Lys Glu Phe Gln Val Thr Leu Lys Glu Ser Gly Pro Gly
        35                  40                  45

Ile Leu Gln Pro Ser Gln Thr Leu Ser Leu Thr Cys Ser Phe Ser Gly
    50                  55                  60

Phe Ser Leu Ser Thr Phe Gly Met Gly Val Gly Trp Ile Arg Gln Pro
65                  70                  75                  80

Ser Gly Lys Gly Leu Glu Trp Leu Ala His Ile Trp Trp Asp Asp Asp
                85                  90                  95

Lys Tyr Tyr Asn Pro Ala Leu Lys Ser Arg Leu Thr Ile Ser Lys Asp
            100                 105                 110

Thr Ser Glu Asn Gln Val Phe Leu Lys Ile Ala Asn Val Asp Thr Ala
        115                 120                 125

Asp Thr Ala Thr Tyr Tyr Cys Val Arg Ile Ala Gly Thr Arg Tyr Phe
    130                 135                 140

Asp Val Trp Gly Thr Gly Thr Thr Val Thr Val Ser Ser Arg Ser Ser
145                 150                 155                 160

Ser Ala Ser Thr Lys Gly Pro Ser Val Phe Pro Leu Ala Pro Ser Ser
                165                 170                 175

Lys Ser Thr Ser Gly Gly Thr Ala Ala Leu Gly Cys Leu Val Lys Asp
            180                 185                 190

Tyr Phe Pro Glu Pro Val Thr Val Ser Trp Asn Ser Gly Ala Leu Thr
        195                 200                 205

Ser Gly Val His Thr Phe Pro Ala Val Leu Gln Ser Ser Gly Leu Tyr
    210                 215                 220

Ser Leu Ser Ser Val Val Thr Val Pro Ser Ser Ser Leu Gly Thr Gln
225                 230                 235                 240

Thr Tyr Ile Cys Asn Val Asn His Lys Pro Ser Asn Thr Lys Val Asp
                245                 250                 255

Lys Lys Val Glu Pro Lys Ser Cys Asp Lys Thr His Thr Cys Pro Pro
            260                 265                 270

Cys Pro Ala Pro Glu Leu Leu Gly Gly Pro Ser Val Phe Leu Phe Pro
        275                 280                 285

Pro Lys Pro Lys Asp Thr Leu Met Ile Ser Arg Thr Pro Glu Val Thr
    290                 295                 300

Cys Val Val Val Asp Val Ser His Glu Asp Pro Glu Val Lys Phe Asn
305                 310                 315                 320

Trp Tyr Val Asp Gly Val Glu Val His Asn Ala Lys Thr Lys Pro Arg
                325                 330                 335

Glu Glu Gln Tyr Ala Ser Thr Tyr Arg Val Val Ser Val Leu Thr Val
            340                 345                 350

Leu His Gln Asp Trp Leu Asn Gly Lys Glu Tyr Lys Cys Lys Val Ser
        355                 360                 365

Asn Lys Ala Leu Pro Ala Pro Ile Glu Lys Thr Ile Ser Lys Ala Lys
    370                 375                 380

Gly Gln Pro Arg Glu Pro Gln Val Tyr Thr Leu Pro Pro Ser Arg Asp
385                 390                 395                 400

Glu Leu Thr Lys Asn Gln Val Ser Leu Thr Cys Leu Val Lys Gly Phe
                405                 410                 415

Tyr Pro Ser Asp Ile Ala Val Glu Trp Glu Ser Asn Gly Gln Pro Glu
            420                 425                 430

Asn Asn Tyr Lys Thr Thr Pro Pro Val Leu Asp Ser Asp Gly Ser Phe
        435                 440                 445

Phe Leu Tyr Ser Lys Leu Thr Val Asp Lys Ser Arg Trp Gln Gln Gly
    450                 455                 460

Asn Val Phe Ser Cys Ser Val Met His Glu Ala Leu His Asn His Tyr
465                 470                 475                 480

Thr Gln Lys Ser Leu Ser Leu Ser Pro Gly Lys Leu Glu Gln Val Thr
                485                 490                 495

Leu Lys Glu Ser Gly Pro Gly Ile Leu Gln Pro Ser Gln Thr Leu Ser
            500                 505                 510

Leu Thr Cys Ser Phe Ser Gly Phe Ser Leu Ser Thr Phe Gly Met Gly
        515                 520                 525

Val Gly Trp Ile Arg Gln Pro Ser Gly Lys Gly Leu Glu Trp Leu Ala
    530                 535                 540

His Ile Trp Trp Asp Asp Asp Lys Tyr Tyr Asn Pro Ala Leu Lys Ser

545                 550                 555                 560

Arg Leu Thr Ile Ser Lys Asp Thr Ser Glu Asn Gln Val Phe Leu Lys
                565                 570                 575

Ile Ala Asn Val Asp Thr Ala Asp Thr Ala Thr Tyr Tyr Cys Val Arg
            580                 585                 590

Ile Ala Gly Thr Arg Tyr Phe Asp Val Trp Gly Thr Gly Thr Thr Val
        595                 600                 605

Thr Val Ser Ser Arg Ser Ser Thr Lys Gly Pro Lys Leu Glu Glu Gly
    610                 615                 620

Glu Phe Ser Glu Ala Gln Leu Asp Ile Gln Met Thr Gln Ser Pro Ser
625                 630                 635                 640

Ser Leu Ser Ala Ser Leu Gly Gly Lys Val Thr Ile Thr Cys Lys Ala
                645                 650                 655

Ser Gln Asp Ile Asn Lys Phe Ile Ala Trp Tyr Gln His Lys Pro Gly
            660                 665                 670

Lys Gly Pro Arg Leu Leu Ile His Tyr Thr Ser Thr Leu Gln Pro Gly
        675                 680                 685

Ile Pro Ser Arg Phe Ser Gly Ser Gly Ser Gly Arg Asp Tyr Ser Phe
    690                 695                 700

Ser Ile Ser Asn Leu Glu Pro Glu Asp Ile Ala Thr Tyr Tyr Cys Leu
705                 710                 715                 720

Gln Tyr Asp Asn Leu Tyr Thr Phe Gly Gly Gly Thr Lys Leu Glu Ile
                725                 730                 735

Lys

<210>  49
<211>  481
<212>  PRT
<213>  artificial sequence

<220>
<223>  pDualyx-027 HC

<400>  49

Met Asn Phe Gly Phe Ser Leu Ile Phe Leu Val Leu Val Leu Lys Gly
1               5                   10                  15

Val Gln Cys Glu Val Lys Leu Val Pro Arg Gln Leu Glu Val Gln Leu
            20                  25                  30

Gln Gln Ser Gly Ala Glu Leu Val Arg Pro Gly Ala Ser Val Lys Leu
        35                  40                  45

Ser Cys Thr Ala Ser Gly Phe Asn Ile Lys Asp Asp Tyr Ile His Trp
    50                  55                  60

Val Lys Gln Arg Pro Glu Gln Gly Leu Glu Trp Ile Gly Trp Ile Asp
65                  70                  75                  80

Pro Glu Asn Ser Asp Thr Glu Tyr Ala Ser Lys Phe Gln Gly Lys Ala
                85                  90                  95

Thr Ile Thr Ala Asp Thr Ser Ser Asn Thr Ala Tyr Leu Gln Leu Ser
            100                 105                 110

Ser Leu Thr Ser Glu Asp Thr Ala Val Tyr Tyr Cys Thr Thr Gly Leu
        115                 120                 125

Trp Leu Arg Thr Ala Ile Asp Tyr Trp Gly Gln Gly Thr Ser Ile Thr
    130                 135                 140

Val Ser Ser Gly Ser Ser Ser Ala Ser Thr Lys Gly Pro Ser Val Phe
145                 150                 155                 160

Pro Leu Ala Pro Ser Ser Lys Ser Thr Ser Gly Gly Thr Ala Ala Leu
                165                 170                 175

Gly Cys Leu Val Lys Asp Tyr Phe Pro Glu Pro Val Thr Val Ser Trp
            180                 185                 190

Asn Ser Gly Ala Leu Thr Ser Gly Val His Thr Phe Pro Ala Val Leu
        195                 200                 205

Gln Ser Ser Gly Leu Tyr Ser Leu Ser Ser Val Val Thr Val Pro Ser
    210                 215                 220

Ser Ser Leu Gly Thr Gln Thr Tyr Ile Cys Asn Val Asn His Lys Pro
225                 230                 235                 240

Ser Asn Thr Lys Val Asp Lys Lys Val Glu Pro Lys Ser Cys Asp Lys
                245                 250                 255

Thr His Thr Cys Pro Pro Cys Pro Ala Pro Glu Leu Leu Gly Gly Pro

260                 265                 270

Ser Val Phe Leu Phe Pro Pro Lys Pro Lys Asp Thr Leu Met Ile Ser
        275                 280                 285

Arg Thr Pro Glu Val Thr Cys Val Val Val Asp Val Ser His Glu Asp
    290                 295                 300

Pro Glu Val Lys Phe Asn Trp Tyr Val Asp Gly Val Glu Val His Asn
305                 310                 315                 320

Ala Lys Thr Lys Pro Arg Glu Glu Gln Tyr Asn Ser Thr Tyr Arg Val
                325                 330                 335

Val Ser Val Leu Thr Val Leu His Gln Asp Trp Leu Asn Gly Lys Glu
            340                 345                 350

Tyr Lys Cys Lys Val Ser Asn Lys Ala Leu Pro Ala Pro Ile Glu Lys
        355                 360                 365

Thr Ile Ser Lys Ala Lys Gly Gln Pro Arg Glu Pro Gln Val Tyr Thr
    370                 375                 380

Leu Pro Pro Ser Arg Asp Glu Leu Thr Lys Asn Gln Val Ser Leu Thr
385                 390                 395                 400

Cys Leu Val Lys Gly Phe Tyr Pro Ser Asp Ile Ala Val Glu Trp Glu
                405                 410                 415

Ser Asn Gly Gln Pro Glu Asn Asn Tyr Lys Thr Thr Pro Pro Val Leu
            420                 425                 430

Asp Ser Asp Gly Ser Phe Phe Leu Tyr Ser Lys Leu Thr Val Asp Lys
        435                 440                 445

Ser Arg Trp Gln Gln Gly Asn Val Phe Ser Cys Ser Val Met His Glu
    450                 455                 460

Ala Leu His Asn His Tyr Thr Gln Lys Ser Leu Ser Leu Ser Pro Gly
465                 470                 475                 480

Lys

<210>  50
<211>  487
<212>  PRT
<213>  artificial sequence

<220>
<223>  pDualyx-085 HC

<400>  50

Met Asn Phe Gly Phe Ser Leu Ile Phe Leu Val Leu Val Leu Lys Gly
1               5                   10                  15

Val Gln Cys Glu Val Lys Leu Val Pro Arg Gln Leu Asp Tyr Lys Asp
            20                  25                  30

Asp Asp Asp Lys Glu Phe Gln Ile Gln Leu Val Gln Ser Gly Pro Glu
        35                  40                  45

Leu Lys Lys Pro Gly Glu Thr Val Lys Ile Ser Cys Lys Ala Ser Gly
    50                  55                  60

Tyr Thr Phe Thr Thr Ala Gly Met Gln Trp Val Gln Lys Met Pro Gly
65                  70                  75                  80

Lys Gly Phe Lys Trp Ile Gly Trp Ile Asn Thr His Ser Gly Glu Pro
                85                  90                  95

Lys Tyr Ala Glu Asp Phe Lys Gly Arg Phe Ala Phe Ser Leu Glu Thr
            100                 105                 110

Ser Ala Ser Thr Ala Tyr Leu Gln Ile Ser Asn Leu Lys Asn Glu Asp
        115                 120                 125

Thr Ala Thr Tyr Phe Cys Ala Arg Trp Asp Gly Thr Gly Tyr Trp Gly
    130                 135                 140

Gln Gly Thr Thr Leu Thr Val Ser Ser Arg Ser Ser Ser Ala Ser Thr
145                 150                 155                 160

Lys Gly Pro Ser Val Phe Pro Leu Ala Pro Ser Ser Lys Ser Thr Ser
                165                 170                 175

Gly Gly Thr Ala Ala Leu Gly Cys Leu Val Lys Asp Tyr Phe Pro Glu
            180                 185                 190

Pro Val Thr Val Ser Trp Asn Ser Gly Ala Leu Thr Ser Gly Val His
        195                 200                 205

Thr Phe Pro Ala Val Leu Gln Ser Ser Gly Leu Tyr Ser Leu Ser Ser
    210                 215                 220

Val Val Thr Val Pro Ser Ser Ser Leu Gly Thr Gln Thr Tyr Ile Cys

225                 230                 235                 240

Asn Val Asn His Lys Pro Ser Asn Thr Lys Val Asp Lys Lys Val Glu
                245                 250                 255

Pro Lys Ser Cys Asp Lys Thr His Thr Cys Pro Pro Cys Pro Ala Pro
            260                 265                 270

Glu Leu Leu Gly Gly Pro Ser Val Phe Leu Phe Pro Pro Lys Pro Lys
        275                 280                 285

Asp Thr Leu Met Ile Ser Arg Thr Pro Glu Val Thr Cys Val Val Val
    290                 295                 300

Asp Val Ser His Glu Asp Pro Glu Val Lys Phe Asn Trp Tyr Val Asp
305                 310                 315                 320

Gly Val Glu Val His Asn Ala Lys Thr Lys Pro Arg Glu Glu Gln Tyr
                325                 330                 335

Ala Ser Thr Tyr Arg Val Val Ser Val Leu Thr Val Leu His Gln Asp
            340                 345                 350

Trp Leu Asn Gly Lys Glu Tyr Lys Cys Lys Val Ser Asn Lys Ala Leu
        355                 360                 365

Pro Ala Pro Ile Glu Lys Thr Ile Ser Lys Ala Lys Gly Gln Pro Arg
    370                 375                 380

Glu Pro Gln Val Tyr Thr Leu Pro Pro Ser Arg Asp Glu Leu Thr Lys
385                 390                 395                 400

Asn Gln Val Ser Leu Thr Cys Leu Val Lys Gly Phe Tyr Pro Ser Asp
                405                 410                 415

Ile Ala Val Glu Trp Glu Ser Asn Gly Gln Pro Glu Asn Asn Tyr Lys
            420                 425                 430

Thr Thr Pro Pro Val Leu Asp Ser Asp Gly Ser Phe Phe Leu Tyr Ser
        435                 440                 445

Lys Leu Thr Val Asp Lys Ser Arg Trp Gln Gln Gly Asn Val Phe Ser
    450                 455                 460

Cys Ser Val Met His Glu Ala Leu His Asn His Tyr Thr Gln Lys Ser
465                 470                 475                 480

Leu Ser Leu Ser Pro Gly Lys
                485

<210>  51
<211>  485
<212>  PRT
<213>  artificial sequence

<220>
<223>  pDualyx-088 HC

<400>  51

Met Asn Phe Gly Phe Ser Leu Ile Phe Leu Val Leu Val Leu Lys Gly
1               5                   10                  15

Val Gln Cys Glu Val Lys Leu Val Pro Arg Gln Leu Asp Tyr Lys Asp
            20                  25                  30

Asp Asp Asp Lys Glu Phe Glu Ile Gln Leu Gln Gln Ser Gly Thr Val
        35                  40                  45

Leu Ala Arg Pro Gly Ala Ser Val Lys Met Ser Cys Lys Thr Ser Gly
    50                  55                  60

Tyr Thr Phe Thr Asn Tyr Trp Met His Trp Leu Lys Gln Arg Pro Gly
65                  70                  75                  80

Gln Gly Leu Glu Trp Ile Gly Thr Ile Tyr Pro Gly Asn Ser Glu Thr
                85                  90                  95

Asn Tyr Asn Pro Asn Phe Lys Gly Lys Ala Lys Leu Thr Ala Val Ser
            100                 105                 110

Ser Ala Ser Thr Ala Tyr Met Glu Leu Ser Ser Leu Thr Asn Glu Asp
        115                 120                 125

Ser Ala Val Tyr Tyr Cys Ser Arg Gly Glu Asp Tyr Trp Gly Gln Gly
    130                 135                 140

Thr Ser Val Thr Val Ser Ser Arg Ser Ser Ser Ala Ser Thr Lys Gly
145                 150                 155                 160

Pro Ser Val Phe Pro Leu Ala Pro Ser Ser Lys Ser Thr Ser Gly Gly
                165                 170                 175

Thr Ala Ala Leu Gly Cys Leu Val Lys Asp Tyr Phe Pro Glu Pro Val
            180                 185                 190

Thr Val Ser Trp Asn Ser Gly Ala Leu Thr Ser Gly Val His Thr Phe

195                 200                 205

Pro Ala Val Leu Gln Ser Ser Gly Leu Tyr Ser Leu Ser Ser Val Val
    210                 215                 220

Thr Val Pro Ser Ser Ser Leu Gly Thr Gln Thr Tyr Ile Cys Asn Val
225                 230                 235                 240

Asn His Lys Pro Ser Asn Thr Lys Val Asp Lys Lys Val Glu Pro Lys
                245                 250                 255

Ser Cys Asp Lys Thr His Thr Cys Pro Pro Cys Pro Ala Pro Glu Leu
            260                 265                 270

Leu Gly Gly Pro Ser Val Phe Leu Phe Pro Pro Lys Pro Lys Asp Thr
        275                 280                 285

Leu Met Ile Ser Arg Thr Pro Glu Val Thr Cys Val Val Val Asp Val
    290                 295                 300

Ser His Glu Asp Pro Glu Val Lys Phe Asn Trp Tyr Val Asp Gly Val
305                 310                 315                 320

Glu Val His Asn Ala Lys Thr Lys Pro Arg Glu Glu Gln Tyr Ala Ser
                325                 330                 335

Thr Tyr Arg Val Val Ser Val Leu Thr Val Leu His Gln Asp Trp Leu
            340                 345                 350

Asn Gly Lys Glu Tyr Lys Cys Lys Val Ser Asn Lys Ala Leu Pro Ala
        355                 360                 365

Pro Ile Glu Lys Thr Ile Ser Lys Ala Lys Gly Gln Pro Arg Glu Pro
    370                 375                 380

Gln Val Tyr Thr Leu Pro Pro Ser Arg Asp Glu Leu Thr Lys Asn Gln
385                 390                 395                 400

Val Ser Leu Thr Cys Leu Val Lys Gly Phe Tyr Pro Ser Asp Ile Ala
                405                 410                 415

Val Glu Trp Glu Ser Asn Gly Gln Pro Glu Asn Asn Tyr Lys Thr Thr
            420                 425                 430

Pro Pro Val Leu Asp Ser Asp Gly Ser Phe Phe Leu Tyr Ser Lys Leu
        435                 440                 445

Thr Val Asp Lys Ser Arg Trp Gln Gln Gly Asn Val Phe Ser Cys Ser
    450                 455                 460

Val Met His Glu Ala Leu His Asn His Tyr Thr Gln Lys Ser Leu Ser
465                 470                 475                 480

Leu Ser Pro Gly Lys
                485

<210>  52
<211>  491
<212>  PRT
<213>  artificial sequence

<220>
<223>  pDualyx-069 HC

<400>  52

Met Asn Phe Gly Phe Ser Leu Ile Phe Leu Val Leu Val Leu Lys Gly
1               5                   10                  15

Val Gln Cys Glu Val Lys Leu Val Pro Arg Gln Leu Asp Tyr Lys Asp
            20                  25                  30

Asp Asp Asp Lys Glu Phe Asp Val Gln Leu Gln Glu Ser Gly Pro Gly
        35                  40                  45

Leu Val Lys Pro Ser Gln Ser Leu Ser Leu Thr Cys Ser Val Thr Gly
    50                  55                  60

Ser Ser Ile Thr Ser Gly Tyr Tyr Trp Asn Trp Ile Arg Gln Phe Pro
65                  70                  75                  80

Gly Asn Lys Leu Glu Trp Met Gly Tyr Ile Thr Asn Asp Gly Ser Asn
                85                  90                  95

Asn Tyr Asn Pro Ser Leu Lys Asn Arg Ile Ser Ile Thr Arg Asp Thr
            100                 105                 110

Ser Glu Asn Gln Phe Phe Leu Lys Leu Asn Ser Val Thr Thr Glu Asp
        115                 120                 125

Thr Ala Thr Tyr Tyr Cys Ala Arg Asp Gly Gly Trp Phe His Tyr Phe
    130                 135                 140

Asp Tyr Trp Gly Gln Gly Thr Thr Leu Thr Val Ser Ser Arg Ser Ser
145                 150                 155                 160

Ser Ala Ser Thr Lys Gly Pro Ser Val Phe Pro Leu Ala Pro Ser Ser

165                 170                 175

Lys Ser Thr Ser Gly Gly Thr Ala Ala Leu Gly Cys Leu Val Lys Asp
        180                 185                 190

Tyr Phe Pro Glu Pro Val Thr Val Ser Trp Asn Ser Gly Ala Leu Thr
    195                 200                 205

Ser Gly Val His Thr Phe Pro Ala Val Leu Gln Ser Ser Gly Leu Tyr
    210                 215                 220

Ser Leu Ser Ser Val Val Thr Val Pro Ser Ser Ser Leu Gly Thr Gln
225                 230                 235                 240

Thr Tyr Ile Cys Asn Val Asn His Lys Pro Ser Asn Thr Lys Val Asp
                245                 250                 255

Lys Lys Val Glu Pro Lys Ser Cys Asp Lys Thr His Thr Cys Pro Pro
            260                 265                 270

Cys Pro Ala Pro Glu Leu Leu Gly Gly Pro Ser Val Phe Leu Phe Pro
        275                 280                 285

Pro Lys Pro Lys Asp Thr Leu Met Ile Ser Arg Thr Pro Glu Val Thr
    290                 295                 300

Cys Val Val Val Asp Val Ser His Glu Asp Pro Glu Val Lys Phe Asn
305                 310                 315                 320

Trp Tyr Val Asp Gly Val Glu Val His Asn Ala Lys Thr Lys Pro Arg
                325                 330                 335

Glu Glu Gln Tyr Ala Ser Thr Tyr Arg Val Val Ser Val Leu Thr Val
            340                 345                 350

Leu His Gln Asp Trp Leu Asn Gly Lys Glu Tyr Lys Cys Lys Val Ser
        355                 360                 365

Asn Lys Ala Leu Pro Ala Pro Ile Glu Lys Thr Ile Ser Lys Ala Lys
    370                 375                 380

Gly Gln Pro Arg Glu Pro Gln Val Tyr Thr Leu Pro Pro Ser Arg Asp
385                 390                 395                 400

Glu Leu Thr Lys Asn Gln Val Ser Leu Thr Cys Leu Val Lys Gly Phe
                405                 410                 415

Tyr Pro Ser Asp Ile Ala Val Glu Trp Glu Ser Asn Gly Gln Pro Glu
            420                 425                 430

Asn Asn Tyr Lys Thr Thr Pro Pro Val Leu Asp Ser Asp Gly Ser Phe
        435                 440                 445

Phe Leu Tyr Ser Lys Leu Thr Val Asp Lys Ser Arg Trp Gln Gln Gly
    450                 455                 460

Asn Val Phe Ser Cys Ser Val Met His Glu Ala Leu His Asn His Tyr
465                 470                 475                 480

Thr Gln Lys Ser Leu Ser Leu Ser Pro Gly Lys
                485                 490

<210>  53
<211>  491
<212>  PRT
<213>  artificial sequence

<220>
<223>  pDualyx-041pre HC

<400>  53

Met Asn Phe Gly Phe Ser Leu Ile Phe Leu Val Leu Val Leu Lys Gly
1               5                   10                  15

Val Gln Cys Glu Val Lys Leu Val Pro Arg Gln Leu Asp Tyr Lys Asp
            20                  25                  30

Asp Asp Asp Lys Glu Phe Gln Val Thr Leu Lys Glu Ser Gly Pro Ala
        35                  40                  45

Leu Val Lys Pro Thr Gln Thr Leu Thr Leu Thr Cys Thr Phe Ser Gly
    50                  55                  60

Phe Ser Leu Ser Thr Ser Gly Met Gly Val Gly Trp Ile Arg Gln Pro
65                  70                  75                  80

Pro Gly Lys Ala Leu Glu Trp Leu Ala His Ile Trp Trp Asp Asp Asp
                85                  90                  95

Lys Phe Tyr Asn Pro Ser Leu Lys Ser Arg Leu Thr Ile Ser Lys Asp
            100                 105                 110

Thr Ser Lys Asn Gln Val Val Leu Thr Met Thr Asn Met Asp Pro Val
        115                 120                 125

Asp Thr Ala Thr Tyr Tyr Cys Ala Arg Leu Thr Gly Thr Arg Tyr Phe

130                 135                 140

Asp Tyr Trp Gly Gln Gly Thr Thr Val Thr Val Ser Ser Arg Ser Ser
145                 150                 155                 160

Ser Ala Ser Thr Lys Gly Pro Ser Val Phe Pro Leu Ala Pro Ser Ser
                165                 170                 175

Lys Ser Thr Ser Gly Gly Thr Ala Ala Leu Gly Cys Leu Val Lys Asp
            180                 185                 190

Tyr Phe Pro Glu Pro Val Thr Val Ser Trp Asn Ser Gly Ala Leu Thr
        195                 200                 205

Ser Gly Val His Thr Phe Pro Ala Val Leu Gln Ser Ser Gly Leu Tyr
    210                 215                 220

Ser Leu Ser Ser Val Val Thr Val Pro Ser Ser Ser Leu Gly Thr Gln
225                 230                 235                 240

Thr Tyr Ile Cys Asn Val Asn His Lys Pro Ser Asn Thr Lys Val Asp
                245                 250                 255

Lys Lys Val Glu Pro Lys Ser Cys Asp Lys Thr His Thr Cys Pro Pro
            260                 265                 270

Cys Pro Ala Pro Glu Leu Leu Gly Gly Pro Ser Val Phe Leu Phe Pro
        275                 280                 285

Pro Lys Pro Lys Asp Thr Leu Met Ile Ser Arg Thr Pro Glu Val Thr
    290                 295                 300

Cys Val Val Val Asp Val Ser His Glu Asp Pro Glu Val Lys Phe Asn
305                 310                 315                 320

Trp Tyr Val Asp Gly Val Glu Val His Asn Ala Lys Thr Lys Pro Arg
                325                 330                 335

Glu Glu Gln Tyr Ala Ser Thr Tyr Arg Val Val Ser Val Leu Thr Val
            340                 345                 350

Leu His Gln Asp Trp Leu Asn Gly Lys Glu Tyr Lys Cys Lys Val Ser
        355                 360                 365

Asn Lys Ala Leu Pro Ala Pro Ile Glu Lys Thr Ile Ser Lys Ala Lys
    370                 375                 380

Gly Gln Pro Arg Glu Pro Gln Val Tyr Thr Leu Pro Pro Ser Arg Asp
385                 390                 395                 400

Glu Leu Thr Lys Asn Gln Val Ser Leu Thr Cys Leu Val Lys Gly Phe
                405                 410                 415

Tyr Pro Ser Asp Ile Ala Val Glu Trp Glu Ser Asn Gly Gln Pro Glu
            420                 425                 430

Asn Asn Tyr Lys Thr Thr Pro Pro Val Leu Asp Ser Asp Gly Ser Phe
        435                 440                 445

Phe Leu Tyr Ser Lys Leu Thr Val Asp Lys Ser Arg Trp Gln Gln Gly
    450                 455                 460

Asn Val Phe Ser Cys Ser Val Met His Glu Ala Leu His Asn His Tyr
465                 470                 475                 480

Thr Gln Lys Ser Leu Ser Leu Ser Pro Gly Lys
                485                 490

<210>  54
<211>  529
<212>  PRT
<213>  artificial sequence

<220>
<223>  pDualyx-149 HC

<400>  54

Met Asn Phe Gly Phe Ser Leu Ile Phe Leu Val Leu Val Leu Lys Gly
1               5                   10                  15

Val Gln Cys Glu Val Lys Leu Val Pro Arg Gln Leu Asp Tyr Lys Asp
            20                  25                  30

Asp Asp Asp Lys Glu Phe Gln Ile Gln Leu Val Gln Ser Gly Pro Glu
        35                  40                  45

Val Lys Lys Pro Gly Glu Thr Val Lys Ile Ser Cys Lys Ala Ser Gly
    50                  55                  60

Tyr Thr Phe Thr Ile His Gly Met Ser Trp Val Lys Gln Ala Pro Gly
65                  70                  75                  80

Lys Gly Leu Lys Trp Met Gly Trp Ile Asn Thr Tyr Ser Gly Val Pro
                85                  90                  95

Thr Tyr Ala Asn Asp Phe Lys Gly Arg Phe Ala Phe Ser Leu Glu Thr

            100                 105                 110

Ser Ala Ser Thr Ala Tyr Leu Gln Ile Asn Asn Leu Lys Asn Glu Asp
        115                 120                 125

Thr Ala Thr Tyr Phe Cys Ala Arg Asp Glu Val Arg Arg Gly Phe Gly
    130                 135                 140

Phe Ala Tyr Trp Gly Gln Gly Thr Leu Val Thr Val Ser Ala Arg Ser
145                 150                 155                 160

Ser Ser Ala Ser Thr Lys Gly Pro Ser Val Phe Pro Leu Ala Pro Ser
                165                 170                 175

Ser Lys Ser Thr Ser Gly Gly Thr Ala Ala Leu Gly Cys Leu Val Lys
            180                 185                 190

Asp Tyr Phe Pro Glu Pro Val Thr Val Ser Trp Asn Ser Gly Ala Leu
        195                 200                 205

Thr Ser Gly Val His Thr Phe Pro Ala Val Leu Gln Ser Ser Gly Leu
    210                 215                 220

Tyr Ser Leu Ser Ser Val Val Thr Val Pro Ser Ser Ser Leu Gly Thr
225                 230                 235                 240

Gln Thr Tyr Ile Cys Asn Val Asn His Lys Pro Ser Asn Thr Lys Val
                245                 250                 255

Asp Lys Lys Val Glu Pro Lys Ser Cys Asp Lys Thr His Thr Cys Pro
            260                 265                 270

Pro Cys Pro Ala Pro Glu Leu Leu Gly Gly Pro Ser Val Phe Leu Phe
        275                 280                 285

Pro Pro Lys Pro Lys Asp Thr Leu Met Ile Ser Arg Thr Pro Glu Val
    290                 295                 300

Thr Cys Val Val Val Asp Val Ser His Glu Asp Pro Glu Val Lys Phe
305                 310                 315                 320

Asn Trp Tyr Val Asp Gly Val Glu Val His Asn Ala Lys Thr Lys Pro
                325                 330                 335

Arg Glu Glu Gln Tyr Ala Ser Thr Tyr Arg Val Val Ser Val Leu Thr
            340                 345                 350

Val Leu His Gln Asp Trp Leu Asn Gly Lys Glu Tyr Lys Cys Lys Val
        355                 360                 365

Ser Asn Lys Ala Leu Pro Ala Pro Ile Glu Lys Thr Ile Ser Lys Ala
    370                 375                 380

Lys Gly Gln Pro Arg Glu Pro Gln Val Tyr Thr Leu Pro Pro Ser Arg
385                 390                 395                 400

Asp Glu Leu Thr Lys Asn Gln Val Ser Leu Thr Cys Leu Val Lys Gly
                405                 410                 415

Phe Tyr Pro Ser Asp Ile Ala Val Glu Trp Glu Ser Asn Gly Gln Pro
            420                 425                 430

Glu Asn Asn Tyr Lys Thr Thr Pro Pro Val Leu Asp Ser Asp Gly Ser
        435                 440                 445

Phe Phe Leu Tyr Ser Lys Leu Thr Val Asp Lys Ser Arg Trp Gln Gln
    450                 455                 460

Gly Asn Val Phe Ser Cys Ser Val Met His Glu Ala Leu His Asn His
465                 470                 475                 480

Tyr Thr Gln Lys Ser Leu Ser Leu Ser Pro Gly Lys Leu Glu Asp Ile
                485                 490                 495

Ala Cys Gly Cys Ala Ala Ala Pro Asp Ile Lys Asp Leu Leu Ser Arg
            500                 505                 510

Leu Glu Glu Leu Glu Gly Leu Val Ser Ser Leu Arg Glu Gln Gly Thr
        515                 520                 525

Gly

<210>  55
<211>  618
<212>  PRT
<213>  artificial sequence

<220>
<223>  pDualyx-150 HC

<400>  55

Met Asn Phe Gly Phe Ser Leu Ile Phe Leu Val Leu Val Leu Lys Gly
1               5                   10                  15

Val Gln Cys Glu Val Lys Leu Val Pro Arg Gln Leu Asp Tyr Lys Asp

20                  25                  30

Asp Asp Asp Lys Glu Phe Gln Ile Gln Leu Val Gln Ser Gly Pro Glu
        35                  40                  45

Val Lys Lys Pro Gly Glu Thr Val Lys Ile Ser Cys Lys Ala Ser Gly
    50                  55                  60

Tyr Thr Phe Thr Ile His Gly Met Ser Trp Val Lys Gln Ala Pro Gly
65                  70                  75                  80

Lys Gly Leu Lys Trp Met Gly Trp Ile Asn Thr Tyr Ser Gly Val Pro
                85                  90                  95

Thr Tyr Ala Asn Asp Phe Lys Gly Arg Phe Ala Phe Ser Leu Glu Thr
            100                 105                 110

Ser Ala Ser Thr Ala Tyr Leu Gln Ile Asn Asn Leu Lys Asn Glu Asp
        115                 120                 125

Thr Ala Thr Tyr Phe Cys Ala Arg Asp Glu Val Arg Arg Gly Phe Gly
    130                 135                 140

Phe Ala Tyr Trp Gly Gln Gly Thr Leu Val Thr Val Ser Ala Arg Ser
145                 150                 155                 160

Ser Thr Lys Gly Pro Lys Leu Glu Glu Gly Glu Phe Ser Glu Ala Gln
                165                 170                 175

Leu Asp Ile Val Met Ala Gln Ser Gln Lys Phe Met Ser Val Ser Val
            180                 185                 190

Gly Asp Arg Val Ser Val Thr Cys Lys Ala Ser Gln Asn Val Gly Thr
        195                 200                 205

Asn Val Ala Trp Tyr Gln Gln Arg Pro Gly Gln Ser Pro Lys Ala Leu
    210                 215                 220

Ile Tyr Ser Ala Ser Tyr Arg Tyr Ser Gly Val Pro Asp Arg Phe Thr
225                 230                 235                 240

Gly Ser Gly Ser Gly Thr Asp Phe Thr Leu Thr Ile Ser Asn Val Gln
                245                 250                 255

Ser Glu Asp Leu Ala Glu Tyr Phe Cys Gln Gln Phe Asp Ser His Pro
            260                 265                 270

Leu Thr Phe Gly Ala Gly Thr Lys Leu Glu Leu Lys Gly Ser Ser Ser
        275                 280                 285

Ala Ser Thr Lys Gly Pro Ser Val Phe Pro Leu Ala Pro Ser Ser Lys
    290                 295                 300

Ser Thr Ser Gly Gly Thr Ala Ala Leu Gly Cys Leu Val Lys Asp Tyr
305                 310                 315                 320

Phe Pro Glu Pro Val Thr Val Ser Trp Asn Ser Gly Ala Leu Thr Ser
                325                 330                 335

Gly Val His Thr Phe Pro Ala Val Leu Gln Ser Ser Gly Leu Tyr Ser
            340                 345                 350

Leu Ser Ser Val Val Thr Val Pro Ser Ser Ser Leu Gly Thr Gln Thr
        355                 360                 365

Tyr Ile Cys Asn Val Asn His Lys Pro Ser Asn Thr Lys Val Asp Lys
    370                 375                 380

Lys Val Glu Pro Lys Ser Cys Asp Lys Thr His Thr Cys Pro Pro Cys
385                 390                 395                 400

Pro Ala Pro Glu Leu Leu Gly Gly Pro Ser Val Phe Leu Phe Pro Pro
                405                 410                 415

Lys Pro Lys Asp Thr Leu Met Ile Ser Arg Thr Pro Glu Val Thr Cys
            420                 425                 430

Val Val Val Asp Val Ser His Glu Asp Pro Glu Val Lys Phe Asn Trp
        435                 440                 445

Tyr Val Asp Gly Val Glu Val His Asn Ala Lys Thr Lys Pro Arg Glu
    450                 455                 460

Glu Gln Tyr Ala Ser Thr Tyr Arg Val Val Ser Val Leu Thr Val Leu
465                 470                 475                 480

His Gln Asp Trp Leu Asn Gly Lys Glu Tyr Lys Cys Lys Val Ser Asn
                485                 490                 495

Lys Ala Leu Pro Ala Pro Ile Glu Lys Thr Ile Ser Lys Ala Lys Gly
            500                 505                 510

Gln Pro Arg Glu Pro Gln Val Tyr Thr Leu Pro Pro Ser Arg Asp Glu
        515                 520                 525

Leu Thr Lys Asn Gln Val Ser Leu Thr Cys Leu Val Lys Gly Phe Tyr
    530                 535                 540

Pro Ser Asp Ile Ala Val Glu Trp Glu Ser Asn Gly Gln Pro Glu Asn
545                 550                 555                 560

Asn Tyr Lys Thr Thr Pro Pro Val Leu Asp Ser Asp Gly Ser Phe Phe
                565                 570                 575

Leu Tyr Ser Lys Leu Thr Val Asp Lys Ser Arg Trp Gln Gln Gly Asn
            580                 585                 590

Val Phe Ser Cys Ser Val Met His Glu Ala Leu His Asn His Tyr Thr
        595                 600                 605

Gln Lys Ser Leu Ser Leu Ser Pro Gly Lys
    610                 615

<210>  56
<211>  655
<212>  PRT
<213>  artificial sequence

<220>
<223>  pDualyx-152 HC

<400>  56

Met Asn Phe Gly Phe Ser Leu Ile Phe Leu Val Leu Val Leu Lys Gly
1               5                   10                  15

Val Gln Cys Glu Val Lys Leu Val Pro Arg Gln Leu Asp Tyr Lys Asp
            20                  25                  30

Asp Asp Asp Lys Glu Phe Gln Ile Gln Leu Val Gln Ser Gly Pro Glu
        35                  40                  45

Val Lys Lys Pro Gly Glu Thr Val Lys Ile Ser Cys Lys Ala Ser Gly
    50                  55                  60

Tyr Thr Phe Thr Ile His Gly Met Ser Trp Val Lys Gln Ala Pro Gly
65                  70                  75                  80

Lys Gly Leu Lys Trp Met Gly Trp Ile Asn Thr Tyr Ser Gly Val Pro
                85                  90                  95

Thr Tyr Ala Asn Asp Phe Lys Gly Arg Phe Ala Phe Ser Leu Glu Thr
            100                 105                 110

Ser Ala Ser Thr Ala Tyr Leu Gln Ile Asn Asn Leu Lys Asn Glu Asp
        115                 120                 125

Thr Ala Thr Tyr Phe Cys Ala Arg Asp Glu Val Arg Arg Gly Phe Gly
    130                 135                 140

Phe Ala Tyr Trp Gly Gln Gly Thr Leu Val Thr Val Ser Ala Arg Ser
145                 150                 155                 160

Ser Thr Lys Gly Pro Lys Leu Glu Glu Gly Glu Phe Ser Glu Ala Gln
                165                 170                 175

Leu Asp Ile Val Met Ala Gln Ser Gln Lys Phe Met Ser Val Ser Val
            180                 185                 190

Gly Asp Arg Val Ser Val Thr Cys Lys Ala Ser Gln Asn Val Gly Thr
        195                 200                 205

Asn Val Ala Trp Tyr Gln Gln Arg Pro Gly Gln Ser Pro Lys Ala Leu
    210                 215                 220

Ile Tyr Ser Ala Ser Tyr Arg Tyr Ser Gly Val Pro Asp Arg Phe Thr
225                 230                 235                 240

Gly Ser Gly Ser Gly Thr Asp Phe Thr Leu Thr Ile Ser Asn Val Gln
                245                 250                 255

Ser Glu Asp Leu Ala Glu Tyr Phe Cys Gln Gln Phe Asp Ser His Pro
            260                 265                 270

Leu Thr Phe Gly Ala Gly Thr Lys Leu Glu Leu Lys Gly Ser Ser Ser
        275                 280                 285

Ala Ser Thr Lys Gly Pro Ser Val Phe Pro Leu Ala Pro Ser Ser Lys
    290                 295                 300

Ser Thr Ser Gly Gly Thr Ala Ala Leu Gly Cys Leu Val Lys Asp Tyr
305                 310                 315                 320

Phe Pro Glu Pro Val Thr Val Ser Trp Asn Ser Gly Ala Leu Thr Ser
                325                 330                 335

Gly Val His Thr Phe Pro Ala Val Leu Gln Ser Ser Gly Leu Tyr Ser
            340                 345                 350

Leu Ser Ser Val Val Thr Val Pro Ser Ser Ser Leu Gly Thr Gln Thr
        355                 360                 365

Tyr Ile Cys Asn Val Asn His Lys Pro Ser Asn Thr Lys Val Asp Lys
    370                 375                 380

Lys Val Glu Pro Lys Ser Cys Asp Lys Thr His Thr Cys Pro Pro Cys
385                 390                 395                 400

Pro Ala Pro Glu Leu Leu Gly Gly Pro Ser Val Phe Leu Phe Pro Pro
                405                 410                 415

Lys Pro Lys Asp Thr Leu Met Ile Ser Arg Thr Pro Glu Val Thr Cys
            420                 425                 430

Val Val Val Asp Val Ser His Glu Asp Pro Glu Val Lys Phe Asn Trp
        435                 440                 445

Tyr Val Asp Gly Val Glu Val His Asn Ala Lys Thr Lys Pro Arg Glu
    450                 455                 460

Glu Gln Tyr Ala Ser Thr Tyr Arg Val Val Ser Val Leu Thr Val Leu
465                 470                 475                 480

His Gln Asp Trp Leu Asn Gly Lys Glu Tyr Lys Cys Lys Val Ser Asn
                485                 490                 495

Lys Ala Leu Pro Ala Pro Ile Glu Lys Thr Ile Ser Lys Ala Lys Gly
            500                 505                 510

Gln Pro Arg Glu Pro Gln Val Tyr Thr Leu Pro Pro Ser Arg Asp Glu
        515                 520                 525

Leu Thr Lys Asn Gln Val Ser Leu Thr Cys Leu Val Lys Gly Phe Tyr
    530                 535                 540

Pro Ser Asp Ile Ala Val Glu Trp Glu Ser Asn Gly Gln Pro Glu Asn
545                 550                 555                 560

Asn Tyr Lys Thr Thr Pro Pro Val Leu Asp Ser Asp Gly Ser Phe Phe
                565                 570                 575

Leu Tyr Ser Lys Leu Thr Val Asp Lys Ser Arg Trp Gln Gln Gly Asn
            580                 585                 590

Val Phe Ser Cys Ser Val Met His Glu Ala Leu His Asn His Tyr Thr
        595                 600                 605

Gln Lys Ser Leu Ser Leu Ser Pro Gly Lys Leu Glu Asp Ile Ala Cys
    610                 615                 620

Gly Cys Ala Ala Ala Pro Asp Ile Lys Asp Leu Leu Ser Arg Leu Glu
625                 630                 635                 640

Glu Leu Glu Gly Leu Val Ser Ser Leu Arg Glu Gln Gly Thr Gly
                645                 650                 655

<210>  57
<211>  439
<212>  PRT
<213>  artificial sequence

<220>
<223>  pDualyx1-153 HC

<400>  57

Met Asn Phe Gly Phe Ser Leu Ile Phe Leu Val Leu Val Leu Lys Gly
1               5                   10                  15

Val Gln Cys Glu Val Lys Leu Val Pro Arg Gln Leu Asp Tyr Lys Asp
            20                  25                  30

Asp Asp Asp Lys Glu Phe Gln Ile Gln Leu Val Gln Ser Gly Pro Glu
        35                  40                  45

Val Lys Lys Pro Gly Glu Thr Val Lys Ile Ser Cys Lys Ala Ser Gly
    50                  55                  60

Tyr Thr Phe Thr Ile His Gly Met Ser Trp Val Lys Gln Ala Pro Gly
65                  70                  75                  80

Lys Gly Leu Lys Trp Met Gly Trp Ile Asn Thr Tyr Ser Gly Val Pro
                85                  90                  95

Thr Tyr Ala Asn Asp Phe Lys Gly Arg Phe Ala Phe Ser Leu Glu Thr
            100                 105                 110

Ser Ala Ser Thr Ala Tyr Leu Gln Ile Asn Asn Leu Lys Asn Glu Asp
        115                 120                 125

Thr Ala Thr Tyr Phe Cys Ala Arg Asp Glu Val Arg Arg Gly Phe Gly
    130                 135                 140

Phe Ala Tyr Trp Gly Gln Gly Thr Leu Val Thr Val Ser Ala Arg Ser
145                 150                 155                 160

Ser Thr Lys Gly Pro Lys Leu Glu Glu Gly Glu Phe Ser Glu Ala Gln
                165                 170                 175

Leu Asp Ile Val Met Ala Gln Ser Gln Lys Phe Met Ser Val Ser Val
        180                 185                 190

Gly Asp Arg Val Ser Val Thr Cys Lys Ala Ser Gln Asn Val Gly Thr
    195                 200                 205

Asn Val Ala Trp Tyr Gln Gln Arg Pro Gly Gln Ser Pro Lys Ala Leu
210                 215                 220

Ile Tyr Ser Ala Ser Tyr Arg Tyr Ser Gly Val Pro Asp Arg Phe Thr
225                 230                 235                 240

Gly Ser Gly Ser Gly Thr Asp Phe Thr Leu Thr Ile Ser Asn Val Gln
                245                 250                 255

Ser Glu Asp Leu Ala Glu Tyr Phe Cys Gln Gln Phe Asp Ser His Pro
            260                 265                 270

Leu Thr Phe Gly Ala Gly Thr Lys Leu Glu Leu Lys Gly Ser Ser Ser
        275                 280                 285

Ala Ser Thr Lys Gly Pro Ser Val Phe Pro Leu Ala Pro Ser Ser Lys
    290                 295                 300

Ser Thr Ser Gly Gly Thr Ala Ala Leu Gly Cys Leu Val Lys Asp Tyr
305                 310                 315                 320

Phe Pro Glu Pro Val Thr Val Ser Trp Asn Ser Gly Ala Leu Thr Ser
                325                 330                 335

Gly Val His Thr Phe Pro Ala Val Leu Gln Ser Ser Gly Leu Tyr Ser
            340                 345                 350

Leu Ser Ser Val Val Thr Val Pro Ser Ser Ser Leu Gly Thr Gln Thr
        355                 360                 365

Tyr Ile Cys Asn Val Asn His Lys Pro Ser Asn Thr Lys Val Asp Lys
    370                 375                 380

Lys Val Glu Pro Lys Ser Cys Asp Lys Thr His Thr Cys Pro Pro Cys
385                 390                 395                 400

Pro Ala Leu Glu Asp Ile Ala Cys Gly Cys Ala Ala Ala Pro Asp Ile
                405                 410                 415

Lys Asp Leu Leu Ser Arg Leu Glu Glu Leu Glu Gly Leu Val Ser Ser
            420                 425                 430

Leu Arg Glu Gln Gly Thr Gly
        435

<210>  58
<211>  530
<212>  PRT
<213>  artificial sequence

<220>
<223>  pDualyx-154 HC

<400>  58

Met Asn Phe Gly Phe Ser Leu Ile Phe Leu Val Leu Val Leu Lys Gly
1               5                   10                  15

Val Gln Cys Glu Val Lys Leu Val Pro Arg Gln Leu Asp Tyr Lys Asp
            20                  25                  30

Asp Asp Asp Lys Glu Phe Glu Val Lys Leu Leu Gln Ser Gly Gly Gly
        35                  40                  45

Leu Val Gln Pro Gly Gly Ser Leu Lys Leu Ser Cys Glu Ala Ser Gly
    50                  55                  60

Ile Asp Phe Ser Arg Tyr Trp Met Ser Trp Val Arg Arg Ala Pro Gly
65                  70                  75                  80

Lys Gly Leu Glu Trp Ile Gly Glu Ser Asp Pro Asp Ser Ser Thr Ile
                85                  90                  95

Asn Tyr Ala Pro Ser Leu Lys Asp Lys Phe Ile Ile Ser Arg Asp Ser
            100                 105                 110

Ala Arg Asn Thr Leu Tyr Leu Gln Met Ser Lys Val Arg Ser Glu Asp
        115                 120                 125

Thr Ala Leu Tyr Tyr Cys Ala Arg Glu Gly Tyr Tyr Asp Asn Glu Gly
    130                 135                 140

Tyr Phe Asp Val Trp Gly Thr Gly Thr Thr Val Thr Val Ser Ser Arg
145                 150                 155                 160

Ser Ser Ser Ala Ser Thr Lys Gly Pro Ser Val Phe Pro Leu Ala Pro
                165                 170                 175

Ser Ser Lys Ser Thr Ser Gly Gly Thr Ala Ala Leu Gly Cys Leu Val
            180                 185                 190

Lys Asp Tyr Phe Pro Glu Pro Val Thr Val Ser Trp Asn Ser Gly Ala
        195                 200                 205

Leu Thr Ser Gly Val His Thr Phe Pro Ala Val Leu Gln Ser Ser Gly
    210                 215                 220

Leu Tyr Ser Leu Ser Ser Val Val Thr Val Pro Ser Ser Ser Leu Gly
225                 230                 235                 240

Thr Gln Thr Tyr Ile Cys Asn Val Asn His Lys Pro Ser Asn Thr Lys
                245                 250                 255

Val Asp Lys Lys Val Glu Pro Lys Ser Cys Asp Lys Thr His Thr Cys
            260                 265                 270

Pro Pro Cys Pro Ala Pro Glu Leu Leu Gly Gly Pro Ser Val Phe Leu
        275                 280                 285

Phe Pro Pro Lys Pro Lys Asp Thr Leu Met Ile Ser Arg Thr Pro Glu
    290                 295                 300

Val Thr Cys Val Val Val Asp Val Ser His Glu Asp Pro Glu Val Lys
305                 310                 315                 320

Phe Asn Trp Tyr Val Asp Gly Val Glu Val His Asn Ala Lys Thr Lys
                325                 330                 335

Pro Arg Glu Glu Gln Tyr Ala Ser Thr Tyr Arg Val Val Ser Val Leu
            340                 345                 350

Thr Val Leu His Gln Asp Trp Leu Asn Gly Lys Glu Tyr Lys Cys Lys
        355                 360                 365

Val Ser Asn Lys Ala Leu Pro Ala Pro Ile Glu Lys Thr Ile Ser Lys
    370                 375                 380

Ala Lys Gly Gln Pro Arg Glu Pro Gln Val Tyr Thr Leu Pro Pro Ser
385                 390                 395                 400

Arg Asp Glu Leu Thr Lys Asn Gln Val Ser Leu Thr Cys Leu Val Lys
                405                 410                 415

Gly Phe Tyr Pro Ser Asp Ile Ala Val Glu Trp Glu Ser Asn Gly Gln
            420                 425                 430

Pro Glu Asn Asn Tyr Lys Thr Thr Pro Pro Val Leu Asp Ser Asp Gly
        435                 440                 445

Ser Phe Phe Leu Tyr Ser Lys Leu Thr Val Asp Lys Ser Arg Trp Gln
    450                 455                 460

Gln Gly Asn Val Phe Ser Cys Ser Val Met His Glu Ala Leu His Asn
465                 470                 475                 480

His Tyr Thr Gln Lys Ser Leu Ser Leu Ser Pro Gly Lys Leu Glu Asp
                485                 490                 495

Ile Ala Cys Gly Cys Ala Ala Ala Pro Asp Ile Lys Asp Leu Leu Ser
            500                 505                 510

Arg Leu Glu Glu Leu Glu Gly Leu Val Ser Ser Leu Arg Glu Gln Gly
        515                 520                 525

Thr Gly
    530

<210>  59
<211>  619
<212>  PRT
<213>  artificial sequence

<220>
<223>  pDualyx-155 HC

<400>  59

Met Asn Phe Gly Phe Ser Leu Ile Phe Leu Val Leu Val Leu Lys Gly
1               5                   10                  15

Val Gln Cys Glu Val Lys Leu Val Pro Arg Gln Leu Asp Tyr Lys Asp
            20                  25                  30

Asp Asp Asp Lys Glu Phe Glu Val Lys Leu Leu Gln Ser Gly Gly Gly
        35                  40                  45

Leu Val Gln Pro Gly Gly Ser Leu Lys Leu Ser Cys Glu Ala Ser Gly
    50                  55                  60

Ile Asp Phe Ser Arg Tyr Trp Met Ser Trp Val Arg Arg Ala Pro Gly
65                  70                  75                  80

Lys Gly Leu Glu Trp Ile Gly Glu Ser Asp Pro Asp Ser Ser Thr Ile
                85                  90                  95

Asn Tyr Ala Pro Ser Leu Lys Asp Lys Phe Ile Ile Ser Arg Asp Ser
            100                 105                 110

Ala Arg Asn Thr Leu Tyr Leu Gln Met Ser Lys Val Arg Ser Glu Asp
        115                 120                 125

Thr Ala Leu Tyr Tyr Cys Ala Arg Glu Gly Tyr Tyr Asp Asn Glu Gly
    130                 135                 140

Tyr Phe Asp Val Trp Gly Thr Gly Thr Thr Val Thr Val Ser Ser Arg
145                 150                 155                 160

Ser Ser Thr Lys Gly Pro Lys Leu Glu Glu Gly Glu Phe Ser Glu Ala
                165                 170                 175

Gln Leu Asn Ile Val Met Thr Gln Ser Pro Lys Ser Leu Ser Met Ser
            180                 185                 190

Val Gly Glu Arg Val Thr Leu Asn Cys Lys Ala Ser Glu Asn Val Lys
        195                 200                 205

Thr Tyr Val Ser Trp Tyr Gln Gln Lys Pro Glu Gln Ser Pro Lys Leu
    210                 215                 220

Leu Ile Tyr Gly Ala Ser Asn Arg Tyr Thr Gly Val Pro Asp Arg Phe
225                 230                 235                 240

Thr Gly Ser Gly Ser Ala Thr Asp Phe Thr Leu Thr Ile Ser Ser Val
                245                 250                 255

Gln Ala Glu Asp Leu Ala Asp Tyr His Cys Gly Gln Ser Tyr Ser Tyr
            260                 265                 270

Pro Phe Thr Phe Gly Ala Gly Thr Lys Leu Glu Arg Lys Gly Ser Ser
        275                 280                 285

Ser Ala Ser Thr Lys Gly Pro Ser Val Phe Pro Leu Ala Pro Ser Ser
    290                 295                 300

Lys Ser Thr Ser Gly Gly Thr Ala Ala Leu Gly Cys Leu Val Lys Asp
305                 310                 315                 320

Tyr Phe Pro Glu Pro Val Thr Val Ser Trp Asn Ser Gly Ala Leu Thr
                325                 330                 335

Ser Gly Val His Thr Phe Pro Ala Val Leu Gln Ser Ser Gly Leu Tyr
            340                 345                 350

Ser Leu Ser Ser Val Val Thr Val Pro Ser Ser Ser Leu Gly Thr Gln
        355                 360                 365

Thr Tyr Ile Cys Asn Val Asn His Lys Pro Ser Asn Thr Lys Val Asp
    370                 375                 380

Lys Lys Val Glu Pro Lys Ser Cys Asp Lys Thr His Thr Cys Pro Pro
385                 390                 395                 400

Cys Pro Ala Pro Glu Leu Leu Gly Gly Pro Ser Val Phe Leu Phe Pro
                405                 410                 415

Pro Lys Pro Lys Asp Thr Leu Met Ile Ser Arg Thr Pro Glu Val Thr
            420                 425                 430

Cys Val Val Val Asp Val Ser His Glu Asp Pro Glu Val Lys Phe Asn
        435                 440                 445

Trp Tyr Val Asp Gly Val Glu Val His Asn Ala Lys Thr Lys Pro Arg
    450                 455                 460

Glu Glu Gln Tyr Ala Ser Thr Tyr Arg Val Val Ser Val Leu Thr Val
465                 470                 475                 480

Leu His Gln Asp Trp Leu Asn Gly Lys Glu Tyr Lys Cys Lys Val Ser
                485                 490                 495

Asn Lys Ala Leu Pro Ala Pro Ile Glu Lys Thr Ile Ser Lys Ala Lys
            500                 505                 510

Gly Gln Pro Arg Glu Pro Gln Val Tyr Thr Leu Pro Pro Ser Arg Asp
        515                 520                 525

Glu Leu Thr Lys Asn Gln Val Ser Leu Thr Cys Leu Val Lys Gly Phe
    530                 535                 540

Tyr Pro Ser Asp Ile Ala Val Glu Trp Glu Ser Asn Gly Gln Pro Glu
545                 550                 555                 560

Asn Asn Tyr Lys Thr Thr Pro Pro Val Leu Asp Ser Asp Gly Ser Phe
                565                 570                 575

Phe Leu Tyr Ser Lys Leu Thr Val Asp Lys Ser Arg Trp Gln Gln Gly
            580                 585                 590

Asn Val Phe Ser Cys Ser Val Met His Glu Ala Leu His Asn His Tyr
        595                 600                 605

Thr Gln Lys Ser Leu Ser Leu Ser Pro Gly Lys

610                 615

<210>  60
<211>  656
<212>  PRT
<213>  artificial sequence

<220>
<223>  pDualyx-157 HC

<400>  60

Met Asn Phe Gly Phe Ser Leu Ile Phe Leu Val Leu Val Leu Lys Gly
1               5                   10                  15

Val Gln Cys Glu Val Lys Leu Val Pro Arg Gln Leu Asp Tyr Lys Asp
            20                  25                  30

Asp Asp Asp Lys Glu Phe Glu Val Lys Leu Leu Gln Ser Gly Gly Gly
        35                  40                  45

Leu Val Gln Pro Gly Gly Ser Leu Lys Leu Ser Cys Glu Ala Ser Gly
    50                  55                  60

Ile Asp Phe Ser Arg Tyr Trp Met Ser Trp Val Arg Arg Ala Pro Gly
65                  70                  75                  80

Lys Gly Leu Glu Trp Ile Gly Glu Ser Asp Pro Asp Ser Ser Thr Ile
                85                  90                  95

Asn Tyr Ala Pro Ser Leu Lys Asp Lys Phe Ile Ile Ser Arg Asp Ser
            100                 105                 110

Ala Arg Asn Thr Leu Tyr Leu Gln Met Ser Lys Val Arg Ser Glu Asp
        115                 120                 125

Thr Ala Leu Tyr Tyr Cys Ala Arg Glu Gly Tyr Tyr Asp Asn Glu Gly
    130                 135                 140

Tyr Phe Asp Val Trp Gly Thr Gly Thr Thr Val Thr Val Ser Ser Arg
145                 150                 155                 160

Ser Ser Thr Lys Gly Pro Lys Leu Glu Glu Gly Glu Phe Ser Glu Ala
                165                 170                 175

Gln Leu Asn Ile Val Met Thr Gln Ser Pro Lys Ser Leu Ser Met Ser
            180                 185                 190

Val Gly Glu Arg Val Thr Leu Asn Cys Lys Ala Ser Glu Asn Val Lys
        195                 200                 205

Thr Tyr Val Ser Trp Tyr Gln Gln Lys Pro Glu Gln Ser Pro Lys Leu
    210                 215                 220

Leu Ile Tyr Gly Ala Ser Asn Arg Tyr Thr Gly Val Pro Asp Arg Phe
225                 230                 235                 240

Thr Gly Ser Gly Ser Ala Thr Asp Phe Thr Leu Thr Ile Ser Ser Val
                245                 250                 255

Gln Ala Glu Asp Leu Ala Asp Tyr His Cys Gly Gln Ser Tyr Ser Tyr
            260                 265                 270

Pro Phe Thr Phe Gly Ala Gly Thr Lys Leu Glu Arg Lys Gly Ser Ser
        275                 280                 285

Ser Ala Ser Thr Lys Gly Pro Ser Val Phe Pro Leu Ala Pro Ser Ser
    290                 295                 300

Lys Ser Thr Ser Gly Gly Thr Ala Ala Leu Gly Cys Leu Val Lys Asp
305                 310                 315                 320

Tyr Phe Pro Glu Pro Val Thr Val Ser Trp Asn Ser Gly Ala Leu Thr
                325                 330                 335

Ser Gly Val His Thr Phe Pro Ala Val Leu Gln Ser Ser Gly Leu Tyr
            340                 345                 350

Ser Leu Ser Ser Val Val Thr Val Pro Ser Ser Ser Leu Gly Thr Gln
        355                 360                 365

Thr Tyr Ile Cys Asn Val Asn His Lys Pro Ser Asn Thr Lys Val Asp
    370                 375                 380

Lys Lys Val Glu Pro Lys Ser Cys Asp Lys Thr His Thr Cys Pro Pro
385                 390                 395                 400

Cys Pro Ala Pro Glu Leu Leu Gly Gly Pro Ser Val Phe Leu Phe Pro
                405                 410                 415

Pro Lys Pro Lys Asp Thr Leu Met Ile Ser Arg Thr Pro Glu Val Thr
            420                 425                 430

Cys Val Val Val Asp Val Ser His Glu Asp Pro Glu Val Lys Phe Asn
        435                 440                 445

Trp Tyr Val Asp Gly Val Glu Val His Asn Ala Lys Thr Lys Pro Arg

450                 455                 460

Glu Glu Gln Tyr Ala Ser Thr Tyr Arg Val Val Ser Val Leu Thr Val
465                 470                 475                 480

Leu His Gln Asp Trp Leu Asn Gly Lys Glu Tyr Lys Cys Lys Val Ser
                485                 490                 495

Asn Lys Ala Leu Pro Ala Pro Ile Glu Lys Thr Ile Ser Lys Ala Lys
            500                 505                 510

Gly Gln Pro Arg Glu Pro Gln Val Tyr Thr Leu Pro Pro Ser Arg Asp
        515                 520                 525

Glu Leu Thr Lys Asn Gln Val Ser Leu Thr Cys Leu Val Lys Gly Phe
    530                 535                 540

Tyr Pro Ser Asp Ile Ala Val Glu Trp Glu Ser Asn Gly Gln Pro Glu
545                 550                 555                 560

Asn Asn Tyr Lys Thr Thr Pro Pro Val Leu Asp Ser Asp Gly Ser Phe
                565                 570                 575

Phe Leu Tyr Ser Lys Leu Thr Val Asp Lys Ser Arg Trp Gln Gln Gly
            580                 585                 590

Asn Val Phe Ser Cys Ser Val Met His Glu Ala Leu His Asn His Tyr
        595                 600                 605

Thr Gln Lys Ser Leu Ser Leu Ser Pro Gly Lys Leu Glu Asp Ile Ala
    610                 615                 620

Cys Gly Cys Ala Ala Ala Pro Asp Ile Lys Asp Leu Leu Ser Arg Leu
625                 630                 635                 640

Glu Glu Leu Glu Gly Leu Val Ser Ser Leu Arg Glu Gln Gly Thr Gly
                645                 650                 655

<210>  61
<211>  440
<212>  PRT
<213>  artificial sequence

<220>
<223>  pDualyx-158 HC

<400>  61

Met Asn Phe Gly Phe Ser Leu Ile Phe Leu Val Leu Val Leu Lys Gly
1               5                   10                  15

Val Gln Cys Glu Val Lys Leu Val Pro Arg Gln Leu Asp Tyr Lys Asp
            20                  25                  30

Asp Asp Asp Lys Glu Phe Glu Val Lys Leu Leu Gln Ser Gly Gly Gly
        35                  40                  45

Leu Val Gln Pro Gly Gly Ser Leu Lys Leu Ser Cys Glu Ala Ser Gly
    50                  55                  60

Ile Asp Phe Ser Arg Tyr Trp Met Ser Trp Val Arg Arg Ala Pro Gly
65                  70                  75                  80

Lys Gly Leu Glu Trp Ile Gly Glu Ser Asp Pro Asp Ser Ser Thr Ile
                85                  90                  95

Asn Tyr Ala Pro Ser Leu Lys Asp Lys Phe Ile Ile Ser Arg Asp Ser
            100                 105                 110

Ala Arg Asn Thr Leu Tyr Leu Gln Met Ser Lys Val Arg Ser Glu Asp
        115                 120                 125

Thr Ala Leu Tyr Tyr Cys Ala Arg Glu Gly Tyr Tyr Asp Asn Glu Gly
    130                 135                 140

Tyr Phe Asp Val Trp Gly Thr Gly Thr Thr Val Thr Val Ser Ser Arg
145                 150                 155                 160

Ser Ser Thr Lys Gly Pro Lys Leu Glu Glu Gly Glu Phe Ser Glu Ala
                165                 170                 175

Gln Leu Asn Ile Val Met Thr Gln Ser Pro Lys Ser Leu Ser Met Ser
            180                 185                 190

Val Gly Glu Arg Val Thr Leu Asn Cys Lys Ala Ser Glu Asn Val Lys
        195                 200                 205

Thr Tyr Val Ser Trp Tyr Gln Gln Lys Pro Glu Gln Ser Pro Lys Leu
    210                 215                 220

Leu Ile Tyr Gly Ala Ser Asn Arg Tyr Thr Gly Val Pro Asp Arg Phe
225                 230                 235                 240

Thr Gly Ser Gly Ser Ala Thr Asp Phe Thr Leu Thr Ile Ser Ser Val
                245                 250                 255

Gln Ala Glu Asp Leu Ala Asp Tyr His Cys Gly Gln Ser Tyr Ser Tyr

260                 265                 270

Pro Phe Thr Phe Gly Ala Gly Thr Lys Leu Glu Arg Lys Gly Ser Ser
        275                 280                 285

Ser Ala Ser Thr Lys Gly Pro Ser Val Phe Pro Leu Ala Pro Ser Ser
    290                 295                 300

Lys Ser Thr Ser Gly Gly Thr Ala Ala Leu Gly Cys Leu Val Lys Asp
305                 310                 315                 320

Tyr Phe Pro Glu Pro Val Thr Val Ser Trp Asn Ser Gly Ala Leu Thr
                325                 330                 335

Ser Gly Val His Thr Phe Pro Ala Val Leu Gln Ser Ser Gly Leu Tyr
            340                 345                 350

Ser Leu Ser Ser Val Val Thr Val Pro Ser Ser Ser Leu Gly Thr Gln
        355                 360                 365

Thr Tyr Ile Cys Asn Val Asn His Lys Pro Ser Asn Thr Lys Val Asp
    370                 375                 380

Lys Lys Val Glu Pro Lys Ser Cys Asp Lys Thr His Thr Cys Pro Pro
385                 390                 395                 400

Cys Pro Ala Leu Glu Asp Ile Ala Cys Gly Cys Ala Ala Ala Pro Asp
                405                 410                 415

Ile Lys Asp Leu Leu Ser Arg Leu Glu Glu Leu Glu Gly Leu Val Ser
            420                 425                 430

Ser Leu Arg Glu Gln Gly Thr Gly
        435                 440

<210>  62
<211>  528
<212>  PRT
<213>  artificial sequence

<220>
<223>  pDualyx-159 HC

<400>  62

Met Asn Phe Gly Phe Ser Leu Ile Phe Leu Val Leu Val Leu Lys Gly
1               5                   10                  15

Val Gln Cys Glu Val Lys Leu Val Pro Arg Gln Leu Asp Tyr Lys Asp
            20                  25                  30

Asp Asp Asp Lys Glu Phe Gln Val Thr Leu Lys Glu Ser Gly Pro Gly
        35                  40                  45

Ile Leu Gln Pro Ser Gln Thr Leu Ser Leu Thr Cys Ser Phe Ser Gly
    50                  55                  60

Phe Ser Leu Ser Thr Phe Gly Met Gly Val Gly Trp Ile Arg Gln Pro
65                  70                  75                  80

Ser Gly Lys Gly Leu Glu Trp Leu Ala His Ile Trp Trp Asp Asp Asp
                85                  90                  95

Lys Tyr Tyr Asn Pro Ala Leu Lys Ser Arg Leu Thr Ile Ser Lys Asp
            100                 105                 110

Thr Ser Glu Asn Gln Val Phe Leu Lys Ile Ala Asn Val Asp Thr Ala
        115                 120                 125

Asp Thr Ala Thr Tyr Tyr Cys Val Arg Ile Ala Gly Thr Arg Tyr Phe
    130                 135                 140

Asp Val Trp Gly Thr Gly Thr Thr Val Thr Val Ser Ser Arg Ser Ser
145                 150                 155                 160

Ser Ala Ser Thr Lys Gly Pro Ser Val Phe Pro Leu Ala Pro Ser Ser
                165                 170                 175

Lys Ser Thr Ser Gly Gly Thr Ala Ala Leu Gly Cys Leu Val Lys Asp
            180                 185                 190

Tyr Phe Pro Glu Pro Val Thr Val Ser Trp Asn Ser Gly Ala Leu Thr
        195                 200                 205

Ser Gly Val His Thr Phe Pro Ala Val Leu Gln Ser Ser Gly Leu Tyr
    210                 215                 220

Ser Leu Ser Ser Val Val Thr Val Pro Ser Ser Ser Leu Gly Thr Gln
225                 230                 235                 240

Thr Tyr Ile Cys Asn Val Asn His Lys Pro Ser Asn Thr Lys Val Asp
                245                 250                 255

Lys Lys Val Glu Pro Lys Ser Cys Asp Lys Thr His Thr Cys Pro Pro
            260                 265                 270

Cys Pro Ala Pro Glu Leu Leu Gly Gly Pro Ser Val Phe Leu Phe Pro

        275                 280                 285

Pro Lys Pro Lys Asp Thr Leu Met Ile Ser Arg Thr Pro Glu Val Thr
    290                 295                 300

Cys Val Val Val Asp Val Ser His Glu Asp Pro Glu Val Lys Phe Asn
305                 310                 315                 320

Trp Tyr Val Asp Gly Val Glu Val His Asn Ala Lys Thr Lys Pro Arg
                325                 330                 335

Glu Glu Gln Tyr Ala Ser Thr Tyr Arg Val Val Ser Val Leu Thr Val
            340                 345                 350

Leu His Gln Asp Trp Leu Asn Gly Lys Glu Tyr Lys Cys Lys Val Ser
        355                 360                 365

Asn Lys Ala Leu Pro Ala Pro Ile Glu Lys Thr Ile Ser Lys Ala Lys
    370                 375                 380

Gly Gln Pro Arg Glu Pro Gln Val Tyr Thr Leu Pro Pro Ser Arg Asp
385                 390                 395                 400

Glu Leu Thr Lys Asn Gln Val Ser Leu Thr Cys Leu Val Lys Gly Phe
                405                 410                 415

Tyr Pro Ser Asp Ile Ala Val Glu Trp Glu Ser Asn Gly Gln Pro Glu
            420                 425                 430

Asn Asn Tyr Lys Thr Thr Pro Pro Val Leu Asp Ser Asp Gly Ser Phe
        435                 440                 445

Phe Leu Tyr Ser Lys Leu Thr Val Asp Lys Ser Arg Trp Gln Gln Gly
    450                 455                 460

Asn Val Phe Ser Cys Ser Val Met His Glu Ala Leu His Asn His Tyr
465                 470                 475                 480

Thr Gln Lys Ser Leu Ser Leu Ser Pro Gly Lys Leu Glu Asp Ile Ala
                485                 490                 495

Cys Gly Cys Ala Ala Ala Pro Asp Ile Lys Asp Leu Leu Ser Arg Leu
            500                 505                 510

Glu Glu Leu Glu Gly Leu Val Ser Ser Leu Arg Glu Gln Gly Thr Gly
        515                 520                 525

<210>  63
<211>  616
<212>  PRT
<213>  artificial sequence

<220>
<223>  pDualyx-160 HC

<400>  63

Met Asn Phe Gly Phe Ser Leu Ile Phe Leu Val Leu Val Leu Lys Gly
1               5                   10                  15

Val Gln Cys Glu Val Lys Leu Val Pro Arg Gln Leu Asp Tyr Lys Asp
            20                  25                  30

Asp Asp Asp Lys Glu Phe Gln Val Thr Leu Lys Glu Ser Gly Pro Gly
        35                  40                  45

Ile Leu Gln Pro Ser Gln Thr Leu Ser Leu Thr Cys Ser Phe Ser Gly
    50                  55                  60

Phe Ser Leu Ser Thr Phe Gly Met Gly Val Gly Trp Ile Arg Gln Pro
65                  70                  75                  80

Ser Gly Lys Gly Leu Glu Trp Leu Ala His Ile Trp Trp Asp Asp Asp
                85                  90                  95

Lys Tyr Tyr Asn Pro Ala Leu Lys Ser Arg Leu Thr Ile Ser Lys Asp
            100                 105                 110

Thr Ser Glu Asn Gln Val Phe Leu Lys Ile Ala Asn Val Asp Thr Ala
        115                 120                 125

Asp Thr Ala Thr Tyr Tyr Cys Val Arg Ile Ala Gly Thr Arg Tyr Phe
    130                 135                 140

Asp Val Trp Gly Thr Gly Thr Thr Val Thr Val Ser Ser Arg Ser Ser
145                 150                 155                 160

Thr Lys Gly Pro Lys Leu Glu Glu Gly Glu Phe Ser Glu Ala Gln Leu
                165                 170                 175

Asp Ile Gln Met Thr Gln Ser Pro Ser Ser Leu Ser Ala Ser Leu Gly
            180                 185                 190

Gly Lys Val Thr Ile Thr Cys Lys Ala Ser Gln Asp Ile Asn Lys Phe
        195                 200                 205

Ile Ala Trp Tyr Gln His Lys Pro Gly Lys Gly Pro Arg Leu Leu Ile

210                 215                 220

His Tyr Thr Ser Thr Leu Gln Pro Gly Ile Pro Ser Arg Phe Ser Gly
225                 230                 235                 240

Ser Gly Ser Gly Arg Asp Tyr Ser Phe Ser Ile Ser Asn Leu Glu Pro
                245                 250                 255

Glu Asp Ile Ala Thr Tyr Tyr Cys Leu Gln Tyr Asp Asn Leu Tyr Thr
            260                 265                 270

Phe Gly Gly Gly Thr Lys Leu Glu Ile Lys Gly Ser Ser Ser Ala Ser
        275                 280                 285

Thr Lys Gly Pro Ser Val Phe Pro Leu Ala Pro Ser Ser Lys Ser Thr
    290                 295                 300

Ser Gly Gly Thr Ala Ala Leu Gly Cys Leu Val Lys Asp Tyr Phe Pro
305                 310                 315                 320

Glu Pro Val Thr Val Ser Trp Asn Ser Gly Ala Leu Thr Ser Gly Val
                325                 330                 335

His Thr Phe Pro Ala Val Leu Gln Ser Ser Gly Leu Tyr Ser Leu Ser
            340                 345                 350

Ser Val Val Thr Val Pro Ser Ser Ser Leu Gly Thr Gln Thr Tyr Ile
        355                 360                 365

Cys Asn Val Asn His Lys Pro Ser Asn Thr Lys Val Asp Lys Lys Val
    370                 375                 380

Glu Pro Lys Ser Cys Asp Lys Thr His Thr Cys Pro Pro Cys Pro Ala
385                 390                 395                 400

Pro Glu Leu Leu Gly Gly Pro Ser Val Phe Leu Phe Pro Pro Lys Pro
                405                 410                 415

Lys Asp Thr Leu Met Ile Ser Arg Thr Pro Glu Val Thr Cys Val Val
            420                 425                 430

Val Asp Val Ser His Glu Asp Pro Glu Val Lys Phe Asn Trp Tyr Val
        435                 440                 445

Asp Gly Val Glu Val His Asn Ala Lys Thr Lys Pro Arg Glu Glu Gln
    450                 455                 460

Tyr Ala Ser Thr Tyr Arg Val Val Ser Val Leu Thr Val Leu His Gln
465                 470                 475                 480

Asp Trp Leu Asn Gly Lys Glu Tyr Lys Cys Lys Val Ser Asn Lys Ala
                485                 490                 495

Leu Pro Ala Pro Ile Glu Lys Thr Ile Ser Lys Ala Lys Gly Gln Pro
            500                 505                 510

Arg Glu Pro Gln Val Tyr Thr Leu Pro Pro Ser Arg Asp Glu Leu Thr
        515                 520                 525

Lys Asn Gln Val Ser Leu Thr Cys Leu Val Lys Gly Phe Tyr Pro Ser
    530                 535                 540

Asp Ile Ala Val Glu Trp Glu Ser Asn Gly Gln Pro Glu Asn Asn Tyr
545                 550                 555                 560

Lys Thr Thr Pro Pro Val Leu Asp Ser Asp Gly Ser Phe Phe Leu Tyr
                565                 570                 575

Ser Lys Leu Thr Val Asp Lys Ser Arg Trp Gln Gln Gly Asn Val Phe
            580                 585                 590

Ser Cys Ser Val Met His Glu Ala Leu His Asn His Tyr Thr Gln Lys
        595                 600                 605

Ser Leu Ser Leu Ser Pro Gly Lys
    610                 615

<210>  64
<211>  653
<212>  PRT
<213>  artificial sequence

<220>
<223>  pDualyx-162 HC

<400>  64

Met Asn Phe Gly Phe Ser Leu Ile Phe Leu Val Leu Val Leu Lys Gly
1               5                   10                  15

Val Gln Cys Glu Val Lys Leu Val Pro Arg Gln Leu Asp Tyr Lys Asp
            20                  25                  30

Asp Asp Asp Lys Glu Phe Gln Val Thr Leu Lys Glu Ser Gly Pro Gly
        35                  40                  45

Ile Leu Gln Pro Ser Gln Thr Leu Ser Leu Thr Cys Ser Phe Ser Gly

50                  55                  60

Phe Ser Leu Ser Thr Phe Gly Met Gly Val Gly Trp Ile Arg Gln Pro
65                  70                  75                  80

Ser Gly Lys Gly Leu Glu Trp Leu Ala His Ile Trp Trp Asp Asp Asp
                85                  90                  95

Lys Tyr Tyr Asn Pro Ala Leu Lys Ser Arg Leu Thr Ile Ser Lys Asp
            100                 105                 110

Thr Ser Glu Asn Gln Val Phe Leu Lys Ile Ala Asn Val Asp Thr Ala
        115                 120                 125

Asp Thr Ala Thr Tyr Tyr Cys Val Arg Ile Ala Gly Thr Arg Tyr Phe
    130                 135                 140

Asp Val Trp Gly Thr Gly Thr Thr Val Thr Val Ser Ser Arg Ser Ser
145                 150                 155                 160

Thr Lys Gly Pro Lys Leu Glu Glu Gly Glu Phe Ser Glu Ala Gln Leu
                165                 170                 175

Asp Ile Gln Met Thr Gln Ser Pro Ser Ser Leu Ser Ala Ser Leu Gly
            180                 185                 190

Gly Lys Val Thr Ile Thr Cys Lys Ala Ser Gln Asp Ile Asn Lys Phe
        195                 200                 205

Ile Ala Trp Tyr Gln His Lys Pro Gly Lys Gly Pro Arg Leu Leu Ile
    210                 215                 220

His Tyr Thr Ser Thr Leu Gln Pro Gly Ile Pro Ser Arg Phe Ser Gly
225                 230                 235                 240

Ser Gly Ser Gly Arg Asp Tyr Ser Phe Ser Ile Ser Asn Leu Glu Pro
                245                 250                 255

Glu Asp Ile Ala Thr Tyr Tyr Cys Leu Gln Tyr Asp Asn Leu Tyr Thr
            260                 265                 270

Phe Gly Gly Gly Thr Lys Leu Glu Ile Lys Gly Ser Ser Ser Ala Ser
        275                 280                 285

Thr Lys Gly Pro Ser Val Phe Pro Leu Ala Pro Ser Ser Lys Ser Thr
    290                 295                 300

Ser Gly Gly Thr Ala Ala Leu Gly Cys Leu Val Lys Asp Tyr Phe Pro
305                 310                 315                 320

Glu Pro Val Thr Val Ser Trp Asn Ser Gly Ala Leu Thr Ser Gly Val
                325                 330                 335

His Thr Phe Pro Ala Val Leu Gln Ser Ser Gly Leu Tyr Ser Leu Ser
            340                 345                 350

Ser Val Val Thr Val Pro Ser Ser Ser Leu Gly Thr Gln Thr Tyr Ile
        355                 360                 365

Cys Asn Val Asn His Lys Pro Ser Asn Thr Lys Val Asp Lys Lys Val
    370                 375                 380

Glu Pro Lys Ser Cys Asp Lys Thr His Thr Cys Pro Pro Cys Pro Ala
385                 390                 395                 400

Pro Glu Leu Leu Gly Gly Pro Ser Val Phe Leu Phe Pro Pro Lys Pro
                405                 410                 415

Lys Asp Thr Leu Met Ile Ser Arg Thr Pro Glu Val Thr Cys Val Val
            420                 425                 430

Val Asp Val Ser His Glu Asp Pro Glu Val Lys Phe Asn Trp Tyr Val
        435                 440                 445

Asp Gly Val Glu Val His Asn Ala Lys Thr Lys Pro Arg Glu Glu Gln
    450                 455                 460

Tyr Ala Ser Thr Tyr Arg Val Val Ser Val Leu Thr Val Leu His Gln
465                 470                 475                 480

Asp Trp Leu Asn Gly Lys Glu Tyr Lys Cys Lys Val Ser Asn Lys Ala
                485                 490                 495

Leu Pro Ala Pro Ile Glu Lys Thr Ile Ser Lys Ala Lys Gly Gln Pro
            500                 505                 510

Arg Glu Pro Gln Val Tyr Thr Leu Pro Pro Ser Arg Asp Glu Leu Thr
        515                 520                 525

Lys Asn Gln Val Ser Leu Thr Cys Leu Val Lys Gly Phe Tyr Pro Ser
    530                 535                 540

Asp Ile Ala Val Glu Trp Glu Ser Asn Gly Gln Pro Glu Asn Asn Tyr
545                 550                 555                 560

Lys Thr Thr Pro Pro Val Leu Asp Ser Asp Gly Ser Phe Phe Leu Tyr
                565                 570                 575

Ser Lys Leu Thr Val Asp Lys Ser Arg Trp Gln Gln Gly Asn Val Phe
            580                 585                 590

Ser Cys Ser Val Met His Glu Ala Leu His Asn His Tyr Thr Gln Lys
        595                 600                 605

Ser Leu Ser Leu Ser Pro Gly Lys Leu Glu Asp Ile Ala Cys Gly Cys
    610                 615                 620

Ala Ala Ala Pro Asp Ile Lys Asp Leu Leu Ser Arg Leu Glu Glu Leu
625                 630                 635                 640

Glu Gly Leu Val Ser Ser Leu Arg Glu Gln Gly Thr Gly
                645                 650

<210> 65
<211> 437
<212> PRT
<213> artificial sequence

<220>
<223> pDualyx-163 HC

<400> 65

Met Asn Phe Gly Phe Ser Leu Ile Phe Leu Val Leu Val Leu Lys Gly
1               5                   10                  15

Val Gln Cys Glu Val Lys Leu Val Pro Arg Gln Leu Asp Tyr Lys Asp
            20                  25                  30

Asp Asp Asp Lys Glu Phe Gln Val Thr Leu Lys Glu Ser Gly Pro Gly
        35                  40                  45

Ile Leu Gln Pro Ser Gln Thr Leu Ser Leu Thr Cys Ser Phe Ser Gly
    50                  55                  60

Phe Ser Leu Ser Thr Phe Gly Met Gly Val Gly Trp Ile Arg Gln Pro
65                  70                  75                  80

Ser Gly Lys Gly Leu Glu Trp Leu Ala His Ile Trp Trp Asp Asp Asp
                85                  90                  95

Lys Tyr Tyr Asn Pro Ala Leu Lys Ser Arg Leu Thr Ile Ser Lys Asp
            100                 105                 110

Thr Ser Glu Asn Gln Val Phe Leu Lys Ile Ala Asn Val Asp Thr Ala
        115                 120                 125

Asp Thr Ala Thr Tyr Tyr Cys Val Arg Ile Ala Gly Thr Arg Tyr Phe
    130                 135                 140

Asp Val Trp Gly Thr Gly Thr Thr Val Thr Val Ser Ser Arg Ser Ser
145                 150                 155                 160

Thr Lys Gly Pro Lys Leu Glu Glu Gly Glu Phe Ser Glu Ala Gln Leu
                165                 170                 175

Asp Ile Gln Met Thr Gln Ser Pro Ser Ser Leu Ser Ala Ser Leu Gly
            180                 185                 190

Gly Lys Val Thr Ile Thr Cys Lys Ala Ser Gln Asp Ile Asn Lys Phe
        195                 200                 205

Ile Ala Trp Tyr Gln His Lys Pro Gly Lys Gly Pro Arg Leu Leu Ile
    210                 215                 220

His Tyr Thr Ser Thr Leu Gln Pro Gly Ile Pro Ser Arg Phe Ser Gly
225                 230                 235                 240

Ser Gly Ser Gly Arg Asp Tyr Ser Phe Ser Ile Ser Asn Leu Glu Pro
                245                 250                 255

Glu Asp Ile Ala Thr Tyr Tyr Cys Leu Gln Tyr Asp Asn Leu Tyr Thr
            260                 265                 270

Phe Gly Gly Gly Thr Lys Leu Glu Ile Lys Gly Ser Ser Ser Ala Ser
        275                 280                 285

Thr Lys Gly Pro Ser Val Phe Pro Leu Ala Pro Ser Ser Lys Ser Thr
    290                 295                 300

Ser Gly Gly Thr Ala Ala Leu Gly Cys Leu Val Lys Asp Tyr Phe Pro
305                 310                 315                 320

Glu Pro Val Thr Val Ser Trp Asn Ser Gly Ala Leu Thr Ser Gly Val
                325                 330                 335

His Thr Phe Pro Ala Val Leu Gln Ser Ser Gly Leu Tyr Ser Leu Ser
            340                 345                 350

Ser Val Val Thr Val Pro Ser Ser Ser Leu Gly Thr Gln Thr Tyr Ile
        355                 360                 365

Cys Asn Val Asn His Lys Pro Ser Asn Thr Lys Val Asp Lys Lys Val
    370                 375                 380

Glu Pro Lys Ser Cys Asp Lys Thr His Thr Cys Pro Pro Cys Pro Ala
385                 390                 395                 400

Leu Glu Asp Ile Ala Cys Gly Cys Ala Ala Ala Pro Asp Ile Lys Asp
                405                 410                 415

Leu Leu Ser Arg Leu Glu Glu Leu Glu Gly Leu Val Ser Ser Leu Arg
            420                 425                 430

Glu Gln Gly Thr Gly
        435

<210>  66
<211>  21
<212>  PRT
<213>  artificial sequence

<220>
<223>  C1 VL CDR1

<400>  66

Arg Ser Ser Thr Gly Ala Val Thr Thr Ser Asn Tyr Ala Asn Gly Thr
1               5                   10                  15

Asn Asn Arg Thr Pro
            20

<210>  67
<211>  7
<212>  PRT
<213>  artificial sequence

<220>
<223>  C1 VL CDR2

<400>  67

Gly Thr Asn Asn Arg Thr Pro
1               5

<210>  68
<211>  9
<212>  PRT
<213>  artificial sequence

<220>
<223>  C1 VL CDR3

<400>  68

Ala Leu Trp Tyr Ser Asn His Trp Val
1               5


<210>  69
<211>  11
<212>  PRT
<213>  artificial sequence

<220>
<223>  C4 VL CDR1

<400>  69

Lys Ala Ser Gln Asp Val Asp Thr Ala Val Ala
1               5                   10


<210>  70
<211>  7
<212>  PRT
<213>  artificial sequence

<220>
<223>  C4 VL CDR2

<400>  70

Trp Ala Ser Thr Arg His Thr
1               5


<210>  71
<211>  9
<212>  PRT
<213>  artificial sequence

<220>
<223>  C4 VL CDR3

<400>  71

Gln Gln Tyr Tyr Ser Val Pro Pro Thr
1               5


<210>  72
<211>  10
<212>  PRT
<213>  artificial sequence

<220>
<223>  C8 VL CDR1

<400>  72

Lys Ala Ser Gln Asp Val Asp Thr Ala Val
1               5                   10


<210>  73
<211>  7
<212>  PRT

<213>  artificial sequence

<220>
<223>  C8 VL CDR2

<400>  73

Trp Ala Ser Thr Arg His Thr
1               5


<210>  74
<211>  9
<212>  PRT
<213>  artificial sequence

<220>
<223>  C8 VL CDR3

<400>  74

Gln Gln Tyr Tyr Ser Val Pro Pro Thr
1               5


<210>  75
<211>  10
<212>  PRT
<213>  artificial sequence

<220>
<223>  C9 VL CDR1

<400>  75

Ser Ala Ser Ser Ser Val Ser Tyr Met His
1               5                   10


<210>  76
<211>  7
<212>  PRT
<213>  artificial sequence

<220>
<223>  C9 VL CDR2

<400>  76

Ser Thr Ser Asn Leu Ala Ser
1               5


<210>  77
<211>  9
<212>  PRT
<213>  artificial sequence

<220>
<223>  C9 VL CDR3

<400>  77

Gln Gln Arg Ser Ser Tyr Pro Pro Thr
1               5

<210>  78
<211>  15
<212>  PRT
<213>  artificial sequence

<220>
<223>  C15 VL CDR1

<400>  78

Arg Ala Ser Glu Asn Ile Asp Asn Tyr Gly Ile Ser Phe Met Tyr
1               5                   10                  15

<210>  79
<211>  7
<212>  PRT
<213>  artificial sequence

<220>
<223>  C15 VL CDR2

<400>  79

Ala Ala Ser Asn Gln Gly Ser
1               5

<210>  80
<211>  9
<212>  PRT
<213>  artificial sequence

<220>
<223>  C15 VL CDR3

<400>  80

Gln Gln Ser Lys Glu Val Pro Tyr Thr
1               5

<210>  81
<211>  11
<212>  PRT
<213>  artificial sequence

<220>
<223>  C16 VL CDR1

<400>  81

Lys Ala Ser Gln Asn Val Gly Thr Asn Val Ala
1               5                   10

<210>  82
<211>  7
<212>  PRT

<213>  artificial sequence

<220>
<223>  C16 VL CDR2

<400>  82

Ser Ala Ser Phe Arg Tyr Ser
1               5


<210>  83
<211>  9
<212>  PRT
<213>  artificial sequence

<220>
<223>  C16 VL CDR3

<400>  83

Gln His Phe Asn Ser Phe Pro Leu Thr
1               5


<210>  84
<211>  11
<212>  PRT
<213>  artificial sequence

<220>
<223>  C19 VL CDR1

<400>  84

Lys Ala Ser Gln Asn Val Gly Thr Asn Val Ala
1               5                   10


<210>  85
<211>  7
<212>  PRT
<213>  artificial sequence

<220>
<223>  C19 VL CDR2

<400>  85

Ser Ala Ser Tyr Arg Tyr Ser
1               5


<210>  86
<211>  9
<212>  PRT
<213>  artificial sequence

<220>
<223>  C19 VL CDR3

<400>  86

Gln Gln Phe Asp Ser His Pro Leu Thr
1               5

<210>  87
<211>  11
<212>  PRT
<213>  artificial sequence

<220>
<223>  C25 VL CDR1

<400>  87

Lys Ala Ser Gln Asn Val Gly Ser Asn Val Asp
1               5                   10

<210>  88
<211>  7
<212>  PRT
<213>  artificial sequence

<220>
<223>  C25 VL CDR2

<400>  88

Ser Ala Ser Tyr Arg Tyr Ser
1               5

<210>  89
<211>  9
<212>  PRT
<213>  artificial sequence

<220>
<223>  C25 VL CDR3

<400>  89

Gln Gln Tyr Asn Ser His Pro Leu Thr
1               5

<210>  90
<211>  16
<212>  PRT
<213>  artificial sequence

<220>
<223>  C31-1 VL CDR1

<400>  90

Arg Ala Ser Gln Ser Leu Val His Ser Asn Gly Asn Thr Tyr Leu Gln
1               5                   10                  15

<210>  91
<211>  7
<212>  PRT

<213>  artificial sequence

<220>
<223>  C31-1 VL CDR2

<400>  91

Lys Val Ser Asn Arg Phe Ser
1               5


<210>  92
<211>  9
<212>  PRT
<213>  artificial sequence

<220>
<223>  C31-1 VL CDR3

<400>  92

Ser Gln Ser Thr His Val Pro Phe Thr
1               5


<210>  93
<211>  14
<212>  PRT
<213>  artificial sequence

<220>
<223>  C31-2 VL CDR1

<400>  93

Arg Ser Ser Thr Gly Ala Val Thr Thr Ser Asn Tyr Ala Asn
1               5                   10


<210>  94
<211>  6
<212>  PRT
<213>  artificial sequence

<220>
<223>  C31-2 VL CDR2

<400>  94

Thr Asn Asn Arg Ala Pro
1               5


<210>  95
<211>  9
<212>  PRT
<213>  artificial sequence

<220>
<223>  C31-2 VL CDR3

<400>  95

Ala Leu Trp Tyr Ser Asn His Trp Val
1               5

<210>  96
<211>  11
<212>  PRT
<213>  artificial sequence

<220>
<223>  C40 VL CDR1

<400>  96

Lys Ala Ser Gln Asp Ile Asn Lys Phe Ile Ala
1               5                   10

<210>  97
<211>  7
<212>  PRT
<213>  artificial sequence

<220>
<223>  C40 VL CDR2

<400>  97

Tyr Thr Ser Thr Leu Gln Pro
1               5

<210>  98
<211>  8
<212>  PRT
<213>  artificial sequence

<220>
<223>  C40 VL CDR3

<400>  98

Leu Gln Tyr Asp Asn Leu Tyr Thr
1               5

<210>  99
<211>  5
<212>  PRT
<213>  artificial sequence

<220>
<223>  C1 VH CDR1

<400>  99

Asp Asp Tyr Ile His
1               5

<210>  100
<211>  20
<212>  PRT

<213>  artificial sequence

<220>
<223>  C1 VH CDR2

<400>  100

Trp Ile Asp Pro Glu Asn Ser Asp Thr Glu Tyr Ala Ser Lys Phe Gln
1               5                   10                  15

Gly Lys Ala Thr
            20

<210>  101
<211>  12
<212>  PRT
<213>  artificial sequence

<220>
<223>  C1 VH CDR3

<400>  101

Thr Thr Gly Leu Trp Leu Arg Thr Ala Ile Asp Tyr
1               5                   10

<210>  102
<211>  5
<212>  PRT
<213>  artificial sequence

<220>
<223>  C4 VH CDR1

<400>  102

Ser Tyr Asp Ile Asn
1               5

<210>  103
<211>  20
<212>  PRT
<213>  artificial sequence

<220>
<223>  C4 VH CDR2

<400>  103

Trp Ile Tyr Pro Arg Asp Gly Asp Thr Lys Tyr Asn Glu Lys Phe Lys
1               5                   10                  15

Gly Lys Ala Ile
            20

<210>  104
<211>  10

<212>  PRT
<213>  artificial sequence

<220>
<223>  C4 VH CDR3

<400>  104

Leu Thr Gly Pro Tyr Trp Tyr Phe Asp Val
1               5                   10


<210>  105
<211>  5
<212>  PRT
<213>  artificial sequence

<220>
<223>  C8 VH CDR1

<400>  105

Asp Asp Tyr Ile His
1               5


<210>  106
<211>  20
<212>  PRT
<213>  artificial sequence

<220>
<223>  C8 VH CDR2

<400>  106

Trp Ile Asp Pro Glu Asn Ser Asp Thr Glu Tyr Ala Ser Lys Phe Gln
1               5                   10                  15


Gly Lys Ala Thr
            20


<210>  107
<211>  10
<212>  PRT
<213>  artificial sequence

<220>
<223>  C8 VH CDR3

<400>  107

Gly Leu Trp Leu Arg Thr Ala Ile Asp Tyr
1               5                   10


<210>  108
<211>  5
<212>  PRT
<213>  artificial sequence

<220>
<223>  C9 VH CDR1

<400>  108

Thr Ala Gly Met Gln
1               5


<210>  109
<211>  20
<212>  PRT
<213>  artificial sequence

<220>
<223>  C9 VH CDR2

<400>  109

Trp Ile Asn Thr His Ser Gly Glu Pro Lys Tyr Ala Glu Asp Phe Lys
1               5                   10                  15


Gly Arg Phe Ala
            20


<210>  110
<211>  6
<212>  PRT
<213>  artificial sequence

<220>
<223>  C9 VH CDR3

<400>  110

Trp Asp Gly Thr Gly Tyr
1               5


<210>  111
<211>  5
<212>  PRT
<213>  artificial sequence

<220>
<223>  C15 VH CDR1

<400>  111

Asp Tyr Glu Met His
1               5


<210>  112
<211>  17
<212>  PRT
<213>  artificial sequence

<220>
<223>  C15 VH CDR2

<400>  112

Val Val Asp Pro Gly Thr Gly Asp Thr Ala Ser Asn Gln Lys Phe Lys
1               5                   10                  15

Gly

<210>  113
<211>  11
<212>  PRT
<213>  artificial sequence

<220>
<223>  C15 VH CDR3

<400>  113

Arg Tyr Tyr Gly Ser Arg Glu Ser Met Asp Tyr
1               5                   10

<210>  114
<211>  5
<212>  PRT
<213>  artificial sequence

<220>
<223>  C16 VH CDR1

<400>  114

Arg Tyr Trp Met Ser
1               5

<210>  115
<211>  20
<212>  PRT
<213>  artificial sequence

<220>
<223>  C16 VH CDR2

<400>  115

Glu Ser Asp Pro Asp Ser Ser Thr Ile Asn Tyr Ala Pro Ser Leu Lys
1               5                   10                  15

Asp Lys Phe Ile
            20

<210>  116
<211>  12
<212>  PRT
<213>  artificial sequence

<220>
<223>  C16 VH CDR3

<400>  116

Glu Gly Tyr Tyr Asp Asn Glu Gly Tyr Phe Asp Val
1               5                   10


<210>  117
<211>  5
<212>  PRT
<213>  artificial sequence

<220>
<223>  C19 VH CDR1

<400>  117

Ile His Gly Met Ser
1               5


<210>  118
<211>  20
<212>  PRT
<213>  artificial sequence

<220>
<223>  C19 VH CDR2

<400>  118

Trp Ile Asn Thr Tyr Ser Gly Val Pro Thr Tyr Ala Asn Asp Phe Lys
1               5                   10                  15


Gly Arg Phe Ala
            20


<210>  119
<211>  13
<212>  PRT
<213>  artificial sequence

<220>
<223>  C19 VH CDR3

<400>  119

Ala Arg Asp Glu Val Arg Arg Gly Phe Gly Phe Ala Tyr
1               5                   10


<210>  120
<211>  5
<212>  PRT
<213>  artificial sequence

<220>
<223>  C25 VH CDR1

<400>  120

Asp Leu Tyr Ile His
1               5

<210>  121
<211>  20
<212>  PRT
<213>  artificial sequence

<220>
<223>  C25 VH CDR2

<400>  121

Trp Ile Asp Pro Glu Thr Asp Asn Thr Ile Tyr Asp Pro Lys Phe Gln
1               5                   10                  15

Gly Lys Ala Ser
            20

<210>  122
<211>  11
<212>  PRT
<213>  artificial sequence

<220>
<223>  C25 VH CDR3

<400>  122

Ser Thr Gly Leu Leu Gln Trp Tyr Phe Asp Val
1               5                   10

<210>  123
<211>  5
<212>  PRT
<213>  artificial sequence

<220>
<223>  C31 VH CDR1

<400>  123

Gly Tyr Tyr Trp Asn
1               5

<210>  124
<211>  19
<212>  PRT
<213>  artificial sequence

<220>
<223>  C31 VH CDR2

<400>  124

Tyr Ile Thr Asn Asp Gly Ser Asn Asn Tyr Asn Pro Ser Leu Lys Asn
1               5                   10                  15

Arg Ile Ser

<210> 125
<211> 10
<212> PRT
<213> artificial sequence

<220>
<223> C31 VH CDR3

<400> 125

Asp Gly Gly Trp Phe His Tyr Phe Asp Tyr
1               5                   10

<210> 126
<211> 5
<212> PRT
<213> artificial sequence

<220>
<223> C31 VH CDR1

<400> 126

Gly Tyr Tyr Trp Asn
1               5

<210> 127
<211> 19
<212> PRT
<213> artificial sequence

<220>
<223> C31 VH CDR2

<400> 127

Tyr Ile Thr Asn Asp Gly Ser Asn Asn Tyr Asn Pro Ser Leu Lys Asn
1               5                   10                  15

Arg Ile Ser

<210> 128
<211> 10
<212> PRT
<213> artificial sequence

<220>
<223> C31 VH CDR3

<400> 128

Asp Gly Gly Trp Phe His Tyr Phe Asp Tyr
1               5                   10

<210>  129
<211>  5
<212>  PRT
<213>  artificial sequence

<220>
<223>  C40 VH CDR1

<400>  129

Gly Met Gly Val Gly
1               5


<210>  130
<211>  19
<212>  PRT
<213>  artificial sequence

<220>
<223>  C40 VH CDR2

<400>  130

His Ile Trp Trp Asp Asp Asp Lys Tyr Tyr Asn Pro Ala Leu Lys Ser
1               5                   10                  15


Arg Leu Thr




<210>  131
<211>  9
<212>  PRT
<213>  Artificial Sequence

<220>
<223>  C40 VH CDR3

<400>  131

Ile Ala Gly Thr Arg Tyr Phe Asp Val
1               5


<210>  132
<211>  233
<212>  PRT
<213>  Homo sapiens

<400>  132

Met Ser Thr Glu Ser Met Ile Arg Asp Val Glu Leu Ala Glu Glu Ala
1               5                   10                  15


Leu Pro Lys Lys Thr Gly Gly Pro Gln Gly Ser Arg Arg Cys Leu Phe
            20                  25                  30

Leu Ser Leu Phe Ser Phe Leu Ile Val Ala Gly Ala Thr Thr Leu Phe
        35                  40                  45

Cys Leu Leu His Phe Gly Val Ile Gly Pro Gln Arg Glu Glu Phe Pro
    50                  55                  60

Arg Asp Leu Ser Leu Ile Ser Pro Leu Ala Gln Ala Val Arg Ser Ser
65                  70                  75                  80

Ser Arg Thr Pro Ser Asp Lys Pro Val Ala His Val Val Ala Asn Pro
                85                  90                  95

Gln Ala Glu Gly Gln Leu Gln Trp Leu Asn Arg Arg Ala Asn Ala Leu
            100                 105                 110

Leu Ala Asn Gly Val Glu Leu Arg Asp Asn Gln Leu Val Val Pro Ser
        115                 120                 125

Glu Gly Leu Tyr Leu Ile Tyr Ser Gln Val Leu Phe Lys Gly Gln Gly
    130                 135                 140

Cys Pro Ser Thr His Val Leu Leu Thr His Thr Ile Ser Arg Ile Ala
145                 150                 155                 160

Val Ser Tyr Gln Thr Lys Val Asn Leu Leu Ser Ala Ile Lys Ser Pro
                165                 170                 175

Cys Gln Arg Glu Thr Pro Glu Gly Ala Glu Ala Lys Pro Trp Tyr Glu
            180                 185                 190

Pro Ile Tyr Leu Gly Gly Val Phe Gln Leu Glu Lys Gly Asp Arg Leu
        195                 200                 205

Ser Ala Glu Ile Asn Arg Pro Asp Tyr Leu Asp Phe Ala Glu Ser Gly
    210                 215                 220

Gln Val Tyr Phe Gly Ile Ile Ala Leu
225                 230

<210>  133
<211>  205
<212>  PRT
<213>  Homo sapiens

<400>  133

Met Thr Pro Pro Glu Arg Leu Phe Leu Pro Arg Val Cys Gly Thr Thr
1               5                   10                  15

Leu His Leu Leu Leu Gly Leu Leu Leu Val Leu Leu Pro Gly Ala
            20                  25                  30

Gln Gly Leu Pro Gly Val Gly Leu Thr Pro Ser Ala Ala Gln Thr Ala
            35                  40                  45

Arg Gln His Pro Lys Met His Leu Ala His Ser Thr Leu Lys Pro Ala
        50                  55                  60

Ala His Leu Ile Gly Asp Pro Ser Lys Gln Asn Ser Leu Leu Trp Arg
65 70 75 80

Ala Asn Thr Asp Arg Ala Phe Leu Gln Asp Gly Phe Ser Leu Ser Asn
85 90 95

Asn Ser Leu Leu Val Pro Thr Ser Gly Ile Tyr Phe Val Tyr Ser Gln
                100                 105                 110

Val Val Phe Ser Gly Lys Ala Tyr Ser Pro Lys Ala Thr Ser Ser Pro
            115                 120                 125

Leu Tyr Leu Ala His Glu Val Gln Leu Phe Ser Ser Gln Tyr Pro Phe
        130                 135                 140

His Val Pro Leu Leu Ser Ser Gln Lys Met Val Tyr Pro Gly Leu Gln
145 150 155 160

Glu Pro Trp Leu His Ser Met Tyr His Gly Ala Ala Phe Gln Leu Thr
165 170 175

Gln Gly Asp Gln Leu Ser Thr His Thr Asp Gly Ile Pro His Leu Val
                180                 185                 190

Leu Ser Pro Ser Thr Val Phe Phe Gly Ala Phe Ala Leu
195 200 205

<210> 134
<211> 559
<212> PRT
<213> artificial sequence

<220>
<223> TNC-sc(mu)TNF(143N/145R)

<400> 134

Met Asn Phe Gly Phe Ser Leu Ile Phe Leu Val Leu Val Leu Lys Gly
1               5                   10                  15

Val Gln Cys Glu Val Lys Leu Val Pro Arg Gly Ser Gly Ser Asp Tyr

20                  25                  30

Lys Asp Asp Asp Asp Lys Asp Ile Ala Cys Gly Cys Ala Ala Ala Pro
        35                  40                  45

Asp Ile Lys Asp Leu Leu Ser Arg Leu Glu Glu Leu Glu Gly Leu Val
    50                  55                  60

Ser Ser Leu Arg Glu Gln Gly Thr Gly Gly Gly Ser Gly Gly Arg Gly
65                  70                  75                  80

Glu Phe Glu Phe Thr Arg Asp Lys Pro Val Ala His Val Val Ala Asn
                85                  90                  95

His Gln Val Glu Glu Gln Leu Glu Trp Leu Ser Gln Arg Ala Asn Ala
            100                 105                 110

Leu Leu Ala Asn Gly Met Asp Leu Lys Asp Asn Gln Leu Val Val Pro
        115                 120                 125

Ala Asp Gly Leu Tyr Leu Val Tyr Ser Gln Val Leu Phe Lys Gly Gln
    130                 135                 140

Gly Cys Pro Asp Tyr Val Leu Leu Thr His Thr Val Ser Arg Phe Ala
145                 150                 155                 160

Ile Ser Tyr Gln Glu Lys Val Asn Leu Leu Ser Ala Val Lys Ser Pro
                165                 170                 175

Cys Pro Lys Asp Thr Pro Glu Gly Ala Glu Leu Lys Pro Trp Tyr Glu
            180                 185                 190

Pro Ile Tyr Leu Gly Gly Val Phe Gln Leu Glu Lys Gly Asp Gln Leu
        195                 200                 205

Ser Ala Glu Val Asn Leu Pro Lys Tyr Leu Asn Phe Arg Glu Ser Gly
    210                 215                 220

Gln Val Tyr Phe Gly Val Ile Ala Leu Gly Gly Gly Ser Gly Gly Gly
225                 230                 235                 240

Ser Gly Gly Gly Ser Gly Gly Gly Ser Asp Lys Pro Val Ala His Val
                245                 250                 255

Val Ala Asn His Gln Val Glu Glu Gln Leu Glu Trp Leu Ser Gln Arg
            260                 265                 270

Ala Asn Ala Leu Leu Ala Asn Gly Met Asp Leu Lys Asp Asn Gln Leu
        275                 280                 285

Val Val Pro Ala Asp Gly Leu Tyr Leu Val Tyr Ser Gln Val Leu Phe
    290                 295                 300

Lys Gly Gln Gly Cys Pro Asp Tyr Val Leu Leu Thr His Thr Val Ser
305                 310                 315                 320

Arg Phe Ala Ile Ser Tyr Gln Glu Lys Val Asn Leu Leu Ser Ala Val
                325                 330                 335

Lys Ser Pro Cys Pro Lys Asp Thr Pro Glu Gly Ala Glu Leu Lys Pro
            340                 345                 350

Trp Tyr Glu Pro Ile Tyr Leu Gly Gly Val Phe Gln Leu Glu Lys Gly
        355                 360                 365

Asp Gln Leu Ser Ala Glu Val Asn Leu Pro Lys Tyr Leu Asn Phe Arg
    370                 375                 380

Glu Ser Gly Gln Val Tyr Phe Gly Val Ile Ala Leu Gly Gly Gly Ser
385                 390                 395                 400

Gly Gly Gly Ser Gly Gly Gly Ser Gly Gly Gly Ser Asp Lys Pro Val
                405                 410                 415

Ala His Val Val Ala Asn His Gln Val Glu Glu Gln Leu Glu Trp Leu
            420                 425                 430

Ser Gln Arg Ala Asn Ala Leu Leu Ala Asn Gly Met Asp Leu Lys Asp
        435                 440                 445

Asn Gln Leu Val Val Pro Ala Asp Gly Leu Tyr Leu Val Tyr Ser Gln
    450                 455                 460

Val Leu Phe Lys Gly Gln Gly Cys Pro Asp Tyr Val Leu Leu Thr His
465                 470                 475                 480

Thr Val Ser Arg Phe Ala Ile Ser Tyr Gln Glu Lys Val Asn Leu Leu
                485                 490                 495

Ser Ala Val Lys Ser Pro Cys Pro Lys Asp Thr Pro Glu Gly Ala Glu
            500                 505                 510

Leu Lys Pro Trp Tyr Glu Pro Ile Tyr Leu Gly Gly Val Phe Gln Leu
        515                 520                 525

Glu Lys Gly Asp Gln Leu Ser Ala Glu Val Asn Leu Pro Lys Tyr Leu
    530                 535                 540

Asn Phe Arg Glu Ser Gly Gln Val Tyr Phe Gly Val Ile Ala Leu
545                 550                 555

<210>  135
<211>  983
<212>  PRT
<213>  artificial sequence

<220>
<223>  Irr-IgG1(N297A)-HC:scFvsc(mu)TNF(143N/145R)*
Heavy chain

<400>  135

Met Asn Phe Gly Phe Ser Leu Ile Phe Leu Val Leu Val Leu Lys Gly
1               5                   10                  15

Val Gln Cys Glu Val Lys Leu Val Pro Arg Gln Leu Asp Tyr Lys Asp
            20                  25                  30

Asp Asp Asp Lys Glu Leu Gln Leu Gln Leu Gln Glu Ser Gly Pro Gly
        35                  40                  45

Leu Val Lys Pro Ser Glu Thr Leu Ser Leu Thr Cys Thr Val Ser Gly
    50                  55                  60

Ala Ser Ile Ser Ala Asn Ser Tyr Tyr Gly Val Trp Val Arg Gln Ser
65                  70                  75                  80

Pro Gly Lys Gly Leu Glu Trp Val Gly Ser Ile Ala Tyr Arg Gly Asn
                85                  90                  95

Ser Asn Ser Gly Ser Thr Tyr Tyr Asn Pro Ser Leu Lys Ser Arg Ala
            100                 105                 110

Thr Val Ser Val Asp Thr Ser Lys Asn Gln Val Ser Leu Arg Leu Thr
        115                 120                 125

Ser Val Thr Ala Ala Asp Thr Ala Leu Tyr Tyr Cys Ala Arg Arg Gln
    130                 135                 140

Leu Leu Asp Asp Gly Thr Gly Tyr Gln Trp Ala Ala Phe Asp Val Trp
145                 150                 155                 160

Gly Gln Gly Thr Met Val Thr Val Ser Ser Gly Ser Ser Ser Ala Ser
                165                 170                 175

Thr Lys Gly Pro Ser Val Phe Pro Leu Ala Pro Ser Ser Lys Ser Thr
        180                 185                 190

Ser Gly Gly Thr Ala Ala Leu Gly Cys Leu Val Lys Asp Tyr Phe Pro
    195                 200                 205

Glu Pro Val Thr Val Ser Trp Asn Ser Gly Ala Leu Thr Ser Gly Val
    210                 215                 220

His Thr Phe Pro Ala Val Leu Gln Ser Ser Gly Leu Tyr Ser Leu Ser
225                 230                 235                 240

Ser Val Val Thr Val Pro Ser Ser Ser Leu Gly Thr Gln Thr Tyr Ile
                245                 250                 255

Cys Asn Val Asn His Lys Pro Ser Asn Thr Lys Val Asp Lys Lys Val
            260                 265                 270

Glu Pro Lys Ser Cys Asp Lys Thr His Thr Cys Pro Pro Cys Pro Ala
        275                 280                 285

Pro Glu Leu Leu Gly Gly Pro Ser Val Phe Leu Phe Pro Pro Lys Pro
    290                 295                 300

Lys Asp Thr Leu Met Ile Ser Arg Thr Pro Glu Val Thr Cys Val Val
305                 310                 315                 320

Val Asp Val Ser His Glu Asp Pro Glu Val Lys Phe Asn Trp Tyr Val
                325                 330                 335

Asp Gly Val Glu Val His Asn Ala Lys Thr Lys Pro Arg Glu Glu Gln
            340                 345                 350

Tyr Ala Ser Thr Tyr Arg Val Val Ser Val Leu Thr Val Leu His Gln
        355                 360                 365

Asp Trp Leu Asn Gly Lys Glu Tyr Lys Cys Lys Val Ser Asn Lys Ala
    370                 375                 380

Leu Pro Ala Pro Ile Glu Lys Thr Ile Ser Lys Ala Lys Gly Gln Pro
385                 390                 395                 400

Arg Glu Pro Gln Val Tyr Thr Leu Pro Pro Ser Arg Asp Glu Leu Thr
                405                 410                 415

Lys Asn Gln Val Ser Leu Thr Cys Leu Val Lys Gly Phe Tyr Pro Ser
            420                 425                 430

Asp Ile Ala Val Glu Trp Glu Ser Asn Gly Gln Pro Glu Asn Asn Tyr
        435                 440                 445

Lys Thr Thr Pro Pro Val Leu Asp Ser Asp Gly Ser Phe Phe Leu Tyr
    450                 455                 460

Ser Lys Leu Thr Val Asp Lys Ser Arg Trp Gln Gln Gly Asn Val Phe
465                 470                 475                 480

Ser Cys Ser Val Met His Glu Ala Leu His Asn His Tyr Thr Gln Lys
                485                 490                 495

Ser Leu Ser Leu Ser Pro Gly Lys Glu Phe Glu Phe Thr Arg Asp Lys
            500                 505                 510

Pro Val Ala His Val Val Ala Asn His Gln Val Glu Glu Gln Leu Glu
        515                 520                 525

Trp Leu Ser Gln Arg Ala Asn Ala Leu Leu Ala Asn Gly Met Asp Leu
    530                 535                 540

Lys Asp Asn Gln Leu Val Val Pro Ala Asp Gly Leu Tyr Leu Val Tyr
545                 550                 555                 560

Ser Gln Val Leu Phe Lys Gly Gln Gly Cys Pro Asp Tyr Val Leu Leu
                565                 570                 575

Thr His Thr Val Ser Arg Phe Ala Ile Ser Tyr Gln Glu Lys Val Asn
            580                 585                 590

Leu Leu Ser Ala Val Lys Ser Pro Cys Pro Lys Asp Thr Pro Glu Gly
        595                 600                 605

Ala Glu Leu Lys Pro Trp Tyr Glu Pro Ile Tyr Leu Gly Gly Val Phe
    610                 615                 620

Gln Leu Glu Lys Gly Asp Gln Leu Ser Ala Glu Val Asn Leu Pro Lys
625                 630                 635                 640

Tyr Leu Asn Phe Arg Glu Ser Gly Gln Val Tyr Phe Gly Val Ile Ala
                645                 650                 655

Leu Gly Gly Gly Ser Gly Gly Gly Ser Gly Gly Gly Ser Gly Gly Gly
            660                 665                 670

Ser Asp Lys Pro Val Ala His Val Val Ala Asn His Gln Val Glu Glu

675                 680                 685

Gln Leu Glu Trp Leu Ser Gln Arg Ala Asn Ala Leu Leu Ala Asn Gly
    690                 695                 700

Met Asp Leu Lys Asp Asn Gln Leu Val Val Pro Ala Asp Gly Leu Tyr
705                 710                 715                 720

Leu Val Tyr Ser Gln Val Leu Phe Lys Gly Gln Gly Cys Pro Asp Tyr
                725                 730                 735

Val Leu Leu Thr His Thr Val Ser Arg Phe Ala Ile Ser Tyr Gln Glu
            740                 745                 750

Lys Val Asn Leu Leu Ser Ala Val Lys Ser Pro Cys Pro Lys Asp Thr
        755                 760                 765

Pro Glu Gly Ala Glu Leu Lys Pro Trp Tyr Glu Pro Ile Tyr Leu Gly
    770                 775                 780

Gly Val Phe Gln Leu Glu Lys Gly Asp Gln Leu Ser Ala Glu Val Asn
785                 790                 795                 800

Leu Pro Lys Tyr Leu Asn Phe Arg Glu Ser Gly Gln Val Tyr Phe Gly
                805                 810                 815

Val Ile Ala Leu Gly Gly Gly Ser Gly Gly Gly Ser Gly Gly Gly Ser
            820                 825                 830

Gly Gly Gly Ser Asp Lys Pro Val Ala His Val Val Ala Asn His Gln
        835                 840                 845

Val Glu Glu Gln Leu Glu Trp Leu Ser Gln Arg Ala Asn Ala Leu Leu
    850                 855                 860

Ala Asn Gly Met Asp Leu Lys Asp Asn Gln Leu Val Val Pro Ala Asp
865                 870                 875                 880

Gly Leu Tyr Leu Val Tyr Ser Gln Val Leu Phe Lys Gly Gln Gly Cys
                885                 890                 895

Pro Asp Tyr Val Leu Leu Thr His Thr Val Ser Arg Phe Ala Ile Ser
            900                 905                 910

Tyr Gln Glu Lys Val Asn Leu Leu Ser Ala Val Lys Ser Pro Cys Pro
        915                 920                 925

Lys Asp Thr Pro Glu Gly Ala Glu Leu Lys Pro Trp Tyr Glu Pro Ile
    930                 935                 940

Tyr Leu Gly Gly Val Phe Gln Leu Glu Lys Gly Asp Gln Leu Ser Ala
945                 950                 955                 960

Glu Val Asn Leu Pro Lys Tyr Leu Asn Phe Arg Glu Ser Gly Gln Val
                965                 970                 975

Tyr Phe Gly Val Ile Ala Leu
            980

<210> 136
<211> 261
<212> PRT
<213> artificial sequence

<220>
<223> Irr-IgG1(N297A)-HC:scFvsc(mu)TNF(143N/145R)*
light chain

<400> 136

Met Asn Phe Gly Phe Ser Leu Ile Phe Leu Val Leu Val Leu Lys Gly
1               5                   10                  15

Val Gln Cys Glu Val Lys Leu Val Pro Arg Gln Leu Asp Tyr Lys Asp
            20                  25                  30

Asp Asp Asp Lys Glu Leu Gln Ser Val Leu Thr Gln Pro Pro Ser Val
        35                  40                  45

Ser Glu Ala Pro Arg Gln Thr Val Thr Ile Ser Cys Ser Gly Asn Ser
    50                  55                  60

Phe Asn Ile Gly Arg Tyr Pro Val Asn Trp Tyr Gln Gln Leu Pro Gly
65                  70                  75                  80

Lys Ala Pro Lys Leu Leu Ile Tyr Tyr Asn Asn Leu Arg Phe Ser Gly
                85                  90                  95

Val Ser Asp Arg Phe Ser Gly Ser Lys Ser Gly Thr Ser Ala Ser Leu
            100                 105                 110

Ala Ile Arg Asp Leu Leu Ser Glu Asp Glu Ala Asp Tyr Tyr Cys Ser
        115                 120                 125

Thr Trp Asp Asp Thr Leu Lys Gly Trp Val Phe Gly Gly Gly Thr Lys
    130                 135                 140

Val Thr Val Leu Gly Gly Ser Glu Ile Lys Arg Thr Val Ala Ala Pro
145                 150                 155                 160

Ser Val Phe Ile Phe Pro Pro Ser Asp Glu Gln Leu Lys Ser Gly Thr
                165                 170                 175

Ala Ser Val Val Cys Leu Leu Asn Asn Phe Tyr Pro Arg Glu Ala Lys
            180                 185                 190

Val Gln Trp Lys Val Asp Asn Ala Leu Gln Ser Gly Asn Ser Gln Glu
        195                 200                 205

Ser Val Thr Glu Gln Asp Ser Lys Asp Ser Thr Tyr Ser Leu Ser Ser
    210                 215                 220

Thr Leu Thr Leu Ser Lys Ala Asp Tyr Glu Lys His Lys Val Tyr Ala
225                 230                 235                 240

Cys Glu Val Thr His Gln Gly Leu Ser Ser Pro Val Thr Lys Ser Phe
                245                 250                 255

Asn Arg Gly Glu Cys
            260

<210>  137
<211>  474
<212>  PRT
<213>  artificial sequence

<220>
<223>  TNFR2(mu)-pCMV-SPORT6 (Tnfrsf1b) (Origene) cDNA clone

<400>  137

Met Ala Pro Ala Ala Leu Trp Val Ala Leu Val Phe Glu Leu Gln Leu
1               5                   10                  15

Trp Ala Thr Gly His Thr Val Pro Ala Gln Val Val Leu Thr Pro Tyr
            20                  25                  30

Lys Pro Glu Pro Gly Tyr Glu Cys Gln Ile Ser Gln Glu Tyr Tyr Asp
        35                  40                  45

Arg Lys Ala Gln Met Cys Cys Ala Lys Cys Pro Pro Gly Gln Tyr Val
    50                  55                  60

Lys His Phe Cys Asn Lys Thr Ser Asp Thr Val Cys Ala Asp Cys Glu
65                  70                  75                  80

Ala Ser Met Tyr Thr Gln Val Trp Asn Gln Phe Arg Thr Cys Leu Ser

                85                  90                  95

Cys Ser Ser Ser Cys Thr Thr Asp Gln Val Glu Ile Arg Ala Cys Thr
            100                 105                 110

Lys Gln Gln Asn Arg Val Cys Ala Cys Glu Ala Gly Arg Tyr Cys Ala
        115                 120                 125

Leu Lys Thr His Ser Gly Ser Cys Arg Gln Cys Met Arg Leu Ser Lys
    130                 135                 140

Cys Gly Pro Gly Phe Gly Val Ala Ser Ser Arg Ala Pro Asn Gly Asn
145                 150                 155                 160

Val Leu Cys Lys Ala Cys Ala Pro Gly Thr Phe Ser Asp Thr Thr Ser
                165                 170                 175

Ser Thr Asp Val Cys Arg Pro His Arg Ile Cys Ser Ile Leu Ala Ile
            180                 185                 190

Pro Gly Asn Ala Ser Thr Asp Ala Val Cys Ala Pro Glu Ser Pro Thr
        195                 200                 205

Leu Ser Ala Ile Pro Arg Thr Leu Tyr Val Ser Gln Pro Glu Pro Thr
    210                 215                 220

Arg Ser Gln Pro Leu Asp Gln Glu Pro Gly Pro Ser Gln Thr Pro Ser
225                 230                 235                 240

Ile Leu Thr Ser Leu Gly Ser Thr Pro Ile Ile Glu Gln Ser Thr Lys
                245                 250                 255

Gly Gly Ile Ser Leu Pro Ile Gly Leu Ile Val Gly Val Thr Ser Leu
            260                 265                 270

Gly Leu Leu Met Leu Gly Leu Val Asn Cys Ile Ile Leu Val Gln Arg
        275                 280                 285

Lys Lys Lys Pro Ser Cys Leu Gln Arg Asp Ala Lys Val Pro His Val
    290                 295                 300

Pro Asp Glu Lys Ser Gln Asp Ala Val Gly Leu Glu Gln Gln His Leu
305                 310                 315                 320

Leu Thr Thr Ala Pro Ser Ser Ser Ser Ser Ser Leu Glu Ser Ser Ala
                325                 330                 335

Ser Ala Gly Asp Arg Arg Ala Pro Pro Gly Gly His Pro Gln Ala Arg
            340                 345                 350

Val Met Ala Glu Ala Gln Gly Phe Gln Glu Ala Arg Ala Ser Ser Arg
        355                 360                 365

Ile Ser Asp Ser Ser His Gly Ser His Gly Thr His Val Asn Val Thr
    370                 375                 380

Cys Ile Val Asn Val Cys Ser Ser Ser Asp His Ser Ser Gln Cys Ser
385                 390                 395                 400

Ser Gln Ala Ser Ala Thr Val Gly Asp Pro Asp Ala Lys Pro Ser Ala
                405                 410                 415

Ser Pro Lys Asp Glu Gln Val Pro Phe Ser Gln Glu Glu Cys Pro Ser
            420                 425                 430

Gln Ser Pro Cys Glu Thr Thr Glu Thr Leu Gln Ser His Glu Lys Pro
        435                 440                 445

Leu Pro Leu Gly Val Pro Asp Met Gly Met Lys Pro Ser Gln Ala Gly
    450                 455                 460

Trp Phe Asp Gln Ile Ala Val Lys Val Ala
465                 470

<210>  138
<211>  445
<212>  PRT
<213>  Macaca fascicularis

<400>  138

Met Ala Val Phe Pro Ser Ser Phe Gln Val Ala Phe Thr Pro Tyr Ala
1               5                   10                  15

Pro Glu Pro Gly Gly Thr Cys Arg Leu Arg Glu Tyr Tyr Asp Gln Thr
            20                  25                  30

Ala Gln Met Cys Cys Ser Lys Cys Pro Pro Gly Gln His Ala Lys Val
        35                  40                  45

Phe Cys Thr Lys Thr Ser Asp Thr Val Cys Asp Ser Cys Glu Asp Ser
    50                  55                  60

Thr Tyr Thr Gln Leu Trp Asn Trp Val Pro Glu Cys Leu Ser Cys Gly
65                  70                  75                  80

Ser Arg Cys Ser Ser Asp Gln Val Glu Thr Gln Ala Cys Thr Arg Glu
                85                  90                  95

Gln Asn Arg Ile Cys Thr Cys Arg Pro Gly Trp Tyr Cys Ala Leu Ser
            100                 105                 110

Lys Gln Glu Gly Cys Arg Leu Cys Ala Gln Leu Arg Lys Cys Arg Pro
        115                 120                 125

Gly Phe Gly Val Ala Arg Pro Gly Thr Glu Thr Ser Asp Val Val Cys
    130                 135                 140

Lys Pro Cys Ala Pro Gly Thr Phe Ser Asn Thr Thr Ser Ser Thr Asp
145                 150                 155                 160

Ile Cys Arg Pro His Gln Ile Cys His Val Val Ala Ile Pro Gly Asn
                165                 170                 175

Ala Ser Met Asp Ala Val Cys Thr Ser Thr Ser Pro Thr Arg Ser Met
            180                 185                 190

Ala Pro Gly Ala Val His Leu Pro Gln Pro Val Ser Thr Arg Ser Gln
        195                 200                 205

His Thr Gln Pro Thr Pro Ala Pro Ser Thr Ala Pro Gly Thr Ser Phe
    210                 215                 220

Leu Leu Pro Val Gly Pro Ser Pro Pro Ala Glu Gly Ser Thr Gly Asp
225                 230                 235                 240

Ile Val Leu Pro Val Gly Leu Ile Val Gly Val Thr Ala Leu Gly Leu
                245                 250                 255

Leu Ile Ile Gly Val Val Asn Cys Val Ile Met Thr Gln Val Lys Lys
            260                 265                 270

Lys Pro Leu Cys Leu Gln Arg Glu Thr Lys Val Pro His Leu Pro Ala
        275                 280                 285

Asp Lys Ala Arg Gly Ala Gln Gly Pro Glu Gln Gln His Leu Leu Thr
    290                 295                 300

Thr Val Pro Ser Ser Ser Ser Ser Ser Leu Glu Ser Ser Ala Ser Ala
305                 310                 315                 320

Leu Asp Arg Arg Ala Pro Thr Arg Asn Gln Pro Gln Ala Pro Gly Ala
                325                 330                 335

Glu Lys Ala Ser Gly Ala Gly Glu Ala Arg Ala Ser Thr Gly Ser Ser
            340                 345                 350

Asp Ser Ser Pro Gly Gly His Gly Thr Gln Val Asn Val Thr Cys Ile
        355                 360                 365

Val Asn Val Cys Ser Ser Ser Asp His Ser Ser Gln Cys Ser Ser Gln
    370                 375                 380

Ala Ser Ser Thr Met Gly Asp Thr Asp Ala Ser Pro Ser Gly Ser Pro
385                 390                 395                 400

Lys Asp Glu Gln Val Pro Phe Ser Lys Glu Glu Ser Ala Phe Arg Ser
                405                 410                 415

Gln Leu Glu Thr Pro Glu Thr Leu Leu Gly Ser Thr Glu Glu Lys Pro
            420                 425                 430

Leu Pro Leu Gly Val Pro Asp Ala Gly Met Lys Pro Ser
        435                 440                 445

<210>  139
<211>  30
<212>  PRT
<213>  Homo sapiens

<400>  139

Ala Cys Gly Cys Ala Ala Ala Pro Asp Val Lys Glu Leu Leu Ser Arg
1               5                   10                  15

Leu Glu Glu Leu Glu Asn Leu Val Ser Ser Leu Arg Glu Gln
            20                  25                  30